# EUROPEAN PATENT SPECIFICATION

(11) **EP 3 833 759 B1**
(45) Date of publication and mention of the grant of the patent: **14.10.2026**
(21) Application number: 19770209.5
(22) Date of filing: 09.08.2019
(51) Int. Cl.: C12N 15/10, C12Q 1/6806

(54) **METHODS FOR ASSESSING INTEGRATED NUCLEIC ACIDS**
VERFAHREN ZUR BEWERTUNG INTEGRIERTER NUKLEINSÄUREN
PROCÉDÉS POUR ÉVALUER DES ACIDES NUCLÉIQUES INTÉGRÉS

(30) Priority: 09.08.2018 US 201862716972 P
(43) Date of publication of application: 16.06.2021
(73) Proprietor: Juno Therapeutics, Inc., Seattle, WA 98109 (US)
(72) Inventor: BRIGGS, Adrian Wrangham, Seattle, Washington 98109 (US)
(74) Representative: Carpmaels & Ransford LLP
(86) International application number: PCT/US2019/046048
(87) International publication number: WO 2020/033916

(56) References cited:
- MARIA P. SERRA ET AL: "Modulation of oat arginine decarboxylase gene expression and genome organization in transgenic Trypanosoma cruzi epimastigotes", FEBS JOURNAL, vol. 273, no. 3, 1 February 2006 (2006-02-01), GB, pages 628 - 637, XP055632693, ISSN: 1742-464X, DOI: 10.1111/j.1742-4658.2005.05098.x
- YE GUANG-NING ET AL: "TOBACCO (NICOTIANA TABACUM) NUCLEAR TRANSGENICS WITH HIGH COPY NUMBER CAN EXPRESS NPTII DRIVEN BY THE CHLOROPLAST PSBA PROMOTER", PLANT CELL REPORTS, SPRINGER, BERLIN/HEIDELBERG, vol. 15, no. 7, 1 January 1996 (1996-01-01), pages 479 - 483, XP009079842, ISSN: 0721-7714, DOI: 10.1007/S002990050058

## Description

### Field

The present disclosure relates in some aspects to methods for assessing nucleic acid sequences integrated into a genome of a genetically engineered cell, such as a genetically engineered cell used in cell therapy. Cells are generally genetically engineered to express a recombinant protein, such as a recombinant receptor, via introduction of a polynucleotide and integration of certain sequences in the polynucleotide, such as a transgene sequence encoding the recombinant protein, into the genome of the cell. In some aspects, the provided methods can be used to distinguish integrated nucleic acids and non-integrated, residual nucleic acids.

### Background

Methods are available to determine the copy number of nucleic acids introduced to genetically engineer a cell, such as for adoptive cell therapy for treating diseases and conditions. For adoptive cell therapies (including those involving the administration of cells expressing recombinant receptors specific for a disease or disorder of interest, such as chimeric antigen receptors (CARs) and/or other recombinant antigen receptors) can require timely and accurate assessment of integrated nucleic acids, and in some cases, non-integrated, residual nucleic acids, prior to administration of cells to a subject. Improved approaches are needed. Provided are methods and kits that meet such needs, wherein only the methods are claimed.
MARIA P. SERRA ET AL, FEBS JOURNAL, vol. 273, no. 3, 1 February 2006, pages 628-637, describes the "Modulation of oat arginine decarboxylase gene expression and genome organization in transgenic Trypanosoma cruzi epimastigotes". D1 investigates whether the genome organisation of transgeneic T. *cruzi* underwent modificaton following modulation of exogenous arginine decarboxylase expression by using 1) PCR amplification assays; 2) restriction mapping; and 3) pulse field gel electrophoresis.

### Summary

Provided herein are methods for assessing genomic integration of a transgene sequence, comprising: (a) separating a high molecular weight fraction of deoxyribonucleic acid (DNA) of greater than or greater than about 10 kilobases (kb) from DNA isolated from one or more cells, said one or more cells comprising, or are suspected of comprising, at least one engineered cell comprising a transgene sequence that includes a nucleic acid sequence encoding a recombinant protein; and (b) from the high molecular weight fraction, determining the presence, absence or amount of the transgene sequence integrated into the genome of the one or more cell.

Provided herein is a method for assessing genomic integration of a transgene sequence, the method comprising: (a) separating a high molecular weight fraction of deoxyribonucleic acid (DNA) of greater than or greater than about 10 kilobases (kb) from DNA isolated from one or more cell, said one or more cell comprising, or suspected of comprising, at least one engineered cell comprising a transgene sequence encoding a recombinant protein; and (b) determining the presence, absence or amount of the transgene sequence integrated into the genome of the one or more cell.

Also provided herein are methods for assessing genomic integration of a transgene sequence, the methods involving: (a) separating a high molecular weight fraction of deoxyribonucleic acid (DNA) of greater than or greater than about 10 kilobases (kb) from DNA isolated from one or more cell, said one or more cell comprising, or suspected of comprising, at least one engineered cell comprising a transgene sequence comprising a nucleic acid sequence encoding a recombinant protein; and (b) determining the presence, absence or amount of the transgene sequence in the high molecular weight fraction.

In some of any embodiments, determining the presence, absence or amount of the transgene sequence in the high molecular weight fraction, thereby assesses the transgene sequences integrated into the genome of the one or more cells.

Also provided herein are methods for assessing genomic integration of a transgene sequence, the methods involving: (a) separating a high molecular weight fraction of deoxyribonucleic acid (DNA) of greater than or greater than about 10 kilobases (kb) from DNA isolated from one or more cell, said one or more cell comprising, or suspected of comprising, at least one engineered cell comprising a transgene sequence comprising a nucleic acid sequence encoding a recombinant protein; and (b) determining the presence, absence or amount of the transgene sequence in the high molecular weight fraction, wherein the transgene sequences in the high molecular weight fraction represents the transgene sequences that have been integrated into the genome of the one or more cell.

The transgene sequences in the high molecular weight fraction represents the transgene sequences that have been integrated into the genome of the one or more cell. In some of any of the provided embodiments, the determining the presence, absence or amount of the transgene sequence integrated into the genome of the one or more cells in (b) comprises determining the mass, weight or copy number of the transgene sequence in the high molecular weight fraction.

In some of any of the provided embodiments, prior to the separating, the methods include isolating deoxyribonucleic acid (DNA) from the one or more cell. In some of any of the provided embodiments, the determining the presence, absence or amount of the transgene sequence comprises determining the mass, weight or copy number of the transgene sequence per diploid genome or per cell in the one or more cells.

In some of any of the provided embodiments, the one or more cell comprises a population of cells in which a plurality of cells of the population comprise the transgene sequence encoding the recombinant protein. In some of any of the provided embodiments, the one or more cell comprises a population of cells in which a plurality of cells of the population is suspected of comprising the transgene sequence encoding the recombinant protein.

In some of any of the provided embodiments, the copy number is an average or mean copy number per diploid genome or per cell among the population of cells.

In some of any of the provided embodiments, prior to the separating, a polynucleotide comprising the transgene sequence encoding the recombinant protein has been introduced into a cell, e.g. of a population of cells, to result in the at least one engineered cell of the one or more cells. In some of any of the provided embodiments, the at least one engineered cell has not been incubated at a temperature greater than 25°C, optionally at or about 37°C ± 2°C, for more than 96 hours following the introduction of the polynucleotide comprising the transgene sequence. In some of any of the provided embodiments, the at least one engineered cell has not been incubated at a temperature greater than 25°C, optionally at or about 37°C ± 2°C, for more than 72 hours following the introduction of the polynucleotide comprising the transgene sequence. In some of any of the provided embodiments, the at least one engineered cell has not been incubated at a temperature greater than 25°C, optionally at or about 37°C ± 2°C, for more than 48 hours following the introduction of the polynucleotide comprising the transgene sequence.

In some of any of the provided embodiments, the one or more cell has been cryopreserved prior to the separating of the high molecular weight fraction of DNA. In some of any of the provided embodiments, the one or more cell is a cell line. In some of any of the provided embodiments, the one or more cell is a primary cell obtained from a sample from a subject. In some of any of the provided embodiments, the one or more cell is an immune cell. In some of any of the provided embodiments, the immune cell is a T cell or an NK cell. In some of any of the provided embodiments, the T cell is a CD3+, CD4+ and/or CD8+ T cells.

Provided herein are methods for assessing a transgene sequence in a biological sample from a subject, comprising: (a) separating a high molecular weight fraction of deoxyribonucleic acid (DNA) of greater than or greater than about 10 kilobases (kb) from DNA isolated from one or more cells present in a biological sample from a subject, wherein the biological sample comprises, or is suspected of comprising, at least one engineered cell comprising a transgene sequence encoding a recombinant protein; and (b) determining the presence, absence or amount of transgene sequence in the high molecular weight fraction, thereby assessing transgene sequences present in all or a portion of the biological sample.

Provided herein is a method for assessing a transgene sequence in a biological sample from a subject, the method comprising: (a) separating a high molecular weight fraction of deoxyribonucleic acid (DNA) of greater than or greater than about 10 kilobases (kb) from DNA isolated from one or more cells present in a biological sample from a subject, wherein the biological sample comprises, or is suspected of comprising, at least one engineered cell comprising a transgene sequence encoding a recombinant protein; and (b) determining the presence, absence or amount of transgene sequence in the high molecular weight fraction, thereby assessing transgene sequences present in all or a portion of the biological sample.

Provided herein is a method for assessing a transgene sequence in a biological sample from a subject, the method comprising: (a) separating a high molecular weight fraction of deoxyribonucleic acid (DNA) of greater than or greater than about 10 kilobases (kb) from DNA isolated from one or more cells present in a biological sample from a subject, wherein the biological sample comprises, or is suspected of comprising, at least one engineered cell comprising a transgene sequence encoding a recombinant protein; and (b) determining the presence, absence or amount of transgene sequence in all or a portion of the biological sample.

In some of any of the provided embodiments, the determining the presence, absence or amount of transgene sequence in (b) comprises determining the mass, weight or copy number of the transgene sequence in all or a portion of the biological sample.

In some of any of the provided embodiments, prior to the separating, isolating the DNA from one or more cells present in the biological sample. In some of any of the provided embodiments, the biological sample is obtained from a subject that had been administered a composition comprising the at least one engineered cell comprising the transgene sequence. In some of any of the provided embodiments, the biological sample is a tissue sample or bodily fluid sample. In some of any of the provided embodiments, the biological sample is a tissue sample and the tissue is a tumor. In some embodiments, the tissue sample is a tumor biopsy. In some of any of the provided embodiments, the biological sample is a bodily fluid sample and the bodily fluid sample is a blood or serum sample.

In some of any of the provided embodiments, prior to the separating, a polynucleotide comprising the transgene sequence encoding the recombinant protein has been introduced into a cell, e.g. of a population of cells, to result in the at least one engineered cell of the one or more cells.

In some of any of the provided embodiments, the one or more cells in the biological sample comprises an immune cell. In some embodiments, the immune cell is a T cell or an NK cell. In some embodiments, the T cell is a CD3+, CD4+ and/or CD8+ T cells.

In some of any of the provided embodiments, the separating is carried out by pulse field gel electrophoresis or size exclusion chromatography. In some embodiments, the separating is carried out by pulse field gel electrophoresis.

Also provided herein are methods for assessing genomic integration of a transgene sequence. In some of any of the embodiments, the methods involve: (a) separating, by pulse field gel electrophoresis, a high molecular weight fraction of deoxyribonucleic acid (DNA) of greater than or greater than about 10 kilobases (kb) from DNA isolated from a population of cells, said population of cells comprising a plurality of engineered cells that each comprise, or are suspected of comprising, a transgene sequence comprising a nucleic acid sequence encoding a recombinant protein; and (b) determining the average or mean copy number per diploid genome or per cell of the transgene sequence sequence in the high molecular weight fraction, thereby assessing transgene sequences integrated into the genome of the plurality of engineered cells of the population of cells.

Also provided herein are methods for assessing genomic integration of a transgene sequence. In some of any of the embodiments, the methods involve: (a) separating, by pulse field gel electrophoresis, a high molecular weight fraction of deoxyribonucleic acid (DNA) of greater than or greater than about 10 kilobases (kb) from DNA isolated from a population of cells, said population of cells comprising a plurality of engineered cells that each comprise, or are suspected of comprising, a transgene sequence comprising a nucleic acid sequence encoding a recombinant protein; and (b) from the high molecular weight fraction, determining the average or mean copy number per diploid genome or per cell of the transgene sequence integrated into the genome of the plurality of engineered cells of the population of cells.

Provided herein is a method for assessing genomic integration of a transgene sequence, the method comprising: (a) separating, by pulse field gel electrophoresis, a high molecular weight fraction of deoxyribonucleic acid (DNA) of greater than or greater than about 10 kilobases (kb) from DNA isolated from a population of cells, said population of cells comprising a plurality of engineered cells that each comprise, or are suspected of comprising, a transgene sequence encoding a recombinant protein; and (b) determining the average or mean copy number per diploid genome or per cell of the transgene sequence integrated into the genome of the plurality of engineered cells of the population of cells.

In some of any of the provided embodiments, prior to the separating, a polynucleotide comprising the transgene sequence encoding the recombinant protein has been introduced into a cell, e.g. of a population of cells, to result in at least one of the plurality of engineered cells of the population of cells. In some of any of the provided embodiments, the population of cells has not been incubated at a temperature greater than 25°C, optionally at or about 37°C ± 2°C, for more than 96 hours following the introduction of the polynucleotide comprising the transgene sequence into the at least one engineered cell. In some of any of the provided embodiments, the population of cells has not been incubated at a temperature greater than 25°C, optionally at or about 37°C ± 2°C, for more than 72 hours following the introduction of the polynucleotide comprising the transgene sequence into the at least one engineered cell. In some of any of the provided embodiments, the population of cells has not been incubated at a temperature greater than 25°C, optionally at or about 37°C ± 2°C, for more than 48 hours following the introduction of the polynucleotide comprising the transgene sequence into the at least one engineered cell. In some of any of the provided embodiments, the population of cells has been cryopreserved prior to the separating of the high molecular weight fraction of DNA.

In some of any of the provided embodiments, the high molecular weight fraction is of greater than or greater than about 15 kilobases (kb). In some of any of the provided embodiments, the high molecular weight fraction is of greater than or greater than about 17.5 kilobases (kb). In some of any of the provided embodiments, the high molecular weight fraction is of greater than or greater than about 20 kilobases (kb).

In some of any embodiments, the transgene sequence comprises a regulatory element linked to a nucleic acid sequence encoding a recombinant protein.

In some of any of the provided embodiments, the determining the presence, absence or amount of the transgene sequence is carried out by polymerase chain reaction (PCR). In some of any of the provided embodiments, the PCR is quantitative polymerase chain reaction (qPCR), digital PCR or droplet digital PCR. In some of any of the provided embodiments, the PCR is droplet digital PCR. In some of any of the provided embodiments, the PCR is carried out using one or more primers that is complementary to or is capable of specifically amplifying at least a portion of the transgene sequence. In some of any embodiments, the one or more primers is complementary to or is capable of specifically amplifying sequences of the regulatory element.

In some of any of the provided embodiments, the determining the amount of the transgene sequence comprises assessing the mass, weight or copy number of the transgene sequence per mass or weight of DNA isolated from the one or more cells, optionally per microgram of DNA isolated from the one or more cells. In some of any of the provided embodiments, the determining the amount of the transgene sequence comprises assessing the mass or weight of transgene sequence in microgram, per microgram of DNA isolated from one or more cells. In some of any of the provided embodiments, the determining the amount of the transgene sequence comprises assessing the mass, weight or copy number of the transgene sequence per the one or more cells, optionally per CD3+, CD4+ and/or CD8+ cell, and/or per cell expressing the recombinant protein.

In some of any of the provided embodiments, the determining the presence, absence or amount of the transgene sequence comprises assessing the mass, weight or copy number of the transgene sequence per diploid genome or per cell in the biological sample. In some of any of the provided embodiments, the copy number is an average or mean copy number per diploid genome or per cell among the one or more cells in the biological sample.

In some of any of the provided embodiments, the determining the amount of the transgene sequence comprises assessing the mass, weight or copy number of the transgene sequence per volume of the biological sample, optionally per microliter or per milliliter of the biological sample. In some of any of the provided embodiments, the determining the amount of the transgene sequence comprises assessing the mass, weight or copy number of the transgene sequence per body weight or body surface area of the subject. In some of any of the provided embodiments, determining the amount of the transgene sequence comprises assessing the mass, weight or copy number of the transgene sequence in the high molecular weight fraction and normalizing the mass, weight or copy number to the mass, weight or copy number of a reference gene in the high molecular weight fraction or to a standard curve. In some of any of the provided embodiments, the reference gene is a housekeeping gene. In some of any of the provided embodiments, the reference gene is a gene encoding albumin *(ALB).* In some of any of the provided embodiments, the reference gene is a gene encoding ribonuclease P protein subunit p30 *(RPP30).* In some of any of the provided embodiments, the copy number of a reference gene in the isolated DNA is carried out by PCR using one or more primers that is complementary to or is capable of specifically amplifying at least a portion of the reference gene.

In some of any of the provided embodiments, the transgene sequence does not encode a complete viral gag protein. In some of any of the provided embodiments, the transgene sequence does not comprise a complete HIV genome, a replication competent viral genome, and/or accessory genes, which accessory genes are optionally Nef, Vpu, Vif, Vpr, and/or Vpx.

In some of any of the provided embodiments, the introduction of the polynucleotide is carried out by transduction with a viral vector comprising the polynucleotide. In some of any of the provided embodiments, the viral vector is a retroviral vector or a gammaretroviral vector. In some of any of the provided embodiments, the viral vector is a lentiviral vector. In some embodiments, the viral vector is an AAV vector, optionally selected from among AAV1, AAV2, AAV3, AAV4, AAV5, AAV6, AAV7 or AAV8 vector.

In some of any of the provided embodiments, the introduction of the polynucleotide is carried out by a physical delivery method, optionally by electroporation.

In some of any of the provided embodiments, the recombinant protein is a recombinant receptor. In some of any of the provided embodiments, the recombinant receptor specifically binds to an antigen associated with a disease or condition or an antigen that is expressed in cells of the environment of a lesion associated with a disease or condition. In some of any of the provided embodiments, the disease or condition is a cancer. In some of any of the provided embodiments, the antigen is selected from αvβ6 integrin (avb6 integrin), B cell maturation antigen (BCMA), B7-H3, B7-H6, carbonic anhydrase 9 (CA9, also known as CAIX or G250), a cancer-testis antigen, cancer/testis antigen 1B (CTAG, also known as NY-ESO-1 and LAGE-2), carcinoembryonic antigen (CEA), a cyclin, cyclin A2, C-C Motif Chemokine Ligand 1 (CCL-1), CD19, CD20, CD22, CD23, CD24, CD30, CD33, CD38, CD44, CD44v6, CD44v7/8, CD123, CD133, CD138, CD171, chondroitin sulfate proteoglycan 4 (CSPG4), epidermal growth factor protein (EGFR), type III epidermal growth factor receptor mutation (EGFR vIII), epithelial glycoprotein 2 (EPG-2), epithelial glycoprotein 40 (EPG-40), ephrinB2, ephrin receptor A2 (EPHa2), estrogen receptor, Fc receptor like 5 (FCRL5; also known as Fc receptor homolog 5 or FCRH5), fetal acetylcholine receptor (fetal AchR), a folate binding protein (FBP), folate receptor alpha, ganglioside GD2, O-acetylated GD2 (OGD2), ganglioside GD3, glycoprotein 100 (gp100), glypican-3 (GPC3), G protein-coupled receptor class C group 5 member D (GPRC5D), Her2/neu (receptor tyrosine kinase erb-B2), Her3 (erb-B3), Her4 (erb-B4), erbB dimers, Human high molecular weight-melanoma-associated antigen (HMW-MAA), hepatitis B surface antigen, Human leukocyte antigen A1 (HLA-A1), Human leukocyte antigen A2 (HLA-A2), IL-22 receptor alpha(IL-22Rα), IL-13 receptor alpha 2 (IL-13Rα2), kinase insert domain receptor (kdr), kappa light chain, L1 cell adhesion molecule (L1-CAM), CE7 epitope of L1-CAM, Leucine Rich Repeat Containing 8 Family Member A (LRRC8A), Lewis Y, Melanoma-associated antigen (MAGE)-A1, MAGE-A3, MAGE-A6, MAGE-A10, mesothelin (MSLN), c-Met, murine cytomegalovirus (CMV), mucin 1 (MUC1), MUC16, natural killer group 2 member D (NKG2D) ligands, melan A (MART-1), neural cell adhesion molecule (NCAM), oncofetal antigen, Preferentially expressed antigen of melanoma (PRAME), progesterone receptor, a prostate specific antigen, prostate stem cell antigen (PSCA), prostate specific membrane antigen (PSMA), Receptor Tyrosine Kinase Like Orphan Receptor 1 (ROR1), survivin, Trophoblast glycoprotein (TPBG also known as 5T4), tumor-associated glycoprotein 72 (TAG72), Tyrosinase related protein 1 (TRP1, also known as TYRP1 or gp75), Tyrosinase related protein 2 (TRP2, also known as dopachrome tautomerase, dopachrome delta-isomerase or DCT), vascular endothelial growth factor receptor (VEGFR), vascular endothelial growth factor receptor 2 (VEGFR2), Wilms Tumor 1 (WT-1), a pathogen-specific or pathogen-expressed antigen, or an antigen associated with a universal tag, and/or biotinylated molecules, and/or molecules expressed by HIV, HCV, HBV or other pathogens. In some of any of the provided embodiments, the recombinant receptor is a recombinant T cell receptor (TCR) or a functional non-T cell receptor.

In some of any of the provided embodiments, the recombinant receptor is a chimeric antigen receptor (CAR). In some of any of the provided embodiments, the CAR comprises an extracellular antigen-recognition domain that specifically binds to the antigen and an intracellular signaling domain comprising an ITAM. In some of any of the provided embodiments, the intracellular signaling domain comprising an ITAM comprises an intracellular domain of a CD3-zeta (CD3ζ) chain, optionally a human CD3-zeta chain. In some of any of the provided embodiments, the intracellular signaling domain further comprises a costimulatory signaling region. In some embodiments, the costimulatory signaling region comprises a signaling domain of CD28 or 4-1BB, optionally human CD28 or human 4-1BB.

Provided herein is a method for assessing a residual non-integrated transgene sequence, the method comprising: (a) performing the method of any of the provided embodiments, to determine the presence, absence or amount of the transgene sequence in the high molecular weight fraction of DNA, thereby assessing genomic integration of a transgene sequence; (b) determining the presence, absence or amount of the transgene sequence in the isolated DNA without separating the high molecular weight fraction; and (c) comparing the amount determined in (a) to the amount determined in (b), thereby determining the amount of the residual non-integrated recombinant sequence.

In some of any of the provided embodiments, the determining the presence, absence or amount of the transgene sequence comprises determining the mass, weight or copy number of the transgene sequence per diploid genome or per cell in the one or more cells. In some of any of the provided embodiments, the one or more cell comprises a population of cells in which a plurality of cells of the population comprise the transgene sequence encoding the recombinant protein. In some of any of the provided embodiments, the one or more cell comprises a population of cells in which a plurality of cells of the population is suspected of comprising the transgene sequence encoding the recombinant protein. In some of any of the provided embodiments, the copy number is an average or mean copy number per diploid genome or per cell among the population of cells.

In some of any of the provided embodiments, comparing the amount comprises subtracting the copy number determined in (a) from the copy number determined in (b). In some of any of the provided embodiments, comparing the amount comprises determining the ratio of the copy number determined in (a) to the copy number determined in (b).

The determining the presence, absence or amount in (b) is carried out by polymerase chain reaction (PCR). In some of any of the provided embodiments, the PCR is quantitative polymerase chain reaction (qPCR), digital PCR or droplet digital PCR. In some of any of the provided embodiments, the PCR is droplet digital PCR. In some of any of the provided embodiments, the PCR is carried out using one or more primers that is complementary to or is capable of specifically amplifying at least a portion of the transgene sequence.

In some of any of the provided embodiments, determining the presence, absence or amount in (b) comprises assessing the mass, weight or copy number of the transgene sequence in the isolated DNA without separating the high molecular weight fraction and normalizing the mass, weight or copy number to the mass, weight or copy number of a reference gene in the isolated DNA without separating the high molecular weight fraction or to a standard curve. In some of any of the provided embodiments, the reference gene is a housekeeping gene. In some of any of the provided embodiments, the reference gene is a gene encoding albumin *(ALB).* In some of any of the provided embodiments, the reference gene is a gene encoding ribonuclease P protein subunit p30 *(RPP30).*

In some of any of the provided embodiments, the determining the mass, weight or copy number of a reference gene in the isolated DNA is carried out by PCR using one or more primers that is complementary to or is capable of specifically amplifying at least a portion of the reference gene.

In some of any of the provided embodiments, the determining the presence, absence or amount in (a) and the determining the presence, absence or amount in (b) is carried out by polymerase chain reaction (PCR) using the same primer or the same sets of primers.

In some of any of the provided embodiments, the residual non-integrated recombinant sequence comprises one or more of vector plasmids, linear complementary DNA (cDNA), autointegrants or long terminal repeat (LTR) circles.

### Brief Description of the Drawings

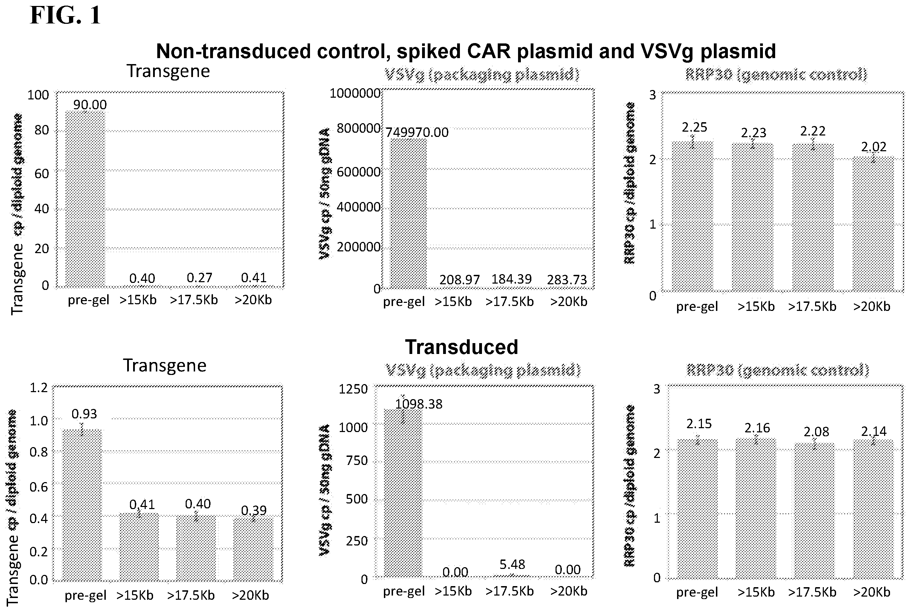

**FIG. 1** shows the copy number as assessed by droplet digital PCR (ddPCR) before (pre-gel; standard vector copy number (VCN) assay) or after separating high molecular weight DNA fraction, above a threshold of 15 kb, 17.5 kb or 20 kb by pulse-field gel electrophoresis (PFGE) (integrated vector copy number (iVCN) assay). The copy number was assessed using primers that specifically amplify a portion of the integrated transgene sequences ("transgene"); packaging plasmid (viral packaging plasmid encoding Vesicular stomatitis Indiana virus G protein ("VSVg")), or a genomic control (gene encoding for ribonuclease P protein subunit p30 ("RRP30")). The assessment was performed in a sample from transduced cells, or in a non-transduced control, spiked CAR plasmid and VSVg plasmid. Copy number of each gene was normalized to the number of diploid genomes (cp/diploid genome; using primers specific for the albumin gene as a reference) or per 50 ng of genomic DNA.

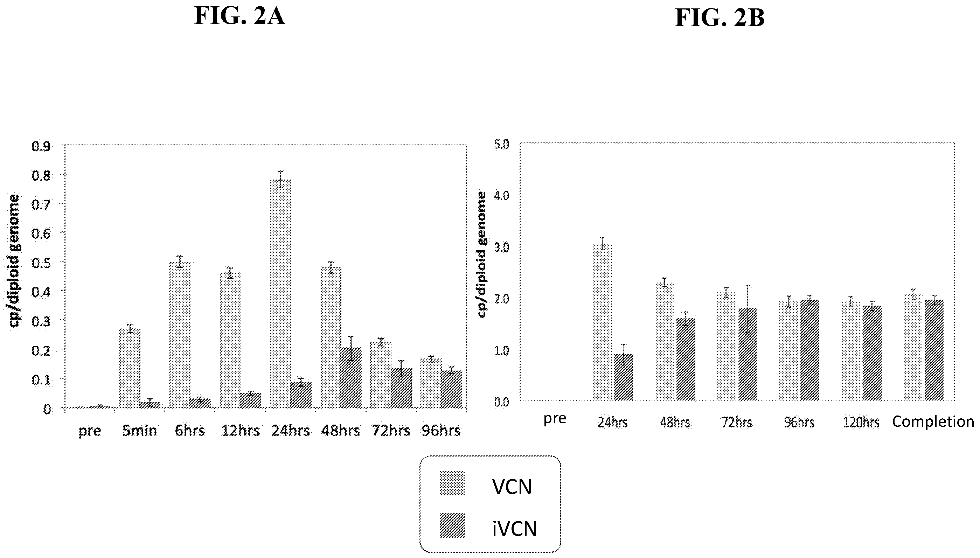

**FIGS. 2A-2B** depict the copy number as assessed by standard vector copy number (VCN) assay (genomic DNA samples that were not subject to PFGE, containing both high- and low-molecular weight DNA) and integrated vectory copy number (iVCN) assay (in high-molecular weight DNA samples after PFGE) of transgene sequences at various time points (prior to transduction ("pre"), at 5 minutes, 6 hours, 12 hours, 24 hours, 48 hours, 72 hours and 96 hours after transduction, or at completion of the engineering process, "completion") for Jurkat T cells **(****FIG. 2A****)** or primary T cells isolated from human subjects **(****FIG. 2B****),** transduced with a lentiviral preparation containing transgene sequences encoding a CAR. Copy number of each gene was normalized to the number of diploid genomes (cp/diploid genome; using primers specific for the albumin gene as a reference).

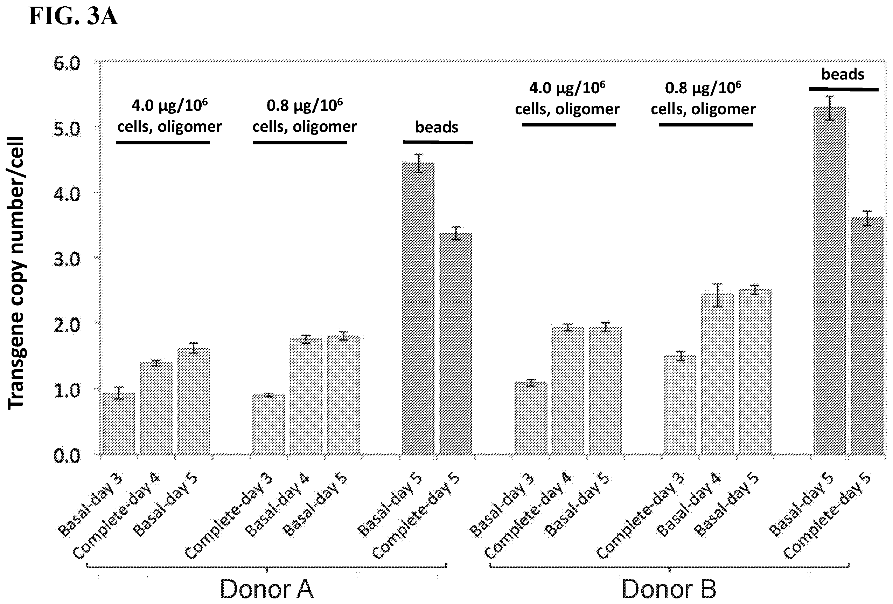

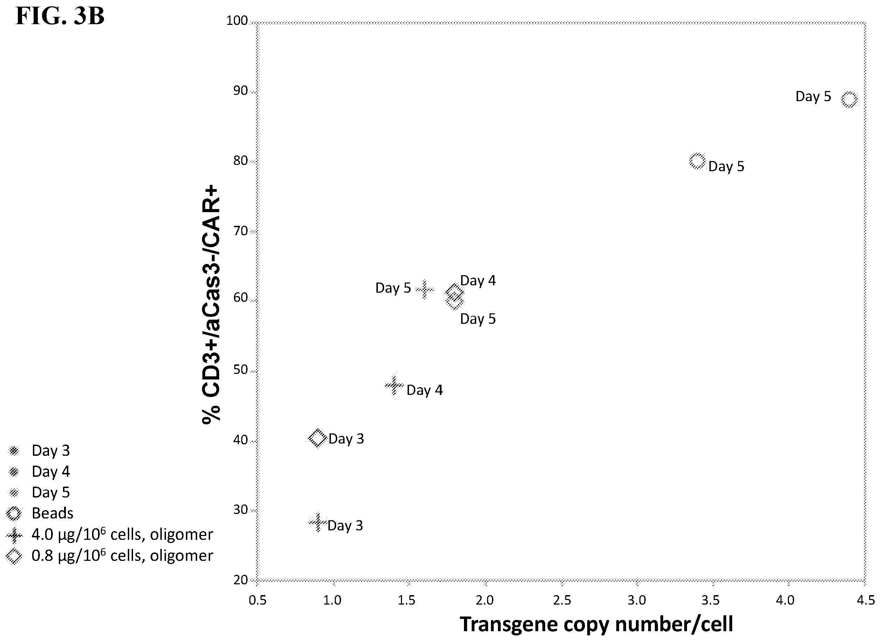

**FIG. 3A** shows the integrated copy number assessed at on day 3, 4 or 5 of exemplary non-expanded T cell composition manufacturing processes, using primary T cells from two different human subjects (Donor A and Donor B) that were stimulated stimulated by incubation with (1) anti-CD3/anti-CD28 antibody conjugated paramagnetic beads ("beads"), (2) anti-CD3/anti-CD28 Fab conjugated oligomeric streptavidin mutein reagents at a concentration of 4.0 µg per 10⁶ cells, or (3) anti-CD3/anti-CD28 Fab conjugated oligomeric streptavidin mutein reagents at a concentration of 0.8 µg per 10⁶ cells, incubated in basal media without serum or growth factors ("basal") or serum free media containing IL-2, IL-5, and IL-15 ("complete") after transduction. **FIG. 3B** depicts the correlation between the copy number as determined by iVCN and the percentage of CAR-expressing cell (as determined by the percentage of CD3+/activated Cas3-/CAR+ cells among CD3+ cells by flow cytometry).

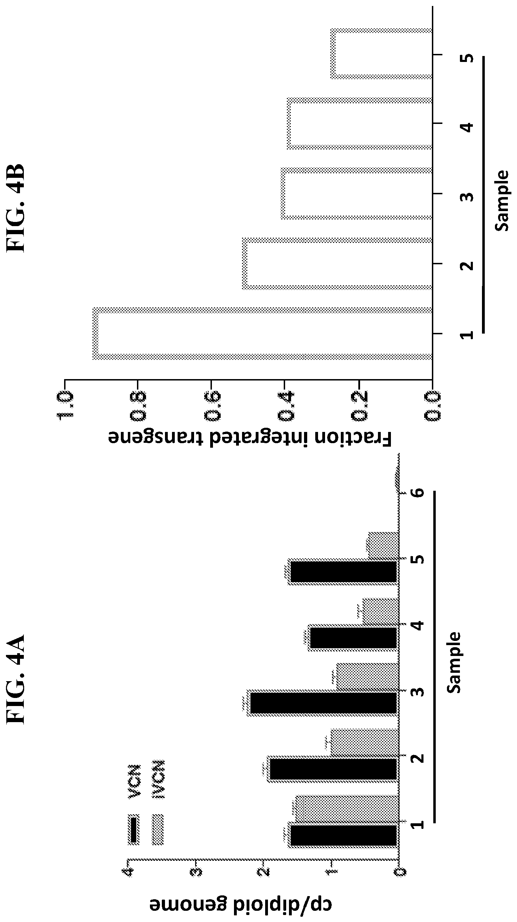

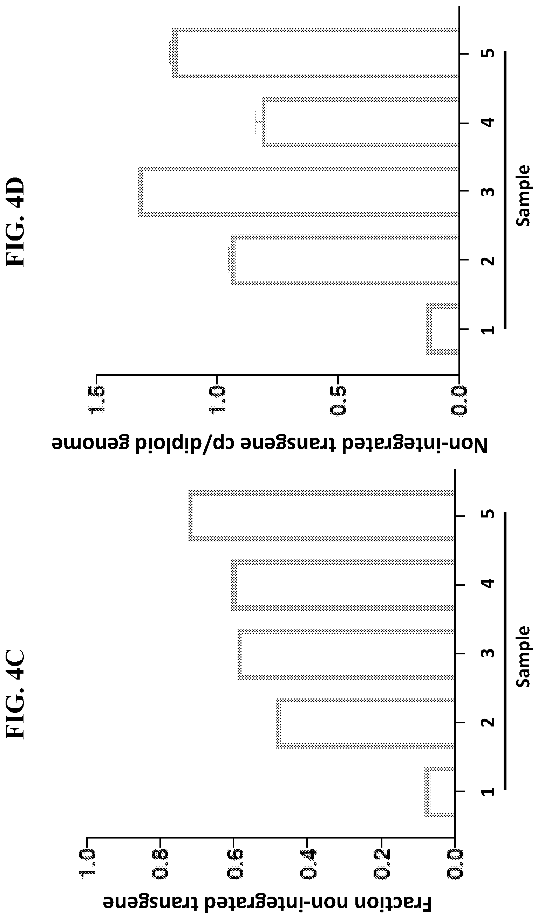

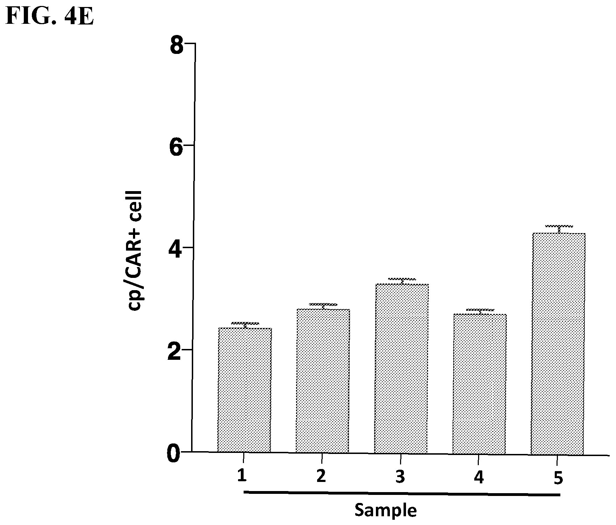

**FIGS. 4A-4E** depict the copy number per diploid genome as assessed by standard VCN (without PFGE) and iVCN (with PFGE) **(****FIG. 4A****),** fraction of integrated transgene **(****FIG. 4B****),** fraction of non-integrated transgene **(****FIG. 4C****),** non-integrated transgene copy number per diploid genome **(****FIG. 4D****),** and integrated copy number per CAR+ cell **(****FIG. 4E****),** during various exemplary expanded or non-expanded T cell composition manufacturing processes that employ different stimulating reagents and collection time, as set forth in **Table E1**.

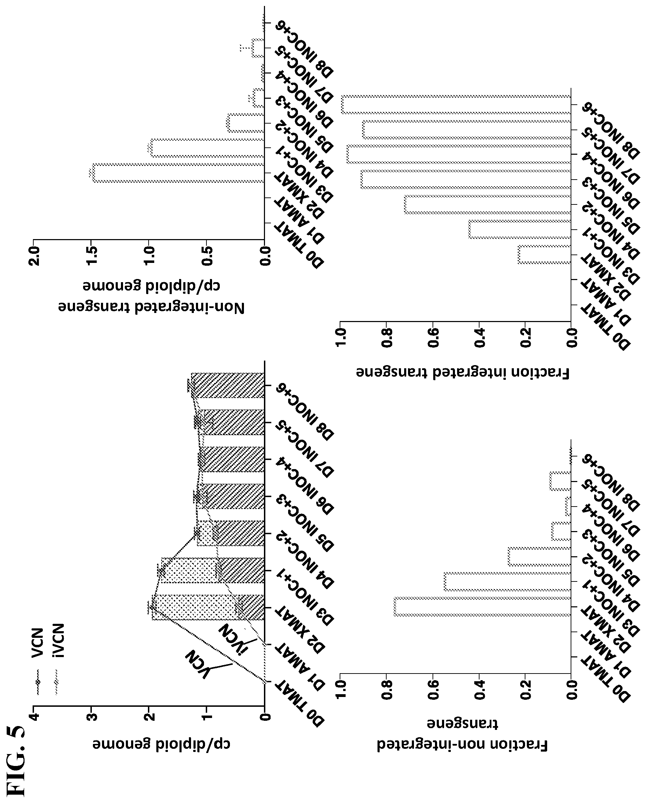

**FIG. 5** depicts the copy number per diploid genome as assessed by standard VCN (without PFGE) and iVCN (with PFGE), non-integrated transgene copy number, fraction of non-integrated transgene and fraction of integrated transgene, during various time points in an exemplary engineering process to engineer primary T cells from various donors to express a chimeric antigen receptor (CAR). Assessed time points include from day 0 to day 8 of the expanded processes, including at thawed material (TMAT; day 0), at activation (AMAT; day 1), at transduction (XMAT; day 2) or at various times after initiation of cultivation (inoc+1 to inoc+6; representing days 3-8 of the process).

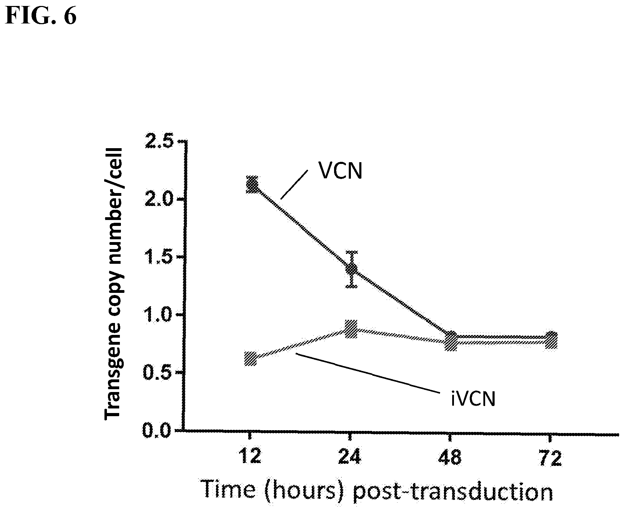

**FIG. 6** shows the copy number per diploid genome as assessed by standard VCN (without PFGE) and iVCN (with PFGE) per cell, in the HT1080 human fibroblast cell line, at 12, 24, 48 or 72 hours after transduction.

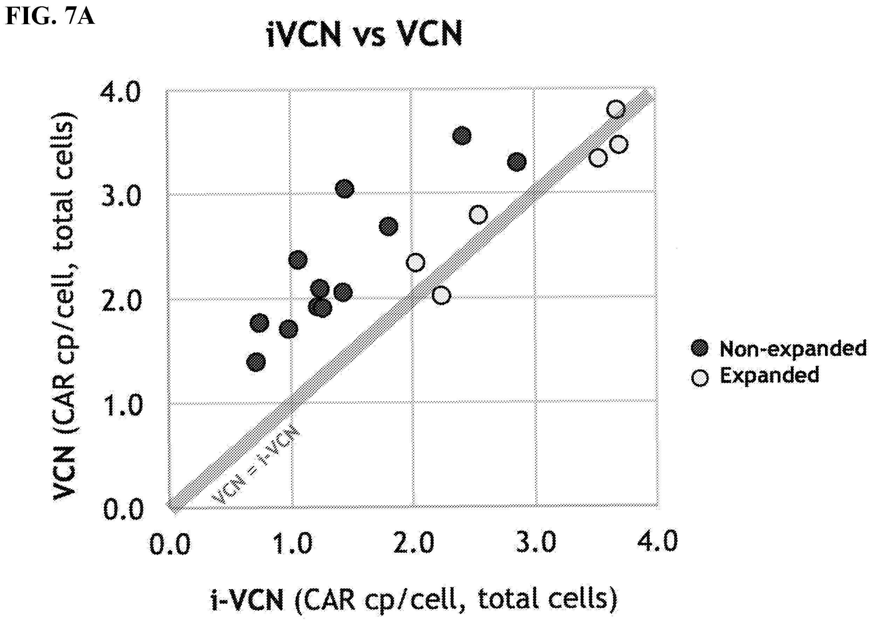

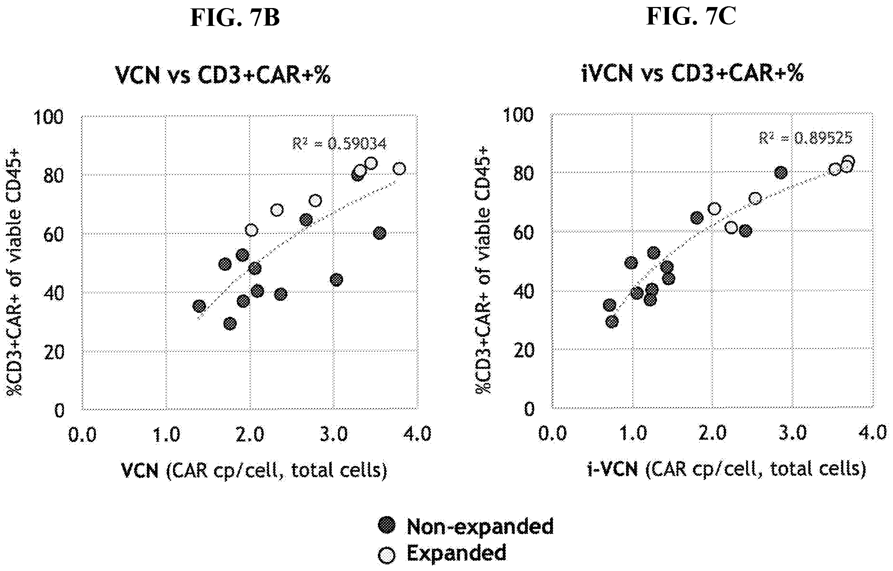

**FIG. 7A** shows the relationship between copy number per cell among total cells as assessed by standard VCN (without PFGE) and iVCN (with PFGE), in cell compositions produced from primary T cells from different human donors that had been engineered to express a CAR using an expanded process (∘) or a non-expanded process (•). **FIGS. 7B-7C** show the relationship between the copy number per cell in the cell compositions as assessed by standard VCN **(****FIG. 7B****)** or iVCN **(****FIG. 7C****)** and the surface expression of the CAR, as indicated by the percentage of CAR-expressing CD3+ cells (%CD3+CAR+) among viable CD45+ cells assessed by flow cytometry.

### Detailed Description

Provided herein are methods for assessing the integration of nucleic acid sequences integrated into a genome of a genetically engineered cell. The methods are used to determine the presence, absence and/or amount of nucleic acid sequences, such as transgene sequences used for genetic engineering of a cell. The methods are used to assess integration of transgene sequences in the genome of a cell, such as a genetically engineered cell used in cell therapy. In some aspects, cells are genetically engineered to express a recombinant protein, such as a recombinant receptor, by the introduction of a polynucleotide containing nucleic acid sequences, such as a transgene sequence encoding a recombinant protein, to be integrated into the genome of the cell. In some aspects, the provided methods can be used to assess integration, and distinguish and/or determine the presence, absence or amount of integrated nucleic acids and non-integrated, residual nucleic acids.

In some aspects, the polynucleotide containing a transgene sequence encoding a recombinant protein is introduced into the cell using various delivery methods such as viral transduction or physical delivery methods such as electroporation. In some embodiments, the engineered cells, such as engineered cells for adoptive cell therapy, are required to be monitored or assessed for various characteristics and features, such as determining the level of expression of the recombinant protein encoded by the transgene sequences, and/or determining the number of copies of the transgene sequences that are integrated into the genome of the cell, such as stably integrated into the genome of the cell. In some embodiments, the engineered cells are required to be monitored for the presence, absence and/or amount of non-integrated, residual nucleic acids. In some aspects, such assessment can be performed at one or more time points during the engineering or manufacturing process. Particularly for engineered cells and cell compositions for use in cell therapy, efficient and accurate determination of the presence, absence, amount, copy number and/or expression of the transgene sequences is critical, such as adoptive cell therapy, to ensure the proper and accurate characterization and definition of the engineered cells, to accurately determine dosing, and to ensure efficacy and safety of the cell compositions when administered to a subject. Improved methods to satisfy such requirements for efficient and accurate assessment of the presence, absence, amount, copy number and/or expression of the integrated and non-integrated, residual nucleic acids, are needed.

In some cases, the assessment may need to be performed during an early stage of the engineering or manufacturing process and/or during a manufacturing process that is shortened or abbreviated, in a timely and reliable manner. Exemplary shortened or abbreviated manufacturing processes include a non-expanded manufacturing process, for example, a process that does not include or includes a shorter or more abbreviated incubation for expansion of the cells after transduction. In some aspects, certain shortened or abbreviated processes may involve an incubation of the cells under conditions that does not substantially expand the cells or only minimally expands the cells. In some cases, such processes may include incubation of the cells at a temperature greater than 25°C, optionally at or about 37°C ± 2°C for no more than 96, 72, or 48 hours following introduction of a recombinant or heterologous polynucleotide, such as by transduction.

In some cases, determining the presence, absence and/or amount of transgene sequences integrated into the genome of a cell can be difficult, particularly in early time points or using processes that are non-expanded, for example processes that have a shorter period of incubation, cultivation or expansion after introduction of nucleic acid sequences for integration, or a shorter period of incubation, cultivation or expansion prior to assessment of characteristics or features of the engineered cells or prior to cryopreservation. In some aspects, the presence, absence or amount determined from DNA isolated from cells during early stages of the engineering or manufacturing process or in a process that is non-expanded, may result in an overestimate, due to the presence of non-integrated species of nucleic acids present in the reaction or in the cells. In some aspects, the provided embodiments permit specific determination of the presence, absence or amount of integrated nucleic acids, and in some cases, non-integrated, residual nucleic acids, prior to administration of cells to a subject.

The provided embodiments offer improved solutions for the requirement of efficiently and accurately assessing the engineered cells or cell compositions. In particular, improved methods are needed for cells engineered using an abbreviated or non-expanded process, or in early time points in the engineering process. In some aspects, the provided embodiments are based on an observation described herein, for example, in Examples 3-5, that an assay method that includes separation of the high molecular weight fraction of DNA from a cell, e.g. greater than about 10 kilobases (kb), and assessing the presence, absence or amount of the transgene sequences in the high molecular weight fraction can reliably detect integrated transgene sequences in a sample. It is found that the provided method, which contains integrated transgene sequences that is separated from non-integrated, residual sequences, offers an advantage, particularly during abbreviated, non-expanded processes.

The provided embodiments are based on methods to separate or isolate large or high molecular weight nucleic acid molecules, which may contain genomic DNA, apart from smaller or low molecular weight nucleic acid molecules that may contain non-integrated or residual molecules, such as episomal plasmids, autointegrants or other fragments. In some aspects, the high molecular weight nucleic acid molecules also include transgene sequences that have been integrated into the genome. The provided embodiments provide an advantage that it can be used to distinguish integrated sequences (e.g., integrated into the genome of the cell) from non-integrated, residual sequences, therefore allowing accurate and reliable determination of integrated sequences, even at early stages manufacturing or in non-expanded processes.

The provided methods permit the efficient and reliable determination of copy number of integrated nucleic acids, in particular, at early time point during the engineering or manufacturing process or for a non-expanded, shortened or expedited engineering or manufacturing processes, thereby improving the accuracy and reliability of characterization of cells for use or administration in cell therapy. In some aspects, the provided embodiments offer an advantage of accurately determining the integrated copy number at particular time points, such as during or at the end of a non-expanded, shortened or expedited engineering or manufacturing processes, without including copy numbers of non-integrated or residual molecules, such as episomal plasmids, autointegrants or other fragments.

In some aspects, the provided embodiments are based on an observation that copy number assessment in the high molecular weight fraction after separation of the fraction can result in accurately determining the copy number of stably integrated transgene sequence, particularly for cells generated using a non-expanded process which may retain free, non-integrated copies of transgene sequences. In comparison, copy number determination without prior separation of high molecular weight, which does not distinguish integrated vs. non-integrated transgene sequences, is limited in accurately determining the number of stably integrated transgene sequences, especially during and after a shorter, non-expanded process. As described herein, for example in Examples 3-5, during an abbreviated, non-expanded process, methods that do not employ separation of high molecular weight fragments (e.g, termed vector copy number (VCN) assay), can result in false positives or overestimation of integrated transgene sequences, as non-integrated, residual species (e.g., episomal plasmids, autointegrants or other fragments) can also be detected and counted. The embodiments provided herein provide various advantages, as they allow for efficient and accurate determination without false positive or overestimation that can result from existing VCN assays.

Also provided, but not claimed, are kits and articles of manufacture that can be used to perform the provided methods.

The section headings used herein are for organizational purposes only and are not to be construed as limiting the subject matter described.

### I. METHODS FOR ASSESSING INTEGRATED NUCLEIC ACIDS

Provided herein are methods for assessing genomic integration (in some cases, can be called an integrated vector copy number (iVCN) assay) of transgene sequences, such as a transgene sequence encoding a recombinant protein, used in genetic engineering of a cell. The methods are used to determine the presence, absence and/or amount of nucleic acid sequences, such as transgene sequences encoding a recombinant protein, such as a recombinant receptor. The methods involve determining the presence, absence or amount of the transgene sequence integrated into the genome of one or more cells, e.g., one or more genetically engineered cells that comprise, or are suspected of comprising, at least one engineered cell comprising a transgene sequence that comprises a nucleic acid sequence encoding a recombinant protein. In some aspects, the provided methods can be employed to assess integration at various time points before, during or after a cell engineering process, e.g., a process used to introduce polynucleotides containing transgene sequences that can be integrated into the genome of the cell. In some aspects, the provided methods can also be employed to assess biological samples obtained from a subject that has been administered engineered cells, to detect or determine the presence, absence and/or amount of transgene sequences in the biological sample.

In some aspects, the provided methods can be used to assess integration, and to distinguish and/or determine the presence, absence and/or amount of integrated nucleic acids and/or non-integrated, residual nucleic acids, such as one or more of vector plasmids, linear complementary DNA (cDNA), autointegrants or long terminal repeat (LTR) circles. In some aspects, the provided embodiments include methods for assessing the presence, absence, copy number of transgene sequence s in a biological sample from a subject that involves isolating deoxyribonucleic acid (DNA) from a biological sample from a subject, such as a subject that has been administered engineered cells.

Provided are methods for assessing genomic integration of a transgene sequence that involves separating a high molecular weight fraction of deoxyribonucleic acid (DNA), such as DNA species that are greater than or greater than about 10 kilobases (kb), from DNA isolated from one or more cell. In some aspects, such separation can be carried out by methods such as pulse field gel electrophoresis (PFGE). In some aspects, the one or more cell contains, or is suspected to contain, at least one engineered cell comprising a transgene sequence encoding a recombinant protein. In some aspects, the transgene sequence is or is to be integrated into the genome of the cell. In some embodiments, the transgene sequences include a nucleic acid sequence encoding the recombinant protein, and other components or elements, including regulatory elements, e.g., promoters, transcriptional and/or post-transcriptional regulatory elements or response elements, or markers, e.g., surrogate markers. In some aspects, the methods involve determining the presence, absence or amount of the transgene sequence integrated into the genome of the one or more cell, for example, by quantitative methods such as quantitative polymerase chain reaction (qPCR), digtal PCR (dPCR) or droplet digital PCR (ddPCR).

In some embodiments, provided are methods for assessing genomic integration of a transgene sequence, that involves separating, by pulse field gel electrophoresis, a high molecular weight fraction of deoxyribonucleic acid (DNA) of greater than or greater than about 10 kilobases (kb) from DNA isolated from a population of cells, said population of cells comprising a plurality of engineered cells that each comprise, or are suspected of comprising, a transgene sequence encoding a recombinant protein; and determining the average or mean copy number per diploid genome of the transgene sequence integrated into the genome of the plurality of engineered cells of the population of cells.

In some embodiments, prior to the separating, a polynucleotide comprising the transgene sequence encoding the recombinant protein has been introduced into at least one of the plurality of engineered cells of the population of cells.

The provided methods involve separating a particular fraction, such as a high molecular weight fraction, from other molecules or species of DNA present in the isolated total DNA from engineered cells. In some aspects, the methods involve separating a high molecular weight fraction, such as containing DNA with a size of about 10 kilobases (kb) or greater. In some aspects, the separation step is performed using methods for separating nucleic acids based on size or molecular weight, such as electrophoresis based methods. In some aspects, the methods also involve, isolating DNA from the one or more cell prior to the separating of the high molecular weight fraction from the isolated DNA.

The methods involve determining the presence, The methods involve determining the presence, absence and/or amount of the transgene sequences in one or more of the separated fractions, such as in the high molecular weight fraction. The methods involve determining the presence, absence and/or amount of the transgene sequences in the high molecular weight fraction. The methods involve, from the high molecular weight fraction, determining the presence, absence or amount of the transgene sequence integrated into the genome of the one or more cell. In some aspects, the determining can be based on methods to detect and/or quantitate nucleic acid sequences, such as quantitative polymerase chain reaction (qPCR) or related methods. In some aspects, the provided methods permit distinguishing of the integrated transgene sequence from the non-integrated transgene sequences that may be present in or near the cells and/or in the analysis sample.

In some embodiments, the methods involve isolating deoxyribonucleic acid (DNA) from a cell that has been introduced with a polynucleotide comprising a transgene sequence under conditions for integration into a genome of the cell, and separating a high molecular weight fraction of greater than or greater than about 10 kilobases (kb) from the isolated DNA. In some embodiments, the methods involve separating a high molecular weight fraction of greater than or greater than about 10 kilobases (kb) from deoxyribonucleic acid (DNA) isolated from a cell, wherein prior to the separating, the cell has been introduced with a polynucleotide comprising a transgene sequence under conditions for integration of the transgene sequence into a genome of the cell. In some aspects, the provided methods involve determining the presence, absence or amount of the transgene sequence in the separated high molecular weight fraction.

Also provided are methods for determining the presence, absence or amount of transgene sequences in a biological sample from a subject that involves isolating deoxyribonucleic acid (DNA) from a biological sample from a subject; separating a high molecular weight fraction of greater than or greater than about 10 kilobases (kb) from the isolated DNA; and determining the presence, absence or amount of the transgene sequence in the high molecular weight fraction. In some aspects, the biological sample is obtained from a subject that had been administered engineered cells comprising the transgene sequence.

In some aspects, also provided are method for assessing a residual non-integrated transgene sequence. In some embodiments, the methods involve performing steps of any of the methods, including isolating deoxyribonucleic acid (DNA) from a cell that has been introduced with a polynucleotide comprising a transgene sequence under conditions for integration into a genome of the cell and separating a high molecular weight fraction of greater than or greater than about 10 kilobases (kb) from the isolated DNA; determining the copy number of the transgene sequence in the high molecular weight fraction, thereby assessing genomic integration of a transgene sequence.

In some aspects, the methods also involve determining the copy number of the transgene sequence in the isolated DNA without separating the high molecular weight fraction, thereby determining the total copy number of transgene sequences. In some aspects, the methods involve determining the copy number of the residual non-integrated transgene sequence by subtracting the copy number determined by assessing the high molecular weight fraction from the copy number determined by assessing the total isolated DNA (such as without separating fractions). In some aspects, the methods involve determining the proportion of residual non-integrated transgene sequence by dividing the copy number determined by assessing the high molecular weight fraction from the copy number determined by the copy number determined by assessing the total isolated DNA (such as without separating fractions). In some embodiments, the residual non-integrated transgene sequence comprises one or more of vector plasmids, linear complementary DNA (cDNA), autointegrants or long terminal repeat (LTR) circles.

### A. Separating High Molecular Weight Fraction

The provided embodiments involve separating nucleic acids, such as deoxyribonucleic acid (DNA) obtained from engineered cells, based on their molecular weight or size. In some aspects, the method also comprises separating or isolating DNA molecules that fall within a size or molecular weight range. In some aspects, particular types of integrated transgene sequences or non-integrated transgene sequences can have a typical range of size or molecular weight. In some aspects, a particular size or molecular weight range can be used to separate or isolate DNA molecules with sizes or molecular weight within that range. In some aspects, the presence, absence or amount of the transgene sequences within a particular size or molecular weight range.

A high molecular weight fraction, for example, containing DNA molecules that are larger than a threshold value or within a size or molecular weight range, are separated or isolated. In some embodiments, the high molecular weight fraction primarily contain large DNA molecules such as chromosomal or genomic DNA, and contain low or almost no molecules that are smaller than the threshold value for size, such as plasmids, non-integrated DNA fragments, linear complementary DNA (cDNA), autointegrants, long terminal repeat (LTR) circles or other residual species or molecules that have not been integrated into the genome. The high molecular weight fraction primarily contain large DNA molecules such as chromosomal or genomic DNA, and are free of molecules that are smaller than the threshold value for size, such as plasmids, non-integrated DNA fragments, linear complementary DNA (cDNA), autointegrants, long terminal repeat (LTR) circles or other residual species or molecules that have not been integrated into the genome. In some embodiments, by determining the presence, absence or amount of the transgene sequences in the high molecular weight fraction, the detected transgene sequences represent those that have been integrated into the genome of the engineered cell, and minimizes the detection of non-integrated transgene sequences.

In some embodiments, the high molecular weight fraction comprises DNA molecules that are greater than or greater than about 10 kilobases (kb) in size. In some embodiments, the high molecular weight fraction comprises DNA molecules that are greater than or greater than about 10, 11, 12, 12.5, 13, 14, 15, 16, 17, 17.5, 18, 19, 20, 25 or 30 kilobases (kb) or more in size. In some embodiments, the high molecular weight fraction comprises DNA molecules that are greater than or greater than about 10, 12.5, 15, 17.5 or 20 kilobases (kb) or more in size. In some aspects, the high molecular weight fraction contains genomic DNA or genomic DNA fragments, and excludes or separates non-integrated or residual nucleic acid species that can be present in the DNA sample. In some aspects, the high molecular weight fraction, e.g., DNA samples that are above a threshold value such as about 10, 11, 12, 12.5, 13, 14, 15, 16, 17, 17.5, 18, 19, 20, 25 or 30 kilobases (kb) or more. In some embodiments, the threshold value is greater than or greater than about 10, 12.5, 15, 17.5 or 20 kilobases (kb) or more. In some embodiments, the high molecular weight fraction is of greater than or greater than about 15 kilobases (kb). In some embodiments, the high molecular weight fraction is of greater than or greater than about 17.5 kilobases (kb). In some embodiments, the high molecular weight fraction is of greater than or greater than about 20 kilobases (kb).

In some aspects, the threshold value is about or at or above 1, 1.5, 2, 2.5, 3, 3.5 or 4 times the size of the introduced polynucleotide. For example, in some aspects, if the introduced polynucleotide containing the transgene sequences is at or about 10 kb, the threshold value can be at or above 20 kb, approximately twice the size of the introduced polynucleotide. In some aspects, using the threshold value to separate high molecular weight fraction, non-integrated sequences, such as linear complementary DNA (cDNA), autointegrants, long terminal repeat (LTR) circles or other residual low molecular weight species or molecules that have not been integrated into the genome, can be separated from the high molecular weight species.

The high molecular weight fraction primarily contain DNA molecules that are larger than the non-integrated and/or residual DNA molecules that can be present in the manufacturing process and/or in the engineered cell, prior to or after completion of the integration of the transgene sequences. In some aspects, by virtue of determining the presence, absence and/or amount of the transgene sequences in the high molecular weight fraction, the copy number of integrated sequences can be accurately determined, without including the copy number of non-integrated or residual nucleic acid molecules containing the transgene sequences in the count. In some cases, inclusion of the copy number of non-integrated or residual nucleic acid molecules containing the transgene sequences can result in over-estimation or inaccurate determination of the copy number. In some aspects, particularly during early stages of the engineering or manufacturing process or in a process that is a non-expanded process, or a shortened process, the presence of non-integrated or residual molecules can affect the determined copy number.

In some embodiments, the method comprises separating or isolating a low molecular weight fraction, e.g., containing DNA molecules that are smaller than a threshold value. In some embodiments, the low molecular weight fraction can contain DNA molecules that are smaller than the threshold value for size, such as plasmids, non-integrated DNA fragments, linear complementary DNA (cDNA), autointegrants, long terminal repeat (LTR) circles or other residual species or molecules that have not been integrated into the genome. In some aspects, the presence, absence or amount of the transgene sequences in the low molecular weight fraction can be determined. In some cases, the presence, absence and/or amount of non-integrated or residual DNA molecules may need to be determined at various stages of manufacturing or engineering, such as to determine the progress of engineering and/or to assess the copy number of residual nucleic acids, such as residual vectors used to introduce the transgene sequences into the cells.

In some embodiments, the low molecular weight fraction comprises DNA molecules that are less than or less than about 20 kilobases (kb) in size. In some embodiments, the high molecular weight fraction comprises DNA molecules that are less than or less than about 20, 19, 18, 17.5, 17, 16, 15, 14, 13, 12.5, 12, 11 or 10 kilobases (kb) or less in size.

In some embodiments, the high- or low- molecular weight fraction can be separated or isolated using electrophoresis-, microfluidics- or chromatography-based methods. In some embodiments, the high molecular weight fraction can be separated or isolated using pulse field gel electrophoresis (PFGE) or size exclusion chromatography.

In some embodiments, the high molecular weight fraction is separated or isolated using pulse field gel electrophoresis (PFGE). In some aspects, PFGE involves introducing an alternating voltage gradient in an electrophoresis system to improve the resolution of larger nucleic acid molecules, such as chromosomal or genomic DNA. In some aspects, the voltage of the electrophoresis system is periodically switched among three directions: one that runs through the central axis of the gel and two that run at an angle of 60 degrees either side. In some aspects, exemplary systems and methods for separating or isolating nucleic acid molecules by PFGE include those described in, e.g., US 9599590; US 2017/0240882; or US 2017/0254774.

In some embodiments, the high molecular weight fraction is separated or isolated using an electrophoresis-based method. In some aspects, electrophoresis separates biomolecules by charge and/or size via mobility through a separating matrix in the presence of an electric field. In some embodiments, electrophoresis systems can be used to fractionate, analyze, and collect particular analytes, including nucleic acid molecules, based on size or molecular weight. In some aspects, a fraction is or includes a subset of the plurality of molecules. In some aspects, a fraction can be defined or determined by size or molecular weight, or in some aspects, by any physical property that causes it to migrate at a faster or slower rate than other molecules or fractions of a plurality when driven to migrate through a buffer composition of the disclosure by the force of an electric field (i.e., electrophoretic mobility).

In some aspects, the electrophoresis, such as PFGE, can be performed using an apparatus or system. In some aspects, the apparatus or system is an automated system or high-throughput system. Exemplary systems for performing PFGE, include, those described in, e.g., US 9599590; US 2017/0240882; or US 2017/0254774, or commercially available apparatus or system, such as Pippin Prep, Blue Pippin or Pippin HT (Sage Science); CHEF Mapper^{®} XA System, CHEF-DR^{®} III Variable Angle System, CHEF-DR II System (Bio-Rad); and Biometra Rotaphor 8 System (Analytik Jena AG).

In some aspects, exemplary samples for assessment include a nucleic acid, an oligonucleotide, a DNA molecule, a RNA molecule, or any combination thereof. In some aspects, the sample can include, an amino acid, a peptide, a protein, or any combination thereof. In some aspects, the sample can be a whole cell lysate, or the DNA or protein fraction of a cell lysate, such as lysate of cells engineered for adoptive cell therapy.

In some aspects, the provided embodiments involve the isolation, separation and analysis of nucleic acid molecules from cells, such as cells engineered for adoptive cell therapy. In some aspects, the provided embodiments involve the isolation, separation and analysis of nucleic acid molecules from cells undergoing various stages or steps of a genetic engineering or manufacturing process. In some aspects, nucleic acid molecule includes the phosphate ester polymeric form of ribonucleosides (adenosine, guanosine, uridine or cytidine; "RNA") or deoxyribonucleosides (deoxyadenosine, deoxyguanosine, deoxythymidine, or deoxycytidine; "DNA"), or any phosphoester analogues thereof, such as phosphorothioates and thioesters, in either single stranded form, or a double-stranded helix. In some aspects, nucleic acid molecule, and in particular DNA or RNA molecule, refers only to the primary and secondary structure of the molecule, and does not limit it to any particular tertiary forms. In some aspects, the nucleic acid molecule can include double-stranded DNA found, in linear or circular DNA molecules (e.g., restriction fragments), plasmids, and chromosomes.

In some embodiments, nucleic acids from the samples can include genomic DNA, double-stranded DNA (dsDNA), single-stranded DNA (ssDNA), coding DNA (or complementary DNA, cDNA), messenger RNA (mRNA), short interfering RNA (siRNA), short-hairpin RNA (shRNA), microRNA (miRNA), single-stranded RNA, double-stranded RNA (dsRNA), a morpholino, RNA interference (RNAi) molecule, mitochondrial nucleic acid, chloroplast nucleic acid, viral DNA, viral RNA, and other organelles with separate genetic material. In some aspects, the nucleic acids from the sample can also include nucleic acid analogs that contain modified, synthetic, or non-naturally occurring nucleotides or structural elements or other alternative/modified nucleic acid chemistries, such as base analogs such as inosine, intercalators (U.S. Pat. No. 4,835,263) and minor groove binders (U.S. Pat. No. 5,801,115).

In some embodiments, prior to isolating or separating a high- or low-molecular weight fraction, the samples can be combined with a reagent that imparts a net negative charge, denatures a peptide or protein, or digests a DNA or RNA molecule prior to assessment in an electrophoresis system. In some aspects, samples can be combined with agents that impart fluorescent, magnetic, or radioactive properties to the sample or fractions thereof for the purpose of detection. In some examples, a dsDNA sample is mixed with ethidium bromide, applied to the electrophoresis cassette, and fractions of the sample are detected using an ultrabright green LED.

In some aspects, a system for separating or isolating the nucleic acid samples, such as an electrophoresis system, can be automated and/or high-throughput. In some aspects, the electrophoresis system can utilize disposable consumables or reagents, such as an electrophoresis cassette.

### B. Determining Presence, Absence or Amount of Nucleic Acids

The methods involve determining the presence, absence or amount of a transgene sequence in a sample, such as a sample containing deoxyribonucleic acid (DNA) from one or more cells, such as a population of cells that includes engineered cells. In some aspects, the methods involve determining the presence, absence or amount of a transgene sequence in a sample, such as a sample containing DNA from one or more cells undergoing one or more stages or steps of genetic engineering or manufacturing. In some aspects, determining the presence, absence or amount of the transgene sequence can be performed using methods for determining the presence, absence or amount of a nucleic acid sequence, e.g., particular sequence of DNA. In particular, methods used to quantitate nucleic acid sequences, such quantitative polymerase chain reaction (qPCR) or related methods, can be employed in determining the copy number of the transgene sequence in a sample containing DNA, or in a particular fraction, such as the high molecular weight fraction, that is separated or isolated from samples containing DNA. In some embodiments, the determining the presence, absence or amount of the transgene sequence comprises determining the copy number, for example, using any one of the exemplary assays described below to quantitate nucleic acid molecules.

In some aspects, the presence, absence and/or amount of a particular sequence can be detected using a probe or a primer, that can specifically bind or recognize all or a portion of the transgene sequence. In some embodiments, copy number can be determined using probes that can specifically detect a portion of the transgene sequence, or primer sequences that can specifically amplify a portion of the transgene sequence. In some aspects, the probe or primer sequences can specifically detect, bind or recognize a portion of the transgene sequence, such as a portion of the transgene sequence that is heterologous, exogenous or transgenic to the cell. In some aspects, the probe or primer sequences can specifically detect, bind or recognize a portion of the transgene sequence, such as a portion of the transgene sequence that is to be or that is integrated into the genome of the cell. In some embodiments, the primers or probe used for qPCR or other nucleic acid-based methods are specific for binding, recognizing and/or amplifying nucleic acids encoding the recombinant protein, and/or other components or elements of the plasmid and/or vector, including regulatory elements, e.g., promoters, transcriptional and/or post-transcriptional regulatory elements or response elements, or markers, e.g., surrogate markers. In some embodiments, primers or probes can bind to nucleic acid sequences that encode the recombinant protein and/or other components or elements, such as regulatory elements, including post-transcriptional regulatory elements. In some of any embodiments, the one or more primers is complementary to or is capable of specifically amplifying sequences of a regulatory element, e.g., a regulatory element operably linked to the nucleic acid sequence encoding the recombinant protein. In some aspects, the probes or primers can be used for exemplary methods to determine the presence, absence and/or amount of transgene sequences, such as quantitative PCR (qPCR), digital PCR (dPCR) or droplet digital PCR (ddPCR).

In some aspects, the determining of the presence, absence or amount comprises determining the amount of the transgene sequence, such as determining the mass, weight, concentration or copy number of the transgene sequences, in one or more cells or in a biological sample containing one or more cells. In some aspects, the determining of the presence, absence or amount of a nucleic acid sequence, or assessing the mass, weight, concentration or copy number of the transgene sequences can be performed in a portion of a population of cells or a portion of a biological sample, and can be normalized, averaged, and/or extrapolated to determine the presence, absence or amount in the entire sample or entire population of cells. In some aspects, the amount of the transgene sequence can include the mass, weight, concentration or copy number of the transgene sequence. In some aspects, the mass, weight, concentration or copy number is an average mass, weight, concentration or copy number. In some aspects, the mass, weight, concentration or copy number is an average mass, weight, concentration or copy number per a unit, such as per cell, per diploid genome, per volume, per mass or equivalent thereof, or otherwise normalized, extrapolated or averaged to be per a unit.

In some embodiments, the determining the presence, absence or amount of the transgene sequence comprises determining the mass, weight, concentration or copy number of the transgene sequence per diploid genome or per cell in the one or more cells. In some embodiments, the one or more cell comprises a population of cells in which a plurality of cells of the population comprise the transgene sequence encoding the recombinant protein. In some of any of the provided embodiments, the one or more cell comprises a population of cells in which a plurality of cells of the population is suspected of comprising the transgene sequence that includes a nucleic acid sequence encoding the recombinant protein, e.g., a recombinant receptor. In some embodiments, the copy number is an average or mean copy number per diploid genome or per cell among the population of cells.

In some aspects, determining the copy number comprises determining the number of copies of the transgene sequences present in one or more cells, or in a biological sample. In some aspects, the copy number can be expressed as an average or mean copy number. In some aspects, the copy number of a particular integrated transgene includes the number of integrants (containing transgene sequences) per cell. In some aspects, the copy number of a particular integrated transgene includes the number of integrants (containing transgene sequences) per diploid genome. In some aspects, the copy number of transgene sequence is expressed as the number of integrated transgene sequences per cell. In some aspects, the copy number of transgene sequence is expressed as the number of integrated transgene sequences per a particular type of cell, e.g., per cell expressing a particular phenotypic marker or per cell that expresses the recombinant protein encoded by the introduced transgene. In some aspects, the copy number of transgene sequence is expressed as the number of integrated transgene sequences per diploid genome. In some aspects, the one or more cell comprises a population of cells in which a plurality of cells of the population comprise the transgene sequence encoding the recombinant protein. In some embodiments, the copy number is an average or mean copy number per diploid genome or per cell among the population of cells.

In some embodiments, the determining the amount of the transgene sequence comprises assessing the mass, weight, concentration or copy number of the transgene sequence per mass or weight of DNA isolated from the one or more cells. In some aspects, the mass, weight, concentration or copy number of the transgene sequence is expressed per microgram of DNA isolated from the one or more cells, for example, one or more cells in a biological sample obtained from a subject or in one or more cells that are in undergoing the engineering process. In some embodiments, the determining the amount of the transgene sequence comprises assessing the mass or weight of transgene sequence in microgram, per microgram of DNA isolated from one or more cells.

In some embodiments, the determining the amount of the transgene sequence comprises assessing the mass, weight, concentration or copy number of the transgene sequence per the one or more cells, optionally per CD3+, CD4+ and/or CD8+ cell, and/or per cell expressing the recombinant protein. In some embodiments, the determining the amount of the transgene sequence comprises assessing the mass, weight, concentration or copy number of the transgene sequence per the one or more cells, optionally per cells that express a particular phenotypic marker, e.g., optionally per CD3+, CD4+ and/or CD8+ cell, per viable cell, activated caspase negative cell (e.g., aCas3-) or CD45+ cell, and/or per cell expressing the recombinant protein. In some aspects, surface markers or phenotypes expressed on the cell can be determined using cell-based methods, such as by flow cytometry or immunostaining. In some aspects, the cells expressing the recombinant protein can be determined using cell-based methods, such as by flow cytometry or immunostaining. In some aspects, the amount of transgene sequences can be normalized to the number of particular cells, such as CD3+, CD4+ and/or CD8+ cell, and/or per cell expressing the recombinant protein, or per total number of cells, such as per total number of cells in the sample or per total number of cells undergoing an engineering process. In some aspects, the amount of transgene sequences can be normalized to the number of particular cells, such as cells that express a particular phenotypic marker, e.g., CD3+, CD4+ and/or CD8+ cells, viable cells, activated caspase negative cells (e.g., aCas3-) or CD45+ cells, and/or per cell expressing the recombinant protein (e.g., per CAR-expressing cell), or per total number of cells, such as per total number of cells in the sample or per total number of cells undergoing an engineering process.

In some embodiments, the determining the presence, absence or amount of the transgene sequence comprises assessing the mass, weight, concentration or copy number of the transgene sequence per diploid genome or per cell in the biological sample. In some embodiments, the copy number is an average or mean copy number per diploid genome or per cell among the one or more cells in the biological sample.

In some embodiments, the determining the amount of the transgene sequence comprises assessing the mass, weight, concentration or copy number of the transgene sequence per volume of the biological sample, optionally per microliter or per milliliter of the biological sample.

In some embodiments, the determining the amount of the transgene sequence comprises assessing the mass, weight, concentration or copy number of the transgene sequence per body weight or body surface area of the subject.

In some embodiments, the determining the amount of the transgene sequence comprises assessing the mass, weight, concentration or copy number of the transgene sequence in the high molecular weight fraction and normalizing the mass, weight, concentration or copy number to the mass, weight, concentration or copy number of a reference gene in the high molecular weight fraction or to a standard curve, such as a standard curve based on samples containing a known amount, concentration, mass, weight, concentration or copy number of transgene sequences.

In some embodiments, the determined copy number is expressed as a normalized value. In some embodiments, the determined copy number is quantified as a number of copy of the transgene sequence per genome or per cell. In some aspects, the per genome value is expressed as copy of the transgene sequence per diploid genome, as a typical somatic cell, such as a T cell, contains a diploid genome. In some aspects, the determined copy number can be normalized against the copy number of a known reference gene in the genome of the cell. In some aspects, the reference gene is *RRP30* (encoding ribonuclease P protein subunit p30), *18S rRNA* (18S ribosomal RNA), *28S rRNA* (28S ribosomal RNA), *TUBA* (α-tubulin), *ACTB* (β-actin), *β2M* (β2-microglobulin), *ALB* (albumin), *RPL32* (ribosomal protein L32), *TBP* (TATA sequence binding protein), *CYCC* (cyclophilin C), *EF1A* (elongation factor 1α), *GAPDH* (glyceraldehyde-3-phosphate dehydrogenase), *HPRT* (hypoxanthine phosphoribosyl transferase) or *RPII* (RNA polymerase II). In some embodiments, the determined copy number is quantified as copy of the transgene sequence per microgram of DNA.

In some aspects, the copy number is an average, mean, or median copy number from a plurality or population of cells, such as a plurality or population of engineered cells. In some aspects, the copy number is an average or mean copy number from a plurality or population of cells, such as a plurality or population of engineered cells In some aspects, the average or mean copy number is determined from a plurality or population of cells, such as a plurality or population of cells undergoing one or more steps of the engineering or manufacturing process, or in a cell composition, such as a cell composition for administration to a subject. In some aspects, a normalized average copy number is determined, for example, as an average or mean copy number of the transgene sequences normalized to a reference gene, such as a known gene that is present in two copies in a diploid genome. In some aspects, normalization to a reference gene that is typically present in two copies per diploid genome, can correspond to the copy number in a cell, such as a diploid cell. Thus, in some aspects, the normalized average or mean copy number can correspond to the average or mean copy number of the detected transgene sequences among a plurality or a population of cells, for example, T cells that typically have a diploid genome.

The determining the presence, absence or amount of the transgene sequence is carried out by polymerase chain reaction (PCR). In some embodiments, the PCR is quantitative polymerase chain reaction (qPCR), digital PCR or droplet digital PCR, such as any described below. In some embodiments, the presence, absence or amount of the transgene sequence is determined by droplet digital PCR. In some embodiments, the PCR is carried out using one or more primers that is complementary to or is capable of specifically amplifying at least a portion of the transgene sequence, and in some cases, one or more primers that is complementary to or is capable of specifically amplifying at least a portion of a reference gene.

In some aspects, the presence, absence and/or amount of the transgene sequences determined in one or more of the fractions, and/or in a particular sample, such as the total DNA isolated from a plurality or population of engineered cells, can be used as a basis to calculate particular ratios or proportions of interest, such as the proportion or number of non-integrated or residual molecules, for example, as described in Section I.C. below.

### 1. Quantitative PCR (qPCR)

In some embodiments, the presence, absence or amount of the transgene sequences, such as transgene sequences encoding a recombinant protein, for integration into the genome of the engineered cell, is determined by quantitative polymerase chain reaction (qPCR; in some cases, also known as real-time PCR).

In some aspects, qPCR can be used to detect the accumulation of amplification product measured as the reaction progresses, in real time, with product quantification after each cycle. Thus, in some aspects, qPCR can be used to determine the copy number of a particular nucleic acid sequence, such as the transgene sequence, in a sample. In some aspects, qPCR employs fluorescent reporter molecule in each reaction well that yields increased fluorescence with an increasing amount of product DNA. In some aspects, fluorescence chemistries employed include DNA-binding dyes and fluorescently labeled sequence-specific primers or probes. In some aspects, qPCR employs a specialized thermal cycler with the capacity to illuminate each sample at a specified wavelength and detect the fluorescence emitted by the excited fluorophore. In some aspects, the measured fluorescence is proportional to the total amount of amplicon; the change in fluorescence over time is used to calculate the amount of amplicon produced in each cycle.

In some aspects, qPCR permits the determination of the initial number of copies of a particular DNA (amplification target sequence, e.g., a transgene sequence encoding a recombinant protein) with accuracy and high sensitivity over a wide dynamic range. In some aspects, qPCR can generate results that are qualitative (the presence or absence of a sequence) or quantitative (copy number).

### 2. Digital PCR (dPCR)

In some embodiments, the presence, absence or amount of the transgene sequences, such as transgene sequences encoding a recombinant protein, for integration into the genome of the engineered cell, is determined using digital polymerase chain reaction (dPCR). In some aspects, dPCR permits determination of the presence, absence or amount of a particular sequence, such as the transgene sequence, with a high accuracy and sensitivity.

In some embodiments, dPCR is a method for detecting and quantifying nucleic acids, and permits accurate quantitative analysis and the highly sensitive detection of a target nucleic acid molecule. In some aspects, dPCR involves a limiting dilution of DNA into a succession of individual PCR reactions (or partitions). In some aspects, limiting dilution can employ the principles of partitioning with nanofluidics and emulsion chemistries, based on random distribution of the template nucleic acid to be assessed, e.g., transgene sequences, and Poisson statistics to measure the quantities of DNA present for a given proportion of positive partitions. In some aspects, dPCR is generally linear and are sensitive, capable of detecting or quantifying very small amounts of DNA. In some aspects, dPCR permits absolute quantification of a DNA sample using a single molecule counting method without a standard curve, and absolute quantification can be obtained from PCR for a single partition per well (see Pohl et al., (2004) Expert Rev. Mol. Diagn. 4(1), 41-47).

In some aspects, dPCR methods can be used to generate a plurality of partitions, such as thousands of partitions, to carry out a plurality of individual PCR reactions in parallel. In some aspects, a sample is partitioned so that individual nucleic acid molecules within the sample are localized and concentrated within many separate regions. Micro well plates, capillaries, micro- or nanofluidics, oil emulsion, emulsion chemistry and/or arrays of miniaturized chambers with nucleic acid binding surfaces can be used to partition the samples. Exemplary compositions for carrying out dPCR can include template nucleic acid (e.g., isolated DNA from engineered cells), fluorescence-quencher probes, primers, and a PCR master mix, which contains DNA polymerase, dNTPs, MgCl₂, and reaction buffers at optimal concentrations. The PCR solution is divided into smaller reactions and are then made to run PCR individually. The partitioning of the sample allows one to estimate the number of different molecules by assuming that the molecule population follows the Poisson distribution, thus accounting for the possibility of multiple target molecules inhabiting a single partition.

In some aspects, dPCR involves analyzing the results by a digital method (because the resultant signal has a binary value: "0" or "1"). In some aspects, dPCR can be used to analyze a large volume, analyze various samples at the same time, and multiple assessments can be performed at the same time. In some aspects, for digital PCR, each partition comprising a sample sequence template (e.g., potentially containing a transgene sequence encoding a recombinant protein) prepared so as to be diluted to an average copy number of the sequence is 0.5-1. The dilution is important to obtain a reliable results for quantification, to signals that appear in a Poisson distribution. In each well, amplification primers specific for the tested sequence (e.g., a portion of the transgene sequences) and a fluorescent probe, is dispensed and emulsion PCR is performed. In some aspects, a well exhibiting a fluorescent signal is counted as a value of "1", because a sample having a sequence copy number of 1 is dispensed into the well and shows the signal after amplification, and a well showing no signal is counted as "0", because a sample not containing a copy of the sequence is dispensed into the well and shows no signal due to no amplification. Using Poisson's law of small numbers, the distribution of target molecule within the sample can be accurately approximated, permitting an absolute quantification of the target sequences in the PCR product.

Exemplary commercially available apparatuses or systems for dPCR include Raindrop^{™} Digital PCR System (Raindance^{™} Technologies); QX200^{™} Droplet Digital^{™} PCR System (Bio-Rad); BioMark^{™} HD System and qdPCR 37K^{™} IFC (Fluidigm Corporation) and QuantStudio^{™} 3D Digital PCR System (Life Technologies^{™}) (see, e.g., Huggett et al. (2013) Clinical Chemistry 59: 1691-1693; Shuga, et al. (2013) Nucleic Acids Research 41(16): e159; Whale et al. (2013) PLoS One 3: e58177).

### 3. Droplet Digital PCR (ddPCR)

In some embodiments, the presence, absence or amount of the transgene sequences, such as transgene sequences encoding a recombinant protein, for integration into the genome of the engineered cell, is determined using droplet digital polymerase chain reaction (ddPCR). ddPCR is a type of digital PCR, in which the PCR solution is divided or partitioned into smaller reactions through a water-oil emulsion chemistry, to generate numerous droplets. In some aspects, particular surfactants can be used to generate the water-in-oil droplets. (see, e.g., Hindson et al., (2011) Anal Chem 83(22): 8604-8610; Pinheiro et al., (2012) Anal Chem 84, 1003-1011). In some aspects, each individual droplet is subsequently run as individual reaction. In some aspects, the PCR sample is partitioned into nanoliter-size samples and encapsulated into oil droplets. In some aspects, the oil droplets are made using a droplet generator that applies a vacuum to each of the wells. In an exemplary case, approximately 20,000 oil droplets for individual reactions can be made from a 20 µL sample volume.

In some aspects, following PCR, each droplet is analyzed or read to determine the fraction of PCR-positive droplets (e.g., binary "0" or "1" assigned in each droplet based on the fluorescence signal) in the original sample. The data are then analyzed using Poisson statistics to determine the target DNA sequence concentration in the original sample. Poisson distribution of the copies of target molecule per droplet (CPD) can be determined based on the fraction of fluorescent droplets (p), represented by the function CPD=-ln(1-p). This model can predict that as the number of samples containing at least one target molecule increases, the probability of the samples containing more than one target molecule increases.

### C. Exemplary Applications

In some aspects, the provided methods can be used to assess engineered cells and cell compositions containing engineered cells, for a variety of applications for assessing integration of nucleic acid sequences and/or characterization of engineered cells. For example, the methods can be used after engineering, prior to formulation, prior to administration and/or administration and/or at various stages and/or time points of the engineering or manufacturing process, to characterize integration of nucleic acids, such as the transgene sequences, and/or the engineered cells. In some embodiments, the methods can be used in cell compositions containing one or more engineered cells.

In some embodiments, the provided methods can be performed at one or more stages or time points during the manufacturing process, including before the engineered cells or cell compositions containing the engineered cells are released for infusion, ready for administration to a subject, and/or administered to a subject. In some embodiments, engineered cells or cell compositions are released for infusion, ready for administration to a subject, and/or administered to a subject after assessing one or more of the provided methods have been performed, e.g., on a portion, fraction, and/or sample of engineered cells or cell compositions. In particular embodiments, the engineered cells or cell compositions are released for infusion, ready for administration to a subject, and/or administered to a subject after the cells are determined to be safe, e.g., sterile and/or free, and/or have desired biological characteristics following the completion of the one or more methods, such as containing less than or more than a required threshold copy number of integrated transgene sequences.

In some aspects, the polynucleotide containing transgene sequences is introduced into the cell using various delivery methods such as viral transduction. In some embodiments, the engineered cells, such as engineered cells for adoptive cell therapy, are required to be monitored or assessed for various characteristics and features, such as determining the level of expression of the recombinant protein encoded by the transgene sequences, and/or determining the number of copies of the transgene sequences that are integrated into the genome of the cell, such as stably integrated into the genome of the cell. In some aspects, such assessment can be performed at one or more time points during the engineering or manufacturing process.

In some embodiments, the provided methods can be used to assess or determine the pharmacokinetic parameters and/or bioavailability of engineered cells after administration of the engineered cell or cell composition to a subject, such as a subject having a disease or condition for therapy. In some aspects, the provided methods can be used as a proxy to characterize the persistence, expansion and/or number of engineered cells, such as engineered cells introduced with a transgene sequence encoding a recombinant protein, such as a recombinant receptor.

In some aspects, described below are exemplary applications of the provided methods. In some aspects, variations of the methods and/or combination with other methods for assessing or characterizing the engineered cells or engineered cell compositions can also be used to determine the features and characteristics of the engineered cells for cell therapy.

### 1. Assessing Integration of Transgene Sequences

The methods for assessing genomic integration of transgene sequences, such as transgene sequences encoding a recombinant protein, such as a recombinant receptor. In some aspects, the provided methods can be used to assess the timing, extent or progression of integration of transgene sequences into a genome of the cell, after introduction of a polynucleotide comprising a transgene sequences under conditions for integration into a cell, for example, for genetic engineering. In some embodiments, In some aspects, the provided methods can be used to assess the timing, extent or progression of integration of transgene sequences into a genome of the cell, after introduction of a polynucleotide comprising a transgene sequences under conditions for integration into a cell, during or after one or more steps or stages of the engineering or manufacturing process. In some aspects, the assessed copy number of integrated transgene sequences is an average copy number of integrated transgene sequences in a plurality or a population of cells.

The methods involve: (a) separating a high molecular weight fraction of deoxyribonucleic acid (DNA) of greater than or greater than about 10 kilobases (kb) from DNA isolated from one or more cells, said one or more cells comprising, or are suspected of comprising, at least one engineered cell comprising a transgene sequence that includes a nucleic acid sequence encoding a recombinant protein; (b) from the high molecular weight fraction, determining the presence, absence or amount of the transgene sequence integrated into the genome of the one or more cell. The transgene sequences in the high molecular weight fraction represents the transgene sequences that have been integrated into the genome of the one or more cell.

The methods involve (a) separating a high molecular weight fraction of deoxyribonucleic acid (DNA) of greater than or greater than about 10 kilobases (kb) from DNA isolated from one or more cell, said one or more cell comprising, or suspected of comprising, at least one engineered cell comprising a transgene sequence comprising a nucleic acid sequence encoding a recombinant protein; and (b) determining the presence, absence or amount of the transgene sequence in the high molecular weight fraction, thereby assessing the transgene sequences integrated into the genome of the one or more cells.

The transgene sequences in the high molecular weight fraction represents the transgene sequences that have been integrated into the genome of the one or more cell. In some of any of the provided embodiments, the determining the presence, absence or amount of the transgene sequence integrated into the genome of the one or more cells in (b) comprises determining the mass, weight or copy number of the transgene sequence in the high molecular weight fraction.

Also provided herein are methods for assessing genomic integration of a transgene sequence. In some of any of the embodiments, the methods involve: (a) separating, by pulse field gel electrophoresis, a high molecular weight fraction of deoxyribonucleic acid (DNA) of greater than or greater than about 10 kilobases (kb) from DNA isolated from a population of cells, said population of cells comprising a plurality of engineered cells that each comprise, or are suspected of comprising, a transgene sequence comprising a nucleic acid sequence encoding a recombinant protein; and (b) determining the average or mean copy number per diploid genome or per cell of the transgene sequence sequence in the high molecular weight fraction, thereby assessing transgene sequences integrated into the genome of the plurality of engineered cells of the population of cells.

Also provided herein are methods for assessing genomic integration of a transgene sequence. In some of any of the embodiments, the methods involve: (a) separating, by pulse field gel electrophoresis, a high molecular weight fraction of deoxyribonucleic acid (DNA) of greater than or greater than about 10 kilobases (kb) from DNA isolated from a population of cells, said population of cells comprising a plurality of engineered cells that each comprise, or are suspected of comprising, a transgene sequence comprising a nucleic acid sequence encoding a recombinant protein; and (b) from the high molecular weight fraction, determining the average or mean copy number per diploid genome or per cell of the transgene sequence integrated into the genome of the plurality of engineered cells of the population of cells.

In some aspects, the copy number of integrated nucleic acid sequences can be determined by assessing the copy number of a particular nucleic acid sequence, such as all or a portion of the transgene sequences, that are present in a high molecular weight fraction of a deoxyribonucleic acid (DNA) sample obtained from a cell or a plurality or population of cells. In some aspects, the high molecular weight fraction contains genomic DNA or genomic DNA fragments, and excludes or separates non-integrated or residual nucleic acid species that can be present in the DNA sample. In some aspects, the high molecular weight fraction, e.g., DNA samples that are above a threshold value such as about 10, 11, 12, 12.5, 13, 14, 15, 16, 17, 17.5, 18, 19, 20, 25 or 30 kilobases (kb) or more. In some embodiments, the threshold value is greater than or greater than about 10, 12.5, 15, 17.5 or 20 kilobases (kb) or more. In some embodiments, the high molecular weight fraction is of greater than or greater than about 15 kilobases (kb). In some embodiments, the high molecular weight fraction is of greater than or greater than about 17.5 kilobases (kb). In some embodiments, the high molecular weight fraction is of greater than or greater than about 20 kilobases (kb).

In some embodiments, the methods involve isolating deoxyribonucleic acid (DNA) from a cell that has been introduced with a polynucleotide comprising a transgene sequence under conditions for integration into a genome of the cell, and separating a high molecular weight fraction of greater than or greater than about 10 kilobases (kb) from the isolated DNA. In some embodiments, the methods involve separating a high molecular weight fraction of greater than or greater than about 10 kilobases (kb) from deoxyribonucleic acid (DNA) isolated from a cell, wherein prior to the separating, the cell has been introduced with a polynucleotide comprising a transgene sequence under conditions for integration of the transgene sequence into a genome of the cell. In some aspects, the provided methods involve determining the presence, absence or amount of the transgene sequence in the separated high molecular weight fraction.

In some embodiments, the methods include involve isolating deoxyribonucleic acid (DNA) from a cell that has been introduced with a polynucleotide comprising a transgene sequence under conditions for integration into a genome of the cell; separating a high molecular weight fraction of greater than or greater than about 10 kilobases (kb) from the isolated DNA; and determining the presence, absence or copy number of the transgene sequence in the high molecular weight fraction.

In some aspects, the fraction of integrated transgene sequences is determined by dividing the integrated copy number (as determined by ddPCR after PFGE, for example an integrated vector copy number (iVCN) assay described herein) by the total copy number (as determined by ddPCR without PFGE, for example, by a standard vector copy number (VCN) assay). In some aspects, other measures can be used for comparison or normalization, e.g., normalized to the copy number of a reference gene, or compared to recombinant receptor expression as detected by methods to detect protein expression, such as compared to the number of CAR+ cells in a population as assessed by flow cytometry.

Provided are methods for assessing genomic integration of transgene sequences, such as a transgene sequence encoding a recombinant protein, such as a recombinant receptor. In some aspects, provided are methods for assessing genomic integration of transgene sequences that contain nucleic acid sequences encoding a recombinant protein, such as a recombinant receptor. In some aspects, provided are methods for assessing genomic integration of transgene sequences, such as a transgene sequence that includes regulatory elements, e.g., promoters, transcriptional and/or post-transcriptional regulatory elements or response elements, or markers, e.g., surrogate markers that are linked to the nucleic acid sequence encoding a recombinant protein. In some of any embodiments, the transgene sequence comprises a regulatory element linked to the nucleic acid sequence encoding the recombinant protein. In some aspects, the provided methods can be used to assess the timing, extent or progress of genetic engineering, e.g., by integration of transgene sequences into a genome of the cell, after introduction of a polynucleotide comprising a transgene sequence under conditions for integration into a cell, for example, for genetic engineering. In some aspects, the assessed copy number of integrated transgene sequences is an average copy number of integrated transgene sequences in a plurality or a population of cells. In some embodiments, the cell is present in a population of cells that have been introduced with the polynucleotide encoding the transgene sequence, wherein the method is carried out on a plurality of cells in the population.

In some aspects, the provided methods can be performed at various time points, steps or stages or using various different samples to determine and compare the timing, extent or progress of genetic engineering, such as integration of the introduced transgene sequences into the genome of the cell into which the transgene sequences are introduced. In some aspects, the provided methods can be used to assess the timing and extent of transgene sequence integration, for example, via a time-course experiment where DNA samples are obtained at various time points after introduction of the polynucleotide encoding the transgene sequence. In some aspects, the methods can be carried out at various stages of an engineering or manufacturing process for engineered cell compositions. For example, the provided methods can be performed at various stages of an expanded engineering process or a non-expanded engineering process.

In some aspects, the introduction of the polynucleotide is carried out by any of the methods described herein, such as those described in Section II.C and II.D herein. In some aspects, the introduction of the polynucleotide is carried out by viral transduction. In some asepcts, the introduction of the polynucleotide is carried out by a physical delivery method, optionally by electroporation.

In some aspects, the methods can be used to determine the time point at which the majority or substantially all of integration is completed. In some aspects, this time point can be used for assessing the integrated copy number, for example, according to the methods provided herein, in an engineered cell composition, such as an engineered cell composition for adoptive cell therapy. In some aspects, the methods can be used to determine the time point at which the majority or substantially all of integration has not been completed, and/or residual, non-integrated sequences are presnet in the cells or cell composition.

In some aspects, the methods can be used to determine a suitable length of incubation of the cell after introduction of the polynucleotides comprising the transgene sequences, at which the majority or substantially all of integration is completed. In some aspects, the methods permit the determination of suitable length of incubation that can maximize integration yet reduces exhaustion of the engineered cells. In some aspects, the cell or population or plurality of cells are not incubated at a temperature greater than 25°C for more than 48, 54, 60, 66, or 72 hours following the introduction of the polynucleotide comprising the transgene sequence into the cell. In some aspects, the cell is not incubated at a temperature greater than 25°C for more than 72 hours following the introduction of the polynucleotide comprising the transgene sequence into the cell. In some aspects, the cell is not incubated at a temperature greater than about 30°C and less than about 40°C for more than 72 hours following the introduction of the polynucleotide comprising the transgene sequence into the cell.

In some embodiments, the cell is not cryopreserved after the introduction of the polynucleotide and the determining of the presence, absence and/or amount of the transgene sequences.

In some aspects, provided are methods for assessing genomic integration of a transgene sequence that involves separating a high molecular weight fraction of greater than or greater than about 10 kilobases (kb) from deoxyribonucleic acid (DNA) isolated from a cell, wherein prior to the separating, the cell has been introduced with a polynucleotide comprising a transgene sequence under conditions for integration of the transgene sequence into a genome of the cell by viral transduction, and the cell is not incubated at a temperature greater than 25°C for more than 48, 54, 60, 66, or 72 hours following the introduction of the polynucleotide comprising the transgene sequence into the cell; and determining the presence, absence or amount of the transgene sequence in the high molecular weight fraction.

In some aspects, provided are methods for assessing genomic integration of a transgene sequence that involves isolating deoxyribonucleic acid (DNA) from a cell that has been introduced with a polynucleotide comprising a transgene sequence under conditions for integration into a genome of the cell by viral transduction, and the cell is not incubated at a temperature greater than 25°C for more than 48, 54, 60, 66, or 72 hours following the introduction of the polynucleotide comprising the transgene sequence into the cell; separating a high molecular weight fraction of greater than or greater than about 10 kilobases (kb) from the isolated DNA; and determining the presence, absence or amount of the transgene sequence in the high molecular weight fraction.

### 2. Assessing the Number of Residual Non-integrated DNA

In some aspects, also provided are methods for assessing a residual non-integrated transgene sequence. In some embodiments, the methods involve performing steps of any of the methods, including isolating deoxyribonucleic acid (DNA) from a cell that has been introduced with a polynucleotide comprising a transgene sequence under conditions for integration into a genome of the cell and separating a high molecular weight fraction, such as DNA samples above a threshold value described herein, such as greater than or greater than about 10 kilobases (kb), from the isolated DNA; determining mass, weight, concentration or copy number of the transgene sequence in the high molecular weight fraction, thereby assessing genomic integration of a transgene sequence.

In some aspects, provided are methods for assessing a residual non-integrated transgene sequence. In some embodiments, the methods involve performing the steps for determining the presence, absence or amount of transgene sequences as described herein, to determine mass, weight, concentration or copy number of the transgene sequences in the high molecular weight fraction of DNA. In some embodiments, the methods also involve determining mass, weight, concentration or copy number of the transgene sequence in the isolated DNA without separating the high molecular weight fraction, thereby determining the total copy number of the transgene sequence; In some embodiments, the methods involve comparing mass, weight, concentration or copy number determined in the high molecular weight fraction to mass, weight, concentration or copy number determined in the total isolated DNA without separating the high molecular weight fraction, thereby determining mass, weight, concentration or copy number of the residual non-integrated recombinant sequence.

In some embodiments, the comparing involves subtracting mass, weight, concentration or copy number determined for the high molecular weight fraction from mass, weight, concentration or copy number determined without separating the high molecular weight fraction. In some embodiments, the comparing involves determining the ratio of mass, weight, concentration or copy number determined for the high molecular weight fraction to mass, weight, concentration or copy number determined without separating the high molecular weight fraction.

The determining of the copy number without separating the high molecular weight DNA is carried out by polymerase chain reaction (PCR), such as any described herein, for example, in Section I.B. In some embodiments, determining of the copy number without separating the high molecular weight DNA is carried out using methods described in, for example, Charrier et al., Gene Therapy (2011) 18, 479-487; Christodoulou et al., Gene Therapy (2016) 23, 113-118; Zhao et al., Human Gene Therapy Methods (2017) 28(4): 205-214; and Milone et al., Molecular Therapy: Methods & Clinical Development (2018) 8:210-221. In some aspects, copy number without separating the high molecular weight DNA include standard vector copy number (VCN) assays.

In some embodiments, the PCR is quantitative polymerase chain reaction (qPCR), digital PCR or droplet digital PCR. In some embodiments, the PCR is droplet digital PCR. In some embodiments, the PCR is carried out using one or more primers that is complementary to or is capable of specifically amplifying at least a portion of the transgene sequence In some of any embodiments, the one or more primers is complementary to or is capable of specifically amplifying all or a portion of the sequences in the transgene that is or is to be integrated into the genome. In some embodiments, the one or more primers is complementary to or is capable of specifically amplifying a portion of the nucleic acid sequence encoding the recombinant protein. In some embodiments, the one or more primers is complementary to or is capable of specifically amplifying sequences of the regulatory element that is operably linked to the nucleic acid sequence encoding the recombinant protein. In some embodiments, the one or more primers is complementary to or is capable of specifically amplifying sequences of a regulatory element that is operably linked to the nucleic acid sequence encoding the recombinant protein. In some embodiments, the one or more primers is complementary to or is capable of specifically amplifying sequences of a post-transcriptional regulatory element that is operably linked to the nucleic acid sequence encoding the recombinant protein. In some embodiments, the one or more primers is complementary to or is capable of specifically amplifying sequences of a woodchuck hepatitis virus post-transcriptional regulatory element (WPRE) that is operably linked to the nucleic acid sequence encoding the recombinant protein.

In some embodiments, the determining the copy number without separating the high molecular weight fraction comprises assessing the copy number of the transgene sequence in the isolated DNA without separating the high molecular weight fraction and normalizing the amount or the concentration to a reference gene in the isolated DNA without separating the high molecular weight fraction or to a standard curve, e.g., of a known copy number. In some embodiments, the reference gene is a housekeeping gene. In some embodiments, the reference gene is a gene encoding albumin *(ALB).* In some embodiments, the reference gene is a gene encoding ribonuclease P protein subunit p30 (*RPP30*).

In some embodiments, the copy number of a reference gene in the isolated DNA is carried out by PCR using one or more primers that is complementary to or is capable of specifically amplifying at least a portion of the reference gene.

In some embodiments, the determining the copy number in the high molecular weight fraction and the determining the copy number without separating the high molecular weight fraction is carried out by polymerase chain reaction (PCR) using the same primer or the same sets of primers.

In some aspects, the methods also involve determining the copy number of the transgene sequence in the isolated DNA without separating the high molecular weight fraction, thereby determining the total copy number of transgene sequences. In some aspects, the methods involve determining the copy number of the residual non-integrated transgene sequence by subtracting the copy number determined by assessing the high molecular weight fraction from the copy number determined by assessing the total isolated DNA (such as without separating fractions). In some aspects, the methods involve determining the proportion of residual non-integrated transgene sequence by dividing the copy number determined by assessing the high molecular weight fraction from the copy number determined by the copy number determined by assessing the total isolated DNA (such as without separating fractions). In some embodiments, the residual non-integrated transgene sequence comprises one or more of vector plasmids, linear complementary DNA (cDNA), autointegrants or long terminal repeat (LTR) circles.

In some aspects, the fraction of integrated transgene sequences can be determined by dividing the integrated copy number (as determined by ddPCR after PFGE, for example an integrated vector copy number (iVCN) assay described herein) by the total copy number (as determined by ddPCR without PFGE, for example, by a standard vector copy number (VCN) assay). In some embodiments, the fraction of non-integrated transgene sequences can be determined as 1 - (fraction of integrated transgene sequences). In some aspects, the non-integrated transgene sequence copy number can be determined by subtracting the integrated copy number from the total copy number.

### 3. Assessing the Amount of Transgene Sequences in a Sample from a Subject Administered Engineered Cells

In some aspects, also provided are methods for assessing the presence, absence or amount of transgene sequences in a biological sample from a subject. In some aspects, the subject has been administered engineered cells, e.g., immune cells engineered by introduction of polynucleotides containing transgene sequences into the cells, for example, for adoptive cell therapy. In some aspects, the methods involve isolating deoxyribonucleic acid (DNA) from a biological sample from a subject; separating a high molecular weight fraction of greater than or greater than about 10 kilobases (kb) from the isolated DNA; and determining the presence, absence or amount of the transgene sequence in the high molecular weight fraction. In some aspects, the biological sample is obtained from a subject that had been administered engineered cells comprising the transgene sequence. In some aspects, the determining the presence, absence or amount of the transgene sequence comprises determining the copy number, such as determining the copy number in a biological sample obtained from the subject.

Provided herein are methods for assessing a transgene sequence in a biological sample from a subject. In some of any embodiments, the provided methods involve: (a) separating a high molecular weight fraction of deoxyribonucleic acid (DNA) of greater than or greater than about 10 kilobases (kb) from DNA isolated from one or more cells present in a biological sample from a subject, wherein the biological sample comprises, or is suspected of comprising, at least one engineered cell comprising a transgene sequence that includes a nucleic acid sequence encoding a recombinant protein; and (b) determining the presence, absence or amount of transgene sequence in the high molecular weight fraction, thereby assessing transgene sequences present in all or a portion of the biological sample.

Provided herein is a method for assessing a transgene sequence in a biological sample from a subject, the method comprising: (a) separating a high molecular weight fraction of deoxyribonucleic acid (DNA) of greater than or greater than about 10 kilobases (kb) from DNA isolated from one or more cells present in a biological sample from a subject, wherein the biological sample comprises, or is suspected of comprising, at least one engineered cell comprising a transgene sequence that includes a nucleic acid sequence encoding a recombinant protein; and (b) determining the presence, absence or amount of transgene sequence in the high molecular weight fraction, thereby assessing transgene sequences present in all or a portion of the biological sample.

In some embodiments, the provided methods for assessing a transgene sequence in a biological sample from a subject involves separating a high molecular weight fraction of deoxyribonucleic acid (DNA) of greater than or greater than about 10 kilobases (kb) from DNA isolated from one or more cells present in a biological sample from a subject, wherein the biological sample comprises, or is suspected of comprising, at least one engineered cell comprising a transgene sequence encoding a recombinant protein; and determining the presence, absence or amount of transgene sequence in all or a portion of the biological sample. In some embodiments, prior to the separating, a polynucleotide comprising the transgene sequence encoding the recombinant protein has been introduced into the at least one engineered cell of the one or more cells. In some embodiments, the determining the presence, absence or amount of transgene sequence comprises determining the mass, weight, concentration or copy number of the transgene sequence in all or a portion of the biological sample.

In some embodiments, the method can be used as a basis to determine pharmacokinetic (PK) or pharmacodynamics (PD) parameters of the administered cells. In some aspects, the PK or PD parameters can be measured based on a nucleic acid-based method, for example, assessing the presence, absence or amount of transgene sequences, such as according to the methods provided herein. In some aspects, the PK and PD parameters can be measured based on measuring cellular properties or phenotypes, for example, by detecting the expression of a recombinant protein on the surface of the cells or cell surface phenotypes or activity. In some aspects, a variety of methods can be used, including the combination of nucleic acid-based methods and cell-based methods. In some embodiments, the methods involve isolating the DNA from one or more cells present in the biological sample prior to the separating the high molecular weight fraction.

In some embodiments, the biological sample is obtained from a subject that had been administered a composition comprising the at least one engineered cell comprising the transgene sequence. In some embodiments, the biological sample is a tissue sample or bodily fluid sample, or any other sample obtained from a subject described herein. In some embodiments, the biological sample is a tissue sample and the tissue is a tumor. In some embodiments, the tissue sample is a tumor biopsy. In some embodiments, the biological sample is a bodily fluid sample and the bodily fluid sample is a blood or serum sample.

In some embodiments, the one or more cells in the biological sample comprises an immune cell, including any immune cell or population thereof described herein. In some embodiments, the immune cell is a T cell or an NK cell. In some embodiments, the T cell is a CD3+, CD4+ and/or CD8+ T cells, or any such cell comprising a particular phenotype.

In some aspects, any of the methods to determine the presence, absence or amount of the transgene sequences and any of the normalization or quantitation methods to determine the mass, weight, concentration or copy number of the sequences described herein can be used.

In some aspects, the provided methods can be used as a basis to assess the exposure, number, concentration, persistence and proliferation of the T cells, *e.g.,* T cells administered for the T cell based therapy. In some embodiments, the exposure, or prolonged expansion and/or persistence of the cells, and/or changes in cell phenotypes or functional activity of the cells, *e.g.,* cells administered for immunotherapy, *e.g.* T cell therapy, in the methods provided herein, can be measured by assessing the characteristics of the T cells *in vitro* or *ex vivo.* In some embodiments, such assays can be used to determine or confirm the function of the T cells used for the immunotherapy, *e.g*. T cell therapy, before or after administering the cell therapy, such as with engineered T cells expressing a recombinant receptor.

In some aspects, the exposure, number, concentration, persistence and proliferation relate to pharmacokinetic parameters. In some cases, pharmacokinetics can be assessed by measuring such parameters as the maximum (peak) plasma concentration (Cₘₐₓ), the peak time (*i.e.* when maximum plasma concentration (Cₘₐₓ) occurs; Tₘₐₓ), the minimum plasma concentration (*i.e.* the minimum plasma concentration between doses of a therapeutic agent, e.g., CAR+ T cells; Cₘᵢₙ), the elimination half-life (T_{1/2}) and area under the curve (*i.e.* the area under the curve generated by plotting time versus plasma concentration of the therapeutic agent engineered cells; AUC), following administration. The concentration of a particular therapeutic agent, e.g., engineered cells, in the plasma following administration can be measured using the provided methods. For example, in some aspects, the copy number of he integrated transgene sequence can be assessed in samples such as blood samples from a subject. In some aspects, other methods for determining PK, such as flow cytometry-based methods, or other assays, such as an immunoassay, ELISA, or chromatography/mass spectrometry-based assays can be used in combination or parallel to determine one or more pharmacokinetic parameters. Other methods to detect and/or extrapolate to total cell numbers include those described in Brentjens et al., Sci Transl Med. 2013 5(177), Park et al, Molecular Therapy 15(4):825-833 (2007), Savoldo et al., JCI 121(5):1822-1826 (2011), Davila et al., (2013) PLoS ONE 8(4):e61338, Davila et al., Oncoimmunology 1(9): 1577-1583 (2012), Lamers, Blood 2011 117:72-82, Jensen et al., Biol Blood Marrow Transplant 2010 September; 16(9): 1245-1256, Brentjens et al., Blood 2011 118(18):4817-4828.

In some embodiments, the pharmacokinetics (PK) of administered cells, e.g., engineered cell composition, are determined to assess the availability, e.g., bioavailability, of the administered cells. In some embodiments, "exposure" can refer to the body exposure of a therapeutic agent, e.g., engineered cells in the plasma (blood or serum) after administration of the therapeutic agent over a certain period of time. In some embodiments exposure can be set forth as the area under the therapeutic agent concentration-time curve (AUC) as determined by pharmacokinetic analysis after administration of a dose of the therapeutic agent, e.g., engineered cells. In some cases, the AUC is expressed in cells*days/µL, for cells administered in cell therapy, or in corresponding units thereof. In some embodiments, the AUC is measured as an average AUC in a patient population, such as a sample patient population, e.g., the average AUC from one or more patient(s). In some embodiments, systemic exposure refers to the area under the curve (AUC) within a certain period of time, e.g., from day 0 to day 1, 2, 3, 4, 5, 6, 7, 8, 9, 10, 11, 12, 13, 14, 21, 28 days or more, or week 1, 2, 3, 4, 5, 6, 7, 8, 9, 10, 11, 12, 13, 14, 15 or more, or month 1, 2, 3, 4, 5, 6, 7, 8, 9, 10, 11, 12, 18, 24, 48 or more. In some embodiments, the AUC is measured as an AUC from day 0 to day 28 (AUC₀₋₂₈) after administration of the therapeutic agent, e.g., engineered cells, including all measured data and data extrapolated from measured pharmacokinetic (PK) parameters, such as an average AUC from a patient population, such as a sample patient population. In some embodiments, to determine exposure over time, e.g., AUC for a certain period of time, such as AUC₀₋₂₈, a therapeutic agent concentration-time curve is generated, using multiple measurements or assessment of parameters, e.g., cell concentrations, over time, e.g., measurements taken every 1, 2, 3, 4, 5, 6, 7, 8, 9, 10, 11, 12, 13, 14, 21 or 28 days or more.

In some embodiments, the presence, absence and/or amount of transgene sequences in the subject following the administration of the T cells and before, during and/or after the administration of the therapy is detected. In some embodiments, the presence, absence and/or amount of cells expressing the recombinant receptor (*e.g.,* CAR-expressing cells administered for T cell based therapy) in the subject following the administration of the T cells and before, during and/or after the administration of the therapy is detected. In some aspects, any of the methods described herein to assess integration of the transgene sequences, can be used to assess the quantity of cells expressing the recombinant protein (*e.g.,* engineered cells expressing a recombinant receptor administered for T cell based therapy) in the blood or serum or organ or tissue sample (*e.g.,* disease site, *e.g.,* tumor sample) of the subject. In some aspects, persistence is quantified as copies of integrated transgene sequences per diploid genome, per volume or area of the sample, *e.g.,* of blood or serum, per microgram of total DNA, or as the number of engineered cells (e.g., recombinant receptor expressing cells) per microliter of the sample, *e.g.,* of blood or serum, or per total number of peripheral blood mononuclear cells (PBMCs) or white blood cells or T cells per microliter of the sample.

In some embodiments, the primers or probe used for any of the provided methods are specific for binding, recognizing and/or amplifying nucleic acids encoding the recombinant receptor, and/or other components or elements of the plasmid and/or vector, including regulatory elements, e.g., promoters, transcriptional and/or post-transcriptional regulatory elements or response elements, or markers, e.g., surrogate markers. In some embodiments, the primers can be specific for regulatory elements. In some embodiments, the primers are specific for a regulatory element operably linked to the nucleic acid sequence encoding the recombinant protein. In somme embodiments, the primers are specific for amplifying all or a portion of a woodchuck hepatitis virus post-transcriptional regulatory element (WPRE) that is operably linked to the nucleic acid sequence encoding the recombinant protein, e.g., recombinant receptor.

In some embodiments, the transgene sequences cells are detected in the subject at or at least at 4, 14, 15, 27, or 28 days following the administration of the engineered cells. In some aspects, the cells are detected at or at least at 2, 4, or 6 weeks following, or 3, 6, or 12, 18, or 24, or 30 or 36 months, or 1, 2, 3, 4, 5, or more years, following the administration of the engineered cells.

In some embodiments, the peak levels and/or AUC are assessed and/or the sample is obtained from the subject at a time that is at least 8 days, 9 days, 10 days, 11 days, 12 days, 13 days, 14 days, 15 days, 16 days, 17 days, 18 days, 19 days, 20 days or 21 days after initiation of administration of the genetically engineered cells. In some embodiments the peak levels and/or AUC are assessed and/or the sample is obtained from the subject at a time that is between or between about 11 to 22 days, 12 to 18 days or 14 to 16 days, each inclusive, after initiation of administration of the genetically engineered cells.

The exposure, e.g., number or concentration of engineered cells administered for adoptive cell therapy, indicative of expansion and/or persistence, may be stated in terms of maximum copy number of the transgene sequences, or maximum numbers or concentration of the cells to which the subject is exposed, duration of detectable cells or cells above a certain number or percentage, area under the curve (AUC) for copy number of the transgene sequences, number or concentration of cells over time, and/or combinations thereof and indicators thereof. Such outcomes may be assessed using the provided methods to detect copy number of transgene sequences encoding a recombinant protein compared to total amount of nucleic acid or DNA in the particular sample, *e.g.,* blood, serum, plasma or tissue, such as a tumor sample, and/or flow cytometric assays detecting cells expressing the receptor generally using antibodies specific for the recombinant protein. Cell-based assays may also be used to detect the number or percentage or concentration of functional cells, such as cells capable of binding to and/or neutralizing and/or inducing responses, *e.g.,* cytotoxic responses, against cells of the disease or condition or expressing the antigen recognized by the recombinant protein that is a recombinant receptor.

In some aspects, increased exposure of the subject to the cells includes increased expansion of the cells. In some embodiments, the receptor expressing cells, *e.g.* CAR-expressing cells, expand in the subject following administration of the T cells, *e.g.,* CAR-expressing T cells.

In some embodiments, cells expressing the receptor are detectable in the serum, plasma, blood or tissue, *e.g.,* tumor sample, of the subject, *e.g.,* by the provided methods or flow cytometry-based detection method, at least 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, or 60 or more days following administration of the engineered cells, for at least at or about 2, 3, 4, 5, 6, 7, 8, 9, 10, 11, 12, 13, 14, 15, 16, 17, 18, 19, 20, 21, 22, 23, or 24 or more weeks following the administration of the engineered cells.

In some aspects, the mass, weight, concentration or copy number of integrated transgene sequences, per 100 cells, for example in the peripheral blood or bone marrow or other compartment, as measured by any of the described method, can be at least 0.01, at least 0.1, at least 1, or at least 10, at about 1 week, about 2 weeks, about 3 weeks, about 4 weeks, about 5 weeks, or at least about 6 weeks, or at least about 2, 3, 4, 5, 6, 7, 8. 9, 10, 11, or 12 months or at least 2 or 3 years following administration of the engineered cells. In some embodiments, the copy number of the transgene sequences, per microgram of genomic DNA is at least 100, at least 1000, at least 5000, or at least 10,000, or at least 15,000 or at least 20,000 at a time about 1 week, about 2 weeks, about 3 weeks, or at least about 4 weeks following administration of the engineered cells or at least 2, 3, 4, 5, 6, 7, 8, 9, 10, 11, or 12 months or at least 2 or 3 years following such administration.

In some aspects, the transgene sequences introduced in the engineered cells ca be detectable by the described methods in the subject, plasma, serum, blood, tissue and/or disease site thereof, *e.g.,* tumor site, at a time that is at least about 3 months, at least about 6 months, at least about 12 months, at least about 1 year, at least about 2 years, at least about 3 years, or more than 3 years, following the administration of the cells, *e.g.,* following the initiation of the administration of the T cells. In some embodiments, the area under the curve (AUC) for concentration of recombinant protein cells in a fluid, plasma, serum, blood, tissue, organ and/or disease site, e.g. tumor site, of the subject over time following the administration of the engineered cell, is measured.

### II. METHODS FOR INTRODUCING A POLYNUCLEOTIDE COMPRISING A TRANSGENE SEQUENCE

In some embodiments, the provided methods for assessing integrated nucleic acids is used or carried out in connection with genetically engineered cells, and at one or more times during an engineering or manufacturing process for generating genetically engineered cells. In some aspects, the cell or plurality of cells to which the provided methods are implemented or carried out, include cells that have been genetically engineered or are in the process of genetic engineering, and can be used in cell therapy, such as adoptive cell therapy. In some embodiments, the cell or plurality of cells to which the provided methods are implemented or carried out, include cells at various steps or stages of engineering. In some aspects, the cells include immune cells, such as T cells, that have been engineered to express a recombinant protein, such as by introduction of a polynucleotide containing transgene sequences that include sequences encoding the recombinant protein. In some aspects, the provided methods are used to assess the integration of such transgene sequences in the genome of the engineered cell.

In some aspects, the engineering or manufacturing process include one or more steps, including stimulation, activation, transduction, expansion, cultivation or proliferation of cells, including immune cells, such as T cells. In some embodiments, provided methods for assessing integrated nucleic acids can be carried out as part of a process for generating or producing a genetically engineered cells, such as genetically-engineered T cells.

In some aspects, the provided embodiments are employed in an engineering or manufacturing process that is shortened, abbreviated or does not include an expansion step, such as using a non-expanded manufacturing process. In some aspects, the shortened or abbreviated manufacturing process includes a shortened or abbreviated expansion step, or does not include an expansion step, after introduction of the nucleic acids encoding the recombinant receptor. In some aspects, the shortened or abbreviated manufacturing process includes processes in which the cells have not been incubated at a temperature greater than 25°C, optionally at or about 37°C ± 2°C, for more than 96 hours following the introduction of the polynucleotide comprising the transgene sequence into the at least one engineered cell.

In some embodiments, the provided methods are used in connection with cells that are undergoing one or more steps or processes involved in the manufacturing, generating or producing cells or cell compositions for a cell therapy. In some aspects, the provided methods can be used to assess integration of nucleic acids, such as transgene sequences that used to engineer the cells. In some embodiments, the cell therapy includes administration of cells, such as T cells, engineered to express recombinant protein, such as a recombinant receptor, e.g., a chimeric antigen receptor (CAR). In some embodiments, the one or more steps comprises the isolation, separation, selection, activation or stimulation, transduction, cultivation, expansion, washing, suspension, dilution, concentration, and/or formulation of the cells.

In some embodiments, the methods of generating or producing a cell therapy include isolating cells from a subject, preparing, processing, culturing under one or stimulating conditions. In some embodiments, the method includes processing steps carried out in an order in which: cells, e.g. primary cells, are first isolated, such as selected or separated, from a biological sample; selected cells are incubated with viral preparations for transduction (e.g., viral preparations containing polynucleotides that include the transgene sequences for integration), optionally subsequent to a step of stimulating the isolated cells in the presence of a stimulation reagent; culturing the transduced cells, such as to expand the cells; formulating the transduced cells in a composition and introducing the composition into a biomedical material vessel.

In some embodiments, the methods for manufacturing or engineering can include one or more of (a) washing a biological sample containing cells (e.g., a whole blood sample, a buffy coat sample, a peripheral blood mononuclear cells (PBMC) sample, an unfractionated T cell sample, a lymphocyte sample, a white blood cell sample, an apheresis product, or a leukapheresis product), (b) isolating, e.g., selecting, from the sample a desired subset or population of cells (e.g., CD4+ and/or CD8+ T cells), for example, by incubation of cells with a selection or immunoaffinity reagent for immunoaffinity-based separation; (c) introducing an agent encoding a recombinant receptor, e.g. a CAR, into the isolated or selected cells, such as by incubating the isolated, such as selected cells, with viral vector particles encoding the recombinant receptor, (d) culturing, cultivating or expanding the cells such using methods as described and (e) formulating the transduced cells, such as in a pharmaceutically acceptable buffer, cryopreservative or other suitable medium. In some embodiments, the methods can further include (f) stimulating cells by exposing cells to stimulating conditions, which can be performed prior to, during and/or subsequent to the incubation of cells with viral vector particles. In some embodiments, one or more further step of washing or suspending step, such as for dilution, concentration and/or buffer exchange of cells, can also be carried out prior to or subsequent to any of the above steps. In some aspects, the resulting engineered cell composition is introduced into one or more biomedical culture vessels.

In some embodiments, the provided methods for preparing or producing genetically engineered cells are carried out such that one, more, or all steps in the preparation of cells for clinical use, e.g., in adoptive cell therapy, are carried out without exposing the cells to non-sterile conditions and without the need to use a sterile room or cabinet. In some embodiments of such a process, the cells are isolated, separated or selected, transduced, washed, optionally activated or stimulated and formulated, all within a closed system. In some aspects of such a process, the cells are expressed from a closed system and introduced into one or more of biomaterial vessels. In some embodiments, the methods are carried out in an automated fashion. In some embodiments, one or more of the steps is carried out apart from the closed system or device.

In some embodiments, a closed system is used for carrying out one or more of the other processing steps of a method for manufacturing, generating or producing a cell therapy. In some embodiments, one or more or all of the processing steps, e.g., isolation, selection and/or enrichment, processing, incubation in connection with transduction and engineering, and formulation steps is carried out using a system, device, or apparatus in an integrated or self-contained system, and/or in an automated or programmable fashion. In some aspects, the system or apparatus includes a computer and/or computer program in communication with the system or apparatus, which allows a user to program, control, assess the outcome of, and/or adjust various aspects of the processing, isolation, engineering, and formulation steps. In one example, the system is a system as described in WO2009/072003, US 20110003380 or WO2016/073602.

### A. Isolation or Selection of Cells

In some embodiments, the cells that are engineered, such as cells or cell compositions that are assessed or analyzed by the provided methods, are primary cells. In some embodiments, the cells are immune cells or enriched immune cells. In some embodiments, the cells are T cells or enriched with T cells. In some embodiments, the cells to be assessed or analyzed using the provided methods are T cells or enriched with T cells. In some embodiments, the cells are CD4+ T cells or enriched CD4+ T cells. In some embodiments, the cells are CD8+ T cells or enriched CD8+ T cells. In some embodiments, the process for manufacturing, generating or producing a cell therapy includes a step in which total T cells, e.g. CD3+ or CD4+/CD8+ T cells, are isolated or selected from a sample obtained from a human subject, prior to carrying out the subsequent steps of the process.

In some embodiments, the method of engineering or manufacturing includes steps for isolation of cells or compositions thereof from biological samples, such as those obtained from or derived from a subject, such as one having a particular disease or condition or in need of a cell therapy or to which cell therapy will be administered. In some aspects, the subject is a human, such as a subject who is a patient in need of a particular therapeutic intervention, such as the adoptive cell therapy for which cells are being isolated, processed, and/or engineered. Accordingly, the cells in some embodiments are primary cells, e.g., primary human cells. In some embodiments, the cells comprise CD4+ and CD8+ T cells. In some embodiments, the cells comprise CD4+ or CD8+ T cells. The samples include tissue, fluid, and other samples taken directly from the subject. The biological sample can be a sample obtained directly from a biological source or a sample that is processed. Biological samples include, but are not limited to, body fluids, such as blood, plasma, serum, cerebrospinal fluid, synovial fluid, urine and sweat, tissue and organ samples, including processed samples derived therefrom.

In some aspects, the sample is blood or a blood-derived sample, or is or is derived from an apheresis or leukapheresis product. Exemplary samples include whole blood, peripheral blood mononuclear cells (PBMCs), leukocytes, bone marrow, thymus, tissue biopsy, tumor, leukemia, lymphoma, lymph node, gut associated lymphoid tissue, mucosa associated lymphoid tissue, spleen, other lymphoid tissues, liver, lung, stomach, intestine, colon, kidney, pancreas, breast, bone, prostate, cervix, testes, ovaries, tonsil, or other organ, and/or cells derived therefrom. Samples include, in the context of cell therapy, e.g., adoptive cell therapy, samples from autologous and allogeneic sources.

In some examples, cells from the circulating blood of a subject are obtained, e.g., by apheresis or leukapheresis. The samples, in some aspects, contain lymphocytes, including T cells, monocytes, granulocytes, B cells, other nucleated white blood cells, red blood cells, and/or platelets, and in some aspects contains cells other than red blood cells and platelets.

In some embodiments, the blood cells collected from the subject are washed, e.g., to remove the plasma fraction and to place the cells in an appropriate buffer or media for subsequent processing steps. In some embodiments, the cells are washed with phosphate buffered saline (PBS). In some embodiments, the wash solution lacks calcium and/or magnesium and/or many or all divalent cations. In some aspects, a washing step is accomplished a semi-automated "flow-through" centrifuge (for example, the Cobe 2991 cell processor, Baxter) according to the manufacturer's instructions. In some aspects, a washing step is accomplished by tangential flow filtration (TFF) according to the manufacturer's instructions. In some embodiments, the cells are resuspended in a variety of biocompatible buffers after washing, such as, for example, Ca++/Mg++ free PBS. In certain embodiments, components of a blood cell sample are removed and the cells directly resuspended in culture media.

In some embodiments, the preparation methods include steps for freezing, e.g., cryopreserving, the cells, either before or after isolation, selection and/or enrichment and/or incubation for transduction and engineering. In some embodiments, the freeze and subsequent thaw step removes granulocytes and, to some extent, monocytes in the cell population. In some embodiments, the cells are suspended in a freezing solution, e.g., following a washing step to remove plasma and platelets. Any of a variety of known freezing solutions and parameters in some aspects may be used. This is then diluted 1: 1 with media so that the final concentration of DMSO and HSA are 10% and 4%, respectively. The cells are generally then frozen to -80° C. at a rate of 1° per minute and stored in the vapor phase of a liquid nitrogen storage tank.

In some embodiments, isolation of the cells or populations includes one or more preparation and/or non-affinity based cell separation steps. In some examples, cells are washed, centrifuged, and/or incubated in the presence of one or more reagents, for example, to remove unwanted components, enrich for desired components, lyse or remove cells sensitive to particular reagents. In some examples, cells are separated based on one or more property, such as density, adherent properties, size, sensitivity and/or resistance to particular components. In some embodiments, the methods include density-based cell separation methods, such as the preparation of white blood cells from peripheral blood by lysing the red blood cells and centrifugation through a Percoll or Ficoll gradient.

In some embodiments, at least a portion of the selection step includes incubation of cells with a selection reagent. The incubation with a selection reagent or reagents, e.g., as part of selection methods which may be performed using one or more selection reagents for selection of one or more different cell types based on the expression or presence in or on the cell of one or more specific molecules, such as surface markers, e.g., surface proteins, intracellular markers, or nucleic acid. In some embodiments, any known method using a selection reagent or reagents for separation based on such markers may be used. In some embodiments, the selection reagent or reagents result in a separation that is affinity- or immunoaffinity-based separation. For example, the selection in some aspects includes incubation with a reagent or reagents for separation of cells and cell populations based on the cells' expression or expression level of one or more markers, typically cell surface markers, for example, by incubation with an antibody or binding partner that specifically binds to such markers, followed generally by washing steps and separation of cells having bound the antibody or binding partner, from those cells having not bound to the antibody or binding partner. In some embodiments, the selection and/or other aspects of the process is as described in WO 2015/164675.

In some aspects of such processes, a volume of cells is mixed with an amount of a desired affinity-based selection reagent. The immunoaffinity-based selection can be carried out using any system or method that results in a favorable energetic interaction between the cells being separated and the molecule specifically binding to the marker on the cell, e.g., the antibody or other binding partner on the solid surface, e.g., particle. In some embodiments, methods are carried out using particles such as beads, e.g. magnetic beads, that are coated with a selection agent (e.g. antibody) specific to the marker of the cells. The particles (e.g. beads) can be incubated or mixed with cells in a container, such as a tube or bag, while shaking or mixing, with a constant cell density-to-particle (e.g., bead) ratio to aid in promoting energetically favored interactions. In other cases, the methods include selection of cells in which all or a portion of the selection is carried out in the internal cavity of a centrifugal chamber, for example, under centrifugal rotation. In some embodiments, incubation of cells with selection reagents, such as immunoaffinity-based selection reagents, is performed in a centrifugal chamber. In certain embodiments, the isolation or separation is carried out using a system, device, or apparatus described in WO2009/072003, US 20110003380 or WO2016/073602.

In some embodiments, by conducting such selection steps or portions thereof (e.g., incubation with antibody-coated particles, e.g., magnetic beads) in the cavity of a centrifugal chamber, the user is able to control certain parameters, such as volume of various solutions, addition of solution during processing and timing thereof, which can provide advantages compared to other available methods. For example, the ability to decrease the liquid volume in the cavity during the incubation can increase the concentration of the particles (e.g. bead reagent) used in the selection, and thus the chemical potential of the solution, without affecting the total number of cells in the cavity. This in turn can enhance the pairwise interactions between the cells being processed and the particles used for selection. In some embodiments, carrying out the incubation step in the chamber, e.g., when associated with the systems, circuitry, and control as described herein, permits the user to effect agitation of the solution at desired time(s) during the incubation, which also can improve the interaction.

In some embodiments, at least a portion of the selection step is performed in a centrifugal chamber, which includes incubation of cells with a selection reagent. In some aspects of such processes, a volume of cells is mixed with an amount of a desired affinity-based selection reagent that is far less than is normally employed when performing similar selections in a tube or container for selection of the same number of cells and/or volume of cells according to manufacturer's instructions. In some embodiments, an amount of selection reagent or reagents that is/are no more than 5%, no more than 10%, no more than 15%, no more than 20%, no more than 25%, no more than 50%, no more than 60%, no more than 70% or no more than 80% of the amount of the same selection reagent(s) employed for selection of cells in a tube or container-based incubation for the same number of cells and/or the same volume of cells according to manufacturer's instructions is employed.

In some embodiments, for selection, e.g., immunoaffinity-based selection of the cells, the cells are incubated in the cavity of the chamber in a composition that also contains the selection buffer with a selection reagent, such as a molecule that specifically binds to a surface marker on a cell that it desired to enrich and/or deplete, but not on other cells in the composition, such as an antibody, which optionally is coupled to a scaffold such as a polymer or surface, e.g., bead, e.g., magnetic bead, such as magnetic beads coupled to monoclonal antibodies specific for CD4 and CD8. In some embodiments, as described, the selection reagent is added to cells in the cavity of the chamber in an amount that is substantially less than (e.g. is no more than 5%, 10%, 20%, 30%, 40%, 50%, 60%, 70% or 80% of the amount) as compared to the amount of the selection reagent that is typically used or would be necessary to achieve about the same or similar efficiency of selection of the same number of cells or the same volume of cells when selection is performed in a tube with shaking or rotation. In some embodiments, the incubation is performed with the addition of a selection buffer to the cells and selection reagent to achieve a target volume with incubation of the reagent of, for example, 10 mL to 200 mL, such as at least or about at least or about or 10 mL, 20 mL, 30 mL, 40 mL, 50 mL, 60 mL, 70 mL, 80 mL, 90 mL, 100 mL, 150 mL or 200 mL. In some embodiments, the selection buffer and selection reagent are pre-mixed before addition to the cells. In some embodiments, the selection buffer and selection reagent are separately added to the cells. In some embodiments, the selection incubation is carried out with periodic gentle mixing condition, which can aid in promoting energetically favored interactions and thereby permit the use of less overall selection reagent while achieving a high selection efficiency.

In some embodiments, the total duration of the incubation with the selection reagent is from or from about 5 minutes to 6 hours, such as 30 minutes to 3 hours, for example, at least or about at least 30 minutes, 60 minutes, 120 minutes or 180 minutes.

In some embodiments, the incubation generally is carried out under mixing conditions, such as in the presence of spinning, generally at relatively low force or speed, such as speed lower than that used to pellet the cells, such as from or from about 600 rpm to 1700 rpm (e.g. at or about or at least 600 rpm, 1000 rpm, or 1500 rpm or 1700 rpm), such as at an RCF at the sample or wall of the chamber or other container of from or from about 80g to 100g (e.g. at or about or at least 80 g, 85 g, 90 g, 95 g, or 100 g). In some embodiments, the spin is carried out using repeated intervals of a spin at such low speed followed by a rest period, such as a spin and/or rest for 1, 2, 3, 4, 5, 6, 7, 8, 9, or 10 seconds, such as a spin at approximately 1 or 2 seconds followed by a rest for approximately 5, 6, 7, or 8 seconds.

In some embodiments, such process is carried out within the entirely closed system to which the chamber is integral. In some embodiments, this process (and in some aspects also one or more additional step, such as a previous wash step washing a sample containing the cells, such as an apheresis sample) is carried out in an automated fashion, such that the cells, reagent, and other components are drawn into and pushed out of the chamber at appropriate times and centrifugation effected, so as to complete the wash and binding step in a single closed system using an automated program.

In some embodiments, after the incubation and/or mixing of the cells and selection reagent and/or reagents, the incubated cells are subjected to a separation to select for cells based on the presence or absence of the particular reagent or reagents. In some embodiments, the separation is performed in the same closed system in which the incubation of cells with the selection reagent was performed. In some embodiments, after incubation with the selection reagents, incubated cells, including cells in which the selection reagent has bound are transferred into a system for immunoaffinity-based separation of the cells. In some embodiments, the system for immunoaffinity-based separation is or contains a magnetic separation column.

Such separation steps can be based on positive selection, in which the cells having bound the reagents, e.g. antibody or binding partner, are retained for further use, and/or negative selection, in which the cells having not bound to the reagent, e.g., antibody or binding partner, are retained. In some examples, both fractions are retained for further use. In some aspects, negative selection can be particularly useful where no antibody is available that specifically identifies a cell type in a heterogeneous population, such that separation is best carried out based on markers expressed by cells other than the desired population.

In some embodiments, the process steps further include negative and/or positive selection of the incubated and cells, such as using a system or apparatus that can perform an affinity-based selection. In some embodiments, isolation is carried out by enrichment for a particular cell population by positive selection, or depletion of a particular cell population, by negative selection. In some embodiments, positive or negative selection is accomplished by incubating cells with one or more antibodies or other binding agent that specifically bind to one or more surface markers expressed or expressed (marker⁺) at a relatively higher level (marker^{high}) on the positively or negatively selected cells, respectively.

The separation need not result in 100 % enrichment or removal of a particular cell population or cells expressing a particular marker. For example, positive selection of or enrichment for cells of a particular type, such as those expressing a marker, refers to increasing the number or percentage of such cells, but need not result in a complete absence of cells not expressing the marker. Likewise, negative selection, removal, or depletion of cells of a particular type, such as those expressing a marker, refers to decreasing the number or percentage of such cells, but need not result in a complete removal of all such cells.

In some examples, multiple rounds of separation steps are carried out, where the positively or negatively selected fraction from one step is subjected to another separation step, such as a subsequent positive or negative selection. In some examples, a single separation step can deplete cells expressing multiple markers simultaneously, such as by incubating cells with a plurality of antibodies or binding partners, each specific for a marker targeted for negative selection. Likewise, multiple cell types can simultaneously be positively selected by incubating cells with a plurality of antibodies or binding partners expressed on the various cell types.

For example, in some aspects, specific subpopulations of T cells, such as cells positive or expressing high levels of one or more surface markers, e.g., CD28⁺, CD62L⁺, CCR7⁺, CD27⁺, CD127⁺, CD4⁺, CD8⁺, CD45RA⁺, and/or CD45RO⁺ T cells, are isolated by positive or negative selection techniques. In some embodiments, such cells are selected by incubation with one or more antibody or binding partner that specifically binds to such markers. In some embodiments, the antibody or binding partner can be conjugated, such as directly or indirectly, to a solid support or matrix to effect selection, such as a magnetic bead or paramagnetic bead. For example, CD3⁺, CD28⁺ T cells can be positively selected using anti-CD3/anti-CD28 conjugated magnetic beads (e.g., DYNABEADS^{®} M-450 CD3/CD28 T Cell Expander, and/or ExpACT^{®} beads).

In some embodiments, T cells are separated from a PBMC sample by negative selection of markers expressed on non-T cells, such as B cells, monocytes, or other white blood cells, such as CD14. In some aspects, a CD4⁺ or CD8⁺ selection step is used to separate CD4⁺ helper and CD8⁺ cytotoxic T cells. Such CD4⁺ and CD8⁺ populations can be further sorted into sub-populations by positive or negative selection for markers expressed or expressed to a relatively higher degree on one or more naive, memory, and/or effector T cell subpopulations.

In some embodiments, CD8⁺ cells are further enriched for or depleted of naive, central memory, effector memory, and/or central memory stem cells, such as by positive or negative selection based on surface antigens associated with the respective subpopulation. In some embodiments, enrichment for central memory T (T_{CM}) cells is carried out to increase efficacy, such as to improve long-term survival, expansion, and/or engraftment following administration, which in some aspects is particularly robust in such sub-populations. See Terakura et al., (2012) Blood.1:72-82; Wang et al. (2012) J Immunother. 35(9):689-701. In some embodiments, combining TCM-enriched CD8⁺ T cells and CD4⁺ T cells further enhances efficacy.

In embodiments, memory T cells are present in both CD62L⁺ and CD62L- subsets of CD8⁺ peripheral blood lymphocytes. PBMC can be enriched for or depleted of CD62L-CD8⁺ and/or CD62L⁺CD8⁺ fractions, such as using anti-CD8 and anti-CD62L antibodies.

In some embodiments, the enrichment for central memory T (TCM) cells is based on positive or high surface expression of CD45RO, CD62L, CCR7, CD28, CD3, and/or CD 127; in some aspects, it is based on negative selection for cells expressing or highly expressing CD45RA and/or granzyme B. In some aspects, isolation of a CD8⁺ population enriched for TCM cells is carried out by depletion of cells expressing CD4, CD14, CD45RA, and positive selection or enrichment for cells expressing CD62L. In one aspect, enrichment for central memory T (TCM) cells is carried out starting with a negative fraction of cells selected based on CD4 expression, which is subjected to a negative selection based on expression of CD14 and CD45RA, and a positive selection based on CD62L. Such selections in some aspects are carried out simultaneously and in other aspects are carried out sequentially, in either order. In some aspects, the same CD4 expression-based selection step used in preparing the CD8⁺ cell population or subpopulation, also is used to generate the CD4⁺ cell population or subpopulation, such that both the positive and negative fractions from the CD4-based separation are retained and used in subsequent steps of the methods, optionally following one or more further positive or negative selection steps. In some embodiments, the selection for the CD4⁺ cell population and the selection for the CD8⁺ cell population are carried out simultaneously. In some embodiments, the CD4⁺ cell population and the selection for the CD8⁺ cell population are carried out sequentially, in either order. In some embodiments, methods for selecting cells can include those as described in published U.S. App. No. US20170037369. In some embodiments, the selected CD4⁺ cell population and the selected CD8⁺ cell population may be combined subsequent to the selecting. In some aspects, the selected CD4⁺ cell population and the selected CD8⁺ cell population may be combined in a container, such as a bag.

In a particular example, a sample of PBMCs or other white blood cell sample is subjected to selection of CD4⁺ cells, where both the negative and positive fractions are retained. The negative fraction then is subjected to negative selection based on expression of CD14 and CD45RA or CD19, and positive selection based on a marker characteristic of central memory T cells, such as CD62L or CCR7, where the positive and negative selections are carried out in either order.

CD4⁺ T helper cells may be sorted into naïve, central memory, and effector cells by identifying cell populations that have cell surface antigens. CD4⁺ lymphocytes can be obtained by standard methods. In some embodiments, naive CD4⁺ T lymphocytes are CD45RO-, CD45RA⁺, CD62L⁺, or CD4⁺ T cells. In some embodiments, central memory CD4⁺ cells are CD62L⁺ and CD45RO⁺. In some embodiments, effector CD4⁺ cells are CD62L- and CD45RO-.

In one example, to enrich for CD4⁺ cells by negative selection, a monoclonal antibody cocktail typically includes antibodies to CD14, CD20, CD11b, CD16, HLA-DR, and CD8. In some embodiments, the antibody or binding partner is bound to a solid support or matrix, such as a magnetic bead or paramagnetic bead, to allow for separation of cells for positive and/or negative selection. For example, in some embodiments, the cells and cell populations are separated or isolated using immunomagnetic (or affinitymagnetic) separation techniques (reviewed in Methods in Molecular Medicine, vol. 58: Metastasis Research Protocols, Vol. 2: Cell Behavior In Vitro and In Vivo, p 17-25 Edited by: S. A. Brooks and U. Schumacher © Humana Press Inc., Totowa, NJ).

In some aspects, the incubated sample or composition of cells to be separated is incubated with a selection reagent containing small, magnetizable or magnetically responsive material, such as magnetically responsive particles or microparticles, such as paramagnetic beads (e.g., such as Dynalbeads or MACS^{®} beads). The magnetically responsive material, e.g., particle, generally is directly or indirectly attached to a binding partner, e.g., an antibody, that specifically binds to a molecule, e.g., surface marker, present on the cell, cells, or population of cells that it is desired to separate, e.g., that it is desired to negatively or positively select.

In some embodiments, the magnetic particle or bead comprises a magnetically responsive material bound to a specific binding member, such as an antibody or other binding partner. Many well-known magnetically responsive materials for use in magnetic separation methods are known, e.g., those described in Molday, U.S. Pat. No. 4,452,773, and in European Patent Specification EP 452342 B. Colloidal sized particles, such as those described in Owen U.S. Pat. No. 4,795,698, and Liberti et al., U.S. Pat. No. 5,200,084 also may be used.

The incubation generally is carried out under conditions whereby the antibodies or binding partners, or molecules, such as secondary antibodies or other reagents, which specifically bind to such antibodies or binding partners, which are attached to the magnetic particle or bead, specifically bind to cell surface molecules if present on cells within the sample.

In certain embodiments, the magnetically responsive particles are coated in primary antibodies or other binding partners, secondary antibodies, lectins, enzymes, or streptavidin. In certain embodiments, the magnetic particles are attached to cells via a coating of primary antibodies specific for one or more markers. In certain embodiments, the cells, rather than the beads, are labeled with a primary antibody or binding partner, and then cell-type specific secondary antibody- or other binding partner (e.g., streptavidin)-coated magnetic particles, are added. In certain embodiments, streptavidin-coated magnetic particles are used in conjunction with biotinylated primary or secondary antibodies.

In some aspects, separation is achieved in a procedure in which the sample is placed in a magnetic field, and those cells having magnetically responsive or magnetizable particles attached thereto will be attracted to the magnet and separated from the unlabeled cells. For positive selection, cells that are attracted to the magnet are retained; for negative selection, cells that are not attracted (unlabeled cells) are retained. In some aspects, a combination of positive and negative selection is performed during the same selection step, where the positive and negative fractions are retained and further processed or subject to further separation steps.

In some embodiments, the affinity-based selection is via magnetic-activated cell sorting (MACS) (Miltenyi Biotec, Auburn, CA). Magnetic Activated Cell Sorting (MACS), e.g., CliniMACS systems are capable of high-purity selection of cells having magnetized particles attached thereto. In certain embodiments, MACS operates in a mode wherein the non-target and target species are sequentially eluted after the application of the external magnetic field. That is, the cells attached to magnetized particles are held in place while the unattached species are eluted. Then, after this first elution step is completed, the species that were trapped in the magnetic field and were prevented from being eluted are freed in some manner such that they can be eluted and recovered. In certain embodiments, the non-target cells are labelled and depleted from the heterogeneous population of cells.

In some embodiments, the magnetically responsive particles are left attached to the cells that are to be subsequently incubated, cultured and/or engineered; in some aspects, the particles are left attached to the cells for administration to a patient. In some embodiments, the magnetizable or magnetically responsive particles are removed from the cells. Methods for removing magnetizable particles from cells are known and include, e.g., the use of competing non-labeled antibodies, magnetizable particles or antibodies conjugated to cleavable linkers, etc. In some embodiments, the magnetizable particles are biodegradable.

In some embodiments, the process for manufacturing, generating or producing a cell therapy includes a step in which the CD4⁺ and CD8⁺ cells are separately isolated and then mixed together prior to the step of introducing a recombinant receptor, e.g. CAR, into the cells. In some embodiments, the CD4⁺ and CD8⁺ cells are mixed at a ratio of 1:5 to 5:1 CD4⁺ to CD8⁺ T cells, such as 1:3 to 3:1, 1:2 to 2:1 or at or about at a ratio of 1:1 CD4⁺ to CD8⁺ cells prior to the introducing of the agent encoding the recombinant receptor and/or one or more of the subsequent processing steps for producing genetically engineered cells.

In some embodiments, the process for manufacturing, generating or producing a cell therapy includes a step of separately isolating the CD4⁺ and CD8⁺ T cells and separately carrying out the one or more subsequent steps on the selected or isolated CD4⁺ T cells and separately carrying out the one or more subsequent steps on the selected or isolated CD8⁺ T cells. In aspects of such an embodiment, the transduced cells, such as separate compositions of CD4⁺ T cells and CD8⁺ T cells genetically engineered with a recombinant receptor, e.g. CAR, can be combined together as a single composition prior to the step of formulating the cells. In other aspects of such an embodiment, the transduced cells, such as the transduced cells, such as separate compositions of CD4⁺ T cells and CD8⁺ T cells genetically engineered with a recombinant receptor, e.g. CAR, are separately formulated, such as for separate administration to a subject.

### B. Activation and Stimulation of Cells

In some embodiments, the methods for manufacturing or engineering cells, such as cells that are assessed or analyzed by the provided methods, include a step of stimulating the isolated cells, such as selected cell populations. The incubation may be prior to or in connection with introduction of the polynucleotide, such as introduction of the polynucleotide containing a transgene sequence for integration, for example by transduction. In some embodiments, the stimulation results in activation and/or proliferation of the cells, for example, prior to transduction.

In some embodiments, the manufacturing or engineering includes steps for incubations of cells, such as selected cells, in which the incubation steps can include culture, cultivation, stimulation, activation, and/or propagation of cells. In some embodiments, the compositions or cells are incubated in the presence of stimulating conditions or a stimulatory agent. Such conditions include those designed to induce proliferation, activation, and/or survival of cells in the population, to mimic antigen exposure, and/or to prime the cells for genetic engineering, such as for the introduction of a recombinant antigen receptor.

In some embodiments, the conditions for stimulation and/or activation can include one or more of particular media, temperature, oxygen content, carbon dioxide content, time, agents, e.g., nutrients, amino acids, antibiotics, ions, and/or stimulatory factors, such as cytokines, chemokines, antigens, binding partners, fusion proteins, recombinant soluble receptors, and any other agents designed to activate the cells.

In some embodiments, the stimulating conditions or agents include one or more agent, e.g., ligand, which is capable of stimulating or activating an intracellular signaling domain of a TCR complex. In some aspects, the agent turns on or initiates TCR/CD3 intracellular signaling cascade in a T cell, such as agents suitable to deliver a primary signal, e.g., to initiate activation of an ITAM-induced signal, such as those specific for a TCR component, e.g., anti-CD3. In some embodiments, the stimulating conditions include one or more agent that promotes a costimulatory signal, such as one specific for a T cell costimulatory receptor, e.g., anti-CD28, or anti-4-1BB. In some embodiments, such agents and/or ligands may be bound to solid support such as a bead, and/or one or more cytokines. Among the stimulating agents are anti-CD3/anti-CD28 beads (e.g., DYNABEADS^{®} M-450 CD3/CD28 T Cell Expander, and/or ExpACT^{®} beads). Optionally, the expansion method may further comprise the step of adding anti-CD3 and/or anti CD28 antibody to the culture medium (e.g., at a concentration of at least about 0.5 ng/mL). In some embodiments, the stimulating agents include IL-2, IL-7 and/or IL-15. In some aspects, the IL-2 concentration is at least about 10 units/mL, at least about 50 units/mL, at least about 100 units/mL or at least about 200 units/mL.

The conditions can include one or more of particular media, temperature, oxygen content, carbon dioxide content, time, agents, e.g., nutrients, amino acids, antibiotics, ions, and/or stimulatory factors, such as cytokines, chemokines, antigens, binding partners, fusion proteins, recombinant soluble receptors, and any other agents designed to activate the cells.

In some aspects, incubation is carried out in accordance with techniques such as those described in US Patent No. 6,040,177 to Riddell et al., Klebanoff et al. (2012) J Immunother. 35(9): 651-660, Terakura et al. (2012) Blood.1:72-82, and/or Wang et al. (2012) J Immunother. 35(9):689-701.

In some embodiments, at least a portion of the incubation in the presence of one or more stimulating conditions or stimulatory agents is carried out in the internal cavity of a centrifugal chamber, for example, under centrifugal rotation, such as described in WO2016/073602. In some embodiments, at least a portion of the incubation performed in a centrifugal chamber includes mixing with a reagent or reagents to induce stimulation and/or activation. In some embodiments, cells, such as selected cells, are mixed with a stimulating condition or stimulatory agent in the centrifugal chamber. In some aspects of such processes, a volume of cells is mixed with an amount of one or more stimulating conditions or agents that is far less than is normally employed when performing similar stimulations in a cell culture plate or other system.

In some embodiments, the stimulating agent is added to cells in the cavity of the chamber in an amount that is substantially less than (e.g. is no more than 5%, 10%, 20%, 30%, 40%, 50%, 60%, 70% or 80% of the amount) as compared to the amount of the stimulating agent that is typically used or would be necessary to achieve about the same or similar efficiency of selection of the same number of cells or the same volume of cells when selection is performed without mixing in a centrifugal chamber, e.g. in a tube or bag with periodic shaking or rotation. In some embodiments, the incubation is performed with the addition of an incubation buffer to the cells and stimulating agent to achieve a target volume with incubation of the reagent of, for example, 10 mL to 200 mL, such as at least or about at least or about or 10 mL, 20 mL, 30 mL, 40 mL, 50 mL, 60 mL, 70 mL, 80 mL, 90 mL, 100 mL, 150 mL, or 200 mL. In some embodiments, the incubation buffer and stimulating agent are pre-mixed before addition to the cells. In some embodiments, the incubation buffer and stimulating agent are separately added to the cells. In some embodiments, the stimulating incubation is carried out with periodic gentle mixing condition, which can aid in promoting energetically favored interactions and thereby permit the use of less overall stimulating agent while achieving stimulating and activation of cells.

In some embodiments, the incubation generally is carried out under mixing conditions, such as in the presence of spinning, generally at relatively low force or speed, such as speed lower than that used to pellet the cells, such as from or from about 600 rpm to 1700 rpm (e.g. at or about or at least 600 rpm, 1000 rpm, or 1500 rpm or 1700 rpm), such as at an RCF at the sample or wall of the chamber or other container of from or from about 80g to 100g (e.g. at or about or at least 80 g, 85 g, 90 g, 95 g, or 100 g). In some embodiments, the spin is carried out using repeated intervals of a spin at such low speed followed by a rest period, such as a spin and/or rest for 1, 2, 3, 4, 5, 6, 7, 8, 9, or 10 seconds, such as a spin at approximately 1 or 2 seconds followed by a rest for approximately 5, 6, 7, or 8 seconds.

In some embodiments, the total duration of the incubation, e.g. with the stimulating agent, is between or between about 1 hour and 96 hours, 1 hour and 72 hours, 1 hour and 48 hours, 4 hours and 36 hours, 8 hours and 30 hours or 12 hours and 24 hours, such as at least or about at least 6 hours, 12 hours, 18 hours, 24 hours, 36 hours or 72 hours. In some embodiments, the further incubation is for a time between or about between 1 hour and 48 hours, 4 hours and 36 hours, 8 hours and 30 hours or 12 hours and 24 hours, inclusive.

### C. Introduction of Polynucleotide Containing Transgene Sequences

In some embodiments, the provided methods can be performed to assess cells that are engineered by introduction of polynucleotides encoding the transgene sequences. In some embodiments, the methods for manufacturing or engineering cells, such as cells that are assessed or analyzed by the provided methods, include a step for introduction of a polynucleotide containing transgene sequences that encode a recombinant protein such as a recombinant receptor. In some embodiments, the engineered cells have been introduced with a polynucleotide comprising a transgene sequence, under conditions for integration of the transgene sequence into a genome of the cell. Introduction of the polynucleotide containing a transgene sequence, such as transgene sequences encoding a recombinant protein, in the cell may be carried out using any of a number of different delivery methods. In some aspects, the condition for integration of the transgene sequence includes any described herein, for example, using viral transduction systems for integration of the transgene sequence into the genome of the cell, e.g., an immune cell.

In some aspects, the polynucleotide containing transgene sequences that encode a recombinant protein such as a recombinant receptor, can be comprised in a vector. In some aspects, uch vectors include viral and non-viral systems, including lentiviral and gammaretroviral systems, as well as transposon-based systems such as PiggyBac or Sleeping Beauty-based gene transfer systems. Exemplary methods include those for introduction or delivery of nucleic acids into a cell, including via viral, e.g., retroviral or lentiviral, transduction, transposons, and electroporation.

In some embodiments, introduction of the polynucleotide is accomplished by first stimulating the cell, such as by combining it with a stimulus that induces a response such as proliferation, survival, and/or activation, e.g., as measured by expression of a cytokine or activation marker, followed by transduction of the activated cells, and expansion in culture to numbers sufficient for clinical applications.

In some embodiments, the polynucleotide is a linear or circular nucleic acid molecule, such as a linear or circular DNA or linear RNA, and can be delivered using any of methods for delivering nucleic acid molecules into the cell. In particular embodiments, the polynucleotide is introduced into the cells in nucleotide form, e.g., as or within a non-viral vector. In some embodiments, the non-viral vector is or includes a polynucleotide, e.g., a DNA or RNA polynucleotide, that is suitable for transduction and/or transfection by any suitable and/or known non-viral method for gene delivery, such as but not limited to microinjection, electroporation, transient cell compression or squeezing (such as described in Lee, et al. (2012) Nano Lett 12: 6322-27), lipid-mediated transfection, peptide-mediated delivery, e.g., cell-penetrating peptides, or a combination thereof.

In some embodiments, recombinant nucleic acids are transferred into T cells via electroporation (see, e.g., Chicaybam et al, (2013) PLoS ONE 8(3): e60298 and Van Tedeloo et al. (2000) Gene Therapy 7(16): 1431-1437). In some embodiments, recombinant nucleic acids are transferred into T cells via transposition (see, e.g., Manuri et al. (2010) Hum Gene Ther 21(4): 427-437; Sharma et al. (2013) Molec Ther Nucl Acids 2, e74; and Huang et al. (2009) Methods Mol Biol 506: 115-126). Other methods of introducing and expressing genetic material in immune cells include calcium phosphate transfection (e.g., as described in Current Protocols in Molecular Biology, John Wiley & Sons, New York. N.Y.), protoplast fusion, cationic liposome-mediated transfection; tungsten particle-facilitated microparticle bombardment (Johnston, Nature, 346: 776-777 (1990)); and strontium phosphate DNA co-precipitation (Brash et al., Mol. Cell Biol., 7: 2031-2034 (1987)).

Other approaches and vectors for transfer of the nucleic acids encoding the recombinant products are those described, e.g., in international patent application, Publication No.: WO2014055668, and U.S. Patent No. 7,446,190.

In some embodiments, the cells, e.g., T cells, may be transduced with either during or after expansion, e.g., with a viral preparation containing polynucleotides that contain the transgene sequences that encode a recombinant protein such as a T cell receptor (TCR) or a chimeric antigen receptor (CAR). This transduction for the introduction of the polynucleotide of the desired receptor can be carried out with any suitable retroviral vector, for example. The genetically modified cell population can then be liberated from the initial stimulus (the anti-CD3/anti-CD28 stimulus, for example) and subsequently be stimulated with a second type of stimulus e.g. via a de novo introduced receptor). This second type of stimulus may include an antigenic stimulus in form of a peptide/MHC molecule, the cognate (cross-linking) ligand of the genetically introduced receptor (e.g. natural ligand of a CAR) or any ligand (such as an antibody) that directly binds within the framework of the new receptor (e.g. by recognizing constant regions within the receptor). See, for example, Cheadle et al, "Chimeric antigen receptors for T-cell based therapy" Methods Mol Biol. 2012; 907:645-66 or Barrett et al., Chimeric Antigen Receptor Therapy for Cancer Annual Review of Medicine Vol. 65: 333-347 (2014).

In some cases, a vector may be used that does not require that the cells, e.g., T cells, are activated. In some such instances, the cells may be selected and/or transduced prior to activation. Thus, the cells may be engineered prior to, or subsequent to culturing of the cells, and in some cases at the same time as or during at least a portion of the culturing.

In some aspects, the cells further are engineered to promote expression of cytokines or other factors. Among additional nucleic acids, e.g., genes for introduction are those to improve the efficacy of therapy, such as by promoting viability and/or function of transferred cells; genes to provide a genetic marker for selection and/or evaluation of the cells, such as to assess in vivo survival or localization; genes to improve safety, for example, by making the cell susceptible to negative selection in vivo as described by Lupton S. D. et al., Mol. and Cell Biol., 11:6 (1991); and Riddell et al., Human Gene Therapy 3:319-338 (1992); see also WO 1992/008796 and WO 1994/028143 describing the use of bifunctional selectable fusion genes derived from fusing a dominant positive selectable marker with a negative selectable marker. See, e.g., Riddell et al., US Patent No. 6,040,177, at columns 14-17.

In some embodiments, the introducing is carried out by contacting one or more cells of a composition with a nucleic acid molecule encoding the recombinant protein, e.g. recombinant receptor. In some embodiments, the contacting can be effected with centrifugation, such as spinoculation (e.g. centrifugal inoculation). Such methods include any of those as described in WO2016/073602. Exemplary centrifugal chambers include those produced and sold by Biosafe SA, including those for use with the Sepax^{®} and Sepax^{®} 2 system, including an A-200/F and A-200 centrifugal chambers and various kits for use with such systems. Exemplary chambers, systems, and processing instrumentation and cabinets are described, for example, in US Patent No. 6,123,655, US Patent No. 6,733,433 and US 2008/0171951 and WO 00/38762Exemplary kits for use with such systems include, but are not limited to, single-use kits sold by BioSafe SA under product names CS-430.1, CS-490.1, CS-600.1 or CS-900.2.

In some embodiments, the system is included with and/or placed into association with other instrumentation, including instrumentation to operate, automate, control and/or monitor aspects of the transduction step and one or more various other processing steps performed in the system, e.g. one or more processing steps that can be carried out with or in connection with the centrifugal chamber system as described herein or in WO2016/073602. This instrumentation in some embodiments is contained within a cabinet. In some embodiments, the instrumentation includes a cabinet, which includes a housing containing control circuitry, a centrifuge, a cover, motors, pumps, sensors, displays, and a user interface. An exemplary device is described in US Patent No. 6,123,655, US Patent No. 6,733,433 and US 2008/0171951.

In some embodiments, the system comprises a series of containers, e.g., bags, tubing, stopcocks, clamps, connectors, and a centrifuge chamber. In some embodiments, the containers, such as bags, include one or more containers, such as bags, containing the cells to be transduced and the viral vector particles, in the same container or separate containers, such as the same bag or separate bags. In some embodiments, the system further includes one or more containers, such as bags, containing medium, such as diluent and/or wash solution, which is pulled into the chamber and/or other components to dilute, resuspend, and/or wash components and/or compositions during the methods. The containers can be connected at one or more positions in the system, such as at a position corresponding to an input line, diluent line, wash line, waste line and/or output line.

In some embodiments, the chamber is associated with a centrifuge, which is capable of effecting rotation of the chamber, such as around its axis of rotation. Rotation may occur before, during, and/or after the incubation in connection with transduction of the cells and/or in one or more of the other processing steps. Thus, in some embodiments, one or more of the various processing steps is carried out under rotation, e.g., at a particular force. The chamber is typically capable of vertical or generally vertical rotation, such that the chamber sits vertically during centrifugation and the side wall and axis are vertical or generally vertical, with the end wall(s) horizontal or generally horizontal.

In some embodiments, the composition containing cells, viral particles and reagent can be rotated, generally at relatively low force or speed, such as speed lower than that used to pellet the cells, such as from or from about 600 rpm to 1700 rpm (e.g. at or about or at least 600 rpm, 1000 rpm, or 1500 rpm or 1700 rpm). In some embodiments, the rotation is carried at a force, e.g., a relative centrifugal force, of from or from about 100 g to 3200 g (e.g. at or about or at least at or about 100 g, 200 g, 300 g, 400 g, 500 g, 1000 g, 1500 g, 2000 g, 2500 g, 3000 g or 3200 g), as measured for example at an internal or external wall of the chamber or cavity. The term "relative centrifugal force" or RCF is generally understood to be the effective force imparted on an object or substance (such as a cell, sample, or pellet and/or a point in the chamber or other container being rotated), relative to the earth's gravitational force, at a particular point in space as compared to the axis of rotation. The value may be determined using well-known formulas, taking into account the gravitational force, rotation speed and the radius of rotation (distance from the axis of rotation and the object, substance, or particle at which RCF is being measured).

### D. Nucleic Acids and Vectors

In some embodiments, the cells assessed or analyzed using the provided methods include immune cells, that have are genetically engineered. In some embodiments, the cells, e.g., T cells, are genetically engineered to express a recombinant protein, such as a recombinant receptor. In some embodiments, the engineering is carried out by introducing one or more polynucleotide(s) that contain transgene sequences encoding the recombinant proteins or portions or components thereof. In some aspects, a portion of the polynucleotide, e.g., containing the transgene sequences, are introduced for integration of the sequence into the genome of the cell, e.g., the cell being engineered. I In some aspects, the polynucleotide is comprised in a vector, such as a viral vector, for introduction of the polynucleotide into the engineered cell.

In some embodiments, the polynucleotide containing the transgene sequences can be comprised in a vector molecule. In some embodiments, the virus is a DNA virus (e.g., dsDNA or ssDNA virus). In some embodiments, the virus is an RNA virus (e.g., an ssRNA virus). Exemplary viral vectors/viruses include, e.g., retroviruses, lentiviruses, adenovirus, adeno-associated virus (AAV), vaccinia viruses, poxviruses, and herpes simplex viruses, or any of the viruses described elsewhere herein. A polynucleotide can be introduced into a cell as part of a vector molecule having additional sequences such as, for example, replication origins, promoters and genes encoding antibiotic resistance. Moreover, polynucleotides can be introduced as naked nucleic acid, as nucleic acid complexed with materials such as a liposome, nanoparticle or poloxamer, or can be delivered by viruses (e.g., adenovirus, AAV, herpesvirus, retrovirus, lentivirus and integrase defective lentivirus (IDLV)).

In some embodiments, the polynucleotide comprising the transgene sequences, such as a transgene sequence encoding a recombinant protein, is transferred into cells using recombinant infectious virus particles, such as, e.g., vectors derived from simian virus 40 (SV40), adenoviruses, adeno-associated virus (AAV), and human immunodeficiency virus (HIV). In some embodiments, recombinant nucleic acids are transferred into T cells using recombinant lentiviral vectors or retroviral vectors, such as gamma-retroviral vectors (see, e.g., Koste et al. (2014) Gene Therapy 2014 Apr 3. doi: 10.1038/gt.2014.25; Carlens et al. (2000) Exp Hematol 28(10): 1137-46; Alonso-Camino et al. (2013) Mol Ther Nucl Acids 2, e93; Park et al., Trends Biotechnol. 2011 November 29(11): 550-557 or HIV-1 derived lentiviral vectors.

In some embodiments, the retroviral vector has a long terminal repeat sequence (LTR), e.g., a retroviral vector derived from the Moloney murine leukemia virus (MoMLV), myeloproliferative sarcoma virus (MPSV), murine embryonic stem cell virus (MESV), murine stem cell virus (MSCV), spleen focus forming virus (SFFV) or human immunodeficiency virus (HIV). In some embodiments, the retroviruses include those derived from any avian or mammalian cell source. The retroviruses typically are amphotropic, meaning that they are capable of infecting host cells of several species, including humans. In some embodiments, the gene to be expressed replaces the retroviral gag, pol and/or env sequences. A number of illustrative retroviral systems have been described (e.g., U.S. Pat. Nos. 5,219,740; 6,207,453; 5,219,740; Miller and Rosman (1989) BioTechniques 7:980-990; Miller, A. D. (1990) Human Gene Therapy 1:5-14; Scarpa et al. (1991) Virology 180:849-852; Burns et al. (1993) Proc. Natl. Acad. Sci. USA 90:8033-8037; and Boris-Lawrie and Temin (1993) Cur. Opin. Genet. Develop. 3:102-109.

Methods of lentiviral transduction are known. Exemplary methods are described in, e.g., Wang et al. (2012) J. Immunother. 35(9): 689-701; Cooper et al. (2003) Blood. 101:1637-1644; Verhoeyen et al. (2009) Methods Mol Biol. 506: 97-114; and Cavalieri et al. (2003) Blood. 102(2): 497-505.

In other aspects, the polynucleotide is delivered by viral and/or non-viral gene transfer methods. In some embodiments, the polynucleotide is delivered to the cell via an adeno associated virus (AAV). Any AAV vector can be used, including, but not limited to, AAV1, AAV2, AAV3, AAV4, AAV5, AAV6, AAV7, AAV8 and combinations thereof. In some instances, the AAV comprises LTRs that are of a heterologous serotype in comparison with the capsid serotype (e.g., AAV2 ITRs with AAV5, AAV6, or AAV8 capsids). In some embodiments, the polynucleotide containing the agent(s) and/or polynucleotide is delivered by a recombinant AAV. In some embodiments, the AAV can incorporate its genome into that of a host cell, e.g., a target cell as described herein. In another embodiment, the AAV is a self-complementary adeno-associated virus (scAAV), e.g., a scAAV that packages both strands which anneal together to form double stranded DNA. AAV serotypes that may be used in the disclosed methods, include AAV1, AAV2, modified AAV2 (e.g., modifications at Y444F, Y500F, Y730F and/or S662V), AAV3, modified AAV3 (e.g., modifications at Y705F, Y731F and/or T492V), AAV4, AAV5, AAV6, modified AAV6 (e.g., modifications at S663V and/or T492V), AAV7, AAV8, AAV 8.2, AAV9, AAV.rh10, modified AAV.rh10, AAV.rh32/33, modified AAV.rh32/33, AAV.rh43, modified AAV.rh43, AAV.rh64R1, modified AAV.rh64R1, and pseudotyped AAV, such as AAV2/8, AAV2/5 and AAV2/6 can also be used in the disclosed methods.

In some embodiments, the transgene sequence is contained in a vector or can be cloned into one or more vector(s). In some embodiments, the one or more vector(s) can be used to transform or transfect a host cell, e.g., a cell for engineering. Exemplary vectors include vectors designed for introduction, propagation and expansion or for expression or both, such as plasmids and viral vectors. In some aspects, the vector is an expression vector, e.g., a recombinant expression vector. In some embodiments, the recombinant expression vectors can be prepared using standard recombinant DNA techniques.

In some embodiments, the vector can be a vector of the pUC series (Fermentas Life Sciences), the pBluescript series (Stratagene, LaJolla, Calif.), the pET series (Novagen, Madison, Wis.), the pGEX series (Pharmacia Biotech, Uppsala, Sweden), or the pEX series (Clontech, Palo Alto, Calif.). In some cases, bacteriophage vectors, such as λG10, λGT11, λZapII (Stratagene), λEMBL4, and λNM1149, also can be used. In some embodiments, plant expression vectors can be used and include pBI01, pBI101.2, pBI101.3, pBI121 and pBIN19 (Clontech). In some embodiments, animal expression vectors include pEUK-Cl, pMAM and pMAMneo (Clontech).

In some embodiments, the vector is a viral vector, such as a retroviral vector. In some embodiments, the transgene sequence and/or additional polypeptide(s) are introduced into the cell via retroviral or lentiviral vectors, or via transposons (see, e.g., Baum et al. (2006) Molecular Therapy: The Journal of the American Society of Gene Therapy. 13:1050-1063; Frecha et al. (2010) Molecular Therapy 18:1748-1757; and Hackett et al. (2010) Molecular Therapy 18:674-683).

In some embodiments, the one or more transgene sequence(s) or vector(s) encoding a recombinant protein, e.g., recombinant receptor, and/or additional polypeptide(s) may be introduced into cells, e.g., T cells, either during or after expansion. This introduction of the transgene sequence(s) or vector(s) can be carried out with any suitable retroviral vector, for example. Resulting genetically engineered cells can then be liberated from the initial stimulus (e.g., anti-CD3/anti-CD28 stimulus) and subsequently be stimulated with a second type of stimulus (e.g., via a de novo introduced recombinant protein, e.g., recombinant receptor,). This second type of stimulus may include an antigenic stimulus in form of a peptide/MHC molecule, the cognate (cross-linking) ligand of the genetically introduced receptor (e.g. natural antigen and/or ligand of a CAR) or any ligand (such as an antibody) that directly binds within the framework of the new receptor (e.g. by recognizing constant regions within the receptor). See, for example, Cheadle et al, "Chimeric antigen receptors for T-cell based therapy" Methods Mol Biol. 2012; 907:645-66 or Barrett et al., Chimeric Antigen Receptor Therapy for Cancer Annual Review of Medicine Vol. 65: 333-347 (2014).

In some cases, a vector may be used that does not require that the cells, e.g., T cells, are activated. In some such instances, the cells may be selected and/or transduced prior to activation or stimulation. Thus, the cells may be engineered prior to, or subsequent to culturing of the cells, and in some cases at the same time as or during at least a portion of the culturing.

### 1. Preparation of Viral Vector Particles for Transduction

In some embodiments, the polynucleotide comprising the transgene sequences, such as a transgene sequence encoding a recombinant protein, is transferred into cells using recombinant infectious virus particles. In some aspects, the polynucleotide is introduced via viral transduction. In some aspects, the transgene sequences are comprised in a viral vector. The viral vector genome is typically constructed in a plasmid form that can be transfected into a packaging or producer cell line. In any of such examples, the transgene sequences encoding a recombinant protein, such as a recombinant receptor, is inserted or located in a region of the viral vector, such as generally in a non-essential region of the viral genome. In some embodiments, the transgene sequences is inserted into the viral genome in the place of certain viral sequences to produce a virus that is replication defective.

Any of a variety of known methods can be used to produce retroviral particles whose genome contains an RNA copy of the viral vector genome. In some embodiments, at least two components are involved in making a virus-based gene delivery system: first, packaging plasmids, encompassing the structural proteins as well as the enzymes necessary to generate a viral vector particle, and second, the viral vector itself, i.e., the genetic material to be transferred. Biosafety safeguards can be introduced in the design of one or both of these components.

In some embodiments, the packaging plasmid can contain all retroviral, such as HIV-1, proteins other than envelope proteins (Naldini et al., 1998). In other embodiments, viral vectors can lack additional viral genes, such as those that are associated with virulence, e.g. vpr, vif, vpu and nef, and/or Tat, a primary transactivator of HIV. In some embodiments, lentiviral vectors, such as HIV-based lentiviral vectors, comprise only three genes of the parental virus: gag, pol and rev, which reduces or eliminates the possibility of reconstitution of a wild-type virus through recombination.

In some embodiments, the viral vector genome is introduced into a packaging cell line that contains all the components necessary to package viral genomic RNA, transcribed from the viral vector genome, into viral particles. Alternatively, the viral vector genome may comprise one or more genes encoding viral components in addition to the one or more transgene sequences, e.g., encoding a recombinant protein, of interest. In some aspects, in order to prevent replication of the genome in the target cell, however, endogenous viral genes required for replication are removed and provided separately in the packaging cell line.

In some embodiments, a packaging cell line is transfected with one or more plasmid vectors containing the components necessary to generate the particles. In some embodiments, a packaging cell line is transfected with a plasmid containing the viral vector genome, including the LTRs, the cis-acting packaging sequence and the sequence of interest, i.e., a nucleic acid encoding an antigen receptor, such as a CAR; and one or more helper plasmids encoding the virus enzymatic and/or structural components, such as Gag, pol and/or rev. In some embodiments, multiple vectors are utilized to separate the various genetic components that generate the retroviral vector particles. In some such embodiments, providing separate vectors to the packaging cell reduces the chance of recombination events that might otherwise generate replication competent viruses. In some embodiments, a single plasmid vector having all of the retroviral components can be used.

In some embodiments, the retroviral vector particle, such as lentiviral vector particle, is pseudotyped to increase the transduction efficiency of host cells. For example, a retroviral vector particle, such as a lentiviral vector particle, in some embodiments is pseudotyped with a VSV-G glycoprotein, which provides a broad cell host range extending the cell types that can be transduced. In some embodiments, a packaging cell line is transfected with a plasmid or polynucleotide encoding a non-native envelope glycoprotein, such as to include xenotropic, polytropic or amphotropic envelopes, such as Sindbis virus envelope, GALV or VSV-G.

In some embodiments, the packaging cell line provides the components, including viral regulatory and structural proteins, that are required in trans for the packaging of the viral genomic RNA into lentiviral vector particles. In some embodiments, the packaging cell line may be any cell line that is capable of expressing lentiviral proteins and producing functional lentiviral vector particles. In some aspects, suitable packaging cell lines include 293 (ATCC CCL X), 293T, HeLA (ATCC CCL 2), D17 (ATCC CCL 183), MDCK (ATCC CCL 34), BHK (ATCC CCL-10) and Cf2Th (ATCC CRL 1430) cells.

In some embodiments, the packaging cell line stably expresses the viral protein(s). For example, in some aspects, a packaging cell line containing the gag, pol, rev and/or other structural genes but without the LTR and packaging components can be constructed. In some embodiments, a packaging cell line can be transiently transfected with nucleic acid molecules encoding one or more viral proteins along with the viral vector genome containing a nucleic acid molecule encoding a heterologous protein, and/or a nucleic acid encoding an envelope glycoprotein.

In some embodiments, the viral vectors and the packaging and/or helper plasmids are introduced via transfection or infection into the packaging cell line. The packaging cell line produces viral vector particles that contain the viral vector genome. Methods for transfection or infection are well known. Non-limiting examples include calcium phosphate, DEAE-dextran and lipofection methods, electroporation and microinjection.

When a recombinant plasmid and the retroviral LTR and packaging sequences are introduced into a special cell line (e.g., by calcium phosphate precipitation for example), the packaging sequences may permit the RNA transcript of the recombinant plasmid to be packaged into viral particles, which then may be secreted into the culture media. The media containing the recombinant retroviruses in some embodiments is then collected, optionally concentrated, and used for gene transfer. For example, in some aspects, after co-transfection of the packaging plasmids and the transfer vector to the packaging cell line, the viral vector particles are recovered from the culture media and titered by standard methods used by those of skill in the art.

In some embodiments, a retroviral vector, such as a lentiviral vector, can be produced in a packaging cell line, such as an exemplary HEK 293T cell line, by introduction of plasmids to allow generation of lentiviral particles. In some embodiments, a packaging cell is transfected and/or contains a polynucleotide encoding gag and pol, and a polynucleotide encoding a recombinant receptor, such as an antigen receptor, for example, a CAR. In some embodiments, the packaging cell line is optionally and/or additionally transfected with and/or contains a polynucleotide encoding a rev protein. In some embodiments, the packaging cell line is optionally and/or additionally transfected with and/or contains a polynucleotide encoding a non-native envelope glycoprotein, such as VSV-G. In some such embodiments, approximately two days after transfection of cells, e.g. HEK 293T cells, the cell supernatant contains recombinant lentiviral vectors, which can be recovered and titered.

Recovered and/or produced retroviral vector particles can be used to transduce target cells using the methods as described. Once in the target cells, the viral RNA is reverse-transcribed, imported into the nucleus and stably integrated into the host genome. One or two days after the integration of the viral RNA, the expression of the recombinant protein, e.g. antigen receptor, such as CAR, can be detected.

### E. Cultivating and/or Expansion of Cells

In some embodiments, the cells, e.g., engineered cells, assessed or analyzed using the provided methods are generated using a method of engineering or manufacturing that does not include a step for cultivation and/or expansion of the cells after introduction of the polynucleotide, or an engineering or manufacturing process that is shortened or abbreviated, such as using a non-expanded manufacturing process. In some aspects, the shortened or abbreviated manufacturing process includes a shortened or abbreviated expansion step, or does not include an expansion step, after introduction of the nucleic acids encoding the recombinant receptor. In some aspects, after introduction of the polynucleotide comprising transgene sequences into the cell, the cells are subject to a shortened or abbreviated incubation step for expansion or is not subjected to an expansion step, for example, is subject to a non-expanded manufacturing process. In some aspects, the provided methods are performed at one or more time points during a non-expanded, shortened or abbreviated manufacturing process.

In some embodiments, the cells, e.g., engineered cells, assessed or analyzed using the provided methods are generated using a method of engineering or manufacturing that can include one or more steps for cultivating engineered cells, e.g., cultivating cells under conditions that promote proliferation and/or expansion. In some embodiments, the provided methods include one or more steps for cultivating engineered cells, e.g., cultivating cells under conditions that promote proliferation and/or expansion. In some embodiments, during at least a part of process for the introduction of the polynucleotide, e.g., via viral transduction, and/or subsequent to the introduction of the polynucleotide, the cells are cultured, such as for cultivation or expansion of the cells. In some aspects, the provided methods can be performed at one or more times after introduction of the polynucleotide, e.g., via viral transduction, such as at one or more time points during the culture or expansion of the cells.

In some aspects, after introduction of the polynucleotide comprising transgene sequences into the cell, the cells are subsequently transferred to a container such as a bag for culturing. In some embodiments, the container for cultivation or expansion of the cells is a bioreactor bag, such as a perfusion bag. In some embodiments, cells cultivated while enclosed, connected, and/or under control of a bioreactor undergo expansion during the cultivation more rapidly than cells that are cultivated without a bioreactor, e.g., cells that are cultivated under static conditions such as without mixing, rocking, motion, and/or perfusion.

In some embodiments, engineered cells are cultivated under conditions that promote proliferation and/or expansion subsequent to a step of genetically engineering, e.g., introducing a recombinant polypeptide to the cells by transduction or transfection. In particular embodiments, the cells are cultivated after the cells have been incubated under stimulating conditions and transduced or transfected with a recombinant polynucleotide, e.g., a polynucleotide encoding a recombinant receptor. In some embodiments, the cultivation produces one or more cultivated compositions of enriched T cells.

In some embodiments, the engineered cells are cultured in a container that can be filled, e.g. via the feed port, with cell media and/or cells for culturing of the added cells. The cells can be from any cell source for which culture of the cells is desired, for example, for expansion and/or proliferation of the cells.

In some aspects, the culture media is an adapted culture medium that supports that growth, cultivation, expansion or proliferation of the cells, such as T cells. In some aspects, the medium can be a liquid containing a mixture of salts, amino acids, vitamins, sugars or any combination thereof. In some embodiments, the culture media further contains one or more stimulating conditions or agents, such as to stimulate the cultivation, expansion or proliferation of cells during the culture. In some embodiments, the stimulating condition is or includes one or more cytokine selected from IL-2, IL-7 or IL-15. In some embodiments, the cytokine is a recombinant cytokine. In some embodiments, the concentration of the one or more cytokine in the culture media during the culturing or incubation, independently, is from or from about 1 IU/mL to 1500 IU/mL, such as from or from about 1 IU/mL to 100 IU/mL, 2 IU/mL to 50 IU/mL, 5 IU/mL to 10 IU/mL, 10 IU/mL to 500 IU/mL, 50 IU/mL to 250 IU/mL or 100 IU/mL to 200 IU/mL, 50 IU/mL to 1500 IU/mL, 100 IU/mL to 1000 IU/mL or 200 IU/mL to 600 IU/mL. In some embodiments, the concentration of the one or more cytokine, independently, is at least or at least about 1 IU/mL, 5 IU/mL, 10 IU/mL, 50 IU/mL, 100 IU/mL, 200 IU/mL, 500 IU/mL, 1000 IU/mL or 1500 IU/mL.

In some aspects, the cells are cultured, such as incubated, for at least a portion of time after transfer of the engineered cells and culture media. In some embodiments, the stimulating conditions generally include a temperature suitable for the growth of primary immune cells, such as human T lymphocytes, for example, at least about 25 degrees Celsius (°C), generally at least about 30°C, and generally at or about 37°C. In some embodiments, the cells are incubated at a temperature of 25 to 38°C, such as 30 to 37°C, for example at or about 37°C ± 2°C. In some embodiments, the incubation is carried out for a time period until the culture, e.g. cultivation or expansion, results in a desired or threshold density, number or dose of cells. In some embodiments, the incubation is greater than or greater than about or is for about or 24 hours, 48 hours, 72 hours, 96 hours, 5 days, 6 days, 7 days, 8 days, 9 days or more.

In some embodiments, the cells are incubated under conditions to maintain a target amount of carbon dioxide in the cell culture. In some aspects, this ensures optimal cultivation, expansion and proliferation of the cells during the growth. In some aspects, the amount of carbon dioxide (CO₂) is between 10% and 0% (v/v) of said gas, such as between 8% and 2% (v/v) of said gas, for example an amount of or about 5% (v/v) CO₂.

In some embodiments, cells are incubated using containers, e.g., bags, which are used in connection with a bioreactor. In some cases, the bioreactor can be subject to motioning or rocking, which, in some aspects, can increase oxygen transfer. Motioning the bioreactor may include, but is not limited to rotating along a horizontal axis, rotating along a vertical axis, a rocking motion along a tilted or inclined horizontal axis of the bioreactor or any combination thereof. In some embodiments, at least a portion of the incubation is carried out with rocking. The rocking speed and rocking angle may be adjusted to achieve a desired agitation. In some embodiments the rock angle is or is about 20°, 19°, 18°, 17°, 16°, 15°, 14°, 13°, 12°, 11°, 10°, 9°, 8°, 7°, 6°, 5°, 4°, 3°, 2° or 1°. In certain embodiments, the rock angle is between 6-16°. In other embodiments, the rock angle is between 7-16°. In other embodiments, the rock angle is between 8-12°. In some embodiments, the rock rate is 1, 2, 3, 4, 5, 6, 7, 8, 9, 10, 11, 12, 13, 14 15, 16, 17, 18, 19, 20, 21, 22, 23, 24, 25, 26, 27, 28, 29, 30, 31, 32, 33, 34, 35, 36, 37, 38, 39 or 40 rpm. In some embodiments, the rock rate is between 4 and 12 rpm, such as between 4 and 6 rpm, inclusive. At least a portion of the cell culture expansion is performed with a rocking motion, such as at an angle of between 5° and 10°, such as 6°, at a constant rocking speed, such as a speed of between 5 and 15 RPM, such as 6 RMP or 10 RPM. The CD4+ and CD8+ cells are each separately expanded until they each reach a threshold amount or cell density.

In some embodiments, at least a portion of the incubation is carried out under static conditions. In some embodiments, at least a portion of the incubation is carried out with perfusion, such as to perfuse out spent media and perfuse in fresh media during the culture. In some embodiments, the method includes a step of perfusing fresh culture medium into the cell culture, such as through a feed port. In some embodiments, the culture media added during perfusion contains the one or more stimulating agents, e.g. one or more recombinant cytokine, such as IL-2, IL-7 and/or IL-15. In some embodiments, the culture media added during perfusion is the same culture media used during a static incubation.

In some embodiments, subsequent to the incubation, the container, e.g., bag, is re-connected to a system for carrying out the one or more other processing steps of for manufacturing, generating or producing the cell therapy, such as is re-connected to the system containing the centrifugal chamber. In some aspects, cultured cells are transferred from the bag to the internal cavity of the chamber for formulation of the cultured cells.

In some embodiments, the incubation is carried out for a time period until the culture, e.g. cultivation or expansion, results in a desired or threshold density, number or dose of cells. In some embodiments, the cells cultivated while enclosed, connected, and/or under control of a bioreactor reach or achieve a threshold expansion, cell count, and/or density within 21 days, 14 days, 10 days, 8 days, 7 days, 6 days, 5 days, 4 days, 3 days, 2 days, 60 hours, 48 hours, 36 hours, 24 hours, or 12 hours. In some embodiments, the incubation is carried out for greater than or greater than about or is for about or for 12 hours, 24 hours, 48 hours, 72 hours, 96 hours, 5 days, 6 days, 7 days, 8 days, 9 days or more. In some aspects, the incubation is shortened or abbreviated, such as for less than or less than about or is for about or for 12 hours, 24 hours, 48 hours, 72 hours or 96 hours, after introduction of the nucleic acid. In some aspects, the incubation is sufficient to permit delivery and integration of the transgene sequence into the genome of the cells.

In some embodiments, cells are cultured for a shortened or abbreviated time or is subject to a non-expanded manufacturing process. In some aspects, the cells are cultured for less than or less than about 1, 2, 3, 4, 5, 6 or 7 days after introduction of the nucleic acids, e.g., via transduction or electroporation. In some embodiments, cells are cultured for about 2-3 days, 3-4 days, 4-5 days, 5-6 days or 6-7 days or less, each inclusive.

In some aspects, the methods can be used to determine a suitable length of incubation of the cell after introduction of the polynucleotides comprising the transgene sequences, at which the majority or substantially all of integration is completed. In some aspects, the methods permit the determination of suitable length of incubation that can maximize integration yet reduces exhaustion of the engineered cells. In some aspects, the cell or population or plurality of cells are not incubated at a temperature greater than 25°C for more than 48, 54, 60, 66, or 72 hours following the introduction of the polynucleotide comprising the transgene sequence into the cell. In some aspects, the cell is not incubated at a temperature greater than 25°C for more than 72 hours following the introduction of the polynucleotide comprising the transgene sequence into the cell. In some aspects, the cell is not incubated at a temperature greater than about 30°C and less than about 40°C for more than 72 hours following the introduction of the polynucleotide comprising the transgene sequence into the cell.

### F. Exemplary Cells for Assessment

In some aspects, the provided methods can be carried out or performed on a plurality of cells or a single isolated cell that has been subject to some or all of the steps of a cell engineering process, including an expanded or non-expanded manufacturing process. In some embodiments, the provided methods are carried out during one or more time points, such as during or after one or more time points of a cell engineering process. In some embodiments, the provided methods are carried out after completion of a cell engineering or cell manufacturing process, including engineering or manufacturing processes that include an expansion step, or that does not include an expansion step, or is a shortened or abbreviated process. In some embodiments, the provided methods can be carried out in a sample from a subject that has been administered a cell therapy. In some aspects, the sample can include the blood or serum or organ or tissue sample (*e*.*g*., disease site, such as a tumor sample) of the subject, obtained after administration of the cell therapy.

In some embodiments, the cells that are engineered and assessed according to the provided methods are primary cells. In some embodiments, the cells are immune cells or enriched immune cells. In some embodiments, the cells are T cells or enriched with T cells. In some embodiments, the cells are CD4+ T cells or enriched CD4+ T cells. In some embodiments, the cells are CD8+ T cells or enriched CD8+ T cells. In some embodiments, the cells comprise genetically engineered cells or an enriched population of genetically-engineered cells. In some embodiments, the cells comprise cells to be genetically engineered or being genetically engineered or have been genetically engineered, such as cells at one or more steps or steps of a manufacturing or engineering process. In some embodiments, the cells comprise an enriched population of cells to be genetically engineered or being genetically engineered. In some embodiments, the cells comprise genetically engineered T cells or an enriched population of genetically engineered T cells. In some embodiments, the cells are engineered to express a recombinant protein, such as a recombinant receptor or a portion thereof. In some embodiments, the recombinant receptor is or comprises a chimeric antigen receptor (CAR). In some embodiments, the cells have been previously cryopreserved. In some embodiments, the cells have not been previously cryopreserved. In some embodiments, the methods can be performed on cells prior to cryopreservation. In some embodiments, the methods can be performed on cells prior to administration of the cells or cell compositions to a subject. In some embodiments, the cells specifically target a tumor cell.

In some embodiments, cells are cultured for a shortened or abbreviated time or is subject to a non-expanded manufacturing process. In some aspects, the cells are cultured for less than or less than about 1, 2, 3, 4, 5, 6 or 7 days after introduction of the nucleic acids encoding the recombinant protein, e.g., via transduction or electroporation. In some embodiments, cells are cultured for about 2-3 days, 3-4 days, 4-5 days, 5-6 days or 6-7 days or less, each inclusive. In some aspects, the cells that are assessed at one or more time point selected from day 0, 1, 2, 3, 4, 5, 6 or 7 after introduction of the nucleic acids, e.g., via transduction or electroporation.

In some aspects, the provided methods can be performed at one or more times after introduction of the polynucleotide but without expansion, e.g., the engineered cell has not been incubated at a temperature greater than 25°C, optionally at or about 37°C ± 2°C, for more than 96, 72, or 48 hours following the introduction of the polynucleotide comprising the transgene sequence.

In particular embodiments, the one or more assessments, such as assessment of integrated and/or non-integrated transgene sequences according to the methods provided herein, are performed before the engineered cells are released for infusion, ready for administration to a subject, and/or administered to a subject, such as for cell therapy. In particular embodiments, engineered cells are released for infusion, ready for administration to a subject, and/or administered to a subject after one or more assessments have been performed, e.g., on a portion, fraction, and/or sample of engineered cells. In particular embodiments, engineered cells are released for infusion, ready for administration to a subject, and/or administered to a subject after the engineered cells are determined to be safe, e.g., sterile and/or free, and/or have desired biological characteristics following the completion of the one or more assessments.

In some aspects, the polynucleotide containing transgene sequences is introduced into the cell using various delivery methods such as viral transduction. In some embodiments, the engineered cells, such as engineered cells for adoptive cell therapy, are required to be monitored or assessed for various characteristics and features, such as determining the level of expression of the recombinant protein encoded by the transgene sequences, and/or determining the number of copies of the transgene sequences that are integrated into the genome of the cell, such as stably integrated into the genome of the cell. In some aspects, such assessment can be performed at one or more time points during the engineering or manufacturing process.

### III. TRANSGENE SEQUENCES

In some aspects, the provided embodiments are used in connection with assessing integration of transgene sequences. In some aspects, the polynucleotides introduced into the cells for engineering, for example, using any of the methods for introducing a polynucleotide sequence described in Section II above, contain transgene sequences that are to be integrated into the genome of the cell. In some aspects, the transgene sequence portion of the polynucleotide is designed to be integrated into the genome of the cell. In some cases, the transgene sequence can refer to a portion of the polynucleotide that is integrated into the genome of the cell.

In some aspects, transgene sequences (also called chimeric sequences, chimeric DNA or recombinant DNA) include nucleic acid sequences that have been formed artificially by combining constituents from different sources, such as different organisms, different genes or different variants. In some aspects, the transgene sequences have undergone a molecular biological manipulation, for example, by artificial combination of different nucleic acid molecules or fragments from different sources. In some embodiments, the transgene sequences contain at least some portion of the sequences that are from a different origin compared to the genomic sequence of the cells into which the polynucleotide containing the transgene sequence is introduced. For example, at least a portion of the transgene sequence is heterologous, exogenous or transgenic to the cell, which in some cases is a primary cell isolated from a subject who is a candidate for administration of the engineered cells.

In some aspects, the transgene sequence that is integrated into the cell, include coding and/or non-coding sequences and/or partial coding sequences thereof, that are inserted or integrated into the genome of the cell. In some aspects, the integration can be random, semi-random or targeted. In some aspects, the transgene sequences can contain nucleic acid sequence fragments comprising arrangements that do not occur naturally, such as fragments from different origins joined together. In some aspects, the transgene sequence is not a naturally occurring sequence. In some embodiments, the transgene sequence does not encode a complete viral gag protein. In some embodiments, the transgene sequence does not comprise a complete HIV genome, a replication competent viral genome, and/or accessory genes, which accessory genes are optionally Nef, Vpu, Vpx, Vif and/or Vpr. In some aspects, the transgene sequence is contained in the polynucleotide that is introduced into the cell, for example, according to the methods for introducing the polynucleotide described herein. In some aspects, the transgene sequence encodes all or a portion of one or more recombinant proteins. In some aspects, the recombinant protein encoded by the transgene sequence is a recombinant receptor, such as a chimeric antigen receptor (CAR) or a recombinant T cell receptor (TCR). In some embodiments, the transgene sequence encodes one or more additional recombinant proteins.

In some aspects, the transgene sequence also contains one or more non-coding, regulatory or control elements, such as a promoter, an enhancer, a post-transcriptional regulatory element (PRE), an intron, an insulator, a polyadenylation signal, a transcription termination sequence, a Kozak consensus sequence, a multicistronic element (e.g., internal ribosome entry sites (IRES), a 2A sequence), sequences corresponding to untranslated regions (UTR) of a messenger RNA (mRNA), and splice acceptor or donor sequences.

In some aspects, any portion of the transgene sequence to be integrated can be detected for determining the presence, absence or amount of the transgene sequence in any of the methods provided herein. In some aspects, the portion of the transgene sequence that is detected can be a portion that is within the sequence that is heterologous, exogenous or transgenic to the cell, such that integrated heterologous, exogenous or transgenic sequence can be specifically detected and distinguished from any similar endogenous genomic sequences of the cell. In some aspects, the method for determining the presence, absence or amount, such as any described herein, for example in Section I above, can be performed using probes that can specifically detect a portion of the transgene sequence, or primer sequences that can specifically amplify a portion of the transgene sequence. In some aspects, the probe or primer sequences can specifically detect, bind or recognize a portion of the transgene sequence, such as a portion of the transgene sequence that is heterologous, exogenous or transgenic to the cell. In some aspects, the probe or primer sequences can specifically detect, bind or recognize a portion of the transgene sequence, such as a portion of the transgene sequence that is integrated into the genome. In some embodiments, the transgene sequence does not encode a viral gag protein.

In some embodiments, the transgene sequence contains one or more promoter that is operatively linked to control expression of the encoded recombinant protein, e.g., recombinant receptor,. In some examples, the transgene sequence contains two, three, or more promoters operatively linked to control expression of the encoded recombinant protein. In some embodiments, transgene sequence can contain regulatory sequences, such as transcription and translation initiation and termination codons, which are specific to the type of host (e.g., bacterium, fungus, plant, or animal) into which the transgene sequence is to be introduced, as appropriate and taking into consideration whether the transgene sequence is DNA- or RNA-based. In some embodiments, the transgene sequence can contain regulatory/control elements, such as a promoter, an enhancer, a post-transcriptional regulatory element (PRE), an intron, a polyadenylation signal, a Kozak consensus sequence, internal ribosome entry sites (IRES), a 2A sequence, and splice acceptor or donor. In some embodiments, the transgene sequence can contain a nonnative promoter operably linked to the nucleotide sequence encoding the recombinant protein and/or one or more additional polypeptide(s). In some embodiments, the promoter is selected from among an RNA pol I, pol II or pol III promoter. In some embodiments, the promoter is recognized by RNA polymerase II (e.g., a CMV, SV40 early region or adenovirus major late promoter). In another embodiment, the promoter is recognized by RNA polymerase III (e.g., a U6 or H1 promoter). In some embodiments, the promoter can be a non-viral promoter or a viral promoter, such as a cytomegalovirus (CMV) promoter, an SV40 promoter, an RSV promoter, and a promoter found in the long-terminal repeat of the murine stem cell virus. Other known promoters also are contemplated.

In some embodiments, the promoter is or comprises a constitutive promoter. Exemplary constitutive promoters include, e.g., simian virus 40 early promoter (SV40), cytomegalovirus immediate-early promoter (CMV), human Ubiquitin C promoter (UBC), human elongation factor 1α promoter (EF1α), mouse phosphoglycerate kinase 1 promoter (PGK), and chicken β-Actin promoter coupled with CMV early enhancer (CAGG). In some embodiments, the constitutive promoter is a synthetic or modified promoter. In some embodiments, the promoter is or comprises an MND promoter, a synthetic promoter that contains the U3 region of a modified MoMuLV LTR with myeloproliferative sarcoma virus enhancer (see Challita et al. (1995) J. Virol. 69(2):748-755). In some embodiments, the promoter is a tissue-specific promoter. In another embodiment, the promoter is a viral promoter. In another embodiment, the promoter is a non-viral promoter. In some embodiments, exemplary promoters can include, but are not limited to, human elongation factor 1 alpha (EF1α) promoter or a modified form thereof or the MND promoter.

In some embodiments, the promoter is a regulated promoter (e.g., inducible promoter). In some embodiments, the promoter is an inducible promoter or a repressible promoter. In some embodiments, the promoter comprises a Lac operator sequence, a tetracycline operator sequence, a galactose operator sequence or a doxycycline operator sequence, or is an analog thereof or is capable of being bound by or recognized by a Lac repressor or a tetracycline repressor, or an analog thereof. In some embodiments, the transgene sequence does not include a regulatory element, e.g. promoter.

In some aspects, the transgene sequence contains a regulatory element that can enhance the expression of the encoded recombinant protein, such as a post-transcriptional regulatory element (PRE), such as cis-acting post-transcriptional regulatory elements. In some aspects, the transgene sequence contains a regulatory element that can enhance the expression of the encoded recombinant protein, such as a post-transcriptional regulatory element (PRE), operably linked, to the nucleotide sequence encoding the recombinant protein. In some embodiments, the regulatory element is a viral post-transcriptional regulatory element or a modified form thereof, such as a PRE derived from hepatitis viruses, such as the woodchuck hepatitis virus (WHV) or hepatitis B virus (HBV), for example, hepatitis post-transcriptional regulatory elements (HPREs), such as woodchuck hepatitis virus post-transcriptional regulatory element (WPRE), hepatitis B virus post-transcriptional regulatory element (HBVPRE). Hepatitis virus-derived PREs, including WPREs and HBVPREs, can generally promote, *e*.*g*., enhance, the expression of coding sequences operably linked thereto, by facilitating post-transcriptional RNA export from the nucleus. Secondary and tertiary structures formed by cis-acting sequences or elements contained within the PREs can promote such functions. For example, wild-type hepatitis virus-derived PREs generally include an alpha subelement and beta subelement, which each independently form stem loop structures, that affect and/or are involved in full PRE post-transcriptional activity (Smith et al. (1998) Nucleic Acids Research, 26:4818-4827). Some wild-type non-human hepatitis virus PREs, such as WPRE, also includes a gamma subelement that can further enhance post-transcriptional activity. Such subelements may have or encode RNAs having structures that promote RNA export from the nucleus, for example, via interaction with CRM1-dependent and/or independent export machinery, provide binding sites for cellular proteins, increase the total amount of RNA, *e*.*g*. recombinant and/or heterologous RNA, transcripts, increase RNA stability, increase the number of poly-adenylated transcripts and/or augment the size of the poly-adenylated tails in such transcripts. In some embodiments, the post-transcriptional regulatory element is a woodchuck hepatitis virus post-transcriptional regulatory element (WPRE).

In some cases, the nucleic acid sequence encoding the recombinant protein, e.g., recombinant receptor, contains a signal sequence that encodes a signal peptide. In some aspects, the signal sequence may encode a signal peptide derived from a native polypeptide. In other aspects, the signal sequence may encode a heterologous or non-native signal peptide, such as the exemplary signal peptide of the GMCSFR alpha chain set forth in SEQ ID NO:25 and encoded by the nucleotide sequence set forth in SEQ ID NO:24. In some cases, the nucleic acid sequence encoding the recombinant protein, e.g., recombinant receptor, such as a chimeric antigen receptor (CAR), contains a signal sequence that encodes a signal peptide. Non-limiting exemplary signal peptides include, for example, the GMCSFR alpha chain signal peptide set forth in SEQ ID NO: 25 and encoded by the nucleotide sequence set forth in SEQ ID NO:24, or the CD8 alpha signal peptide set forth in SEQ ID NO:26.

In some embodiments, the transgene sequence contains a nucleic acid sequence encoding one or more additional polypeptides, e.g., one or more marker(s) and/or one or more effector molecules. In some embodiments, the one or more marker(s) includes a transduction marker, a surrogate marker and/or a selection marker. Among additional nucleic acid sequences introduced, e.g., encoding for one or more additional polypeptide(s), include nucleic acid sequences that can improve the efficacy of therapy, such as by promoting viability and/or function of transferred cells; nucleic acid sequences to provide a genetic marker for selection and/or evaluation of the cells, such as to assess *in vivo* survival or localization; ucleic acid sequences to improve safety, for example, by making the cell susceptible to negative selection in vivo as described by Lupton S. D. et al., Mol. and Cell Biol., 11:6 (1991); and Riddell et al., Human Gene Therapy 3:319-338 (1992); see also WO 1992008796 and WO 1994028143 describing the use of bifunctional selectable fusion genes derived from fusing a dominant positive selectable marker with a negative selectable marker, and US Patent No. 6,040,177.

In some embodiments, the marker is a transduction marker or a surrogate marker. A transduction marker or a surrogate marker can be used to detect cells that have been introduced with the nucleic acid sequence sequence encoding a recombinant protein, e.g., recombinant receptor,. In some embodiments, the transduction marker can indicate or confirm modification of a cell. In some embodiments, the surrogate marker is a protein that is made to be co-expressed on the cell surface with the recombinant protein, e.g., recombinant receptor such as a CAR. In particular embodiments, such a surrogate marker is a surface protein that has been modified to have little or no activity. In certain embodiments, the surrogate marker is encoded on the same transgene sequence that encodes the recombinant protein, e.g., recombinant receptor. In some embodiments, the nucleic acid sequence encoding the recombinant protein, e.g., recombinant receptor, is operably linked to a nucleic acid sequence encoding a marker, optionally separated by an internal ribosome entry site (IRES), or a nucleic acid encoding a self-cleaving peptide or a peptide that causes ribosome skipping, such as a 2A sequence. Extrinsic marker genes may in some cases be utilized in connection with engineered cell to permit detection or selection of cells and, in some cases, also to promote cell elimination and/or cell suicide.

Exemplary surrogate markers can include truncated forms of cell surface polypeptides, such as truncated forms that are non-functional and to not transduce or are not capable of transducing a signal or a signal ordinarily transduced by the full-length form of the cell surface polypeptide, and/or do not or are not capable of internalizing. Exemplary truncated cell surface polypeptides including truncated forms of growth factors or other receptors such as a truncated human epidermal growth factor receptor 2 (tHER2), a truncated epidermal growth factor receptor (tEGFR, exemplary tEGFR sequence set forth in SEQ ID NO: 7 or 16) or a prostate-specific membrane antigen (PSMA) or modified form thereof, such as a truncated PSMA (tPSMA). In some aspects, tEGFR may contain an epitope recognized by the antibody cetuximab (Erbitux^{®}) or other therapeutic anti-EGFR antibody or binding molecule, which can be used to identify or select cells that have been engineered with the tEGFR construct and an encoded exogenous protein, and/or to eliminate or separate cells expressing the encoded exogenous protein. See U.S. Patent No. 8,802,374 and Liu et al., Nature Biotech. 2016 April; 34(4): 430-434). In some aspects, the marker, *e.g.* surrogate marker, includes all or part (*e.g*., truncated form) of CD34, a NGFR, a CD19 or a truncated CD19, e.g., a truncated non-human CD19. An exemplary polypeptide for a truncated EGFR (*e.g.* tEGFR) comprises the sequence of amino acids set forth in SEQ ID NO: 7 or 16 or a sequence of amino acids that exhibits at least 85%, 86%, 87%, 88%, 89%, 90%, 91%, 92%, 93%, 94%, 95%, 96%, 97%, 98%, 99% or more sequence identity to SEQ ID NO: 7 or 16.

In some embodiments, the marker is or comprises a detectable protein, such as a fluorescent protein, such as green fluorescent protein (GFP), enhanced green fluorescent protein (EGFP), such as super-fold GFP (sfGFP), red fluorescent protein (RFP), such as tdTomato, mCherry, mStrawberry, AsRed2, DsRed or DsRed2, cyan fluorescent protein (CFP), blue green fluorescent protein (BFP), enhanced blue fluorescent protein (EBFP), and yellow fluorescent protein (YFP), and variants thereof, including species variants, monomeric variants, codon-optimized, stabilized and/or enhanced variants of the fluorescent proteins. In some embodiments, the marker is or comprises an enzyme, such as a luciferase, the lacZ gene from *E*. *coli,* alkaline phosphatase, secreted embryonic alkaline phosphatase (SEAP), chloramphenicol acetyl transferase (CAT). Exemplary light-emitting reporter genes include luciferase (luc), β-galactosidase, chloramphenicol acetyltransferase (CAT), β-glucuronidase (GUS) or variants thereof. In some aspects, expression of the enzyme can be detected by addition of a substrate that can be detected upon the expression and functional activity of the enzyme.

In some embodiments, the marker is a selection marker. In some embodiments, the selection marker is or comprises a polypeptide that confers resistance to exogenous agents or drugs. In some embodiments, the selection marker is an antibiotic resistance gene. In some embodiments, the selection marker is an antibiotic resistance gene confers antibiotic resistance to a mammalian cell. In some embodiments, the selection marker is or comprises a Puromycin resistance gene, a Hygromycin resistance gene, a Blasticidin resistance gene, a Neomycin resistance gene, a Geneticin resistance gene or a Zeocin resistance gene or a modified form thereof.

Any of the recombinant protein, e.g., recombinant receptor, and/or the additional polypeptide(s) described herein can be encoded by one or more transgene sequences containing one or more nucleic acid sequences encoding, recombinant protein, e.g., recombinant receptor, in any combinations, orientation or arrangements. For example, one, two, three or more transgene sequences can encode one, two, three or more different polypeptides, e.g., recombinant receptor, or portions or components thereof, and/or one or more additional polypeptide(s), e.g., a marker and/or an effector molecule. In some embodiments, one transgene sequence contains a nucleic acid sequence encoding a recombinant protein, e.g., recombinant receptor such as a CAR, or portion or components thereof, and a nucleic acid sequence encoding one or more additional polypeptide(s). In some embodiments, one vector or construct contains a nucleic acid sequence encoding a recombinant protein, e.g., recombinant receptor such as a CAR, or portion or components thereof, and a separate vector or construct contains a nucleic acid sequence encoding one or more additional polypeptide(s). In some embodiments, the nucleic acid sequence encoding the recombinant protein, e.g., recombinant receptor, and the nucleic acid sequence encoding the one or more additional polypeptide(s) are operably linked to two different promoters. In some embodiments, the nucleic acid encoding the recombinant protein, e.g., recombinant receptor, is present upstream of the nucleic acid encoding the one or more additional polypeptide(s). In some embodiments, the nucleic acid encoding the recombinant protein, e.g., recombinant receptor, is present downstream of the nucleic acid encoding one or more additional polypeptide(s).

In certain cases, one transgene sequence contains nucleic acid sequences encode two or more different polypeptide chains, e.g., a recombinant protein, e.g., recombinant receptor, and one or more additional polypeptide(s), e.g., a marker and/or an effector molecule. In some embodiments, the nucleic acid sequences encoding two or more different polypeptide chains, e.g., a recombinant proteins, e.g., recombinant receptor, and one or more additional polypeptide(s), are present in two separate transgene sequences. For example, two separate transgene sequences are provided, and each can be individually transferred or introduced into the cell for expression in the cell. In some embodiments, the nucleic acid sequences encoding the marker and the nucleic acid sequences encoding the recombinant protein, e.g., recombinant receptor, are present or inserted at different locations within the genome of the cell. In some embodiments, the nucleic acid sequences encoding the marker and the nucleic acid sequences encoding the recombinant protein, e.g., recombinant receptor, are operably linked to two different promoters.

In some embodiments, such as those where the transgene sequence contains a first and second nucleic acid sequence, the coding sequences encoding each of the different polypeptide chains can be operatively linked to a promoter, which can be the same or different. In some embodiments, the nucleic acid molecule can contain a promoter that drives the expression of two or more different polypeptide chains. In some embodiments, such nucleic acid molecules can be multicistronic (bicistronic or tricistronic, see *e.g.,* U.S. Patent No. 6,060,273). In some embodiments, the nucleic acid sequences encoding the recombinant protein, e.g., recombinant receptor, and the nucleic acid sequences encoding the one or more additional polypeptide(s) are operably linked to the same promoter and are optionally separated by an internal ribosome entry site (IRES), or a nucleic acid encoding a self-cleaving peptide or a peptide that causes ribosome skipping, such as a 2A element. For example, an exemplary marker, and optionally a ribosome skipping sequence sequence, can be any as disclosed in PCT Pub. No. WO2014031687.

In some embodiments, transcription units can be engineered as a bicistronic unit containing an IRES, which allows coexpression of gene products (*e*.*g*. encoding the recombinant protein, e.g., recombinant receptor, and the additional polypeptide) by a message from a single promoter. Alternatively, in some cases, a single promoter may direct expression of an RNA that contains, in a single open reading frame (ORF), two or three genes (e.g. encoding the marker and encoding the recombinant protein, e.g., recombinant receptor,) separated from one another by sequences encoding a self-cleavage peptide (e.g., 2A sequences) or a protease recognition site (e.g., furin). The ORF thus encodes a single polypeptide, which, either during (in the case of 2A) or after translation, is processed into the individual proteins. In some cases, the peptide, such as a T2A, can cause the ribosome to skip (ribosome skipping) synthesis of a peptide bond at the C-terminus of a 2A element, leading to separation between the end of the 2A sequence and the next peptide downstream (see, e.g., de Felipe, Genetic Vaccines and Ther. 2:13 (2004) and de Felipe et al. Traffic 5:616-626 (2004)). Various 2A elements are known. Examples of 2A sequences that can be used in the methods and system disclosed herein, without limitation, 2A sequences from the foot-and-mouth disease virus (F2A, *e.g.,* SEQ ID NO: 21), equine rhinitis A virus (E2A, *e.g.,* SEQ ID NO: 20), Thosea asigna virus (T2A, *e.g.,* SEQ ID NO: 6 or 17), and porcine teschovirus-1 (P2A, *e.g.,* SEQ ID NO: 18 or 19) as described in U.S. Patent Pub. No. 20070116690.

### A. Recombinant Receptors

In some embodiments, the cells or cell compositions that are assessed for the integration of transgene sequences, contain transgene sequences encoding a recombinant protein. In some embodiments, the transgene sequences include nucleic acid sequences encoding a recombinant receptor, and other elements as described above. In some embodiments, the recombinant protein encoded by the integrated transgene sequences is a recombinant receptor. In some aspects, the transgene sequences encode a recombinant receptor or a portion thereof. In some embodiments, the cells that are treated, processed, engineered, and/or produced as described herein, e.g., in Section I, contain or express, or are engineered to contain or express, a recombinant protein, such as a recombinant receptor, e.g., a chimeric antigen receptor (CAR), or a T cell receptor (TCR). In certain embodiments, the methods for manufacturing or engineering described produce and/or are capable of producing cells, or populations or compositions containing and/or enriched for cells, that are engineered to express or contain a recombinant protein such as a recombinant receptor, by virtue of integration of the transgene sequences. In some embodiments, T cells, or populations or compositions of T cells, are treated, processed, engineered, and/or produced.

In some aspects, the encoded recombinant receptor is a chimeric antigen receptor (CAR) or a recombinant T cell receptor (TCR). Among the recombinant receptors are chimeric receptors, antigen receptors and receptors containing one or more component of chimeric receptors or antigen receptors. The recombinant receptors may include those containing ligand-binding domains or binding fragments thereof and intracellular signaling domains or regions. In some embodiments, the recombinant receptors encoded by the engineered cells include functional non-TCR antigen receptors, chimeric antigen receptors (CARs), chimeric autoantibody receptor (CAAR), recombinant T cell receptors (TCRs) and regions, domains or components of any of the foregoing, including one or more polypeptide chains of a multi-chain recombinant receptor. The recombinant receptor, such as a CAR, generally includes the extracellular antigen (or ligand) binding domain linked to one or more intracellular signaling components, in some aspects via linkers and/or transmembrane domain(s). In some embodiments, exemplary recombinant receptors expressed from the engineered cell include multi-chain receptors that contain two or more receptor polypeptides, which, in some cases, contain different components, domains or regions. In some aspects, the recombinant receptor contains two or more polypeptides that together comprise a functional recombinant receptor. In some aspects, the multi-chain receptor is a dual-chain receptor, comprising two polypeptides that together comprise a functional recombinant receptor. In some embodiments, the recombinant receptor is a TCR comprising two different receptor polypeptides, for example, a TCR alpha (TCRα) and a TCR beta (TCRβ) chain; or a TCR gamma (TCRγ) and a TCR delta (TCRδ) chain. In some embodiments, the recombinant receptor is a multi-chain receptor in which one or more of the polypeptides regulates, modifies or controls the expression, activity or function of another receptor polypeptide. In some aspects, multi-chain receptors allows spatial or temporal regulation or control of specificity, activity, antigen (or ligand) binding, function and/or expression of the receptor.

### 1. Chimeric Antigen Receptors (CARs)

In some embodiments, the encoded recombinant receptor is a chimeric antigen receptor (CAR) with specificity for a particular antigen (or marker or ligand), such as an antigen expressed on the surface of a particular cell type. In some embodiments, the antigen is a polypeptide. In some embodiments, it is a carbohydrate or other molecule. In some embodiments, the antigen is selectively expressed or overexpressed on cells of the disease or condition, e.g., the tumor or pathogenic cells, as compared to normal or non-targeted cells or tissues. In other embodiments, the antigen is expressed on normal cells and/or is expressed on the engineered cells.

In particular embodiments, the recombinant receptor, such as chimeric receptor, contains an intracellular signaling region, which includes a cytoplasmic signaling domain or region (also interchangeably called an intracellular signaling domain or region), such as a cytoplasmic (intracellular) region capable of inducing a primary activation signal in a T cell, for example, a cytoplasmic signaling domain or region of a T cell receptor (TCR) component (e.g. a cytoplasmic signaling domain or region of a zeta chain of a CD3-zeta (CD3ζ) chain or a functional variant or signaling portion thereof) and/or that comprises an immunoreceptor tyrosine-based activation motif (ITAM).

In some embodiments, the chimeric receptor further contains an extracellular ligand-binding domain that specifically binds to a ligand (e.g. antigen) antigen. In some embodiments, the chimeric receptor is a CAR that contains an extracellular antigen-recognition domain that specifically binds to an antigen. In some embodiments, the ligand, such as an antigen, is a protein expressed on the surface of cells. In some embodiments, the CAR is a TCR-like CAR and the antigen is a processed peptide antigen, such as a peptide antigen of an intracellular protein, which, like a TCR, is recognized on the cell surface in the context of a major histocompatibility complex (MHC) molecule.

Exemplary antigen receptors, including CARs, and methods for engineering and introducing such receptors into cells, include those described, for example, in international patent application publication numbers WO200014257, WO2013126726, WO2012/129514, WO2014031687, WO2013/166321, WO2013/071154, WO2013/123061, U.S. patent application publication numbers US2002131960, US2013287748, US20130149337, U.S. Patent Nos.: 6,451,995, 7,446,190, 8,252,592, 8,339,645, 8,398,282, 7,446,179, 6,410,319, 7,070,995, 7,265,209, 7,354,762, 7,446,191, 8,324,353, and 8,479,118, and European patent application number EP2537416, and/or those described by Sadelain et al., Cancer Discov. 2013 April; 3(4): 388-398; Davila et al. (2013) PLoS ONE 8(4): e61338; Turtle et al., Curr. Opin. Immunol., 2012 October; 24(5): 633-39; Wu et al., Cancer, 2012 March 18(2): 160-75. In some aspects, the antigen receptors include a CAR as described in U.S. Patent No.: 7,446,190, and those described in International Patent Application Publication No.: WO/2014055668 A1. Examples of the CARs include CARs as disclosed in any of the aforementioned publications, such as WO2014031687, US 8,339,645, US 7,446,179, US 2013/0149337, U.S. Patent No.: 7,446,190, US Patent No.: 8,389,282, Kochenderfer et al., 2013, Nature Reviews Clinical Oncology, 10, 267-276 (2013); Wang et al. (2012) J. Immunother. 35(9): 689-701; and Brentjens et al., Sci Transl Med. 2013 5(177). See also WO2014031687, US 8,339,645, US 7,446,179, US 2013/0149337, U.S. Patent No.: 7,446,190, and US Patent No.: 8,389,282.

In some embodiments, the CAR is constructed with a specificity for a particular antigen (or marker or ligand), such as an antigen expressed in a particular cell type to be targeted by adoptive therapy, *e.g.,* a cancer marker, and/or an antigen intended to induce a dampening response, such as an antigen expressed on a normal or non-diseased cell type. Thus, the CAR typically includes in its extracellular portion one or more antigen binding molecules, such as one or more antigen-binding fragment, domain, or portion, or one or more antibody variable domains, and/or antibody molecules. In some embodiments, the CAR includes an antigen-binding portion or portions of an antibody molecule, such as a single-chain antibody fragment (scFv) derived from the variable heavy (V_{H}) and variable light (V_{L}) chains of a monoclonal antibody (mAb).

In some embodiments, the antibody or antigen-binding portion thereof is expressed on cells as part of a recombinant receptor, such as an antigen receptor. Among the antigen receptors are functional non-TCR antigen receptors, such as chimeric antigen receptors (CARs). Generally, a CAR containing an antibody or antigen-binding fragment that exhibits TCR-like specificity directed against peptide-MHC complexes also may be referred to as a TCR-like CAR. In some embodiments, the extracellular antigen binding domain specific for an MHC-peptide complex of a TCR-like CAR is linked to one or more intracellular signaling components, in some aspects via linkers and/or transmembrane domain(s). In some embodiments, such molecules can typically mimic or approximate a signal through a natural antigen receptor, such as a TCR, and, optionally, a signal through such a receptor in combination with a costimulatory receptor.

In some embodiments, the recombinant receptor, such as a chimeric receptor (e.g. CAR), includes a ligand-binding domain that binds, such as specifically binds, to an antigen (or a ligand). Among the antigens targeted by the chimeric receptors are those expressed in the context of a disease, condition, or cell type to be targeted via the adoptive cell therapy. Among the diseases and conditions are proliferative, neoplastic, and malignant diseases and disorders, including cancers and tumors, including hematologic cancers, cancers of the immune system, such as lymphomas, leukemias, and/or myelomas, such as B, T, and myeloid leukemias, lymphomas, and multiple myelomas.

In some embodiments, the antigen (or a ligand) is a polypeptide. In some embodiments, it is a carbohydrate or other molecule. In some embodiments, the antigen (or a ligand) is selectively expressed or overexpressed on cells of the disease or condition, *e*.*g*., the tumor or pathogenic cells, as compared to normal or non-targeted cells or tissues. In other embodiments, the antigen is expressed on normal cells and/or is expressed on the engineered cells.

In some embodiments, the CAR contains an antibody or an antigen-binding fragment (*e.g*. scFv) that specifically recognizes an antigen, such as an intact antigen, expressed on the surface of a cell.

In some embodiments, the antigen (or a ligand) is a tumor antigen or cancer marker. In some embodiments, the antigen (or a ligand) the antigen is or includes αvβ6 integrin (avb6 integrin), B cell maturation antigen (BCMA), B7-H3, B7-H6, carbonic anhydrase 9 (CA9, also known as CAIX or G250), a cancer-testis antigen, cancer/testis antigen 1B (CTAG, also known as NY-ESO-1 and LAGE-2), carcinoembryonic antigen (CEA), a cyclin, cyclin A2, C-C Motif Chemokine Ligand 1 (CCL-1), CD19, CD20, CD22, CD23, CD24, CD30, CD33, CD38, CD44, CD44v6, CD44v7/8, CD123, CD133, CD138, CD171, chondroitin sulfate proteoglycan 4 (CSPG4), epidermal growth factor protein (EGFR), type III epidermal growth factor receptor mutation (EGFR vIII), epithelial glycoprotein 2 (EPG-2), epithelial glycoprotein 40 (EPG-40), ephrinB2, ephrin receptor A2 (EPHa2), estrogen receptor, Fc receptor like 5 (FCRL5; also known as Fc receptor homolog 5 or FCRH5), fetal acetylcholine receptor (fetal AchR), a folate binding protein (FBP), folate receptor alpha, ganglioside GD2, O-acetylated GD2 (OGD2), ganglioside GD3, glycoprotein 100 (gp100), glypican-3 (GPC3), G protein-coupled receptor class C group 5 member D (GPRC5D), Her2/neu (receptor tyrosine kinase erb-B2), Her3 (erb-B3), Her4 (erb-B4), erbB dimers, Human high molecular weight-melanoma-associated antigen (HMW-MAA), hepatitis B surface antigen, Human leukocyte antigen A1 (HLA-A1), Human leukocyte antigen A2 (HLA-A2), IL-22 receptor alpha(IL-22Rα), IL-13 receptor alpha 2 (IL-13Rα2), kinase insert domain receptor (kdr), kappa light chain, L1 cell adhesion molecule (L1-CAM), CE7 epitope of L1-CAM, Leucine Rich Repeat Containing 8 Family Member A (LRRC8A), Lewis Y, Melanoma-associated antigen (MAGE)-A1, MAGE-A3, MAGE-A6, MAGE-A10, mesothelin (MSLN), c-Met, murine cytomegalovirus (CMV), mucin 1 (MUC1), MUC16, natural killer group 2 member D (NKG2D) ligands, melan A (MART-1), neural cell adhesion molecule (NCAM), oncofetal antigen, Preferentially expressed antigen of melanoma (PRAME), progesterone receptor, a prostate specific antigen, prostate stem cell antigen (PSCA), prostate specific membrane antigen (PSMA), Receptor Tyrosine Kinase Like Orphan Receptor 1 (ROR1), survivin, Trophoblast glycoprotein (TPBG also known as 5T4), tumor-associated glycoprotein 72 (TAG72), Tyrosinase related protein 1 (TRP1, also known as TYRP1 or gp75), Tyrosinase related protein 2 (TRP2, also known as dopachrome tautomerase, dopachrome delta-isomerase or DCT), vascular endothelial growth factor receptor (VEGFR), vascular endothelial growth factor receptor 2 (VEGFR2), Wilms Tumor 1 (WT-1), a pathogen-specific or pathogen-expressed antigen, or an antigen associated with a universal tag, and/or biotinylated molecules, and/or molecules expressed by HIV, HCV, HBV or other pathogens. Antigens targeted by the receptors in some embodiments include antigens associated with a B cell malignancy, such as any of a number of known B cell marker. In some embodiments, the antigen is or includes CD20, CD19, CD22, ROR1, CD45, CD21, CD5, CD33, Igkappa, Iglambda, CD79a, CD79b or CD30.

In some embodiments, the CAR is an anti-BCMA CAR that is specific for BCMA, e.g. human BCMA. Chimeric antigen receptors containing anti-BCMA antibodies, including mouse anti-human BCMA antibodies and human anti-human antibodies, and cells expressing such chimeric receptors have been previously described. See Carpenter et al., Clin Cancer Res., 2013, 19(8):2048-2060, WO 2016/090320, WO2016090327, WO2010104949A2 and WO2017173256. In some embodiments, the anti-BCMA CAR contains an antigen-binding domain, such as an scFv, containing a variable heavy (V_{H}) and/or a variable light (V_{L}) region derived from an antibody described in WO 2016/090320 or WO2016090327. In some embodiments, the antigen-binding domain, such as an scFv, contains a V_{H} set forth in SEQ ID NO: 30 and a V_{L} set forth in SEQ ID NO:31. In some embodiments, the antigen-binding domain, such as an scFv, contains a V_{H} set forth in SEQ ID NO: 32 and a V_{L} set forth in SEQ ID NO:33. In some embodiments, the antigen-binding domain, such as an scFv, contains a VH set forth in SEQ ID NO: 34 and a V_{L} set forth in SEQ ID NO: 35. In some embodiments, the antigen-binding domain, such as an scFv, contains a V_{H} set forth in SEQ ID NO: 27 and a V_{L} set forth in SEQ ID NO:28. In some embodiment the antigen-binding domain, such as an scFv, contains a V_{H} set forth in SEQ ID NO: 41 and a V_{L} set forth in SEQ ID NO: 42. In some embodiments, the antigen-binding domain, such as an scFv, contains a V_{H} set forth in SEQ ID NO: 43 and a V_{L} set forth in SEQ ID NO: 44. In some embodiments, the antigen-binding domain, such as an scFv, contains a V_{H} set forth in SEQ ID NO: 45 and a V_{L} set forth in SEQ ID NO: 46. In some embodiments, the V_{H} or V_{L} has a sequence of amino acids that exhibits at least 85%, 86%, 87%, 88%, 89%, 90%, 91%, 92%, 93%, 94%, 95%, 96%, 97%, 98%, 99% or more sequence identity to any of the foregoing V_{H} or V_{L} sequences, and retains binding to BCMA. In some embodiments, the V_{H} region is amino-terminal to the V_{L} region. In some embodiments, the V_{H} region is carboxy-terminal to the V_{L} region.

In some embodiments, the CAR is an anti-CD19 CAR that is specific for CD19, e.g. human CD19. In some embodiments the scFv and/or V_{H} domains is derived from FMC63. FMC63 generally refers to a mouse monoclonal IgG1 antibody raised against Nalm-1 and -16 cells expressing CD19 of human origin (Ling, N. R., et al. (1987). Leucocyte typing III. 302). In some embodiments, the FMC63 antibody comprises a CDR-H1 and a CDR-H2 set forth in SEQ ID NOS: 50 and 51 respectively, and a CDR-H3 set forth in SEQ ID NO: 52 or 66 and a CDR-L1 set forth in SEQ ID NO: 47 and a CDR-L2 set forth in SEQ ID NO: 48 or 67 and a CDR-L3 sequences set forth in SEQ ID NO: 49 or 68. In some embodiments, the FMC63 antibody comprises a heavy chain variable region (V_{H}) comprising the amino acid sequence of SEQ ID NO: 53 and a light chain variable region (V_{L}) comprising the amino acid sequence of SEQ ID NO: 54.

In some embodiments, the scFv comprises a variable light chain containing a CDR-L1 sequence of SEQ ID NO:47, a CDR-L2 sequence of SEQ ID NO:48, and a CDR-L3 sequence of SEQ ID NO:49 and/or a variable heavy chain containing a CDR-H1 sequence of SEQ ID NO:50, a CDR-H2 sequence of SEQ ID NO:51, and a CDR-H3 sequence of SEQ ID NO:52. In some embodiments, the scFv comprises a variable heavy chain region set forth in SEQ ID NO:53 and a variable light chain region set forth in SEQ ID NO:54. In some embodiments, the variable heavy and variable light chains are connected by a linker. In some embodiments, the linker is set forth in SEQ ID NO:29. In some embodiments, the scFv comprises, in order, a V_{H}, a linker, and a V_{L}. In some embodiments, the scFv comprises, in order, a V_{L}, a linker, and a V_{H}. In some embodiments, the scFv is encoded by a sequence of nucleotides set forth in SEQ ID NO:69 or a sequence that exhibits at least 85%, 86%, 87%, 88%, 89%, 90%, 91%, 92%, 93%, 94%, 95%, 96%, 97%, 98%, or 99% sequence identity to SEQ ID NO:69. In some embodiments, the scFv comprises the sequence of amino acids set forth in SEQ ID NO:55 or a sequence that exhibits at least 85%, 86%, 87%, 88%, 89%, 90%, 91%, 92%, 93%, 94%, 95%, 96%, 97%, 98%, or 99% sequence identity to SEQ ID NO:55.

In some embodiments the scFv is derived from SJ25C1. SJ25C1 is a mouse monoclonal IgG1 antibody raised against Nalm-1 and -16 cells expressing CD19 of human origin (Ling, N. R., et al. (1987). Leucocyte typing III. 302). In some embodiments, the SJ25C1 antibody comprises a CDR-H1, a CDR-H2 and a CDR-H3 sequence set forth in SEQ ID NOS: 59-61, respectively, and a CDR-L1, a CDR-L2 and a CDR-L3 sequence set forth in SEQ ID NOS: 56-58, respectively. In some embodiments, the SJ25C1 antibody comprises a heavy chain variable region (V_{H}) comprising the amino acid sequence of SEQ ID NO: 62 and a light chain variable region (V_{L}) comprising the amino acid sequence of SEQ ID NO: 63. In some embodiments, the svFv comprises a variable light chain containing a CDR-L1 sequence of SEQ ID NO:56, a CDR-L2 sequence of SEQ ID NO: 57, and a CDR-L3 sequence of SEQ ID NO:58 and/or a variable heavy chain containing a CDR-H1 sequence of SEQ ID NO:59, a CDR-H2 sequence of SEQ ID NO:60, and a CDR-H3 sequence of SEQ ID NO:61. In some embodiments, the scFv comprises a variable heavy chain region set forth in SEQ ID NO:62 and a variable light chain region set forth in SEQ ID NO:63. In some embodiments, the variable heavy and variable light chain are connected by a linker. In some embodiments, the linker is set forth in SEQ ID NO:64. In some embodiments, the scFv comprises, in order, a V_{H}, a linker, and a V_{L}. In some embodiments, the scFv comprises, in order, a V_{L}, a linker, and a V_{H}. In some embodiments, the scFv comprises the sequence of amino acids set forth in SEQ ID NO:65 or a sequence that exhibits at least 85%, 86%, 87%, 88%, 89%, 90%, 91%, 92%, 93%, 94%, 95%, 96%, 97%, 98%, or 99% sequence identity to SEQ ID NO:65.

In some embodiments, the CAR is an anti-CD20 CAR that is specific for CD20. In some embodiments, the scFv contains a V_{H} and a V_{L} derived from an antibody or an antibody fragment specific to CD20. In some embodiments, the antibody or antibody fragment that binds CD20 is an antibody that is or is derived from Rituximab, such as is Rituximab scFv.

In some embodiments, the CAR is an anti-CD22 CAR that is specific for CD22. In some embodiments, the scFv contains a V_{H} and a V_{L} derived from an antibody or an antibody fragment specific to CD22. In some embodiments, the antibody or antibody fragment that binds CD22 is an antibody that is or is derived from m971, such as is m971 scFv.

In some embodiments, the CAR is an anti-GPRC5D CAR that is specific for GPRC5D. In some embodiments, the scFv contains a V_{H} and a V_{L} derived from an antibody or an antibody fragment specific to GPRC5D. In some embodiments, the antibody or antibody fragment that binds GPRC5D is or contains a V_{H} and a V_{L} from an antibody or antibody fragment set forth in International Patent Applications, Publication Number WO 2016/090329 and WO 2016/090312.

In some embodiments, the antibody is an antigen-binding fragment, such as a scFv, that includes one or more linkers joining two antibody domains or regions, such as a heavy chain variable (V_{H}) region and a light chain variable (V_{L}) region. The linker typically is a peptide linker, e.g., a flexible and/or soluble peptide linker. Among the linkers are those rich in glycine and serine and/or in some cases threonine. In some embodiments, the linkers further include charged residues such as lysine and/or glutamate, which can improve solubility. In some embodiments, the linkers further include one or more proline. In some aspects, the linkers rich in glycine and serine (and/or threonine) include at least 80%, 85%, 90%, 91%, 92%, 93%, 94%, 95%, 96%, 97%, 98%, or 99% such amino acid(s). In some embodiments, they include at least at or about 50%, 55%, 60%, 70%, or 75%, glycine, serine, and/or threonine. In some embodiments, the linker is comprised substantially entirely of glycine, serine, and/or threonine. The linkers generally are between about 5 and about 50 amino acids in length, typically between at or about 10 and at or about 30, e.g., 10, 11, 12, 13, 14, 15, 16, 17, 18, 19, 20, 21, 22, 23, 24, 25, 26, 27, 28, 29, or 30, and in some examples between 10 and 25 amino acids in length. Exemplary linkers include linkers having various numbers of repeats of the sequence GGGGS (4GS; SEQ ID NO:36) or GGGS (3GS; SEQ ID NO:37), such as between 2, 3, 4, and 5 repeats of such a sequence. Exemplary linkers include those having or consisting of an sequence set forth in SEQ ID NO:38 (GGGGSGGGGSGGGGS), SEQ ID NO:39 (GSTSGSGKPGSGEGSTKG) or SEQ ID NO: 40 (SRGGGGSGGGGSGGGGSLEMA).

In some embodiments, the antigen is or includes a pathogen-specific or pathogen-expressed antigen. In some embodiments, the antigen is a viral antigen (such as a viral antigen from HIV, HCV, HBV, etc.), bacterial antigens, and/or parasitic antigens. In some embodiments, the CAR contains a TCR-like antibody, such as an antibody or an antigen-binding fragment (*e*.*g*. scFv) that specifically recognizes an intracellular antigen, such as a tumor-associated antigen, presented on the cell surface as a MHC-peptide complex. In some embodiments, an antibody or antigen-binding portion thereof that recognizes an MHC-peptide complex can be expressed on cells as part of a recombinant receptor, such as an antigen receptor. Among the antigen receptors are functional non-TCR antigen receptors, such as chimeric antigen receptors (CARs). Generally, a CAR containing an antibody or antigen-binding fragment that exhibits TCR-like specificity directed against peptide-MHC complexes also may be referred to as a TCR-like CAR.

Reference to "Major histocompatibility complex" (MHC) refers to a protein, generally a glycoprotein, that contains a polymorphic peptide binding site or binding groove that can, in some cases, complex with peptide antigens of polypeptides, including peptide antigens processed by the cell machinery. In some cases, MHC molecules can be displayed or expressed on the cell surface, including as a complex with peptide, *i.e.* MHC-peptide complex, for presentation of an antigen in a conformation recognizable by an antigen receptor on T cells, such as a TCRs or TCR-like antibody. Generally, MHC class I molecules are heterodimers having a membrane spanning α chain, in some cases with three α domains, and a non-covalently associated β2 microglobulin. Generally, MHC class II molecules are composed of two transmembrane glycoproteins, α and β, both of which typically span the membrane. An MHC molecule can include an effective portion of an MHC that contains an antigen binding site or sites for binding a peptide and the sequences necessary for recognition by the appropriate antigen receptor. In some embodiments, MHC class I molecules deliver peptides originating in the cytosol to the cell surface, where a MHC-peptide complex is recognized by T cells, such as generally CD8⁺ T cells, but in some cases CD4+ T cells. In some embodiments, MHC class II molecules deliver peptides originating in the vesicular system to the cell surface, where they are typically recognized by CD4⁺ T cells. Generally, MHC molecules are encoded by a group of linked loci, which are collectively termed H-2 in the mouse and human leukocyte antigen (HLA) in humans. Hence, typically human MHC can also be referred to as human leukocyte antigen (HLA).

The term "MHC-peptide complex" or "peptide-MHC complex" or variations thereof, refers to a complex or association of a peptide antigen and an MHC molecule, such as, generally, by non-covalent interactions of the peptide in the binding groove or cleft of the MHC molecule. In some embodiments, the MHC-peptide complex is present or displayed on the surface of cells. In some embodiments, the MHC-peptide complex can be specifically recognized by an antigen receptor, such as a TCR, TCR-like CAR or antigen-binding portions thereof.

In some embodiments, a peptide, such as a peptide antigen or epitope, of a polypeptide can associate with an MHC molecule, such as for recognition by an antigen receptor. Generally, the peptide is derived from or based on a fragment of a longer biological molecule, such as a polypeptide or protein. In some embodiments, the peptide typically is about 8 to about 24 amino acids in length. In some embodiments, a peptide has a length of from or from about 9 to 22 amino acids for recognition in the MHC Class II complex. In some embodiments, a peptide has a length of from or from about 8 to 13 amino acids for recognition in the MHC Class I complex. In some embodiments, upon recognition of the peptide in the context of an MHC molecule, such as MHC-peptide complex, the antigen receptor, such as TCR or TCR-like CAR, produces or triggers an activation signal to the T cell that induces a T cell response, such as T cell proliferation, cytokine production, a cytotoxic T cell response or other response.

In some embodiments, a TCR-like antibody or antigen-binding portion, are known or can be produced by known methods (see e.g. US Published Application Nos. US 2002/0150914; US 2003/0223994; US 2004/0191260; US 2006/0034850; US 2007/00992530; US20090226474; US20090304679; and International PCT Publication No. WO 03/068201).

In some embodiments, an antibody or antigen-binding portion thereof that specifically binds to a MHC-peptide complex, can be produced by immunizing a host with an effective amount of an immunogen containing a specific MHC-peptide complex. In some cases, the peptide of the MHC-peptide complex is an epitope of antigen capable of binding to the MHC, such as a tumor antigen, for example a universal tumor antigen, myeloma antigen or other antigen as described below. In some embodiments, an effective amount of the immunogen is then administered to a host for eliciting an immune response, wherein the immunogen retains a three-dimensional form thereof for a period of time sufficient to elicit an immune response against the three-dimensional presentation of the peptide in the binding groove of the MHC molecule. Serum collected from the host is then assayed to determine if desired antibodies that recognize a three-dimensional presentation of the peptide in the binding groove of the MHC molecule is being produced. In some embodiments, the produced antibodies can be assessed to confirm that the antibody can differentiate the MHC-peptide complex from the MHC molecule alone, the peptide of interest alone, and a complex of MHC and irrelevant peptide. The desired antibodies can then be isolated.

In some embodiments, an antibody or antigen-binding portion thereof that specifically binds to an MHC-peptide complex can be produced by employing antibody library display methods, such as phage antibody libraries. In some embodiments, phage display libraries of mutant Fab, scFv or other antibody forms can be generated, for example, in which members of the library are mutated at one or more residues of a CDR or CDRs. See *e.g.* US published application No. US20020150914, US2014/0294841; and Cohen CJ. et al. (2003) J Mol. Recogn. 16:324-332.

The term "antibody" herein is used in the broadest sense and includes polyclonal and monoclonal antibodies, including intact antibodies and functional (antigen-binding) antibody fragments, including fragment antigen binding (Fab) fragments, F(ab')₂ fragments, Fab' fragments, Fv fragments, recombinant IgG (rIgG) fragments, variable heavy chain (V_{H}) regions capable of specifically binding the antigen, single chain antibody fragments, including single chain variable fragments (scFv), and single domain antibodies (*e*.*g*., sdAb, sdFv, nanobody) fragments. The term encompasses genetically engineered and/or otherwise modified forms of immunoglobulins, such as intrabodies, peptibodies, chimeric antibodies, fully human antibodies, humanized antibodies, and heteroconjugate antibodies, multispecific, *e*.*g*., bispecific, antibodies, diabodies, triabodies, and tetrabodies, tandem di-scFv, tandem tri-scFv. Unless otherwise stated, the term "antibody" should be understood to encompass functional antibody fragments thereof. The term also encompasses intact or full-length antibodies, including antibodies of any class or sub-class, including IgG and sub-classes thereof, IgM, IgE, IgA, and IgD.

The terms "complementarity determining region," and "CDR," synonymous with "hypervariable region" or "HVR," are known, in some cases, to refer to non-contiguous sequences of amino acids within antibody variable regions, which confer antigen specificity and/or binding affinity. In general, there are three CDRs in each heavy chain variable region (CDR-H1, CDR-H2, CDR-H3) and three CDRs in each light chain variable region (CDR-L1, CDR-L2, CDR-L3). "Framework regions" and "FR" are known, in some cases,to refer to the non-CDR portions of the variable regions of the heavy and light chains. In general, there are four FRs in each full-length heavy chain variable region (FR-H1, FR-H2, FR-H3, and FR-H4), and four FRs in each full-length light chain variable region (FR-L1, FR-L2, FR-L3, and FR-L4).

The precise amino acid sequence boundaries of a given CDR or FR can be readily determined using any of a number of well-known schemes, including those described by Kabat et al. (1991), "Sequences of Proteins of Immunological Interest," 5th Ed. Public Health Service, National Institutes of Health, Bethesda, MD ("Kabat" numbering scheme); Al-Lazikani et al., (1997) JMB 273,927-948 ("Chothia" numbering scheme); MacCallum et al., J. Mol. Biol. 262:732-745 (1996), "Antibody-antigen interactions: Contact analysis and binding site topography," J. Mol. Biol. 262, 732-745." ("Contact" numbering scheme); Lefranc MP et al., "IMGT unique numbering for immunoglobulin and T cell receptor variable domains and Ig superfamily V-like domains," Dev Comp Immunol, 2003 Jan;27(1):55-77 ("IMGT" numbering scheme); Honegger A and Plückthun A, "Yet another numbering scheme for immunoglobulin variable domains: an automatic modeling and analysis tool," J Mol Biol, 2001 Jun 8;309(3):657-70, ("Aho" numbering scheme); and Martin et al., "Modeling antibody hypervariable loops: a combined algorithm," PNAS, 1989, 86(23):9268-9272, ("AbM" numbering scheme).

The boundaries of a given CDR or FR may vary depending on the scheme used for identification. For example, the Kabat scheme is based on structural alignments, while the Chothia scheme is based on structural information. Numbering for both the Kabat and Chothia schemes is based upon the most common antibody region sequence lengths, with insertions accommodated by insertion letters, for example, "30a," and deletions appearing in some antibodies. The two schemes place certain insertions and deletions ("indels") at different positions, resulting in differential numbering. The Contact scheme is based on analysis of complex crystal structures and is similar in many respects to the Chothia numbering scheme. The AbM scheme is a compromise between Kabat and Chothia definitions based on that used by Oxford Molecular's AbM antibody modeling software.

**Table 1,** below, lists exemplary position boundaries of CDR-L1, CDR-L2, CDR-L3 and CDR-H1, CDR-H2, CDR-H3 as identified by Kabat, Chothia, AbM, and Contact schemes, respectively. For CDR-H1, residue numbering is listed using both the Kabat and Chothia numbering schemes. FRs are located between CDRs, for example, with FR-L1 located before CDR-L1, FR-L2 located between CDR-L1 and CDR-L2, FR-L3 located between CDR-L2 and CDR-L3 and so forth. It is noted that because the shown Kabat numbering scheme places insertions at H35A and H35B, the end of the Chothia CDR-H1 loop when numbered using the shown Kabat numbering convention varies between H32 and H34, depending on the length of the loop.

**Table 1. Boundaries of CDRs according to various numbering schemes.**

| **CDR** | **Kabat** | **Chothia** | **AbM** | **Contact** |
|---|---|---|---|---|
| CDR-L1 | L24--L34 | L24--L34 | L24--L34 | L30--L36 |
| CDR-L2 | L50--L56 | L50--L56 | L50--L56 | L46--L55 |
| CDR-L3 | L89--L97 | L89--L97 | L89--L97 | L89--L96 |
| CDR-H1 (Kabat Numbering¹) | H31--H35B | H26--H32..34 | H26--H3 5B | H30--H35B |
| CDR-H1 (Chothia Numbering²) | H31--H35 | H26--H32 | H26--H35 | H30--H35 |
| CDR-H2 | H50--H65 | H52--H56 | H50--H58 | H47--H58 |
| CDR-H3 | H95--H102 | H95--H102 | H95--H102 | H93--H101 |

| | | | | |
|---|---|---|---|---|
| 1 - Kabat et al. (1991), "Sequences of Proteins of Immunological Interest," 5th Ed. Public Health Service, National Institutes of Health, Bethesda, MD 2 - Al-Lazikani et al., (1997) JMB 273,927-948 | | | | |

Thus, unless otherwise specified, a "CDR" or "complementary determining region," or individual specified CDRs *(e.g.,* CDR-H1, CDR-H2, CDR-H3), of a given antibody or region thereof, such as a variable region thereof, should be understood to encompass a (or the specific) complementary determining region as defined by any of the aforementioned schemes, or other known schemes. For example, where it is stated that a particular CDR *(e.g.,* a CDR-H3) contains the amino acid sequence of a corresponding CDR in a given V_{H} or V_{L} region amino acid sequence, it is understood that such a CDR has a sequence of the corresponding CDR (*e.g.,* CDR-H3) within the variable region, as defined by any of the aforementioned schemes, or other known schemes. In some embodiments, specific CDR sequences are specified. Exemplary CDR sequences of provided antibodies are described using various numbering schemes, although it is understood that a provided antibody can include CDRs as described according to any of the other aforementioned numbering schemes or other numbering schemes known to a skilled artisan.

Likewise, unless otherwise specified, a FR or individual specified FR(s) *(e.g.,* FR-H1, FR-H2, FR-H3, FR-H4), of a given antibody or region thereof, such as a variable region thereof, should be understood to encompass a (or the specific) framework region as defined by any of the known schemes. In some instances, the scheme for identification of a particular CDR, FR, or FRs or CDRs is specified, such as the CDR as defined by the Kabat, Chothia, AbM or Contact method, or other known schemes. In other cases, the particular amino acid sequence of a CDR or FR is given.

In some embodiments, the antigen-binding proteins, antibodies and antigen binding fragments thereof specifically recognize an antigen of a full-length antibody. In some embodiments, the heavy and light chains of an antibody can be full-length or can be an antigen-binding portion (a Fab, F(ab')2, Fv or a single chain Fv fragment (scFv)). In other embodiments, the antibody heavy chain constant region is chosen from, *e.g.,* IgG1, IgG2, IgG3, IgG4, IgM, IgA1, IgA2, IgD, and IgE, particularly chosen from, *e.g.,* IgG1, IgG2, IgG3, and IgG4, more particularly, IgG1 (*e.g.,* human IgG1). In another embodiment, the antibody light chain constant region is chosen from, *e.g.,* kappa or lambda, particularly kappa.

Among the provided antibodies are antibody fragments. An "antibody fragment" refers to a molecule other than an intact antibody that comprises a portion of an intact antibody that binds the antigen to which the intact antibody binds. Examples of antibody fragments include but are not limited to Fv, Fab, Fab', Fab'-SH, F(ab')₂; diabodies; linear antibodies; variable heavy chain (V_{H}) regions, single-chain antibody molecules such as scFvs and single-domain V_{H} single antibodies; and multispecific antibodies formed from antibody fragments. In particular embodiments, the antibodies are single-chain antibody fragments comprising a variable heavy chain region and/or a variable light chain region, such as scFvs.

The term "variable region" or "variable domain" refers to the domain of an antibody heavy or light chain that is involved in binding the antibody to antigen. The variable domains of the heavy chain and light chain (V_{H} and V_{L}, respectively) of a native antibody generally have similar structures, with each domain comprising four conserved framework regions (FRs) and three CDRs. (See, *e.g.,* Kindt et al. Kuby Immunology, 6th ed., W.H. Freeman and Co., page 91 (2007). A single V_{H} or V_{L} domain may be sufficient to confer antigen-binding specificity. Furthermore, antibodies that bind a particular antigen may be isolated using a V_{H} or V_{L} domain from an antibody that binds the antigen to screen a library of complementary V_{L} or V_{H} domains, respectively. See, *e.g*., Portolano et al., J. Immunol. 150:880-887 (1993); Clarkson et al., Nature 352:624-628 (1991).

Among the antibodies included in the provided CARs are antibody fragments. An "antibody fragment" or "antigen-binding fragment" refers to a molecule other than an intact antibody that comprises a portion of an intact antibody that binds the antigen to which the intact antibody binds. Examples of antibody fragments include but are not limited to Fv, Fab, Fab', Fab'-SH, F(ab')₂; diabodies; linear antibodies; heavy chain variable (V_{H}) regions, single-chain antibody molecules such as scFvs and single-domain antibodies comprising only the V_{H} region; and multispecific antibodies formed from antibody fragments. In some embodiments, the antigen-binding domain in the provided CARs is or comprises an antibody fragment comprising a variable heavy chain (V_{H}) and a variable light chain (V_{L}) region. In particular embodiments, the antibodies are single-chain antibody fragments comprising a heavy chain variable (V_{H}) region and/or a light chain variable (V_{L}) region, such as scFvs.

The term "variable region" or "variable domain" refers to the domain of an antibody heavy or light chain that is involved in binding the antibody to antigen. The variable domains of the heavy chain and light chain (V_{H} and V_{L}, respectively) of a native antibody generally have similar structures, with each domain comprising four conserved framework regions (FRs) and three CDRs. (See, *e.g.,* Kindt et al. Kuby Immunology, 6th ed., W.H. Freeman and Co., page 91 (2007). A single V_{H} or V_{L} domain may be sufficient to confer antigen-binding specificity. Furthermore, antibodies that bind a particular antigen may be isolated using a V_{H} or V_{L} domain from an antibody that binds the antigen to screen a library of complementary V_{L} or V_{H} domains, respectively. See, *e.g*., Portolano et al., J. Immunol. 150:880-887 (1993); Clarkson et al., Nature 352:624-628 (1991).

Single-domain antibodies are antibody fragments comprising all or a portion of the heavy chain variable domain or all or a portion of the light chain variable domain of an antibody. In certain embodiments, a single-domain antibody is a human single-domain antibody. In some embodiments, the CAR comprises an antibody heavy chain domain that specifically binds the antigen, such as a cancer marker or cell surface antigen of a cell or disease to be targeted, such as a tumor cell or a cancer cell, such as any of the target antigens described herein or known.

Antibody fragments can be made by various techniques, including but not limited to proteolytic digestion of an intact antibody as well as production by recombinant host cells. In some embodiments, the antibodies are recombinantly-produced fragments, such as fragments comprising arrangements that do not occur naturally, such as those with two or more antibody regions or chains joined by synthetic linkers, *e.g*., peptide linkers, and/or that are may not be produced by enzyme digestion of a naturally-occurring intact antibody. In some embodiments, the antibody fragments are scFvs.

A "humanized" antibody is an antibody in which all or substantially all CDR amino acid residues are derived from non-human CDRs and all or substantially all FR amino acid residues are derived from human FRs. A humanized antibody optionally may include at least a portion of an antibody constant region derived from a human antibody. A "humanized form" of a non-human antibody, refers to a variant of the non-human antibody that has undergone humanization, typically to reduce immunogenicity to humans, while retaining the specificity and affinity of the parental non-human antibody. In some embodiments, some FR residues in a humanized antibody are substituted with corresponding residues from a non-human antibody *(e.g.,* the antibody from which the CDR residues are derived), *e.g.,* to restore or improve antibody specificity or affinity.

Thus, in some embodiments, the chimeric antigen receptor, including TCR-like CARs, includes an extracellular portion containing an antibody or antibody fragment. In some embodiments, the antibody or fragment includes an scFv. In some aspects, the chimeric antigen receptor includes an extracellular portion containing the antibody or fragment and an intracellular signaling region. In some embodiments, the intracellular signaling region comprises an intracellular signaling domain. In some embodiments, the intracellular signaling domain is or comprises a primary signaling domain, a signaling domain that is capable of inducing a primary activation signal in a T cell, a signaling domain of a T cell receptor (TCR) component, and/or a signaling domain comprising an immunoreceptor tyrosine-based activation motif (ITAM).

In some embodiments, the recombinant receptor such as the CAR, such as the antibody portion thereof, further includes a spacer, which may be or include at least a portion of an immunoglobulin constant region or variant or modified version thereof, such as a hinge region, e.g., an IgG4 hinge region, and/or a C_{H}1/C_{L} and/or Fc region. In some embodiments, the recombinant receptor further comprises a spacer and/or a hinge region. In some embodiments, the constant region or portion is of a human IgG, such as IgG4 or IgG1. In some aspects, the portion of the constant region serves as a spacer region between the antigen-recognition component, e.g., scFv, and transmembrane domain. The spacer can be of a length that provides for increased responsiveness of the cell following antigen binding, as compared to in the absence of the spacer.

In some examples, the spacer is at or about 12 amino acids in length or is no more than 12 amino acids in length. Exemplary spacers include those having at least about 10 to 229 amino acids, about 10 to 200 amino acids, about 10 to 175 amino acids, about 10 to 150 amino acids, about 10 to 125 amino acids, about 10 to 100 amino acids, about 10 to 75 amino acids, about 10 to 50 amino acids, about 10 to 40 amino acids, about 10 to 30 amino acids, about 10 to 20 amino acids, or about 10 to 15 amino acids, and including any integer between the endpoints of any of the listed ranges. In some embodiments, a spacer region has about 12 amino acids or less, about 119 amino acids or less, or about 229 amino acids or less. Exemplary spacers include IgG4 hinge alone, IgG4 hinge linked to C_{H}2 and C_{H}3 domains, or IgG4 hinge linked to the C_{H}3 domain. Exemplary spacers include, but are not limited to, those described in Hudecek et al. (2013) Clin. Cancer Res., 19:3153, Hudecek et al. (2015) Cancer Immunol Res. 3(2): 125-135 or International Pat. App. Pub. No. WO2014031687, U.S. Patent No. 8,822,647 or US2014/0271635. In some embodiments, the spacer includes a sequence of an immunoglobulin hinge region, a C_{H}2 and C_{H}3 region. In some embodiments, one of more of the hinge, C_{H}2 and C_{H}3 is derived all or in part from IgG4 or IgG2. In some cases, the hinge, C_{H}2 and C_{H}3 is derived from IgG4. In some aspects, one or more of the hinge, C_{H}2 and C_{H}3 is chimeric and contains sequence derived from IgG4 and IgG2. In some examples, the spacer contains an IgG4/2 chimeric hinge, an IgG2/4 C_{H}2, and an IgG4 C_{H}3 region.

In some embodiments, the spacer can be derived all or in part from IgG4 and/or IgG2. In some embodiments, the spacer can be a chimeric polypeptide containing one or more of a hinge, C_{H}2 and/or C_{H}3 sequence(s) derived from IgG4, IgG2, and/or IgG2 and IgG4. In some embodiments, the spacer can contain mutations, such as one or more single amino acid mutations in one or more domains. In some examples, the amino acid modification is a substitution of a proline (P) for a serine (S) in the hinge region of an IgG4. In some embodiments, the amino acid modification is a substitution of a glutamine (Q) for an asparagine (N) to reduce glycosylation heterogeneity, such as an N to Q substitution at a position corresponding to position 177 in the C_{H}2 region of the IgG4 heavy chain constant region sequence set forth in SEQ ID NO: 70 (Uniprot Accession No. P01861; position corresponding to position 297 by EU numbering and position 79 of the hinge-C_{H}2-C_{H}3 spacer sequence set forth in SEQ ID NO:4) or an N to Q substitution at a position corresponding to position 176 in the C_{H}2 region of the IgG2 heavy chain constant region sequence set forth in SEQ ID NO: 71 (Uniprot Accession No. P01859; position corresponding to position 297 by EU numbering).

In some aspects, the spacer contains only a hinge region of an IgG, such as only a hinge of IgG4 or IgG1, such as the hinge only spacer set forth in SEQ ID NO: 1, and is encoded by the sequence set forth in SEQ ID NO: 2. In other embodiments, the spacer is an Ig hinge, e.g., and IgG4 hinge, linked to a C_{H}2 and/or C_{H}3 domains. In some embodiments, the spacer is an Ig hinge, e.g., an IgG4 hinge, linked to C_{H}2 and C_{H}3 domains, such as set forth in SEQ ID NO:3. In some embodiments, the spacer is an Ig hinge, e.g., an IgG4 hinge, linked to a C_{H}3 domain only, such as set forth in SEQ ID NO:4. In some embodiments, the spacer is or comprises a glycine-serine rich sequence or other flexible linker such as known flexible linkers. In some embodiments, the constant region or portion is of IgD. In some embodiments, the spacer has the sequence set forth in SEQ ID NO: 5. In some embodiments, the spacer has a sequence of amino acids that exhibits at least or at least about 85%, 86%, 87%, 88%, 89%, 90%, 91%, 92%, 93%, 94%, 95%, 96%, 97%, 98%, 99% or more sequence identity to any of SEQ ID NOS: 1, 3, 4 and 5.

The antigen recognition domain generally is linked to one or more intracellular signaling components, such as signaling components that mimic stimulation or activation through an antigen receptor complex, such as a TCR complex, in the case of a CAR, and/or signal via another cell surface receptor. Thus, in some embodiments, the antigen binding component (e.g., antibody) is linked to one or more transmembrane and intracellular signaling regions. In some embodiments, the transmembrane domain is fused to the extracellular domain. In one embodiment, a transmembrane domain that naturally is associated with one of the domains in the receptor, e.g., CAR, is used. In some instances, the transmembrane domain is selected or modified by amino acid substitution to avoid binding of such domains to the transmembrane domains of the same or different surface membrane proteins to minimize interactions with other members of the receptor complex.

The transmembrane domain in some embodiments is derived either from a natural or from a synthetic source. Where the source is natural, the domain in some aspects is derived from any membrane-bound or transmembrane protein. Transmembrane regions include those derived from (i.e. comprise at least the transmembrane region(s) of) the alpha, beta or zeta chain of the T-cell receptor, CD28, CD3 epsilon, CD45, CD4, CD5, CD8, CD9, CD16, CD22, CD33, CD37, CD64, CD80, CD86, CD134, CD137, CD154. Alternatively the transmembrane domain in some embodiments is synthetic. In some aspects, the synthetic transmembrane domain comprises predominantly hydrophobic residues such as leucine and valine. In some aspects, a triplet of phenylalanine, tryptophan and valine will be found at each end of a synthetic transmembrane domain. In some embodiments, the linkage is by linkers, spacers, and/or transmembrane domain(s).

Among the intracellular signaling region are those that mimic or approximate a signal through a natural antigen receptor, a signal through such a receptor in combination with a costimulatory receptor, and/or a signal through a costimulatory receptor alone. In some embodiments, a short oligo- or polypeptide linker, for example, a linker of between 2 and 10 amino acids in length, such as one containing glycines and serines, e.g., glycine-serine doublet, is present and forms a linkage between the transmembrane domain and the cytoplasmic signaling domain of the CAR.

The receptor, e.g., the CAR, generally includes at least one intracellular signaling component or components. In some embodiments, the receptor includes an intracellular component of a TCR complex, such as a TCR CD3 chain that mediates T-cell activation and cytotoxicity, e.g., CD3 zeta chain. Thus, in some aspects, the ROR1-binding antibody is linked to one or more cell signaling modules. In some embodiments, cell signaling modules include CD3 transmembrane domain, CD3 intracellular signaling domains, and/or other CD transmembrane domains. In some embodiments, the receptor, e.g., CAR, further includes a portion of one or more additional molecules such as Fc receptor y, CD8, CD4, CD25, or CD16. For example, in some aspects, the CAR includes a chimeric molecule between CD3-zeta (CD3-ζ) or Fc receptor γ and CD8, CD4, CD25 or CD16.

In some embodiments, upon ligation of the CAR, the cytoplasmic domain or intracellular signaling region of the CAR activates at least one of the normal effector functions or responses of the immune cell, e.g., T cell engineered to express the CAR. For example, in some contexts, the CAR induces a function of a T cell such as cytolytic activity or T-helper activity, such as secretion of cytokines or other factors. In some embodiments, a truncated portion of an intracellular signaling region of an antigen receptor component or costimulatory molecule is used in place of an intact immunostimulatory chain, for example, if it transduces the effector function signal. In some embodiments, the intracellular signaling regions, e.g., comprising intracellular domain or domains, include the cytoplasmic sequences of the T cell receptor (TCR), and in some aspects also those of co-receptors that in the natural context act in concert with such receptor to initiate signal transduction following antigen receptor engagement, and/or any derivative or variant of such molecules, and/or any synthetic sequence that has the same functional capability.

In the context of a natural TCR, full activation generally requires not only signaling through the TCR, but also a costimulatory signal. Thus, in some embodiments, to promote full activation, a component for generating secondary or co-stimulatory signal is also included in the CAR. In other embodiments, the CAR does not include a component for generating a costimulatory signal. In some aspects, an additional CAR is expressed in the same cell and provides the component for generating the secondary or costimulatory signal.

T cell activation is in some aspects described as being mediated by two classes of cytoplasmic signaling sequences: those that initiate antigen-dependent primary activation through the TCR (primary cytoplasmic signaling sequences), and those that act in an antigenindependent manner to provide a secondary or co-stimulatory signal (secondary cytoplasmic signaling sequences). In some aspects, the CAR includes one or both of such signaling components.

In some aspects, the CAR includes a primary cytoplasmic signaling sequence that regulates primary activation of the TCR complex. Primary cytoplasmic signaling sequences that act in a stimulatory manner may contain signaling motifs which are known as immunoreceptor tyrosine-based activation motifs or ITAMs. Examples of ITAM containing primary cytoplasmic signaling sequences include those derived from TCR or CD3 zeta, FcR gamma or FcR beta. In some embodiments, cytoplasmic signaling molecule(s) in the CAR contain(s) a cytoplasmic signaling domain, portion thereof, or sequence derived from CD3 zeta.

In some embodiments, the CAR includes a signaling region and/or transmembrane portion of a costimulatory receptor, such as CD28, 4-1BB, OX40, DAP10, and ICOS. In some aspects, the same CAR includes both the signaling region and costimulatory components.

In some embodiments, the signaling region is included within one CAR, whereas the costimulatory component is provided by another CAR recognizing another antigen. In some embodiments, the CARs include activating or stimulatory CARs, and costimulatory CARs, both expressed on the same cell (*see* WO2014/055668).

In certain embodiments, the intracellular signaling region comprises a CD28 transmembrane and signaling domain linked to a CD3 (e.g., CD3-zeta) intracellular domain. In some embodiments, the intracellular signaling region comprises a chimeric CD28 and CD137 (4-1BB, TNFRSF9) co-stimulatory domains, linked to a CD3 zeta intracellular domain.

In some embodiments, the CAR encompasses one or more, e.g., two or more, costimulatory domains and an activation domain, e.g., primary activation domain, in the cytoplasmic portion. Exemplary CARs include intracellular components of CD3-zeta, CD28, and 4-1BB.

In some cases, CARs are referred to as first, second, and/or third generation CARs. In some aspects, a first generation CAR is one that solely provides a CD3-chain induced signal upon antigen binding; in some aspects, a second-generation CARs is one that provides such a signal and costimulatory signal, such as one including an intracellular signaling domain from a costimulatory receptor such as CD28 or CD137; in some aspects, a third generation CAR in some aspects is one that includes multiple costimulatory domains of different costimulatory receptors.

In some embodiments, the chimeric antigen receptor includes an extracellular portion containing the antibody or fragment described herein. In some aspects, the chimeric antigen receptor includes an extracellular portion containing the antibody or fragment described herein and an intracellular signaling domain. In some embodiments, the antibody or fragment includes an scFv or a single-domain V_{H} antibody and the intracellular domain contains an ITAM. In some aspects, the intracellular signaling domain includes a signaling domain of a zeta chain of a CD3-zeta (CD3ζ) chain. In some embodiments, the chimeric antigen receptor includes a transmembrane domain disposed between the extracellular domain and the intracellular signaling region.

In some aspects, the transmembrane domain contains a transmembrane portion of CD28. The extracellular domain and transmembrane can be linked directly or indirectly. In some embodiments, the extracellular domain and transmembrane are linked by a spacer, such as any described herein. In some embodiments, the chimeric antigen receptor contains an intracellular domain of a T cell costimulatory molecule, such as between the transmembrane domain and intracellular signaling domain. In some aspects, the T cell costimulatory molecule is CD28 or 4-1BB.

In some embodiments, the CAR contains an antibody, e.g., an antibody fragment, a transmembrane domain that is or contains a transmembrane portion of CD28 or a functional variant thereof, and an intracellular signaling domain containing a signaling portion of CD28 or functional variant thereof and a signaling portion of CD3 zeta or functional variant thereof. In some embodiments, the CAR contains an antibody, e.g., antibody fragment, a transmembrane domain that is or contains a transmembrane portion of CD28 or a functional variant thereof, and an intracellular signaling domain containing a signaling portion of a 4-1BB or functional variant thereof and a signaling portion of CD3 zeta or functional variant thereof. In some such embodiments, the receptor further includes a spacer containing a portion of an Ig molecule, such as a human Ig molecule, such as an Ig hinge, e.g. an IgG4 hinge, such as a hinge-only spacer.

In some embodiments, the transmembrane domain of the receptor, e.g., the CAR is a transmembrane domain of human CD28 or variant thereof, e.g., a 27-amino acid transmembrane domain of a human CD28 (Accession No.: P10747.1), or is a transmembrane domain that comprises the sequence of amino acids set forth in SEQ ID NO: 8 or a sequence of amino acids that exhibits at least or at least about85%, 86%, 87%, 88%, 89%, 90%, 91%, 92%, 93%, 94%, 95%, 96%, 97%, 98%, 99% or more sequence identity to SEQ ID NO:8; in some embodiments, the transmembrane-domain containing portion of the recombinant receptor comprises the sequence of amino acids set forth in SEQ ID NO: 9 or a sequence of amino acids having at least or at least about 85%, 86%, 87%, 88%, 89%, 90%, 91%, 92%, 93%, 94%, 95%, 96%, 97%, 98%, 99% or more sequence identity thereto.

In some embodiments, the chimeric antigen receptor contains an intracellular domain of a T cell costimulatory molecule. In some aspects, the T cell costimulatory molecule is CD28 or 4-1BB.

In some embodiments, the intracellular signaling region comprises an intracellular costimulatory signaling domain of human CD28 or functional variant or portion thereof, such as a 41 amino acid domain thereof and/or such a domain with an LL to GG substitution at positions 186-187 of a native CD28 protein. In some embodiments, the intracellular signaling domain can comprise the sequence of amino acids set forth in SEQ ID NO: 10 or 11 or a sequence of amino acids that exhibits at least or at least about 85%, 86%, 87%, 88%, 89%, 90%, 91%, 92%, 93%, 94%, 95%, 96%, 97%, 98%, 99% or more sequence identity to SEQ ID NO: 10 or 11. In some embodiments, the intracellular region comprises an intracellular costimulatory signaling domain of 4-1BB or functional variant or portion thereof, such as a 42-amino acid cytoplasmic domain of a human 4-1BB (Accession No. Q07011.1) or functional variant or portion thereof, such as the sequence of amino acids set forth in SEQ ID NO: 12 or a sequence of amino acids that exhibits at least or at least about 85%, 86%, 87%, 88%, 89%, 90%, 91%, 92%, 93%, 94%, 95%, 96%, 97%, 98%, 99% or more sequence identity to SEQ ID NO: 12.

In some embodiments, the intracellular signaling region comprises a human CD3 chain, optionally a CD3 zeta stimulatory signaling domain or functional variant thereof, such as an 112 AA cytoplasmic domain of isoform 3 of human CD3ζ (Accession No.: P20963.2) or a CD3 zeta signaling domain as described in U.S. Patent No.: 7,446,190 or U.S. Patent No. 8,911,993. In some embodiments, the intracellular signaling region comprises the sequence of amino acids set forth in SEQ ID NO: 13, 14 or 15 or a sequence of amino acids that exhibits at least or at least about 85%, 86%, 87%, 88%, 89%, 90%, 91%, 92%, 93%, 94%, 95%, 96%, 97%, 98%, 99% or more sequence identity to SEQ ID NO: 13, 14 or 15.

In some aspects, the spacer contains only a hinge region of an IgG, such as only a hinge of IgG4 or IgG1, such as the hinge only spacer set forth in SEQ ID NO:1. In other embodiments, the spacer is an Ig hinge, e.g., and IgG4 hinge, linked to a C_{H}2 and/or C_{H}3 domains. In some embodiments, the spacer is an Ig hinge, e.g., an IgG4 hinge, linked to C_{H}2 and C_{H}3 domains, such as set forth in SEQ ID NO:3. In some embodiments, the spacer is an Ig hinge, e.g., an IgG4 hinge, linked to a C_{H}3 domain only, such as set forth in SEQ ID NO:4. In some embodiments, the spacer is or comprises a glycine-serine rich sequence or other flexible linker such as known flexible linkers.

### 2. T Cell Receptors (TCRs)

In some embodiments, the encoded recombinant receptor is a T cell receptor (TCR) or antigen-binding portion thereof that recognizes an peptide epitope or T cell epitope of a target polypeptide, such as an antigen of a tumor, viral or autoimmune protein.

In some embodiments, a "T cell receptor" or "TCR" is a molecule that contains a variable α and β chains (also known as TCRα and TCRβ, respectively) or a variable γ and δ chains (also known as TCRα and TCRβ, respectively), or antigen-binding portions thereof, and which is capable of specifically binding to a peptide bound to an MHC molecule. In some embodiments, the TCR is in the αβ form. Typically, TCRs that exist in αβ and γδ forms are generally structurally similar, but T cells expressing them may have distinct anatomical locations or functions. A TCR can be found on the surface of a cell or in soluble form. Generally, a TCR is found on the surface of T cells (or T lymphocytes) where it is generally responsible for recognizing antigens bound to major histocompatibility complex (MHC) molecules.

Unless otherwise stated, the term "TCR" should be understood to encompass full TCRs as well as antigen-binding portions or antigen-binding fragments thereof. In some embodiments, the TCR is an intact or full-length TCR, including TCRs in the αβ form or γδ form. In some embodiments, the TCR is an antigen-binding portion that is less than a full-length TCR but that binds to a specific peptide bound in an MHC molecule, such as binds to an MHC-peptide complex. In some cases, an antigen-binding portion or fragment of a TCR can contain only a portion of the structural domains of a full-length or intact TCR, but yet is able to bind the peptide epitope, such as MHC-peptide complex, to which the full TCR binds. In some cases, an antigen-binding portion contains the variable domains of a TCR, such as variable α chain and variable β chain of a TCR, sufficient to form a binding site for binding to a specific MHC-peptide complex. Generally, the variable chains of a TCR contain complementarity determining regions involved in recognition of the peptide, MHC and/or MHC-peptide complex.

In some embodiments, the variable domains of the TCR contain hypervariable loops, or complementarity determining regions (CDRs), which generally are the primary contributors to antigen recognition and binding capabilities and specificity. In some embodiments, a CDR of a TCR or combination thereof forms all or substantially all of the antigen-binding site of a given TCR molecule. The various CDRs within a variable region of a TCR chain generally are separated by framework regions (FRs), which generally display less variability among TCR molecules as compared to the CDRs (see, e.g., Jores et al., Proc. Nat'l Acad. Sci. U.S.A. 87:9138, 1990; Chothia et al., EMBO J. 7:3745, 1988; see also Lefranc et al., Dev. Comp. Immunol. 27:55, 2003). In some embodiments, CDR3 is the main CDR responsible for antigen binding or specificity, or is the most important among the three CDRs on a given TCR variable region for antigen recognition, and/or for interaction with the processed peptide portion of the peptide-MHC complex. In some contexts, the CDR1 of the alpha chain can interact with the N-terminal part of certain antigenic peptides. In some contexts, CDR1 of the beta chain can interact with the C-terminal part of the peptide. In some contexts, CDR2 contributes most strongly to or is the primary CDR responsible for the interaction with or recognition of the MHC portion of the MHC-peptide complex. In some embodiments, the variable region of the β-chain can contain a further hypervariable region (CDR4 or HVR4), which generally is involved in superantigen binding and not antigen recognition (Kotb (1995) Clinical Microbiology Reviews, 8:411-426).

In some embodiments, a TCR also can contain a constant domain, a transmembrane domain and/or a short cytoplasmic tail (see, e.g., Janeway et al., Immunobiology: The Immune System in Health and Disease, 3rd Ed., Current Biology Publications, p. 4:33, 1997). In some aspects, each chain of the TCR can possess one N-terminal immunoglobulin variable domain, one immunoglobulin constant domain, a transmembrane region, and a short cytoplasmic tail at the C-terminal end. In some embodiments, a TCR is associated with invariant proteins of the CD3 complex involved in mediating signal transduction.

In some embodiments, a TCR chain contains one or more constant domain. For example, the extracellular portion of a given TCR chain (e.g., α-chain or β-chain) can contain two immunoglobulin-like domains, such as a variable domain (e.g., Vα or Vβ; typically amino acids 1 to 116 based on Kabat numbering Kabat et al., "Sequences of Proteins of Immunological Interest, US Dept. Health and Human Services, Public Health Service National Institutes of Health, 1991, 5th ed.) and a constant domain (e.g., α-chain constant domain or Cα, typically positions 117 to 259 of the chain based on Kabat numbering or β chain constant domain or C_{β}, typically positions 117 to 295 of the chain based on Kabat) adjacent to the cell membrane. For example, in some cases, the extracellular portion of the TCR formed by the two chains contains two membrane-proximal constant domains, and two membrane-distal variable domains, which variable domains each contain CDRs. The constant domain of the TCR may contain short connecting sequences in which a cysteine residue forms a disulfide bond, thereby linking the two chains of the TCR. In some embodiments, a TCR may have an additional cysteine residue in each of the α and β chains, such that the TCR contains two disulfide bonds in the constant domains.

In some embodiments, the TCR chains contain a transmembrane domain. In some embodiments, the transmembrane domain is positively charged. In some cases, the TCR chain contains a cytoplasmic tail. In some cases, the structure allows the TCR to associate with other molecules like CD3 and subunits thereof. For example, a TCR containing constant domains with a transmembrane region may anchor the protein in the cell membrane and associate with invariant subunits of the CD3 signaling apparatus or complex. The intracellular tails of CD3 signaling subunits (e.g. CD3γ, CD3δ, CD3ε and CD3ζ chains) contain one or more immunoreceptor tyrosine-based activation motif or ITAM that are involved in the signaling capacity of the TCR complex.

In some embodiments, the TCR may be a heterodimer of two chains α and β (or optionally γ and δ) or it may be a single chain TCR construct. In some embodiments, the TCR is a heterodimer containing two separate chains (α and β chains or γ and δ chains) that are linked, such as by a disulfide bond or disulfide bonds.

In some embodiments, the TCR can be generated from a known TCR sequence(s), such as sequences of Vα,β chains, for which a substantially full-length coding sequence is readily available. Methods for obtaining full-length TCR sequences, including V chain sequences, from cell sources are well known. In some embodiments, nucleic acids encoding the TCR can be obtained from a variety of sources, such as by polymerase chain reaction (PCR) amplification of TCR-encoding nucleic acids within or isolated from a given cell or cells, or synthesis of publicly available TCR DNA sequences.

In some embodiments, the TCR is obtained from a biological source, such as from cells such as from a T cell (e.g. cytotoxic T cell), T-cell hybridomas or other publicly available source. In some embodiments, the T-cells can be obtained from *in vivo* isolated cells. In some embodiments, the TCR is a thymically selected TCR. In some embodiments, the TCR is a neoepitope-restricted TCR. In some embodiments, the T- cells can be a cultured T-cell hybridoma or clone. In some embodiments, the TCR or antigen-binding portion thereof or antigen-binding fragment thereof can be synthetically generated from knowledge of the sequence of the TCR.

In some embodiments, the TCR is generated from a TCR identified or selected from screening a library of candidate TCRs against a target polypeptide antigen, or target T cell epitope thereof. TCR libraries can be generated by amplification of the repertoire of Vα and Vβ from T cells isolated from a subject, including cells present in PBMCs, spleen or other lymphoid organ. In some cases, T cells can be amplified from tumor-infiltrating lymphocytes (TILs). In some embodiments, TCR libraries can be generated from CD4+ or CD8+ cells. In some embodiments, the TCRs can be amplified from a T cell source of a normal of healthy subject, i.e. normal TCR libraries. In some embodiments, the TCRs can be amplified from a T cell source of a diseased subject, i.e. diseased TCR libraries. In some embodiments, degenerate primers are used to amplify the gene repertoire of Vα and Vβ, such as by RT-PCR in samples, such as T cells, obtained from humans. In some embodiments, scTv libraries can be assembled from naive Vα and Vβ libraries in which the amplified products are cloned or assembled to be separated by a linker. Depending on the source of the subject and cells, the libraries can be HLA allele-specific. Alternatively, in some embodiments, TCR libraries can be generated by mutagenesis or diversification of a parent or scaffold TCR molecule. In some aspects, the TCRs are subjected to directed evolution, such as by mutagenesis, e.g., of the α or β chain. In some aspects, particular residues within CDRs of the TCR are altered. In some embodiments, selected TCRs can be modified by affinity maturation. In some embodiments, antigen-specific T cells may be selected, such as by screening to assess CTL activity against the peptide. In some aspects, TCRs, e.g. present on the antigen-specific T cells, may be selected, such as by binding activity, e.g., particular affinity or avidity for the antigen.

In some embodiments, the genetically engineered antigen receptors include recombinant T cell receptors (TCRs) and/or TCRs cloned from naturally occurring T cells. In some embodiments, a high-affinity T cell clone for a target antigen (e.g., a cancer antigen) is identified, isolated from a patient, and introduced into the cells. In some embodiments, the TCR clone for a target antigen has been generated in transgenic mice engineered with human immune system genes (e.g., the human leukocyte antigen system, or HLA). See, e.g., tumor antigens (see, e.g., Parkhurst et al. (2009) Clin Cancer Res. 15:169-180 and Cohen et al. (2005) J Immunol. 175:5799-5808. In some embodiments, phage display is used to isolate TCRs against a target antigen (see, e.g., Varela-Rohena et al. (2008) Nat Med. 14:1390-1395 and Li (2005) Nat Biotechnol. 23:349-354.

In some embodiments, the TCR or antigen-binding portion thereof is one that has been modified or engineered. In some embodiments, directed evolution methods are used to generate TCRs with altered properties, such as with higher affinity for a specific MHC-peptide complex. In some embodiments, directed evolution is achieved by display methods including, but not limited to, yeast display (Holler et al. (2003) Nat Immunol, 4, 55-62; Holler et al. (2000) Proc Natl Acad Sci U S A, 97, 5387-92), phage display (Li et al. (2005) Nat Biotechnol, 23, 349-54), or T cell display (Chervin et al. (2008) J Immunol Methods, 339, 175-84). In some embodiments, display approaches involve engineering, or modifying, a known, parent or reference TCR. For example, in some cases, a wild-type TCR can be used as a template for producing mutagenized TCRs in which in one or more residues of the CDRs are mutated, and mutants with an desired altered property, such as higher affinity for a desired target antigen, are selected.

In some embodiments, peptides of a target polypeptide for use in producing or generating a TCR of interest are known or can be readily identified by a skilled artisan. In some embodiments, peptides suitable for use in generating TCRs or antigen-binding portions can be determined based on the presence of an HLA-restricted motif in a target polypeptide of interest, such as a target polypeptide described below. In some embodiments, peptides are identified using available computer prediction models. In some embodiments, for predicting MHC class I binding sites, such models include, but are not limited to, ProPred1 (Singh and Raghava (2001) Bioinformatics 17(12):1236-1237, and SYFPEITHI (see Schuler et al. (2007) Immunoinformatics Methods in Molecular Biology, 409(1): 75-93 2007). In some embodiments, the MHC-restricted epitope is HLA-A0201, which is expressed in approximately 39-46% of all Caucasians and therefore, represents a suitable choice of MHC antigen for use preparing a TCR or other MHC-peptide binding molecule.

HLA-A0201-binding motifs and the cleavage sites for proteasomes and immuneproteasomes using computer prediction models are known. For predicting MHC class I binding sites, such models include, but are not limited to, ProPred1 (described in more detail in Singh and Raghava, ProPred: prediction of HLA-DR binding sites. BIOINFORMATICS 17(12):1236-1237 2001), and SYFPEITHI (see Schuler et al. SYFPEITHI, Database for Searching and T-Cell Epitope Prediction. in Immunoinformatics Methods in Molecular Biology, vol 409(1): 75-93 2007).

In some embodiments, the TCR or antigen binding portion thereof may be a recombinantly produced natural protein or mutated form thereof in which one or more property, such as binding characteristic, has been altered. In some embodiments, a TCR may be derived from one of various animal species, such as human, mouse, rat, or other mammal. A TCR may be cell-bound or in soluble form. In some embodiments, for purposes of the provided methods, the TCR is in cell-bound form expressed on the surface of a cell.

In some embodiments, the TCR is a full-length TCR. In some embodiments, the TCR is an antigen-binding portion. In some embodiments, the TCR is a dimeric TCR (dTCR). In some embodiments, the TCR is a single-chain TCR (sc-TCR). In some embodiments, a dTCR or scTCR have the structures as described in WO 03/020763, WO 04/033685, WO2011/044186.

In some embodiments, the TCR contains a sequence corresponding to the transmembrane sequence. In some embodiments, the TCR does contain a sequence corresponding to cytoplasmic sequences. In some embodiments, the TCR is capable of forming a TCR complex with CD3. In some embodiments, any of the TCRs, including a dTCR or scTCR, can be linked to signaling domains that yield an active TCR on the surface of a T cell. In some embodiments, the TCR is expressed on the surface of cells.

In some embodiments a dTCR contains a first polypeptide wherein a sequence corresponding to a TCR α chain variable region sequence is fused to the N terminus of a sequence corresponding to a TCR α chain constant region extracellular sequence, and a second polypeptide wherein a sequence corresponding to a TCR β chain variable region sequence is fused to the N terminus a sequence corresponding to a TCR β chain constant region extracellular sequence, the first and second polypeptides being linked by a disulfide bond. In some embodiments, the bond can correspond to the native inter-chain disulfide bond present in native dimeric αβ TCRs. In some embodiments, the interchain disulfide bonds are not present in a native TCR. For example, in some embodiments, one or more cysteines can be incorporated into the constant region extracellular sequences of dTCR polypeptide pair. In some cases, both a native and a non-native disulfide bond may be desirable. In some embodiments, the TCR contains a transmembrane sequence to anchor to the membrane.

In some embodiments, a dTCR contains a TCR α chain containing a variable α domain, a constant α domain and a first dimerization motif attached to the C-terminus of the constant α domain, and a TCR β chain comprising a variable β domain, a constant β domain and a first dimerization motif attached to the C-terminus of the constant β domain, wherein the first and second dimerization motifs easily interact to form a covalent bond between an amino acid in the first dimerization motif and an amino acid in the second dimerization motif linking the TCR α chain and TCR β chain together.

In some embodiments, the TCR is a scTCR. Typically, a scTCR can be generated using methods known, See e.g., Soo Hoo, W. F. et al. PNAS (USA) 89, 4759 (1992); Wülfing, C. and Plückthun, A., J. Mol. Biol. 242, 655 (1994); Kurucz, I. et al. PNAS (USA) 90 3830 (1993); International published PCT Nos. WO 96/13593, WO 96/18105, WO99/60120, WO99/18129, WO 03/020763, WO2011/044186; and Schlueter, C. J. et al. J. Mol. Biol. 256, 859 (1996). In some embodiments, a scTCR contains an introduced non-native disulfide interchain bond to facilitate the association of the TCR chains (see e.g. International published PCT No. WO 03/020763). In some embodiments, a scTCR is a non-disulfide linked truncated TCR in which heterologous leucine zippers fused to the C-termini thereof facilitate chain association (see e.g. International published PCT No. WO99/60120). In some embodiments, a scTCR contain a TCRα variable domain covalently linked to a TCRβ variable domain via a peptide linker (see e.g., International published PCT No. WO99/18129).

In some embodiments, a scTCR contains a first segment constituted by an amino acid sequence corresponding to a TCR α chain variable region, a second segment constituted by an amino acid sequence corresponding to a TCR β chain variable region sequence fused to the N terminus of an amino acid sequence corresponding to a TCR β chain constant domain extracellular sequence, and a linker sequence linking the C terminus of the first segment to the N terminus of the second segment.

In some embodiments, a scTCR contains a first segment constituted by an α chain variable region sequence fused to the N terminus of an α chain extracellular constant domain sequence, and a second segment constituted by a β chain variable region sequence fused to the N terminus of a sequence β chain extracellular constant and transmembrane sequence, and, optionally, a linker sequence linking the C terminus of the first segment to the N terminus of the second segment.

In some embodiments, a scTCR contains a first segment constituted by a TCR β chain variable region sequence fused to the N terminus of a β chain extracellular constant domain sequence, and a second segment constituted by an α chain variable region sequence fused to the N terminus of a sequence α chain extracellular constant and transmembrane sequence, and, optionally, a linker sequence linking the C terminus of the first segment to the N terminus of the second segment.

In some embodiments, the linker of a scTCRs that links the first and second TCR segments can be any linker capable of forming a single polypeptide strand, while retaining TCR binding specificity. In some embodiments, the linker sequence may, for example, have the formula -P-AA-P- wherein P is proline and AA represents an amino acid sequence wherein the amino acids are glycine and serine. In some embodiments, the first and second segments are paired so that the variable region sequences thereof are orientated for such binding. Hence, in some cases, the linker has a sufficient length to span the distance between the C terminus of the first segment and the N terminus of the second segment, or vice versa, but is not too long to block or reduces bonding of the scTCR to the target ligand. In some embodiments, the linker can contain from or from about 10 to 45 amino acids, such as 10 to 30 amino acids or 26 to 41 amino acids residues, for example 29, 30, 31 or 32 amino acids. In some embodiments, the linker has the formula -PGGG-(SGGGG)₅-P- wherein P is proline, G is glycine and S is serine (SEQ ID NO:22). In some embodiments, the linker has the sequence GSADDAKKDAAKKDGKS (SEQ ID NO:23)

In some embodiments, the scTCR contains a covalent disulfide bond linking a residue of the immunoglobulin region of the constant domain of the α chain to a residue of the immunoglobulin region of the constant domain of the β chain. In some embodiments, the interchain disulfide bond in a native TCR is not present. For example, in some embodiments, one or more cysteines can be incorporated into the constant region extracellular sequences of the first and second segments of the scTCR polypeptide. In some cases, both a native and a nonnative disulfide bond may be desirable.

In some embodiments of a dTCR or scTCR containing introduced interchain disulfide bonds, the native disulfide bonds are not present. In some embodiments, the one or more of the native cysteines forming a native interchain disulfide bonds are substituted to another residue, such as to a serine or alanine. In some embodiments, an introduced disulfide bond can be formed by mutating non-cysteine residues on the first and second segments to cysteine. Exemplary non-native disulfide bonds of a TCR are described in published International PCT No. WO2006/000830.

In some embodiments, the TCR or antigen-binding fragment thereof exhibits an affinity with an equilibrium binding constant for a target antigen of between or between about 10⁻⁵ and 10⁻¹² M and all individual values and ranges therein. In some embodiments, the target antigen is an MHC-peptide complex or ligand.

In some embodiments, nucleic acid or nucleic acids encoding a TCR, such as α and β chains, can be amplified by PCR, cloning or other suitable means and cloned into a suitable expression vector or vectors. The expression vector can be any suitable recombinant expression vector, and can be used to transform or transfect any suitable host. Suitable vectors include those designed for propagation and expansion or for expression or both, such as plasmids and viruses.

In some embodiments, the recombinant expression vectors can be prepared using standard recombinant DNA techniques. In some embodiments, vectors can contain regulatory sequences, such as transcription and translation initiation and termination codons, which are specific to the type of host (e.g., bacterium, fungus, plant, or animal) into which the vector is to be introduced, as appropriate and taking into consideration whether the vector is DNA- or RNA-based. In some embodiments, the vector can contain a nonnative promoter operably linked to the nucleotide sequence encoding the TCR or antigen-binding portion (or other MHC-peptide binding molecule). In some embodiments, the promoter can be a non-viral promoter or a viral promoter, such as a cytomegalovirus (CMV) promoter, an SV40 promoter, an RSV promoter, and a promoter found in the long-terminal repeat of the murine stem cell virus. Other known promoters also are contemplated.

In some embodiments, after the T-cell clone is obtained, the TCR alpha and beta chains are isolated and cloned into a gene expression vector. In some embodiments, the TCR alpha and beta genes are linked via a picornavirus 2A ribosomal skip peptide so that both chains are coexpression. In some embodiments, genetic transfer of the TCR is accomplished via retroviral or lentiviral vectors, or via transposons (see, e.g., Baum et al. (2006) Molecular Therapy: The Journal of the American Society of Gene Therapy. 13:1050-1063; Frecha et al. (2010) Molecular Therapy: The Journal of the American Society of Gene Therapy. 18:1748-1757; and Hackett et al. (2010) Molecular Therapy: The Journal of the American Society of Gene Therapy. 18:674-683.

In some embodiments, to generate a vector encoding a TCR, the α and β chains are PCR amplified from total cDNA isolated from a T cell clone expressing the TCR of interest and cloned into an expression vector. In some embodiments, the α and β chains are cloned into the same vector. In some embodiments, the α and β chains are cloned into different vectors. In some embodiments, the generated α and β chains are incorporated into a retroviral, e.g. lentiviral, vector.

### 3. Chimeric Auto-Antibody Receptor (CAAR)

In some embodiments, the encoded recombinant receptor is a chimeric autoantibody receptor (CAAR). In some embodiments, the CAAR is specific for an autoantibody. In some embodiments, a cell expressing the CAAR, such as a T cell engineered to express a CAAR, can be used to specifically bind to and kill autoantibody-expressing cells, but not normal antibody expressing cells. In some embodiments, CAAR-expressing cells can be used to treat an autoimmune disease associated with expression of self-antigens, such as autoimmune diseases. In some embodiments, CAAR-expressing cells can target B cells that ultimately produce the autoantibodies and display the autoantibodies on their cell surfaces, mark these B cells as disease-specific targets for therapeutic intervention. In some embodiments, CAAR-expressing cells can be used to efficiently targeting and killing the pathogenic B cells in autoimmune diseases by targeting the disease-causing B cells using an antigen-specific chimeric autoantibody receptor. In some embodiments, the recombinant receptor is a CAAR, such as any described in U.S. Patent Application Pub. No. US 2017/0051035.

In some embodiments, the CAAR comprises an autoantibody binding domain, a transmembrane domain, and an intracellular signaling region. In some embodiments, the intracellular signaling region comprises an intracellular signaling domain. In some embodiments, the intracellular signaling domain is or comprises a primary signaling domain, a signaling domain that is capable of inducing a primary activation signal in a T cell, a signaling domain of a T cell receptor (TCR) component, and/or a signaling domain comprising an immunoreceptor tyrosine-based activation motif (ITAM). In some embodiments, the intracellular signaling region comprises a secondary or costimulatory signaling region (secondary intracellular signaling regions).

In some embodiments, the autoantibody binding domain comprises an autoantigen or a fragment thereof. The choice of autoantigen can depend upon the type of autoantibody being targeted. For example, the autoantigen may be chosen because it recognizes an autoantibody on a target cell, such as a B cell, associated with a particular disease state, e.g. an autoimmune disease, such as an autoantibody-mediated autoimmune disease. In some embodiments, the autoimmune disease includes pemphigus vulgaris (PV). Exemplary autoantigens include desmoglein 1 (Dsg1) and Dsg3.

### 4. Multi-targeting

In some embodiments, the cells and methods include multi-targeting strategies, such as expression of two or more genetically engineered receptors on the cell, each recognizing the same of a different antigen and typically each including a different intracellular signaling component. Such multi-targeting strategies are described, for example, in WO 2014055668 (describing combinations of activating and costimulatory CARs, e.g., targeting two different antigens present individually on off-target, e.g., normal cells, but present together only on cells of the disease or condition to be treated) and Fedorov et al., Sci. Transl. Medicine, 5(215) (December, 2013) (describing cells expressing an activating and an inhibitory CAR, such as those in which the activating CAR binds to one antigen expressed on both normal or non-diseased cells and cells of the disease or condition to be treated, and the inhibitory CAR binds to another antigen expressed only on the normal cells or cells which it is not desired to treat).

For example, in some embodiments, the cells include a receptor expressing a first genetically engineered antigen receptor (e.g., CAR or TCR) which is capable of inducing an activating or stimulating signal to the cell, generally upon specific binding to the antigen recognized by the first receptor, e.g., the first antigen. In some embodiments, the cell further includes a second genetically engineered antigen receptor (e.g., CAR or TCR), e.g., a chimeric costimulatory receptor, which is capable of inducing a costimulatory signal to the immune cell, generally upon specific binding to a second antigen recognized by the second receptor. In some embodiments, the first antigen and second antigen are the same. In some embodiments, the first antigen and second antigen are different.

In some embodiments, the first and/or second genetically engineered antigen receptor (e.g. CAR or TCR) is capable of inducing an activating or stimulating signal to the cell. In some embodiments, the receptor includes an intracellular signaling component containing ITAM or ITAM-like motifs. In some embodiments, the activation induced by the first receptor involves a signal transduction or change in protein expression in the cell resulting in initiation of an immune response, such as ITAM phosphorylation and/or initiation of ITAM-mediated signal transduction cascade, formation of an immunological synapse and/or clustering of molecules near the bound receptor (e.g. CD4 or CD8, etc.), activation of one or more transcription factors, such as NF-κB and/or AP-1, and/or induction of gene expression of factors such as cytokines, proliferation, and/or survival.

In some embodiments, the first and/or second receptor includes intracellular signaling domains of costimulatory receptors such as CD28, CD137 (4-1BB), OX40, and/or ICOS. In some embodiments, the first and second receptor include an intracellular signaling domain of a costimulatory receptor that are different. In one embodiment, the first receptor contains a CD28 costimulatory signaling region and the second receptor contain a 4-1BB costimulatory signaling region or vice versa.

In some embodiments, the first and/or second receptor includes both an intracellular signaling domain containing ITAM or ITAM-like motifs and an intracellular signaling domain of a costimulatory receptor.

In some embodiments, the first receptor contains an intracellular signaling domain containing ITAM or ITAM-like motifs and the second receptor contains an intracellular signaling domain of a costimulatory receptor. The costimulatory signal in combination with the activating or stimulating signal induced in the same cell is one that results in an immune response, such as a robust and sustained immune response, such as increased gene expression, secretion of cytokines and other factors, and T cell mediated effector functions such as cell killing.

In some embodiments, neither ligation of the first receptor alone nor ligation of the second receptor alone induces a robust immune response. In some aspects, if only one receptor is ligated, the cell becomes tolerized or unresponsive to antigen, or inhibited, and/or is not induced to proliferate or secrete factors or carry out effector functions. In some such embodiments, however, when the plurality of receptors are ligated, such as upon encounter of a cell expressing the first and second antigens, a desired response is achieved, such as full immune activation or stimulation, e.g., as indicated by secretion of one or more cytokine, proliferation, persistence, and/or carrying out an immune effector function such as cytotoxic killing of a target cell.

In some embodiments, the two receptors induce, respectively, an activating and an inhibitory signal to the cell, such that binding by one of the receptor to its antigen activates the cell or induces a response, but binding by the second inhibitory receptor to its antigen induces a signal that suppresses or dampens that response. Examples are combinations of activating CARs and inhibitory CARs or iCARs. Such a strategy may be used, for example, in which the activating CAR binds an antigen expressed in a disease or condition but which is also expressed on normal cells, and the inhibitory receptor binds to a separate antigen which is expressed on the normal cells but not cells of the disease or condition.

In some embodiments, the multi-targeting strategy is employed in a case where an antigen associated with a particular disease or condition is expressed on a non-diseased cell and/or is expressed on the engineered cell itself, either transiently (e.g., upon stimulation in association with genetic engineering) or permanently. In such cases, by requiring ligation of two separate and individually specific antigen receptors, specificity, selectivity, and/or efficacy may be improved.

In some embodiments, the plurality of antigens, e.g., the first and second antigens, are expressed on the cell, tissue, or disease or condition being targeted, such as on the cancer cell. In some aspects, the cell, tissue, disease or condition is multiple myeloma or a multiple myeloma cell. In some embodiments, one or more of the plurality of antigens generally also is expressed on a cell which it is not desired to target with the cell therapy, such as a normal or non-diseased cell or tissue, and/or the engineered cells themselves. In such embodiments, by requiring ligation of multiple receptors to achieve a response of the cell, specificity and/or efficacy is achieved.

### IV. METHODS OF ADMINISTRATION WHICH ARE NOT CLAIMED

In some aspects, the engineered cells, e.g., cells in which the transgene sequences are integrated, can be used in connection with a method of treatment, e.g., including administering any of the engineered cells or compositions containing engineered cells that have been assessed using the methods provided herein. In some aspects, the provided methods can be used to test, evaluate, characterize and/or assess the engineered cells or cell compositions prior to administration, e.g., for testing the presence, absence and/or amount of the integrated nucleic acids (e.g., transgene sequences) during one or more steps of the engineering or manufacturing process and/or for post-formulation testing, assessment for released for administration, and/or ready to be administered to the subject.

In some embodiments, the engineered cells expressing a recombinant receptor or compositions comprising the same, assessed or evaluated using the embodiments described herein, are useful in a variety of therapeutic, diagnostic and prophylactic indications. For example, the engineered cells or compositions comprising the engineered cells are useful in treating a variety of diseases and disorders in a subject. Methods and uses include therapeutic methods and uses, for example, involving administration of the engineered cells, or compositions containing the same, to a subject having a disease, condition, or disorder, such as a tumor or cancer. In some embodiments, the engineered cells or compositions assessed or evaluated using the embodiments provided herein are administered in an effective amount to effect treatment of the disease or disorder. Uses include uses of the engineered cells or compositions in such methods and treatments, and in the preparation of a medicament in order to carry out such therapeutic methods. In some embodiments, the methods, e.g., therapeutic methods, are carried out by administering the assessed or evaluated engineered cells, or compositions comprising the same, to the subject having or suspected of having the disease or condition. In some embodiments, these methods thereby treat the disease or condition or disorder in the subject.

In some aspects, the engineered cells or engineered cell composition can be administered to a subject, such as a subject that has a disease or disorder. Methods for administration of cells for adoptive cell therapy are known and may be used in connection with the provided methods and compositions. For example, adoptive T cell therapy methods are described, e.g., in US Pat. App. Pub. No. 2003/0170238 to Gruenberg et al; US Patent No. 4,690,915 to Rosenberg; Rosenberg (2011) Nat Rev Clin Oncol. 8(10):577-85). See, e.g., Themeli et al. (2013) Nat Biotechnol. 31(10): 928-933; Tsukahara et al. (2013) Biochem Biophys Res Commun 438(1): 84-9; Davila et al. (2013) PLoS ONE 8(4): e61338.

The disease or condition that is treated can be any in which expression of an antigen is associated with and/or involved in the etiology of a disease condition or disorder, e.g. causes, exacerbates or otherwise is involved in such disease, condition, or disorder. Exemplary diseases and conditions can include diseases or conditions associated with malignancy or transformation of cells (e.g. cancer), autoimmune or inflammatory disease, or an infectious disease, e.g. caused by a bacterial, viral or other pathogen. Exemplary antigens, which include antigens associated with various diseases and conditions that can be treated, are described above. In particular embodiments, the chimeric antigen receptor or transgenic TCR specifically binds to an antigen associated with the disease or condition.

Among the diseases, conditions, and disorders are tumors, including solid tumors, hematologic malignancies, and melanomas, and including localized and metastatic tumors, infectious diseases, such as infection with a virus or other pathogen, e.g., HIV, HCV, HBV, CMV, HPV, and parasitic disease, and autoimmune and inflammatory diseases. In some embodiments, the disease, disorder or condition is a tumor, cancer, malignancy, neoplasm, or other proliferative disease or disorder. Such diseases include but are not limited to leukemia, lymphoma, e.g., acute myeloid (or myelogenous) leukemia (AML), chronic myeloid (or myelogenous) leukemia (CML), acute lymphocytic (or lymphoblastic) leukemia (ALL), chronic lymphocytic leukemia (CLL), hairy cell leukemia (HCL), small lymphocytic lymphoma (SLL), Mantle cell lymphoma (MCL), Marginal zone lymphoma, Burkitt lymphoma, Hodgkin lymphoma (HL), non-Hodgkin lymphoma (NHL), Anaplastic large cell lymphoma (ALCL), follicular lymphoma, refractory follicular lymphoma, diffuse large B-cell lymphoma (DLBCL) and multiple myeloma (MM). In some embodiments, disease or condition is a B cell malignancy selected from among acute lymphoblastic leukemia (ALL), adult ALL, chronic lymphoblastic leukemia (CLL), non-Hodgkin lymphoma (NHL), and Diffuse Large B-Cell Lymphoma (DLBCL). In some embodiments, the disease or condition is NHL and the NHL is selected from the group consisting of aggressive NHL, diffuse large B cell lymphoma (DLBCL), NOS (de novo and transformed from indolent), primary mediastinal large B cell lymphoma (PMBCL), T cell/histocyte-rich large B cell lymphoma (TCHRBCL), Burkitt's lymphoma, mantle cell lymphoma (MCL), and/or follicular lymphoma (FL), optionally, follicular lymphoma Grade 3B (FL3B).

In some embodiments, the disease or condition is an infectious disease or condition, such as, but not limited to, viral, retroviral, bacterial, and protozoal infections, immunodeficiency, Cytomegalovirus (CMV), Epstein-Barr virus (EBV), adenovirus, BK polyomavirus. In some embodiments, the disease or condition is an autoimmune or inflammatory disease or condition, such as arthritis, e.g., rheumatoid arthritis (RA), Type I diabetes, systemic lupus erythematosus (SLE), inflammatory bowel disease, psoriasis, scleroderma, autoimmune thyroid disease, Grave's disease, Crohn's disease, multiple sclerosis, asthma, and/or a disease or condition associated with transplant.

In some embodiments, the antigen associated with the disease or disorder is or includes αvβ6 integrin (avb6 integrin), B cell maturation antigen (BCMA), B7-H3, B7-H6, carbonic anhydrase 9 (CA9, also known as CAIX or G250), a cancer-testis antigen, cancer/testis antigen _{1B} (CTAG, also known as NY-ESO-1 and LAGE-2), carcinoembryonic antigen (CEA), a cyclin, cyclin A2, C-C Motif Chemokine Ligand 1 (CCL-1), CD19, CD20, CD22, CD23, CD24, CD30, CD33, CD38, CD44, CD44v6, CD44v7/8, CD123, CD133, CD138, CD171, epidermal growth factor protein (EGFR), truncated epidermal growth factor protein (tEGFR), type III epidermal growth factor receptor mutation (EGFR vIII), epithelial glycoprotein 2 (EPG-2), epithelial glycoprotein 40 (EPG-40), ephrinB2, ephrine receptor A2 (EPHa2), estrogen receptor, Fc receptor like 5 (FCRL5; also known as Fc receptor homolog 5 or FCRH5), fetal acetylcholine receptor (fetal AchR), a folate binding protein (FBP), folate receptor alpha, ganglioside GD2, O-acetylated GD2 (OGD2), ganglioside GD3, glycoprotein 100 (gp100), glypican-3 (GPC3), G protein-coupled receptor class C group 5 member D (GPRC5D), Her2/neu (receptor tyrosine kinase erb-B2), Her3 (erb-B3), Her4 (erb-B4), erbB dimers, Human high molecular weightmelanoma-associated antigen (HMW-MAA), hepatitis B surface antigen, Human leukocyte antigen A1 (HLA-A1), Human leukocyte antigen A2 (HLA-A2), IL-22 receptor alpha (IL-22Rα), IL-13 receptor alpha 2 (IL-13Rα2), kinase insert domain receptor (kdr), kappa light chain, L1 cell adhesion molecule (L1-CAM), CE7 epitope of L1-CAM, Leucine Rich Repeat Containing 8 Family Member A (LRRC8A), Lewis Y, Melanoma-associated antigen (MAGE)-A1, MAGE-A3, MAGE-A6, MAGE-A10, mesothelin (MSLN), c-Met, murine cytomegalovirus (CMV), mucin 1 (MUC1), MUC16, natural killer group 2 member D (NKG2D) ligands, melan A (MART-1), neural cell adhesion molecule (NCAM), oncofetal antigen, Preferentially expressed antigen of melanoma (PRAME), progesterone receptor, a prostate specific antigen, prostate stem cell antigen (PSCA), prostate specific membrane antigen (PSMA), Receptor Tyrosine Kinase Like Orphan Receptor 1 (ROR1), survivin, Trophoblast glycoprotein (TPBG also known as 5T4), tumor-associated glycoprotein 72 (TAG72), Tyrosinase related protein 1 (TRP1, also known as TYRP1 or gp75), Tyrosinase related protein 2 (TRP2, also known as dopachrome tautomerase, dopachrome delta-isomerase or DCT) vascular endothelial growth factor receptor (VEGFR), vascular endothelial growth factor receptor 2 (VEGFR2), Wilms Tumor 1 (WT-1), a pathogen-specific or pathogen-expressed antigen, or an antigen associated with a universal tag, and/or biotinylated molecules, and/or molecules expressed by HIV, HCV, HBV or other pathogens. Antigens targeted by the receptors in some embodiments include antigens associated with a B cell malignancy, such as any of a number of known B cell marker. In some embodiments, the antigen is or includes CD20, CD19, CD22, ROR1, CD45, CD21, CD5, CD33, Igkappa, Iglambda, CD79a, CD79b or CD30. In some embodiments, the antigen is or includes a pathogen-specific or pathogen-expressed antigen, such as a viral antigen (e.g., a viral antigen from HIV, HCV, HBV), bacterial antigens, and/or parasitic antigens.

In some embodiments, the antibody or an antigen-binding fragment (e.g. scFv or V_{H} domain) specifically recognizes an antigen, such as CD19. In some embodiments, the antibody or antigen-binding fragment is derived from, or is a variant of, antibodies or antigen-binding fragment that specifically binds to CD19. In some embodiments, the cell therapy, e.g., adoptive T cell therapy, is carried out by autologous transfer, in which the cells are isolated and/or otherwise prepared from the subject who is to receive the cell therapy, or from a sample derived from such a subject. Thus, in some aspects, the cells are derived from a subject, e.g., patient, in need of a treatment and the cells, following isolation and processing are administered to the same subject.

In some embodiments, the disease or condition is a B cell malignancy. In some embodiments, the B cell malignancy is a leukemia or a lymphoma. In some aspects, the disease or condition is acute lymphoblastic leukemia (ALL), adult ALL, chronic lymphoblastic leukemia (CLL), non-Hodgkin lymphoma (NHL), or Diffuse Large B-Cell Lymphoma (DLBCL). In some cases, the disease or condition is an NHL, such as or including an NHL that is an aggressive NHL, diffuse large B cell lymphoma (DLBCL), NOS (de novo and transformed from indolent), primary mediastinal large B cell lymphoma (PMBCL), T cell/histocyte-rich large B cell lymphoma (TCHRBCL), Burkitt's lymphoma, mantle cell lymphoma (MCL), and/or follicular lymphoma (FL), optionally, follicular lymphoma Grade 3B (FL3B). In some aspects, the recombinant receptor, such as a CAR, specifically binds to an antigen associated with the disease or condition or expressed in cells of the environment of a lesion associated with the B cell malignancy. Antigens targeted by the receptors in some embodiments include antigens associated with a B cell malignancy, such as any of a number of known B cell marker. In some embodiments, the antigen targeted by the receptor is CD20, CD19, CD22, ROR1, CD45, CD21, CD5, CD33, Igkappa, Iglambda, CD79a, CD79b or CD30, or combinations thereof.

In some embodiments, the disease or condition is a myeloma, such as a multiple myeloma. In some aspects, the recombinant receptor, such as a CAR, specifically binds to an antigen associated with the disease or condition or expressed in cells of the environment of a lesion associated with the multiple myeloma. Antigens targeted by the receptors in some embodiments include antigens associated with multiple myeloma. In some aspects, the antigen, e.g., the second or additional antigen, such as the disease-specific antigen and/or related antigen, is expressed on multiple myeloma, such as B cell maturation antigen (BCMA), G protein-coupled receptor class C group 5 member D (GPRC5D), CD38 (cyclic ADP ribose hydrolase), CD138 (syndecan-1, syndecan, SYN-1), CS-1 (CS1, CD2 subset 1, CRACC, SLAMF7, CD319, and 19A24), BAFF-R, TACI and/or FcRH5. Other exemplary multiple myeloma antigens include CD56, TIM-3, CD33, CD123, CD44, CD20, CD40, CD74, CD200, EGFR, β2-Microglobulin, HM1.24, IGF-1R, IL-6R, TRAIL-R1, and the activin receptor type IIA (ActRIIA). See Benson and Byrd, J. Clin. Oncol. (2012) 30(16): 2013-15; Tao and Anderson, Bone Marrow Research (2011):924058; Chu et al., Leukemia (2013) 28(4):917-27; Garfall et al., Discov Med. (2014) 17(91):37-46. In some embodiments, the antigens include those present on lymphoid tumors, myeloma, AIDS-associated lymphoma, and/or post-transplant lymphoproliferations, such as CD38. Antibodies or antigen-binding fragments directed against such antigens are known and include, for example, those described in U.S. Patent No. 8,153,765; 8,603477, 8,008,450; U.S. Pub. No. US20120189622 or US20100260748; and/or International PCT Publication Nos. WO2006099875, WO2009080829 or WO2012092612 or WO2014210064. In some embodiments, such antibodies or antigen-binding fragments thereof (e.g. scFv) are contained in multispecific antibodies, multispecific chimeric receptors, such as multispecific CARs, and/or multispecific cells.

In some embodiments, the disease or disorder is associated with expression of G protein-coupled receptor class C group 5 member D (GPRC5D) and/or expression of B cell maturation antigen (BCMA).

In some embodiments, the disease or disorder is a B cell-related disorder. In some of any of the provided embodiments of the provided methods, the disease or disorder associated with BCMA is an autoimmune disease or disorder. In some of any of the provided embodiments of the provided methods, the autoimmune disease or disorder is systemic lupus erythematosus (SLE), lupus nephritis, inflammatory bowel disease, rheumatoid arthritis, ANCA associated vasculitis, idiopathic thrombocytopenia purpura (ITP), thrombotic thrombocytopenia purpura (TTP), autoimmune thrombocytopenia, Chagas' disease, Grave's disease, Wegener's granulomatosis, poly-arteritis nodosa, Sjogren's syndrome, pemphigus vulgaris, scleroderma, multiple sclerosis, psoriasis, IgA nephropathy, IgM polyneuropathies, vasculitis, diabetes mellitus, Reynaud's syndrome, anti-phospholipid syndrome, Goodpasture's disease, Kawasaki disease, autoimmune hemolytic anemia, myasthenia gravis, or progressive glomerulonephritis.

In some embodiments, the disease or disorder is a cancer. In some embodiments, the cancer is a GPRC5D-expressing cancer. In some embodiments, the cancer is a plasma cell malignancy and the plasma cell malignancy is multiple myeloma (MM) or plasmacytoma. In some embodiments, the cancer is multiple myeloma (MM). In some embodiments, the cancer is a relapsed/refractory multiple myeloma.

In some embodiments, the cell therapy, e.g., adoptive T cell therapy, is carried out by allogeneic transfer, in which the cells are isolated and/or otherwise prepared from a subject other than a subject who is to receive or who ultimately receives the cell therapy, e.g., a first subject. In such embodiments, the cells then are administered to a different subject, e.g., a second subject, of the same species. In some embodiments, the first and second subjects are genetically identical. In some embodiments, the first and second subjects are genetically similar. In some embodiments, the second subject expresses the same HLA class or supertype as the first subject.

The cells can be administered by any suitable means, for example, by bolus infusion, by injection, e.g., intravenous or subcutaneous injections, intraocular injection, periocular injection, subretinal injection, intravitreal injection, trans-septal injection, subscleral injection, intrachoroidal injection, intracameral injection, subconjectval injection, subconjuntival injection, sub-Tenon's injection, retrobulbar injection, peribulbar injection, or posterior juxtascleral delivery. In some embodiments, they are administered by parenteral, intrapulmonary, and intranasal, and, if desired for local treatment, intralesional administration. Parenteral infusions include intramuscular, intravenous, intraarterial, intraperitoneal, or subcutaneous administration. In some embodiments, a given dose is administered by a single bolus administration of the cells. In some embodiments, it is administered by multiple bolus administrations of the cells, for example, over a period of no more than 3 days, or by continuous infusion administration of the cells. In some embodiments, administration of the cell dose or any additional therapies, e.g., the lymphodepleting therapy, intervention therapy and/or combination therapy, is carried out via outpatient delivery.

For the prevention or treatment of disease, the appropriate dosage may depend on the type of disease to be treated, the type of cells or recombinant receptors, the severity and course of the disease, whether the cells are administered for preventive or therapeutic purposes, previous therapy, the subject's clinical history and response to the cells, and the discretion of the attending physician. The compositions and cells are in some embodiments suitably administered to the subject at one time or over a series of treatments.

In some embodiments, the cells are administered as part of a combination treatment, such as simultaneously with or sequentially with, in any order, another therapeutic intervention, such as an antibody or engineered cell or receptor or agent, such as a cytotoxic or therapeutic agent. The cells in some embodiments are co-administered with one or more additional therapeutic agents or in connection with another therapeutic intervention, either simultaneously or sequentially in any order. In some contexts, the cells are co-administered with another therapy sufficiently close in time such that the cell populations enhance the effect of one or more additional therapeutic agents, or vice versa. In some embodiments, the cells are administered prior to the one or more additional therapeutic agents. In some embodiments, the cells are administered after the one or more additional therapeutic agents. In some embodiments, the one or more additional agents include a cytokine, such as IL-2, for example, to enhance persistence. In some embodiments, the methods comprise administration of a chemotherapeutic agent.

In some embodiments, the subject is administered a chemotherapeutic agent, e.g., a conditioning chemotherapeutic agent, for example, to reduce tumor burden prior to the administration.

Preconditioning subjects with immunodepleting (e.g., lymphodepleting) therapies in some aspects can improve the effects of adoptive cell therapy (ACT).

Thus, in some embodiments, the subject is administered a preconditioning agent, such as a lymphodepleting or chemotherapeutic agent, such as cyclophosphamide, fludarabine, or combinations thereof, to a subject prior to the initiation of the cell therapy. For example, the subject may be administered a preconditioning agent at least 2 days prior, such as at least 3, 4, 5, 6, or 7 days prior, to the initiation of the cell therapy. In some embodiments, the subject is administered a preconditioning agent no more than 7 days prior, such as no more than 6, 5, 4, 3, or 2 days prior, to the initiation of the cell therapy.

In some embodiments, the subject is preconditioned with cyclophosphamide at a dose between or between about 20 mg/kg and 100 mg/kg, such as between or between about 40 mg/kg and 80 mg/kg. In some aspects, the subject is preconditioned with or with about 60 mg/kg of cyclophosphamide. In some embodiments, the cyclophosphamide can be administered in a single dose or can be administered in a plurality of doses, such as given daily, every other day or every three days. In some embodiments, the cyclophosphamide is administered once daily for one or two days. In some embodiments, where the lymphodepleting agent comprises cyclophosphamide, the subject is administered cyclophosphamide at a dose between or between about 100 mg/m² and 500 mg/m², such as between or between about 200 mg/m² and 400 mg/m², or 250 mg/m² and 350 mg/m², inclusive. In some instances, the subject is administered about 300 mg/m² of cyclophosphamide. In some embodiments, the cyclophosphamide can be administered in a single dose or can be administered in a plurality of doses, such as given daily, every other day or every three days. In some embodiments, cyclophosphamide is administered daily, such as for 1-5 days, for example, for 3 to 5 days. In some instances, the subject is administered about 300 mg/m² of cyclophosphamide, daily for 3 days, prior to initiation of the cell therapy.

In some embodiments, where the lymphodepleting agent comprises fludarabine, the subject is administered fludarabine at a dose between or between about 1 mg/m² and 100 mg/m², such as between or between about 10 mg/m² and 75 mg/m², 15 mg/m² and 50 mg/m², 20 mg/m² and 40 mg/m², or 24 mg/m² and 35 mg/m², inclusive. In some instances, the subject is administered about 30 mg/m² of fludarabine. In some embodiments, the fludarabine can be administered in a single dose or can be administered in a plurality of doses, such as given daily, every other day or every three days. In some embodiments, fludarabine is administered daily, such as for 1-5 days, for example, for 3 to 5 days. In some instances, the subject is administered about 30 mg/m² of fludarabine, daily for 3 days, prior to initiation of the cell therapy.

In some embodiments, the lymphodepleting agent comprises a combination of agents, such as a combination of cyclophosphamide and fludarabine. Thus, the combination of agents may include cyclophosphamide at any dose or administration schedule, such as those described above, and fludarabine at any dose or administration schedule, such as those described above. For example, in some aspects, the subject is administered 60 mg/kg (~2 g/m²) of cyclophosphamide and 3 to 5 doses of 25 mg/m² fludarabine prior to the first or subsequent dose.

Following administration of the cells, the biological activity of the engineered cell populations in some embodiments is measured, e.g., by any of a number of known methods. Parameters to assess include specific binding of an engineered or natural T cell or other immune cell to antigen, in vivo, e.g., by imaging, or ex vivo, e.g., by ELISA or flow cytometry. In certain embodiments, the ability of the engineered cells to destroy target cells can be measured using any suitable known methods, such as cytotoxicity assays described in, for example, Kochenderfer et al., J. Immunotherapy, 32(7): 689-702 (2009), and Herman et al. J. Immunological Methods, 285(1): 25-40 (2004). In certain embodiments, the biological activity of the cells is measured by assaying expression and/or secretion of one or more cytokines, such as CD107a, IFNγ, IL-2, and TNF. In some aspects the biological activity is measured by assessing clinical outcome, such as reduction in tumor burden or load.

In certain embodiments, the engineered cells are further modified in any number of ways, such that their therapeutic or prophylactic efficacy is increased. For example, the engineered CAR or TCR expressed by the population can be conjugated either directly or indirectly through a linker to a targeting moiety. The practice of conjugating compounds, e.g., the CAR or TCR, to targeting moieties is known. See, for instance, Wadwa et al., J. Drug Targeting 3: 1 1 1 (1995), and U.S. Patent 5,087,616.

In some embodiments, the cells are administered as part of a combination treatment, such as simultaneously with or sequentially with, in any order, another therapeutic intervention, such as an antibody or engineered cell or receptor or agent, such as a cytotoxic or therapeutic agent. The cells in some embodiments are co-administered with one or more additional therapeutic agents or in connection with another therapeutic intervention, either simultaneously or sequentially in any order. In some contexts, the cells are co-administered with another therapy sufficiently close in time such that the cell populations enhance the effect of one or more additional therapeutic agents, or vice versa. In some embodiments, the cells are administered prior to the one or more additional therapeutic agents. In some embodiments, the cells are administered after the one or more additional therapeutic agents. In some embodiments, the one or more additional agent includes a cytokine, such as IL-2, for example, to enhance persistence.

### A. Dosing

In some embodiments, a dose of cells is administered to subjects in accord with the provided methods, and/or with the provided articles of manufacture or compositions. In some embodiments, the size or timing of the doses is determined as a function of the particular disease or condition in the subject. In some cases, the size or timing of the doses for a particular disease in view of the provided description may be empirically determined.

In some embodiments, the dose of cells comprises between at or about 2 x 10⁵ of the cells/kg and at or about 2 x 10⁶ of the cells/kg, such as between at or about 4 x 10⁵ of the cells/kg and at or about 1 x 10⁶ of the cells/kg or between at or about 6 x 10⁵ of the cells/kg and at or about 8 x 10⁵ of the cells/kg. In some embodiments, the dose of cells comprises no more than 2 x 10⁵ of the cells (e.g. antigen-expressing, such as CAR-expressing cells) per kilogram body weight of the subject (cells/kg), such as no more than at or about 3 x 10⁵ cells/kg, no more than at or about 4 x 10⁵ cells/kg, no more than at or about 5 x 10⁵ cells/kg, no more than at or about 6 x 10⁵ cells/kg, no more than at or about 7 x 10⁵ cells/kg, no more than at or about 8 x 10⁵ cells/kg, no more than at or about 9 x 10⁵ cells/kg, no more than at or about 1 x 10⁶ cells/kg, or no more than at or about 2 x 10⁶ cells/kg. In some embodiments, the dose of cells comprises at least or at least about or at or about 2 x 10⁵ of the cells (e.g. antigen-expressing, such as CAR-expressing cells) per kilogram body weight of the subject (cells/kg), such as at least or at least about or at or about 3 x 10⁵ cells/kg, at least or at least about or at or about 4 x 10⁵ cells/kg, at least or at least about or at or about 5 x 10⁵ cells/kg, at least or at least about or at or about 6 x 10⁵ cells/kg, at least or at least about or at or about 7 x 10⁵ cells/kg, at least or at least about or at or about 8 x 10⁵ cells/kg, at least or at least about or at or about 9 x 10⁵ cells/kg, at least or at least about or at or about 1 x 10⁶ cells/kg, or at least or at least about or at or about 2 x 10⁶ cells/kg.

In certain embodiments, the cells, or individual populations of sub-types of cells, are administered to the subject at a range of at or about 0.1 million to at or about 100 billion cells and/or that amount of cells per kilogram of body weight of the subject, such as, *e.g.,* at or about 0.1 million to at or about 50 billion cells *(e.g.,* at or about 5 million cells, at or about 25 million cells, at or about 500 million cells, at or about 1 billion cells, at or about 5 billion cells, at or about 20 billion cells, at or about 30 billion cells, at or about 40 billion cells, or a range defined by any two of the foregoing values), at or about 1 million to at or about 50 billion cells *(e.g.,* at or about 5 million cells, at or about 25 million cells, at or about 500 million cells, at or about 1 billion cells, at or about 5 billion cells, at or about 20 billion cells, at or about 30 billion cells, at or about 40 billion cells, or a range defined by any two of the foregoing values), such as at or about 10 million to at or about 100 billion cells *(e.g.,* at or about 20 million cells, at or about 30 million cells, at or about 40 million cells, at or about 60 million cells, at or about 70 million cells, at or about 80 million cells, at or about 90 million cells, at or about 10 billion cells, at or about 25 billion cells, at or about 50 billion cells, at or about 75 billion cells, at or about 90 billion cells, or a range defined by any two of the foregoing values), and in some cases at or about 100 million cells to at or about 50 billion cells *(e.g.,* at or about 120 million cells, at or about 250 million cells, at or about 350 million cells, about 450 million cells, at or about 650 million cells, at or about 800 million cells, at or about 900 million cells, at or about 3 billion cells, at or about 30 billion cells, at or about 45 billion cells) or any value in between these ranges and/or per kilogram of body weight of the subject. Dosages may vary depending on attributes particular to the disease or disorder and/or patient and/or other treatments.

In some embodiments, the dose of cells is a flat dose of cells or fixed dose of cells such that the dose of cells is not tied to or based on the body surface area or weight of a subject. In some embodiments, such values refer to numbers of recombinant receptor-expressing cells; in other embodiments, they refer to number of T cells or PBMCs or total cells administered.

In some embodiments, for example, where the subject is a human, the dose includes fewer than about 5 x 10⁸ total recombinant receptor (e.g., CAR)-expressing cells, T cells, or peripheral blood mononuclear cells (PBMCs), e.g., in the range of about 1 x 10⁶ to 5 x 10⁸ such cells, such as 2 x 10⁶, 5 x 10⁶, 1 x 10⁷, 5 x 10⁷, 1 x 10⁸, or 5 x 10⁸, at or about 1 x 10⁶ to at or about 5 x 10⁸ such cells, such as at or about 2 x 10⁶, 5 x 10⁶, 1 x 10⁷, 5 x 10⁷, 1 x 10⁸, 1.5 x 10⁸, or 5 x 10⁸ total such cells, or the range between any two of the foregoing values. In some embodiments, for example, where the subject is a human, the dose includes more than at or about 1 x 10⁶ total recombinant receptor (e.g., CAR)-expressing cells, T cells, or peripheral blood mononuclear cells (PBMCs) and fewer than at or about 2 x 10⁹ total recombinant receptor (e.g., CAR)-expressing cells, T cells, or peripheral blood mononuclear cells (PBMCs), e.g., in the range of at or about 2.5 x 10⁷ to at or about 1.2 x 10⁹ such cells, such as at or about 2.5 x 10⁷, 5 x 10⁷, 1 x 10⁸, 1.5 x 10⁸, 8 x 10⁸,or 1.2 x 10⁹ total such cells, or the range between any two of the foregoing values.

In some embodiments, the dose of genetically engineered cells comprises from at or about 1 x 10⁵ to at or about 5 x 10⁸ total CAR-expressing (CAR+) T cells, from at or about 1 x 10⁵ to at or about 2.5 x 10⁸ total CAR-expressing T cells, from at or about 1 x 10⁵ to at or about 1 x 10⁸ total CAR-expressing T cells, from at or about 1 x 10⁵ to at or about 5 x 10⁷ total CAR-expressing T cells, from at or about 1 x 10⁵ to at or about 2.5 x 10⁷ total CAR-expressing T cells, from at or about 1 x 10⁵ to at or about 1 x 10⁷ total CAR-expressing T cells, from at or about 1 x 10⁵ to at or about 5 x 10⁶ total CAR-expressing T cells, from at or about 1 x 10⁵ to at or about 2.5 x 10⁶ total CAR-expressing T cells, from at or about 1 x 10⁵ to at or about 1 x 10⁶ total CAR-expressing T cells, from at or about 1 x 10⁶ to at or about 5 x 10⁸ total CAR-expressing T cells, from at or about 1 x 10⁶ to at or about 2.5 x 10⁸ total CAR-expressing T cells, from at or about 1 x 10⁶ to at or about 1 x 10⁸ total CAR-expressing T cells, from at or about 1 x 10⁶ to at or about 5 x 10⁷ total CAR-expressing T cells, from at or about 1 x 10⁶ to at or about 2.5 x 10⁷ total CAR-expressing T cells, from at or about 1 x 10⁶ to at or about 1 x 10⁷ total CAR-expressing T cells, from at or about 1 x 10⁶ to at or about 5 x 10⁶ total CAR-expressing T cells, from at or about 1 x 10⁶ to at or about 2.5 x 10⁶ total CAR-expressing T cells, from at or about 2.5 x 10⁶ to at or about 5 x 10⁸ total CAR-expressing T cells, from at or about 2.5 x 10⁶ to at or about 2.5 x 10⁸ total CAR-expressing T cells, from at or about 2.5 x 10⁶ to at or about 1 x 10⁸ total CAR-expressing T cells, from at or about 2.5 x 10⁶ to at or about 5 x 10⁷ total CAR-expressing T cells, from at or about 2.5 x 10⁶ to at or about 2.5 x 10⁷ total CAR-expressing T cells, from at or about 2.5 x 10⁶ to at or about 1 x 10⁷ total CAR-expressing T cells, from at or about 2.5 x 10⁶ to at or about 5 x 10⁶ total CAR-expressing T cells, from at or about 5 x 10⁶ to at or about 5 x 10⁸ total CAR-expressing T cells, from at or about 5 x 10⁶ to at or about 2.5 x 10⁸ total CAR-expressing T cells, from at or about 5 x 10⁶ to at or about 1 x 10⁸ total CAR-expressing T cells, from at or about 5 x 10⁶ to at or about 5 x 10⁷ total CAR-expressing T cells, from at or about 5 x 10⁶ to at or about 2.5 x 10⁷ total CAR-expressing T cells, from at or about 5 x 10⁶ to at or about 1 x 10⁷ total CAR-expressing T cells, from at or about 1 x 10⁷ to at or about 5 x 10⁸ total CAR-expressing T cells, from at or about 1 x 10⁷ to at or about 2.5 x 10⁸ total CAR-expressing T cells, from at or about 1 x 10⁷ to at or about 1 x 10⁸ total CAR-expressing T cells, from at or about 1 x 10⁷ to at or about 5 x 10⁷ total CAR-expressing T cells, from at or about 1 x 10⁷ to at or about 2.5 x 10⁷ total CAR-expressing T cells, from at or about 2.5 x 10⁷ to at or about 5 x 10⁸ total CAR-expressing T cells, from at or about 2.5 x 10⁷ to at or about 2.5 x 10⁸ total CAR-expressing T cells, from at or about 2.5 x 10⁷ to at or about 1 x 10⁸ total CAR-expressing T cells, from at or about 2.5 x 10⁷ to at or about 5 x 10⁷ total CAR-expressing T cells, from at or about 5 x 10⁷ to at or about 5 x 10⁸ total CAR-expressing T cells, from at or about 5 x 10⁷ to at or about 2.5 x 10⁸ total CAR-expressing T cells, from at or about 5 x 10⁷ to at or about 1 x 10⁸ total CAR-expressing T cells, from at or about 1 x 10⁸ to at or about 5 x 10⁸ total CAR-expressing T cells, from at or about 1 x 10⁸ to at or about 2.5 x 10⁸ total CAR-expressing T cells, from at or about or 2.5 x 10⁸ to at or about 5 x 10⁸ total CAR-expressing T cells. In some embodiments, the dose of genetically engineered cells comprises from or from about 2.5 x 10⁷ to at or about 1.5 x 10⁸ total CAR-expressing T cells, such as from or from about 5 x 10⁷ to or to about 1 x 10⁸ total CAR-expressing T cells.

In some embodiments, the dose of genetically engineered cells comprises at least at or about 1 x 10⁵ CAR-expressing cells, at least at or about 2.5 x 10⁵ CAR-expressing cells, at least at or about 5 x 10⁵ CAR-expressing cells, at least at or about 1 x 10⁶ CAR-expressing cells, at least at or about 2.5 x 10⁶ CAR-expressing cells, at least at or about 5 x 10⁶ CAR-expressing cells, at least at or about 1 x 10⁷ CAR-expressing cells, at least at or about 2.5 x 10⁷ CAR-expressing cells, at least at or about 5 x 10⁷ CAR-expressing cells, at least at or about 1 x 10⁸ CAR-expressing cells, at least at or about 1.5 x 10⁸ CAR-expressing cells, at least about 5 x 10⁶ CAR-expressing cells, at least or at least about 1 x 10⁷ CAR-expressing cells, at least or at least about 2.5 x 10⁷ CAR-expressing cells, at least or at least about 5 x 10⁷ CAR-expressing cells, at least or at least about 1 x 10⁸ CAR-expressing cells, at least or at least about 2.5 x 10⁸ CAR-expressing cells, or at least or at least about 5 x 10⁸ CAR-expressing cells.

In some embodiments, the cell therapy comprises administration of a dose comprising a number of cell from or from about 1 x 10⁵ to or to about 5 x 10⁸ total recombinant receptor-expressing cells, total T cells, or total peripheral blood mononuclear cells (PBMCs), from or from about 5 x 10⁵ to or to about 1 x 10⁷ total recombinant receptor-expressing cells, total T cells, or total peripheral blood mononuclear cells (PBMCs) or from or from about 1 x 10⁶ to or to about 1 x 10⁷ total recombinant receptor-expressing cells, total T cells, or total peripheral blood mononuclear cells (PBMCs), each inclusive. In some embodiments, the cell therapy comprises administration of a dose of cells comprising a number of cells at least or at least about 1 x 10⁵ total recombinant receptor-expressing cells, total T cells, or total peripheral blood mononuclear cells (PBMCs), such at least or at least 1 x 10⁶, at least or at least about 1 x 10⁷, at least or at least about 1 x 10⁸ of such cells. In some embodiments, the number is with reference to the total number of CD3-expressing or CD8-expressing, in some cases also recombinant receptor-expressing (e.g. CAR-expressing) cells. In some embodiments, the cell therapy comprises administration of a dose comprising a number of cell from or from about 1 x 10⁵ to or to about 5 x 10⁸ CD3-expressing or CD8-expressing total T cells or CD3-expressing or CD8-expressing recombinant receptor-expressing cells, from or from about 5 x 10⁵ to or to about 1 x 10⁷ CD3-expressing or CD8-expressing total T cells or CD3-expressing or CD8-expressing recombinant receptor-expressing cells, or from or from about 1 x 10⁶ to or to about 1 x 10⁷ CD3-expressing or CD8-expressing total T cells or CD3-expressing or CD8-expressingrecombinant receptor-expressing cells, each inclusive. In some embodiments, the cell therapy comprises administration of a dose comprising a number of cell from or from about 1 x 10⁵ to or to about 5 x 10⁸ total CD3-expressing/CAR-expressing or CD8-expressing/CAR-expressing cells, from or from about 5 x 10⁵ to or to about 1 x 10⁷ total CD3-expressing/CAR-expressing or CD8-expressing/CAR-expressing cells, or from or from about 1 x 10⁶ to or to about 1 x 10⁷ total CD3-expressing/CAR-expressing or CD8-expressing/CAR-expressing cells, each inclusive.

In some embodiments, the T cells of the dose include CD4+ T cells, CD8+ T cells or CD4+ and CD8+ T cells.

In some embodiments, for example, where the subject is human, the CD8⁺ T cells of the dose, including in a dose including CD4⁺ and CD8⁺ T cells, includes between at or about 1 x 10⁶ and at or about 5 x 10⁸ total recombinant receptor (e.g., CAR)-expressing CD8⁺cells, e.g., in the range of from at or about 5 x 10⁶ to at or about 1 x 10⁸ such cells, such as 1 x 10⁷, 2.5 x 10⁷, 5 x 10⁷, 7.5 x 10⁷, 1 x 10⁸, 1.5 x 10⁸, or 5 x 10⁸ total such cells, or the range between any two of the foregoing values. In some embodiments, the patient is administered multiple doses, and each of the doses or the total dose can be within any of the foregoing values. In some embodiments, the dose of cells comprises the administration of from or from about 1 x 10⁷ to or to about 0.75 x 10⁸ total recombinant receptor-expressing CD8⁺ T cells, from or from about 1 x 10⁷ to or to about 5 x 10⁷ total recombinant receptor-expressing CD8⁺ T cells, from or from about 1 x 10⁷ to or to about 0.25 x 10⁸ total recombinant receptor-expressing CD8⁺ T cells, each inclusive. In some embodiments, the dose of cells comprises the administration of at or about 1 x 10⁷ , 2.5 x 10⁷, 5 x 10⁷, 7.5 x 10⁷, 1 x 10⁸, 1.5 x 10⁸, 2.5 x 10⁸, or 5 x 10⁸ total recombinant receptor-expressing CD8⁺ T cells.

In some embodiments, the dose of cells, e.g., recombinant receptor-expressing T cells, is administered to the subject as a single dose or is administered only one time within a period of two weeks, one month, three months, six months, 1 year or more.

In the context of adoptive cell therapy, administration of a given "dose" encompasses administration of the given amount or number of cells as a single composition and/or single uninterrupted administration, e.g., as a single injection or continuous infusion, and also encompasses administration of the given amount or number of cells as a split dose or as a plurality of compositions, provided in multiple individual compositions or infusions, over a specified period of time, such as over no more than 3 days. Thus, in some contexts, the dose is a single or continuous administration of the specified number of cells, given or initiated at a single point in time. In some contexts, however, the dose is administered in multiple injections or infusions over a period of no more than three days, such as once a day for three days or for two days or by multiple infusions over a single day period.

Thus, in some aspects, the cells of the dose are administered in a single pharmaceutical composition. In some embodiments, the cells of the dose are administered in a plurality of compositions, collectively containing the cells of the dose.

In some embodiments, the term "split dose" refers to a dose that is split so that it is administered over more than one day. This type of dosing is encompassed by the present methods and is considered to be a single dose.

Thus, the dose of cells may be administered as a split dose, e.g., a split dose administered over time. For example, in some embodiments, the dose may be administered to the subject over 2 days or over 3 days. Exemplary methods for split dosing include administering 25% of the dose on the first day and administering the remaining 75% of the dose on the second day. In other embodiments, 33% of the dose may be administered on the first day and the remaining 67% administered on the second day. In some aspects, 10% of the dose is administered on the first day, 30% of the dose is administered on the second day, and 60% of the dose is administered on the third day. In some embodiments, the split dose is not spread over more than 3 days.

In some embodiments, cells of the dose may be administered by administration of a plurality of compositions or solutions, such as a first and a second, optionally more, each containing some cells of the dose. In some aspects, the plurality of compositions, each containing a different population and/or sub-types of cells, are administered separately or independently, optionally within a certain period of time. For example, the populations or sub-types of cells can include CD8⁺ and CD4⁺ T cells, respectively, and/or CD8⁺- and CD4⁺-enriched populations, respectively, e.g., CD4⁺ and/or CD8⁺ T cells each individually including cells genetically engineered to express the recombinant receptor. In some embodiments, the administration of the dose comprises administration of a first composition comprising a dose of CD8⁺ T cells or a dose of CD4⁺ T cells and administration of a second composition comprising the other of the dose of CD4⁺ T cells and the CD8⁺ T cells.

In some embodiments, the administration of the composition or dose, e.g., administration of the plurality of cell compositions, involves administration of the cell compositions separately. In some aspects, the separate administrations are carried out simultaneously, or sequentially, in any order. In some embodiments, the dose comprises a first composition and a second composition, and the first composition and second composition are administered from at or about 0 to at or about 12 hours apart, from at or about 0 to at or about 6 hours apart or from at or about 0 to at or about 2 hours apart. In some embodiments, the initiation of administration of the first composition and the initiation of administration of the second composition are carried out no more than at or about 2 hours, no more than at or about 1 hour, or no more than at or about 30 minutes apart, no more than at or about 15 minutes, no more than at or about 10 minutes or no more than at or about 5 minutes apart. In some embodiments, the initiation and/or completion of administration of the first composition and the completion and/or initiation of administration of the second composition are carried out no more than at or about 2 hours, no more than at or about 1 hour, or no more than at or about 30 minutes apart, no more than at or about 15 minutes, no more than at or about 10 minutes or no more than at or about 5 minutes apart.

In some composition, the first composition, e.g., first composition of the dose, comprises CD4⁺ T cells. In some composition, the first composition, e.g., first composition of the dose, comprises CD8⁺ T cells. In some embodiments, the first composition is administered prior to the second composition.

In some embodiments, the dose or composition of cells includes a defined or target ratio of CD4⁺ cells expressing a recombinant receptor to CD8⁺ cells expressing a recombinant receptor and/or of CD4⁺ cells to CD8⁺ cells, which ratio optionally is approximately 1:1 or is between approximately 1:3 and approximately 3:1, such as approximately 1:1. In some aspects, the administration of a composition or dose with the target or desired ratio of different cell populations (such as CD4⁺:CD8⁺ ratio or CAR⁺CD4⁺:CAR+CD8⁺ ratio, e.g., 1:1) involves the administration of a cell composition containing one of the populations and then administration of a separate cell composition comprising the other of the populations, where the administration is at or approximately at the target or desired ratio. In some aspects, administration of a dose or composition of cells at a defined ratio leads to improved expansion, persistence and/or antitumor activity of the T cell therapy.

In some embodiments, the subject receives multiple doses, e.g., two or more doses or multiple consecutive doses, of the cells. In some embodiments, two doses are administered to a subject. In some embodiments, the subject receives the consecutive dose, e.g., second dose, is administered approximately 4, 5, 6, 7, 8, 9, 10, 11, 12, 13, 14, 15, 16, 17, 18, 19, 20 or 21 days after the first dose. In some embodiments, multiple consecutive doses are administered following the first dose, such that an additional dose or doses are administered following administration of the consecutive dose. In some aspects, the number of cells administered to the subject in the additional dose is the same as or similar to the first dose and/or consecutive dose. In some embodiments, the additional dose or doses are larger than prior doses.

In some aspects, the size of the first and/or consecutive dose is determined based on one or more criteria such as response of the subject to prior treatment, e.g. chemotherapy, disease burden in the subject, such as tumor load, bulk, size, or degree, extent, or type of metastasis, stage, and/or likelihood or incidence of the subject developing toxic outcomes, e.g., CRS, macrophage activation syndrome, tumor lysis syndrome, neurotoxicity, and/or a host immune response against the cells and/or recombinant receptors being administered.

In some aspects, the time between the administration of the first dose and the administration of the consecutive dose is about 9 to about 35 days, about 14 to about 28 days, or 15 to 27 days. In some embodiments, the administration of the consecutive dose is at a time point more than about 14 days after and less than about 28 days after the administration of the first dose. In some aspects, the time between the first and consecutive dose is about 21 days. In some embodiments, an additional dose or doses, e.g. consecutive doses, are administered following administration of the consecutive dose. In some aspects, the additional consecutive dose or doses are administered at least about 14 and less than about 28 days following administration of a prior dose. In some embodiments, the additional dose is administered less than about 14 days following the prior dose, for example, 4, 5, 6, 7, 8, 9, 10, 11, 12, or 13 days after the prior dose. In some embodiments, no dose is administered less than about 14 days following the prior dose and/or no dose is administered more than about 28 days after the prior dose.

In some embodiments, the dose of cells, e.g., recombinant receptor-expressing cells, comprises two doses (e.g., a double dose), comprising a first dose of the T cells and a consecutive dose of the T cells, wherein one or both of the first dose and the second dose comprises administration of the split dose of T cells.

In some embodiments, the dose of cells is generally large enough to be effective in reducing disease burden.

In some embodiments, the cells are administered at a desired dosage, which in some aspects includes a desired dose or number of cells or cell type(s) and/or a desired ratio of cell types. Thus, the dosage of cells in some embodiments is based on a total number of cells (or number per kg body weight) and a desired ratio of the individual populations or sub-types, such as the CD4⁺ to CD8⁺ ratio. In some embodiments, the dosage of cells is based on a desired total number (or number per kg of body weight) of cells in the individual populations or of individual cell types. In some embodiments, the dosage is based on a combination of such features, such as a desired number of total cells, desired ratio, and desired total number of cells in the individual populations.

In some embodiments, the populations or sub-types of cells, such as CD8⁺ and CD4⁺ T cells, are administered at or within a tolerated difference of a desired dose of total cells, such as a desired dose of T cells. In some aspects, the desired dose is a desired number of cells or a desired number of cells per unit of body weight of the subject to whom the cells are administered, e.g., cells/kg. In some aspects, the desired dose is at or above a minimum number of cells or minimum number of cells per unit of body weight. In some aspects, among the total cells, administered at the desired dose, the individual populations or sub-types are present at or near a desired output ratio (such as CD4⁺ to CD8⁺ ratio), e.g., within a certain tolerated difference or error of such a ratio.

In some embodiments, the cells are administered at or within a tolerated difference of a desired dose of one or more of the individual populations or sub-types of cells, such as a desired dose of CD4⁺ cells and/or a desired dose of CD8⁺ cells. In some aspects, the desired dose is a desired number of cells of the sub-type or population, or a desired number of such cells per unit of body weight of the subject to whom the cells are administered, e.g., cells/kg. In some aspects, the desired dose is at or above a minimum number of cells of the population or sub-type, or minimum number of cells of the population or sub-type per unit of body weight.

Thus, in some embodiments, the dosage is based on a desired fixed dose of total cells and a desired ratio, and/or based on a desired fixed dose of one or more, e.g., each, of the individual sub-types or sub-populations. Thus, in some embodiments, the dosage is based on a desired fixed or minimum dose of T cells and a desired ratio of CD4⁺ to CD8⁺ cells, and/or is based on a desired fixed or minimum dose of CD4⁺ and/or CD8⁺ cells.

In some embodiments, the cells are administered at or within a tolerated range of a desired output ratio of multiple cell populations or sub-types, such as CD4⁺ and CD8⁺ cells or sub-types. In some aspects, the desired ratio can be a specific ratio or can be a range of ratios. for example, in some embodiments, the desired ratio (e.g., ratio of CD4⁺ to CD8⁺ cells) is between at or about 5:1 and at or about 5:1 (or greater than about 1:5 and less than about 5:1), or between at or about 1:3 and at or about 3:1 (or greater than about 1:3 and less than about 3:1), such as between at or about 2:1 and at or about 1:5 (or greater than about 1:5 and less than about 2:1, such as at or about 5:1, 4.5:1, 4:1, 3.5:1, 3:1, 2.5:1, 2:1, 1.9:1, 1.8:1, 1.7:1, 1.6:1, 1.5:1, 1.4:1, 1.3:1, 1.2:1, 1.1:1, 1:1, 1:1.1, 1:1.2, 1:1.3, 1:1.4, 1:1.5, 1:1.6, 1:1.7, 1:1.8, 1:1.9: 1:2, 1:2.5, 1:3, 1:3.5, 1:4, 1:4.5, or 1:5. In some aspects, the tolerated difference is within about 1%, about 2%, about 3%, about 4% about 5%, about 10%, about 15%, about 20%, about 25%, about 30%, about 35%, about 40%, about 45%, about 50% of the desired ratio, including any value in between these ranges.

In particular embodiments, the numbers and/or concentrations of cells refer to the number of recombinant receptor (e.g., CAR)-expressing cells. In other embodiments, the numbers and/or concentrations of cells refer to the number or concentration of all cells, T cells, or peripheral blood mononuclear cells (PBMCs) administered.

In some aspects, the size of the dose is determined based on one or more criteria such as response of the subject to prior treatment, e.g. chemotherapy, disease burden in the subject, such as tumor load, bulk, size, or degree, extent, or type of metastasis, stage, and/or likelihood or incidence of the subject developing toxic outcomes, e.g., CRS, macrophage activation syndrome, tumor lysis syndrome, neurotoxicity, and/or a host immune response against the cells and/or recombinant receptors being administered.

In some embodiments, the methods also include administering one or more additional doses of cells expressing a chimeric antigen receptor (CAR) and/or lymphodepleting therapy, and/or one or more steps of the methods are repeated. In some embodiments, the one or more additional dose is the same as the initial dose. In some embodiments, the one or more additional dose is different from the initial dose, e.g., higher, such as 2-fold, 3-fold, 4-fold, 5-fold, 6-fold, 7-fold, 8-fold, 9-fold or 10-fold or more higher than the initial dose, or lower, such as e.g., higher, such as 2-fold, 3-fold, 4-fold, 5-fold, 6-fold, 7-fold, 8-fold, 9-fold or 10-fold or more lower than the initial dose. In some embodiments, administration of one or more additional doses is determined based on response of the subject to the initial treatment or any prior treatment, disease burden in the subject, such as tumor load, bulk, size, or degree, extent, or type of metastasis, stage, and/or likelihood or incidence of the subject developing toxic outcomes, e.g., CRS, macrophage activation syndrome, tumor lysis syndrome, neurotoxicity, and/or a host immune response against the cells and/or recombinant receptors being administered.

### B. Assessment of Administered Cells

In some cases, the provided methods can be used to determine the presence, absence or number of engineered cells in a sample from a subject, after the administration of the engineered cells or cell compositions, e.g., containing cells in which transgene sequences are integrated. In some aspects, the provided methods can be used to assess the presence, absence or amount of the transgene sequence in a particular sample obtained from a subject that has been administered the engineered cells or cell composition, such as those generated using methods described herein, for example, in Section II above. In some aspects, the provided methods can be used to assess pharmacokinetic (PK) or pharmacodynamic (PD) parameters of the administered engineered cells. In some embodiments, the pharmacokinetic parameters include maximum (peak) plasma concentration (Cₘₐₓ), the peak time (*i.e.* when maximum plasma concentration (Cₘₐₓ) occurs; Tₘₐₓ), the minimum plasma concentration (*i.e.* the minimum plasma concentration between doses of a therapeutic agent, e.g., CAR⁺ T cells; Cₘᵢₙ), the elimination half-life (T_{1/2}) and area under the curve (*i.e.* the area under the curve generated by plotting time versus plasma concentration of the therapeutic agent CAR⁺ T cells; AUC), following administration.

In some aspects, an exemplary embodiment involve assessing and/or monitoring pharmacokinetic parameters, e.g., number or concentration of CAR⁺ T cells in a sample obtained from a subject that has been administered the engineered cells or composition containing the engineered cells, e.g., in the blood, and/or the amount or concentration of transgene sequences present a sample from the subject. In some aspects, an exemplary embodiment involve assessing and/or monitoring pharmacokinetic parameters, e.g., number or concentration of CAR⁺ T cells in the blood. In some embodiments, the methods involve monitoring CAR⁺ T cell numbers and/or concentration in the blood, e.g., by determining the presence, absence and/or amount of the transgene sequence in the blood, such as described in Section I.C.3 above.

### V. KITS AND ARTICLES OF MANUFACTURE WHICH ARE NOT CLAIMED

Also provided are kits and articles of manufacture, such as those containing reagents for performing the methods provided herein, e.g., reagents for assessing the presence, absence and/or amount of integrated transgene sequences. In some aspects, the kits or articles of manufacture can also contain reagents and/or nucleic acids for use in engineering or manufacturing processes to generate the engineered cells.

In some embodiments, the kits can contain reagents and/or consumables required for isolating nucleic acids from the samples and/or separating or isolating the nucleic acids based on size or molecular weight and/or determining the presence, absence and/or amount of the integrated transgene sequence. In some embodiments, the kits contain reagents and/or consumables for separating or isolating the high- or low-molecular weight fraction of the DNA, such as reagents and consumables for pulse field gel electrophoresis (PFGE). In some embodiments, the kits contain a matrix or gel or cartridges that contain matrix or gel to carry out the steps of separating or isolating the high- or low-molecular weight fraction of the DNA. In some embodiments, provided are kits that comprise one or more probes and/or one or more primers, such as a pair of primers, specific for all or a portion of the transgene sequence. In some aspects, the probes and/or primer can specifically bind to or recognize or detect all or a portion of the transgene sequence. In some aspects, the kit can contain reagents and consumables required for polymerase chain reaction (PCR), such as for quantitative PCR (qPCR), digital PCR (dPCR) or droplet digital PCR (ddPCR).

In some embodiments, the kits optionally contain other components, for example: PCR primers, PCR reagents such as polymerase, buffer, nucleotides, reagents for additional assays, e.g., intracellular cytokine staining, flow cytometry, chromatin immunoprecipitation and/or additional analysis. In some embodiments, the reagents for additional assays include components for performing an *in vitro* assay to measure the expression or level of particular molecules. In some cases, the *in vitro* assay is an immunoassay, an aptamer-based assay, a histological or cytological assay, or an mRNA expression level assay. In some embodiments, the *in vitro* assay is selected from among an enzyme linked immunosorbent assay (ELISA), immunoblotting, immunoprecipitation, radioimmunoassay (RIA), immunostaining, flow cytometry assay, surface plasmon resonance (SPR), chemiluminescence assay, lateral flow immunoassay, inhibition assay and avidity assay. In some aspects, the reagent is a binding reagent that specifically binds the molecules. In some cases, the binding reagent is an antibody or antigen-binding fragment thereof, an aptamer or a nucleic acid probe. The various components of the kit may be present in separate containers or certain compatible components may be precombined into a single container. In some embodiments, the kits further contain instructions for using the components of the kit to practice the provided methods.

### VI. DEFINITIONS

Unless defined otherwise, all terms of art, notations and other technical and scientific terms or terminology used herein are intended to have the same meaning as is commonly understood by one of ordinary skill in the art to which the claimed subject matter pertains. In some cases, terms with commonly understood meanings are defined herein for clarity and/or for ready reference, and the inclusion of such definitions herein should not necessarily be construed to represent a substantial difference over what is generally understood in the art.

As used herein, the term "transgene" or "transgene sequences" (in some cases, also called chimeric, recombinant, heterologous, exogenous sequences or chimeric, recombinant, heterologous, exogenous DNA) refer to nucleic acid sequences that have been formed artificially by combining constituents from different sources, such as different organisms, different genes or different variants. In some aspects, the transgene sequences have undergone a molecular biological manipulation, for example, by artificial combination of different nucleic acid molecules or fragments from different sources. In some embodiments, the transgene sequences contain at least some portion of the sequences that are from a different origin compared to the genomic sequence of the cells into which the polynucleotide containing the transgene sequence is introduced. In some cases, at least a portion of the transgene sequence is heterologous, exogenous or transgenic to the cell into which the transgene sequence is introduced, and can include coding and/or non-coding sequences. In some aspects, the transgene sequence can refer to sequences that are integrated into the genome of the cell into which the transgene sequence is introduced.

As used herein, the singular forms "a," "an," and "the" include plural referents unless the context clearly dictates otherwise. For example, "a" or "an" means "at least one" or "one or more." It is understood that aspects and variations described herein include "consisting" and/or "consisting essentially of" aspects and variations.

The term "about" as used herein refers to the usual error range for the respective value readily known to the skilled person in this technical field. Reference to "about" a value or parameter herein includes (and describes) embodiments that are directed to that value or parameter *per se.* For example, description referring to "about X" includes description of "X".

Throughout this disclosure, various aspects of the claimed subject matter are presented in a range format. It should be understood that the description in range format is merely for convenience and brevity and should not be construed as an inflexible limitation on the scope of the claimed subject matter. Accordingly, the description of a range should be considered to have specifically disclosed all the possible sub-ranges as well as individual numerical values within that range. For example, where a range of values is provided, it is understood that each intervening value, between the upper and lower limit of that range and any other stated or intervening value in that stated range is encompassed within the claimed subject matter. The upper and lower limits of these smaller ranges may independently be included in the smaller ranges, and are also encompassed within the claimed subject matter, subject to any specifically excluded limit in the stated range. Where the stated range includes one or both of the limits, ranges excluding either or both of those included limits are also included in the claimed subject matter. This applies regardless of the breadth of the range.

As used herein, a composition refers to any mixture of two or more products, substances, or compounds, including cells. It may be a solution, a suspension, liquid, powder, a paste, aqueous, non-aqueous or any combination thereof.

As used herein, a statement that a cell or population of cells is "positive" for a particular marker refers to the detectable presence on or in the cell of a particular marker, typically a surface marker. When referring to a surface marker, the term refers to the presence of surface expression as detected by flow cytometry, for example, by staining with an antibody that specifically binds to the marker and detecting said antibody, wherein the staining is detectable by flow cytometry at a level substantially above the staining detected carrying out the same procedure with an isotype-matched control under otherwise identical conditions and/or at a level substantially similar to that for cell known to be positive for the marker, and/or at a level substantially higher than that for a cell known to be negative for the marker.

As used herein, a statement that a cell or population of cells is "negative" for a particular marker refers to the absence of substantial detectable presence on or in the cell of a particular marker, typically a surface marker. When referring to a surface marker, the term refers to the absence of surface expression as detected by flow cytometry, for example, by staining with an antibody that specifically binds to the marker and detecting said antibody, wherein the staining is not detected by flow cytometry at a level substantially above the staining detected carrying out the same procedure with an isotype-matched control under otherwise identical conditions, and/or at a level substantially lower than that for cell known to be positive for the marker, and/or at a level substantially similar as compared to that for a cell known to be negative for the marker.

The term "vector," as used herein, refers to a nucleic acid molecule capable of propagating another nucleic acid to which it is linked. The term includes the vector as a selfreplicating nucleic acid structure as well as the vector incorporated into the genome of a host cell into which it has been introduced. Certain vectors are capable of directing the expression of nucleic acids to which they are operatively linked. Such vectors are referred to herein as "expression vectors."

As used herein, "percent (%) amino acid sequence identity" and "percent identity" when used with respect to an amino acid sequence (reference polypeptide sequence) is defined as the percentage of amino acid residues in a candidate sequence (e.g., the subject antibody or fragment) that are identical with the amino acid residues in the reference polypeptide sequence, after aligning the sequences and introducing gaps, if necessary, to achieve the maximum percent sequence identity, and not considering any conservative substitutions as part of the sequence identity. Alignment for purposes of determining percent amino acid sequence identity can be achieved in various known ways, for instance, using publicly available computer software such as BLAST, BLAST-2, ALIGN or Megalign (DNASTAR) software. Appropriate parameters for aligning sequences can be determined, including any algorithms needed to achieve maximal alignment over the full length of the sequences being compared.

In some embodiments, "operably linked" may include the association of components, such as a DNA sequence, e.g. a heterologous nucleic acid) and a regulatory sequence(s), in such a way as to permit gene expression when the appropriate molecules (e.g. transcriptional activator proteins) are bound to the regulatory sequence. Hence, it means that the components described are in a relationship permitting them to function in their intended manner.

An amino acid substitution may include replacement of one amino acid in a polypeptide with another amino acid. The substitution may be a conservative amino acid substitution or a non-conservative amino acid substitution. Amino acid substitutions may be introduced into a binding molecule, e.g., antibody, of interest and the products screened for a desired activity, e.g., retained/improved antigen binding, decreased immunogenicity, or improved ADCC or CDC.

Amino acids generally can be grouped according to the following common sidechain properties:
(1) hydrophobic: Norleucine, Met, Ala, Val, Leu, Ile;
(2) neutral hydrophilic: Cys, Ser, Thr, Asn, Gln;
(3) acidic: Asp, Glu;
(4) basic: His, Lys, Arg;
(5) residues that influence chain orientation: Gly, Pro;
(6) aromatic: Trp, Tyr, Phe.

In some embodiments, conservative substitutions can involve the exchange of a member of one of these classes for another member of the same class. In some embodiments, non-conservative amino acid substitutions can involve exchanging a member of one of these classes for another class.

As used herein, a "subject" is a mammal, such as a human or other animal, and typically is human.

### VIII. EXAMPLES

The following examples are included for illustrative purposes only and are not intended to limit the scope of the invention, which are defined in the appended claims.

### Example 1: Method to Assess Integrated Transgene Copy Number in Cells Engineered to Express a Recombinant Protein with Pulse Field Gel Electrophoresis

The use of pulse field gel electrophoresis (PFGE) was investigated as a strategy to separate high molecular weight DNA in methods for assessing vector copy number in cells transduced with a viral vector containing a transgene sequence encoding a recombinant protein.

A Jurkat T cell line was transduced with a lentiviral preparation containing a transgene sequence encoding a recombinant protein, in this case a chimeric antigen receptor (CAR). The cells were cultured for 3 days, and then genomic DNA was isolated from the cells. As a control, genomic DNA was isolated from cells that had not been transduced with a lentivirus encoding the transgene sequence, and the isolated genomic DNA was spiked with a known amount of a plasmid encoding the recombinant protein and a non-integrating viral packaging plasmid encoding Vesicular stomatitis Indiana virus G protein (VSVg).

The DNA samples were subjected to automated pulse field gel electrophoresis (PFGE) using the BluePippin (Sage Science, Beverly, MA) device to separate high molecular weight DNA species from low molecular weight, non-chromosomal DNA species below a threshold of 15 kb, 17.5 kb or 20 kb.

Exemplary quantitative polymerase chain reaction (qPCR) methods, such as droplet digital PCR (ddPCR), was carried out on the high molecular weight DNA sample using primers specific for a sequence unique to the transgene sequence ("transgene"). For comparison, ddPCR also was carried out with primers specific for VSVg-encoding sequences ("packaging plasmid") to detect the spiked DNA that is not expected to integrate into the chromosome of the cells or residual packaging plasmids in the transduced cells, and with primers specific for a housekeeping gene to detect genomic DNA in all samples (e.g., primers for a gene encoding ribonuclease P protein subunit p30 (*RRP30*; "genomic control"). Primers specific for the albumin (*ALB*) gene was used as as a reference for normalization. Reactions were also carried out on DNA samples that were not subject to PFGE (pre-gel). For ddPCR, samples were added to a mixture containing each primer set and probes, droplets were generated using a droplet generator, generated droplets were transferred to a PCR plate and amplification was carried out under the following PCR conditions: 95°C 10 min; [94°C 30 sec; 60°C 1 min] x 39 cycles at 2°C/sec ramp rate; 98°C 10 min; and 4°C indefintely. Following amplification, signal from the droplets were measured on a droplet reader. Copy number of each gene was normalized to the number of diploid genomes (cp/diploid genome, using amplification with primers specific for the albumin (*ALB*) gene as a reference) or per 50 ng of genomic DNA.

As shown in **FIG. 1****,** in non-transduced sample that contained spiked-in CAR-encoding plasmid and VSVg packaging plasmid, transgene sequences and VSVg sequences were only detected in samples that had not undergone PFGE (pre-gel). In contrast, genomic control sequences were detected in all samples subjected to PFGE for higher molecular weight DNA of 15 kb, 17.5 kb or 20 kb or higher. This result demonstrated that PFGE prior to PCR amplification of separated DNA, achieved separation of non-integrated lower molecular weight plasmids.

In transduced samples, the transgene sequences were detected in both the samples prior to PFGE (pre-gel) and in the samples subject to PFGE above 15 kb, 17.5 kb or 20 kb (FIG. 1 bottom panel). The VSVg packaging plasmid sequence was found in the pre-gel samples and were almost undetectable in the higher molecular weight DNA. These sequences, which possibly derive from residual plasmid sequences from viral production, were not expected to integrate into the genome of the cells. Genomic DNA, including chromosomomal DNA, was also detected in all samples: the copy number of the *RRP30* housekeeping gene was observed to be approximately 2 copies per diploid genome in all samples.

The assay including PFGE permitted detection of integrated or genomic sequences while removing non-integrated, low molecular weight nucleic acid species. Thus, the results show that the use of PFGE to separate high molecular weight DNA, prior to PCR amplification of transgene sequences from the isolated DNA, can be used as an integrated vector copy number (iVCN) assay to facilitate the specific determination of the copy number of transgene sequences that has integrated into the genome.

### Example 2: Comparison of Transgene Copy Number Assessed by Droplet Digital PCR (ddPCR) with or without Pulse Field Gel Electrophoresis (PFGE) at Various Time Points during Cell Manufacturing Process

The iVCN method described in Example 1, involving separation of high-molecular weight species by PFGE prior to vector copy number analysis by qPCR, was used to assess integrated copy number of an exemplary transgene sequences encoding a chimeric antigen receptor (CAR) at various time points during cell engineering processes in both a Jurkat T cell line and primary T cells. The method was compared to a standard vector copy number (VCN) assay that did not include separation of the high- and low-molecular weight DNA species by pulse field gel electrophoresis (PFGE).

For the studies, genomic DNA was prepared from the cells and subjected to assessment of transgene sequence copy number by either (1) the iVCN method, generally as described in Example 1 above, using a threshold value for separation of > 15 kb ("iVCN") and the PippinHT (Sage Science, Beverly, MA) device, or (2) a standard VCN method in which genomic DNA was not first separated by PFGE ("VCN"). In both assays, transgene copy number was determined by ddPCR using primers specific for a sequence unique to the transgene, and normalized to a diploid genome, as determined using primers specific for a reference gene (e.g., albumin *(ALB)* gene).

### A. Jurkat Cell Line

Jurkat T cells were transduced with a lentiviral preparation containing transgene sequences encoding a CAR, generally as described in Example 1 above. Samples of cells were obtained prior to transduction ("pre"), at 5 minutes, 6 hours, 12 hours, 24 hours, 48 hours, 72 hours and 96 hours after transduction, and used for analysis of transgene copy number.

As shown in FIG. 2A, transgene copy number assessment with PFGE for detecting integrated copy number (iVCN) in the genome during various time points in the process of engineering Jurkat cells showed that transgene integration was not detectable until at around 6 hours in these cells, and increased until about 48 hours, at which time the detected copy number generally plateaued. In contrast, transgene copy number assessment without PFGE, which detected the copy number of the transgene in the cells by the standard VCN method, demonstrated substantial detection of transgene sequence starting at much earlier time points. This observation is consistent with the detection of non-integrating transgene sequences by the standard VCN method, such as producer plasmids, linear or circular complementary DNA (cDNA) or autointegrants, at these early time points after cell engineering. The transgene copy number as determined without PFGE later decreased to levels similar to those detected by the iVCN integrated copy number assessment (with PFGE) around 96 hours, indicating that although transgene integration was complete by approximately 48 hours, standard VCN method without PFGE was not be accurate until around 96 hours or after transduction in these cells.

### B. Primary Cell Engineering

Primary T cells from human subjects were engineered to express a CAR using an exemplary engineering process. Separate compositions of CD4+ and CD8+ cells were selected from isolated PBMCs from a leukapheresis sample, and the selected cell compositions were cryopreserved. The separate compositions of CD4+ and CD8+ T cells were subsequently thawed and mixed at a ratio of 1:1 of viable CD4+ T cells to viable CD8+ T cells. Approximately 300 x 10⁶ T cells (150 x 10⁶ CD4+ and 150 x 10⁶ CD8+ T cells) of the mixed composition were stimulated in the presence of paramagnetic polystyrene-coated beads with attached anti-CD3 and anti-CD28 antibodies at a 1:1 bead to cell ratio in serum free media containing recombinant IL-2, IL-7 and IL-15 for between 18 to 30 hours.

Following the incubation, approximately 100 x10⁶ viable cells from the stimulated cell composition were washed and resuspended in the serum free media containing recombinant IL-2, IL-7 and IL-15. The cells were transduced with a lentiviral preparation encoding an anti-BCMA CAR by spinoculation at approximately 1600 g for 60 minutes. After spinoculation, the cells were washed and resuspended in the serum free media containing recombinant IL-2, IL-7 and IL-15, and incubated for about 18 to 30 hours at about 37 °C. The anti-BCMA CAR contained an scFv antigen-binding domain specific for BCMA, a CD28 transmembrane region, a 4-1BB costimulatory signaling region, and a CD3-zeta derived intracellular signaling domain.

The cells were then cultivated for expansion by transfer to a bioreactor (e.g. a rocking motion bioreactor) in about 500 mL of media containing twice the concentration of cytokines as used during the incubation and transduction steps. When a set viable cell density was achieved, perfusion was initiated, where media was replaced by semi-continuous perfusion with continual mixing. The cells were cultivated in the bioreactor until a threshold number of cells (TNC) was achieved of about 3 x10⁹ cells, which typically occurred in a process involving 6-7 days of expansion. The anti-CD3 and anti-CD28 antibody conjugated paramagnetic beads were removed from the cell composition by exposure to a magnetic field. The cells were then collected and formulated with a cryoprotectant.

For analysis of DNA by iVCN and standard VCN methods as described above, samples of cells were obtained prior to transduction ("pre"), at 24 hours, 48 hours, 72 hours, 96 hours, 120 hours after transduction and at completion of the engineering process ("completion").

As shown in **FIG. 2B****,** transgene copy number, as determined following PFGE (iVCN), was not detected until about 24 hours post-transduction and increased until about 48 to 72 hours. In the assay without PFGE (VCN), transgene copy number was much higher at the early time points (24 and 48 hours), but later decreased to levels similar to those detected by the integrated copy number assessment (iVCN) around 96 hours. In both assays, the assessed copy number was similar in samples obtained 96 hours or longer post-transduction.

The results confirm that assessment of vector copy number with PFGE (iVCN) reveal consistent timing of transgene integration at time points after transduction. The observations further show that assessment by standard VCN methods that do not involve PFGE can detect transgene sequences in cells at times before integration into the genome has occurred, and thus demonstrated that standard VCN methods may not be entirely appropriate to assess vector copy number on samples less than 4 days post-transduction.

### Example 3: Assessment of Integrated Transgene Copy Number with Pulse Field Gel Electrophoresis (PFGE) during Various Non-expanded Manufacturing Processes

Copy number of transgenes encoding a chimeric antigen receptor (CAR) was assessed using the iVCN and VCN methods generally as described in Examples 1 and 2, at various time points during exemplary processes for producing a genetically engineering a T cell composition. The exemplary processes did not involve an expansion step after transduction, and differed in the stimulatory reagent, culture media, and harvesting times.

For each process, separate compositions of CD4+ and CD8+ primary human T cells were selected from two different human subjects (Donor A and Donor B) from isolated PBMCs from human leukapheresis samples. The selected cells were cryopreserved, subsequently thawed and mixed at a ratio of 1:1 of viable CD4+ T cells to viable CD8+ T cells. The mixed cell composition was stimulated by incubation with a stimulatory reagent as follows: (1) anti-CD3/anti-CD28 antibody conjugated paramagnetic beads ("beads"), (2) anti-CD3/anti-CD28 Fab conjugated oligomeric streptavidin mutein reagents at a concentration of 4.0 µg per 10⁶ cells, or (3) anti-CD3/anti-CD28 Fab conjugated oligomeric streptavidin mutein reagents at a concentration of 0.8 µg per 10⁶ cells. The incubation was carried out in serum free media with recombinant cytokines for approximately 18 to 30 hours.

The cells were then washed and transduced by spinoculation with a lentiviral preparation containing transgene sequences encoding a CAR. Cells were then incubated with basal media without serum, growth factors or recombinant cytokines ("basal") or serum free media containing recombinant IL-2, IL-5, and IL-15 cytokines ("complete"). After 96 hours after initiation of the incubation with the bead reagent, cells were exposed to a magnetic field to remove the paramagnetic beads. After 24 hours, 48 hours or 96 hours after initiation of the incubation with the oligomeric stimulatory reagent, cells were exposed to biotin for 10 minutes to dissociate and remove oligomeric streptavidin reagents. The cells were washed and formulated with a cryoprotectant.

The cells were thawed and genomic DNA was prepared from harvested cells. For assessment of transgene copy number, ddPCR using primers specific to the transgene sequence was carried out on DNA that had been subject to PFGE for high molecular weight fraction >15 kb, as described in Examples 1 and 2 above. The cell samples were also assessed by flow cytometry, staining for expression of the CAR, CD3 and activated caspase 3 (aCas3).

The results are shown in **FIGS. 3A-3B****.** As shown in FIG. 3A, for cells stimulated using the oligomeric reagent, integrated transgene copy number measured by iVCN increased in samples in which the incubation was carried out for 24 hours or 48 hours after initiation of the stimulation, but did not increase further. Transgene copy number as measured by standard VCN (without PFGE) remained substantially higher than the integrated transgene copy number as measured by iVCN, until about 96 hours after initiation of the stimulation. This result is consistent with an observation that a standard VCN method (without PFGE) was not accurate until the 96 hour time-point in this process. For cells stimulated using the bead reagent, at 96 hours, higher integrated copy number was observed for cells incubated with basal media compared to cells incubated with complete media. As shown in FIG. 3B, the integrated transgene copy number correlated with the percentage of CAR-expressing cell (as determined by the percentage of CD3+/activated Cas3-/CAR+ cells among CD3+ cells by flow cytometry). This result further supports the utility of the iVCN assay for assessing integrated copy number, particularly when assessing impact of different parameters, including time of incubation, media or reagents, in a process for engineering cells.

### Example 4: Comparison of Integrated and Non-integrated Transgene Copy Number Assessed by Droplet Digital PCR (ddPCR) with or without Pulse Field Gel Electrophoresis (PFGE) during Various Cell Manufacturing Processes

The number of integrated and non-integrated transgenes encoding a chimeric antigen receptor (CAR) was assessed by ddPCR on DNA samples, with or without pulse field gel electrophoresis (PFGE), obtained during various exemplary processes for producing a genetically engineering a T cell composition.

### A. Exemplary processes for engineering T cells

The exemplary processes included processes in which engineered cells were subjected to cell expansion (expanded process) and processes in which cells were not expanded (non-expanded process).

### 1) Expanded process - anti-CD3/anti-CD28 beads

An expanded process generally as described in Example 2.B was carried out.

### 2) Non-expanded Process - anti-CD3/anti-CD28 beads

A non-expanded process using anti-CD3/anti-CD28 antibody conjugated beads was carried out similar to as described in Example 3. CD4+ and CD8+ T cells were selected and mixed at a 1:1 ratio to produce an input composition containing approximately 600 x 10⁶ T cells (300 x 10⁶ CD4+ and 300 x 10⁶ CD8+ T cells). The mixed input cell composition were stimulated by incubating the cells for 18-30 hours in the presence of anti-CD3/anti-CD28 antibody conjugated beads at a 1:1 bead to cell ratio in serum free media containing recombinant IL-2, IL-7 and IL-15. Following the stimulation, the cells were washed and resuspended in the serum free media containing recombinant IL-2, IL-7 and IL-15. The cells were then transduced with a lentiviral vector encoding the same anti-BCMA CAR used in the expanded process by spinoculation at approximately 693 g for 30 minutes.

In one arm, after spinoculation, the cells were resuspended in basal media without serum and without added growth factors or recombinant cytokines (basal media) and allowed to incubate at about 37.0 °C in an incubator for about 96 hours after initiation of the stimulation with the anti-CD3/anti-CD28 beads. In another arm, a similar process was carried out except that, after spinoculation, the cells were resuspended in basal media without added growth factors or recombinant cytokines (basal media) and allowed to incubate at about 37.0 °C in an incubator for about 72 hours after initiation of the stimulation with the anti-CD3/anti-CD28 beads.

### 3) Non-expanded Process - anti-CD3/anti-CD28 Fab oligomeric reagent

A non-expanded process using anti-CD3/anti-CD28 Fab oligomeric reagent was carried out similar to as described in Example 3. CD4+ and CD8+ T cells were selected and mixed at a 1:1 ratio to produce an input composition containing approximately 600 x 10⁶ T cells (300 x 10⁶ CD4+ and 300 x 10⁶ CD8+ T cells). Cells from the mixed input cell composition were stimulated by incubation with 480 µg (or 0.8 µg per 1x10⁶ cells) anti-CD3/anti-CD28 Fab conjugated oligomeric streptavidin mutein reagents generated as described in Example 2, which was carried out in serum free media containing recombinant IL-2, IL-7 and IL-15 for between 18-30 hours. After stimulation, the cells were transduced with a lentiviral vector encoding the same anti-BCMA CAR used in the expanded process, by spinoculation at 693 g for 30 minutes.

In one arm of the process, after the spinoculation, the cells were washed and resupended in basal media without serum, added growth factors or recombinant cytokines (basal media), and incubated at about 37.0 °C in an incubator. About 24 hours after initiation of transduction (approximately 48 hours after initiation of stimulation), 1.0 mM D-biotin was added during the incubation and mixed with the cells to dissociate anti-CD3 and anti-CD28 Fab reagents from oligomeric streptavidin reagents. The cells were further incubated for about 48 hours (96 hours after initiation of stimulation), and then were washed and formulated with a cryoprotectant.

In another arm of the process, after the spinoculation, the cells were washed and resupended in basal media without serum, added growth factors or recombinant cytokines (basal media) and incubated at about 37.0 °C in an incubator. About 24 hours after initiation of the transduction, 1.0 mM D-biotin was added during the incubation and mixed with the cells to dissociate anti-CD3 and anti-CD28 Fab reagents from oligomeric streptavidin reagent. The cells were further incubated for an additional 24 hours (72 hours after initiation of stimulation), and then were washed and formulated with a cryoprotectant.

### 4) Summary of Processes

**Table E1** summarizes features of the processes as described above, in addition to a process using a mock empty vector.

**Table E1: Summary of Processes**

| **Arm** | **Stim. Reagent** | **Cell Number** | **Transduction** | **Expansion** | **Removal of stimulatory reagent** | **Harvest** |
|---|---|---|---|---|---|---|
| 1 | Beads | 1.5 x 10⁶ CD4 and 1.5x 10⁶ CD8 | Spinoculation & incubation for 18-30 hours | Until TNC of ~ 3x10⁹ cells, about 6-7 days | Wash and debead | Following debead |
| | | | | | Day following threshold number | |
| 2 | Beads | 3.0x10⁶ CD4 and 3.0x10⁶ CD8 | Spinoculation & incubation for about 72 hours in basal media | None | Wash and debead | Following debead |
| | | | | | About 96 hours after initiation of stimulation | |
| 3 | Beads | 3.0x10⁶ CD4 and 3.0x10⁶ CD8 | Spinoculation & incubation for about 48 hours in basal media | None | Wash and debead | Following debead |
| | | | | | About 72 hours after initiation of stimulation | |
| 4 | Oligo. 0.2X | 30x10⁶ CD4 and 3.0x10⁶ CD8 | Spinoculation & incubation for 72 hours in basal media | None | Biotin added during incubation about 24 hours after stimulation | 96 hours after initiation of stimulation |
| 5 | Oligo. 0.2X | 30x10⁶ CD4 and 3.0x10⁶ CD8 | Spinoculation& incubation for 48 hours in basal media | None | Biotin added during incubation about 24 hours after initiation of stimulation | 72 hours after initiation of stimulation |
| 6 | (Mock) Oligo. 0.2X | 1.5 x 10⁶ CD4 and 1.5x 10⁶ CD8 | Mock transduction & static incubation for 24 hours in complete media | None | Biotin added about 24 hours after initiation of stimulation | 96 hours after initiation of stimulation |

### B. Assessment of Transgene Copy Number

The cells were thawed and genomic DNA was prepared from harvested cells. For assessment of integrated transgene copy number, ddPCR using primers specific to the transgene sequence was carried out on DNA that had been subject to PFGE for high molecular weight fraction > 10 kb generally as described in Examples 1 and 2 ("iVCN"). For comparison, ddPCR using the primers specific to the transgene sequence also was carried out on DNA that had not been subject to PFGE ("VCN", both high- and low-molecular weight DNA). The cell samples were also assessed by flow cytometry.

The results are shown in **FIGS. 4A-4E****.** As shown in **FIG. 4A****,** each of the shorter non-expanded processes produced cells that exhibited copy number as determined by VCN (without PFGE) that was higher than copy number as determined by iVCN (with PFGE), indicating that non-integrated transgene sequences were present in the samples that were engineered by these shorter processes. The fraction of integrated transgene, which was determined by dividing copy number using iVCN by VCN, was substantially lower in cells produced using the non-expanded processes **(****FIG. 4B****).** Similarly, the fraction of non-integrated transgene, which was determined as 1 - fraction of integrated transgene, was substantially higher in cells produced using the non-expanded processes **(****FIG. 4C****).** As shown in **FIG. 4D****,** the non-integrated transgene copy number, determined by subtracting the iVCN from VCN, was between about 0. 8 and 1.3 on average, in cells produced using the shorter processes. Using a standard VCN without PFGE, the transgene copy number per CAR+ cell are shown in **FIG. 4E****.** This results further supports the utility of the iVCN method for assessing integrated and non-integrated transgene copy number in a process for engineering cells.

### Example 5: Comparison of Integrated and Non-integrated Transgene Copy Number during Various Expanded Cell Manufacturing Processes

Transgene copy number in DNA with or without pulse field gel electrophoresis (PFGE), was assessed by ddPCR at various time points in a process for genetically engineering T cells that included a step of expanding the cells after transduction.

Primary T cells from different human donors were engineered to express a CAR, as described in Example 2.B. Samples were obtained daily starting from day 0 to day 8 of the expanded process, including at thawed material (TMAT; day 0), at activation (AMAT; day 1), at transduction (XMAT; day 2) or at various times after initiation of cultivation to expand the cells (inoc+1 to inoc+6; representing days 3-8 of the process). Genomic DNA was prepared from the cell samples. For assessment of integrated transgene copy number, ddPCR using primers specific to the transgene sequence was carried out on DNA that had been subject to PFGE for high molecular weight fraction as described in Examples 1 and 2 ("iVCN"). For comparison, ddPCR using the primers specific to the transgene sequence also was carried out on DNA that was not subject to PFGE ("VCN", both high- and low-molecular weight DNA). The non-integrated transgene copy number, the fraction of integrated transgene and the fraction of non-integrated transgene were also determined, generally as described in Example 4 above.

Representative results are shown in **FIG. 5****.** As shown, on the day of transduction or at early time points after transduction (e.g., inoc+1, inoc+2), copy number as determined by the standard VCN method, determined from genomic DNA samples not subject to PFGE, included a substantial fraction of non-integrated transgenes. At later time points after transduction, copy number as determined by iVCN (with PFGE) and standard VCN (without PFGE) were about the same, indicating that non-integrated copies of the transgene were no longer present in the cells at these time points.

### Example 6: Assessment of Transgene Integration in Fibroblasts

Integration of transgene introduced to a fibroblast cell line via lentiviral transduction was assessed by analysis of transgene copy number by ddPCR on genomic DNA that had been subject to pulse field gel electrophoresis (PFGE).

HT1080 human fibrosarcoma cell line (ATCC^{®} CCL-121^{™}) was transduced with a lentiviral preparation containing a transgene sequence encoding a recombinant protein, in this case a chimeric antigen receptor (CAR). After transduction the cells were cultured, and were harvested 12, 24, 48 or 72 hours post-transduction. Genomic DNA was prepared from harvested cells. Transgene copy number was determined by ddPCR using primers specific for the transgene, in high-molecular weight DNA samples after PFGE ("iVCN") and in DNA samples that were not subject to PFGE ("VCN", both high- and low-molecular weight DNA), generally as described in Examples 1 and 2 above.

As shown in **FIG. 6****,** transgene integration into HT1080 fibroblast cell line was complete by 24 hours post-transduction. The timing of complete integration in the fibroblast cell line was faster than observed in T cells, including Jurkat cells and primary human T cells, as shown in Examples above. This result reveals that certain cell types undergo faster lentiviral integration into the genome of the cells.

### Example 7: Assessment of Recombinant Receptor-expressing T Cells to Determine the Amount of Transgene Sequence and Pharmacokinetic Parameters of Administered Engineered T Cells

An exemplary method employing ddPCR and PFGE is used to assess the amount of a transgene sequence encoding a recombinant protein, such as a chimeric antigen receptor (CAR), in a subject that has been administered engineered T cells.

Subjects that have a disease or disorder, such as a proliferative disease or disorder, for example, a cancer, is administered engineered cells, such as T cells, expressing a recombinant protein, such as a recombinant receptor that can target a particular antigen that is expressed by cells associated with the disease or disorder, such as cancer cells. In some examples, the recombinant receptor is a chimeric antigen receptor (CAR) that is specific for a cancer antigen, such as an antigen expressed by or associated with cancer cells. In some aspects, therapeutic T cell compositions are generated by introduction of transgene sequences encoding the recombinant receptor into isolated primary human T cells, such as isolated primary CD4+ and/or CD8+ human T cells, such as by using an expanded or non-expanded process as described in Examples 1-6 above. Subjects are administered a therapeutic T cell composition comprising the engineered cells.

At various time points after administration of the cell composition, the amount of transgene sequences that are present in biological samples from a subject that has been administered the engineered cells is determined using the methods as described herein to assess integrated transgene copy number. In some aspects, cells in the blood or serum or organ or tissue sample (*e.g.,* disease site, *e.g.,* tumor sample) of the subject are obtained, before, during and/or after administration of the therapeutic T cell composition. Genomic DNA is prepared from the samples. In some aspects, the amount of transgene sequences that are present in a biological sample is determined by ddPCR using primers that can specifically amplify a portion of the transgene, from high-molecular weight DNA samples after PFGE ("iVCN"), generally as described in Examples 1 and 2 above. In some aspects, copy number is also assessed by ddPCR using the same primers from DNA samples that were not subject to PFGE ("VCN", both high- and low-molecular weight DNA). In some aspects, the cell samples in some cases can also be assessed by flow cytometry, staining for expression of the recombinant protein, e.g., CAR, to determine the proportion of cells in the sample that express the recombinant protein, e.g., CAR.

In some examples, the amount of transgene sequences in a biological sample is quantified as copies integrated transgene encoding the recombinant protein, *e.g.,* CAR, per amount of DNA, such as microgram of DNA, or per volume of sample, such as microliter of the sample, *e.g.,* of blood or serum, or per total number of cells, such as per total peripheral blood mononuclear cells (PBMCs) or white blood cells or T cells, optionally per volume, e.g., per microliter of the sample, or per cell, such as per diploid T cell genome or per CAR-expressing cell optionally per volume, e.g., per microliter of the sample. Exemplary pharmacokinetic (PK) parameters that can be determined based on the iVCN method or by other methods such as flow cytometry, include maximum (peak) plasma concentrations (Cₘₐₓ) of transgene sequences or particular cells expressing the recombinant protein, such as Cₘₐₓ of CD3⁺ CAR⁺ cells, CD4⁺ CAR⁺ cells and or CD8⁺ CAR⁺ T cells; the time point at which Cₘₐₓ is achieved (Tₘₐₓ), such as the Tₘₐₓ of transgene sequences, CD3⁺ CAR⁺ cells, CD4⁺ CAR⁺ cells and or CD8⁺ CAR⁺ T cells, and or area under the curve (AUC) for a specific time after administration, such as the AUC₀₋₂₈, of transgene sequences, CD3⁺ CAR⁺ cells, CD4⁺ CAR⁺ cells and or CD8⁺ CAR⁺ T cells.

### Example 8: Correlation of Standard VCN Assay and iVCN Assay to Surface Expression of Recombinant Receptor during Cell Manufacturing Processes

Vector copy number (VCN) and iVCN assays described above were used to determine copy number of a transgene encoding a recombinant receptor, e.g. chimeric antigen receptor (CAR), introduced into T cells by lentiviral transduction, and the results were correlated to surface expression of the CAR. In this study, the assays were carried out on cell compositions produced from primary T cells from different human donors that had been engineered to express a CAR using either an expanded process, generally as described in Example 2.B, or a non-expanded process generally as described in Example 4.A.3, with a modification in which the cells were activated with an anti-CD3/anti-CD28 Fab conjugated oligomeric streptavidin mutein reagent followed by transduction and a short incubation in basal media that does not include addition of recombinant cytokines (cytokine-free media) before harvesting.

Genomic DNA was prepared from the cell samples at the end of the process for engineering in the shortened, non-expanded process or in the expanded process. The VCN and iVCN assays were carried out as described in Examples 1 using a threshold value for separation of >15 kb ("iVCN") and the PippinHT (Sage Science, Beverly, MA) device, or (2) a standard VCN method in which genomic DNA was not first separated by PFGE ("VCN").

The results showed that transgene copy number assessed using the VCN assay generally correlated with the transgene copy number assessed by iVCN **(****FIG. 7A****).** However, for cells manufactured using the non-expanded process, the values obtained by VCN were higher than the values obtained by iVCN, consistent with the VCN assay detecting non-integrated transgene sequences that could be present in samples containing cells generated using the non-expanded process. In contrast, for cells manufactured using the expanded process, the value obtained by VCN and iVCN were nearly identical (near the VCN = iVCN line). These differences are likely due to the presence of a greater amount of free, non-integrated copies of transgene sequences in samples in the shorter non-expanded process compared to the expanded process. These results are consistent with the observation that a standard VCN assay that is able to detect both high and low molecular weight DNA has limitations compared to an iVCN assay, particularly when used to assess cells early after transgene introduction, such as in a shortened process for engineering T cells, where free, non-integrated copies of transgene sequences may still be present in the sample.

To assess the degree of correlation of the iVCN or VCN assay to surface expression of the CAR, cell samples from the non-expanded or expanded process were assessed by flow cytometry for expression of CD3, CD45 and the CAR to determine the percentage of CD3+CAR+ cells among viable CD45+ cells. As shown in **FIGS. 7B****,** the VCN assay exhibited better correlation to the percentage of CAR+ cells for samples engineered by the expanded process than by the non-expanded process, likely due to the presence of non-integrated CAR DNA sequences that did not contribute to surface CAR expression. As shown in **FIG. 7C****,** the iVCN showed similar correlation to expression of the CAR among cells that had been engineered by either the non-expanded or expanded process. For all samples, the correlation of CAR expression with the copy number per cell was higher by the iVCN assay (R² = 0.8952) compared to the copy number per cell as determined by the VCN assay (R² = 0.5903).

The results support the utility of the iVCN assay for accurately determining the copy number of stably integrated transgene sequence, particularly for cells generated using a non-expanded process which may retain free, non-integrated copies of transgene sequences. The VCN assay, which does not distinguish integrated vs. non-integrated transgene sequences, is limited in accurately determining the number of stably integrated transgene sequences, especially during and after a shorter, non-expanded process.

### Sequences

| # | **SEQUENCE** | **ANNOTATION** |
|---|---|---|
| 1 | ESKYGPPCPPCP | spacer (IgG4hinge) (aa) Homo sapiens |
| 2 | GAATCTAAGTACGGACCGCCCTGCCCCCCTTGCCCT | spacer (IgG4hinge) (nt) Homo sapiens |
| 3 | ESKYGPPCPPCPGQPREPQVYTLPPSQEEMTKNQVSLTCLVKGFYPSD IAVEWESNGQPENNYKTTPPVLDSDGSFFLYSRLTVDKSRWQEGNVFS CSVMHEALHNHYTQKSLSLSLGK | Hinge-CH3 spacer Homo sapiens |
| 4 | ESKYGPPCPPCPAPEFLGGPSVFLFPPKPKDTLMISRTPEVTCVVVDV SQEDPEVQFNWYVDGVEVHNAKTKPREEQFNSTYRVVSVLTVLHQDWL NGKEYKCKVSNKGLPSSIEKTISKAKGQPREPQVYTLPPSQEEMTKNQ VSLTCLVKGFYPSDIAVEWESNGQPENNYKTTPPVLDSDGSFFLYSRL TVDKSRWQEGNVFSCSVMHEALHNHYTQKSLSLSLGK | Hinge-CH2-CH3 spacer Homo sapiens |
| 5 | RWPESPKAQASSVPTAQPQAEGSLAKATTAPATTRNTGRGGEEKKKEK EKEEQEERETKTPECPSHTQPLGVYLLTPAVQDLWLRDKATFTCFVVG SDLKDAHLTWEVAGKVPTGGVEEGLLERHSNGSQSQHSRLTLPRSLWN AGTSVTCTLNHPSLPPQRLMALREPAAQAPVKLSLNLLASSDPPEAAS WLLCEVSGFSPPNILLMWLEDQREVNTSGFAPARPPPQPGSTTFWAWS VLRVPAPPSPQPATYTCVVSHEDSRTLLNASRSLEVSYVTDH | IgD-hinge-Fc Homo sapiens |
| 6 | LEGGGEGRGSLLTCGDVEENPGPR | T2A artificial |
| 7 | MLLLVTSLLLCELPHPAFLLIPRKVCNGIGIGEFKDSLSINATNIKHF KNCTSISGDLHILPVAFRGDSFTHTPPLDPQELDILKTVKEITGFLLI QAWPENRTDLHAFENLEIIRGRTKQHGQFSLAVVSLNITSLGLRSLKE ISDGDVIISGNKNLCYANTINWKKLFGTSGQKTKIISNRGENSCKATG QVCHALCSPEGCWGPEPRDCVSCRNVSRGRECVDKCNLLEGEPREFVE NSECIQCHPECLPQAMNITCTGRGPDNCIQCAHYIDGPHCVKTCPAGV MGENNTLVWKYADAGHVCHLCHPNCTYGCTGPGLEGCPTNGPKIPSIA TGMVGALLLLLVVALGIGLFM | tEGFR artificial |
| 8 | FWVLVVVGGVLACYSLLVTVAFIIFWV | CD28 (amino acids 153-179 of Accession No. P10747) Homo sapiens |
| 9 | IEVMYPPPYLDNEKSNGTIIHVKGKHLCPSPLFPGPSKP FWVLVVVGGVLACYSLLVTVAFIIFWV | CD28 (amino acids 114-179 of Accession No. P10747) Homo sapiens |
| 10 | RSKRSRLLHSDYMNMTPRRPGPTRKHYQPYAPPRDFAAYRS | CD28 (amino acids 180-220 of P10747) Homo sapiens |
| 11 | RSKRSRGGHSDYMNMTPRRPGPTRKHYQPYAPPRDFAAYRS | CD28 (LL to GG) Homo sapiens |
| 12 | KRGRKKLLYIFKQPFMRPVQTTQEEDGCSCRFPEEEEGGCEL | 4-1BB (amino acids 214-255 of Q07011.1) Homo sapiens |
| 13 | RVKFSRSADAPAYQQGQNQLYNELNLGRREEYDVLDKRRGRDPEMGGK PRRKNPQEGLYNELQKDKMAEAYSEIGMKGERRRGKGHDGLYQGLSTA TKDTYDALHMQALPPR | CD3 zeta Homo sapiens |
| 14 | RVKFSRSAEPPAYQQGQNQLYNELNLGRREEYDVLDKRRGRDPEMGGK PRRKNPQEGLYNELQKDKMAEAYSEIGMKGERRRGKGHDGLYQGLSTA TKDTYDALHMQALPPR | CD3 zeta Homo sapiens |
| 15 | RVKFSRSADAPAYKQGQNQLYNELNLGRREEYDVLDKRRGRDPEMGGK PRRKNPQEGLYNELQKDKMAEAYSEIGMKGERRRGKGHDGLYQGLSTA TKDTYDALHMQALPPR | CD3 zeta Homo sapiens |
| 16 | RKVCNGIGIGEFKDSLSINATNIKHFKNCTSISGDLHILPVAFRGDSF THTPPLDPQELDILKTVKEITGFLLIQAWPENRTDLHAFENLEIIRGR TKQHGQFSLAVVSLNITSLGLRSLKEISDGDVIISGNKNLCYANTINW KKLFGTSGQKTKIISNRGENSCKATGQVCHALCSPEGCWGPEPRDCVS CRNVSRGRECVDKCNLLEGEPREFVENSECIQCHPECLPQAMNITCTG RGPDNCIQCAHYIDGPHCVKTCPAGVMGENNTLVWKYADAGHVCHLCH PNCTYGCTGPGLEGCPTNGPKIPSIATGMVGALLLLLVVALGIGLFM | tEGFR artificial |
| 17 | EGRGSLLTCGDVEENPGP | T2A artificial |
| 18 | GSGATNFSLLKQAGDVEENPGP | P2A |
| 19 | ATNFSLLKQAGDVEENPGP | P2A |
| 20 | QCTNYALLKLAGDVESNPGP | E2A |
| 21 | VKQTLNFDLLKLAGDVESNPGP | F2A |
| 22 | -PGGG- (SGGGG) 5-P- wherein P is proline, G is glycine and S is serine | Linker |
| 23 | GSADDAKKDAAKKDGKS | Linker |
| 24 | atgcttctcctggtgacaagccttctgctctgtgagttaccacaccca gcattcctcctgatccca | GMCSFR alpha chain signal sequence |
| 25 | MLLLVTSLLLCELPHPAFLLIP | GMCSFR alpha chain signal sequence |
| 26 | MALPVTALLLPLALLLHA | CD8 alpha signal peptide |
| 27 | EVQLVQSGAEMKKPGASLKLSCKASGYTFIDYYVYWMRQAPGQGLESM GWINPNSGGTNYAQKFQGRVTMTRDTSISTAYMELSRLRSDDTAMYYC ARSQRDGYMDYWGQGTLVTVSS | Variable heavy (VH) Anti-BCMA |
| 28 | QSALTQPASVSASPGQSIAISCTGTSSDVGWYQQHPGKAPKLMIYEDS KRPSGVSNRFSGSKSGNTASLTISGLQAEDEADYYCSSNTRSSTLVFG GGTKLTVLG | Variable light (VL) Anti-BCMA |
| 29 | GSTSGSGKPGSGEGSTKG | linker |
| 30 | QIQLVQSGPELKKPGETVKISCKASGYTFTDYSINWVKRAPGKGLKWM GWINTETREPAYAYDFRGRFAFSLETSASTAYLQINNLKYEDTATYFC ALDYSYAMDYWGQGTSVTVSS | Variable heavy (VH) Anti-BCMA |
| 31 | DIVLTQSPPSLAMSLGKRATISCRASESVTILGSHLIHWYQQKPGQPP TLLIQLASNVQTGVPARFSGSGSRTDFTLTIDPVEEDDVAVYYCLQSR TIPRTFGGGTKLEIK | Variable light (VL) Anti-BCMA |
| 32 | QIQLVQSGPDLKKPGETVKLSCKASGYTFTNFGMNWVKQAPGKGFKWM AWINTYTGESYFADDFKGRFAFSVETSATTAYLQINNLKTEDTATYFC ARGEIYYGYDGGFAYWGQGTLVTVSA | Variable heavy (VH) Anti-BCMA |
| 33 | DVVMTQSHRFMSTSVGDRVSITCRASQDVNTAVSWYQQKPGQSPKLLI FSASYRYTGVPDRFTGSGSGADFTLTISSVQAEDLAVYYCQQHYSTPW TFGGGTKLDIK | Variable light (VL) Anti-BCMA |
| 34 | EVQLVQSGAEVKKPGESLKISCKGSGYSFTSYWIGWVRQMPGKGLEWM GIIYPGDSDTRYSPSFQGHVTISADKSISTAYLQWSSLKASDTAMYYC ARYSGSFDNWGQGTLVTVSS | Variable heavy (VH) Anti-BCMA |
| 35 | SYELTQPPSASGTPGQRVTMSCSGTSSNIGSHSVNWYQQLPGTAPKLL IYTNNQRPSGVPDRFSGSKSGTSASLAISGLQSEDEADYYCAAWDGSL NGLVFGGGTKLTVLG | Variable light (VL) Anti-BCMA |
| 36 | GGGGS | Linker |
| 37 | GGGS | Linker |
| 38 | GGGGSGGGGSGGGGS | Linker |
| 39 | GSTSGSGKPGSGEGSTKG | Linker |
| 40 | SRGGGGSGGGGSGGGGSLEMA | Linker |
| 41 | EVQLVQSGAEVKKPGSSVKVSCKASGGTFSSYAISWVRQAPGQGLEWM GRIIPILGIANYAQKFQGRVTMTEDTSTDTAYMELSSLRSEDTAVYYC ARSGYSKSIVSYMDYWGQGTLVTVSS | Variable heavy (VH) Anti-BCMA |
| 42 | LPVLTQPPSTSGTPGQRVTVSCSGSSSNIGSNVVFWYQQLPGTAPKLV IYRNNQRPSGVPDRFSVSKSGTSASLAISGLRSEDEADYYCAAWDDSL SGYVFGTGTKVTVLG | Variable light (VL) Anti-BCMA |
| 43 | QVQLVQSGAEVKKPGSSVKVSCKASGGTFSSYAISWVRQAPGQGLEWM GRIIPILGTANYAQKFQGRVTITADESTSTAYMELSSLRSEDTAVYYC ARSGYGSYRWEDSWGQGTLVTVSS | Variable heavy (VH) Anti-BCMA |
| 44 | QAVLTQPPSASGTPGQRVTISCSGSSSNIGSNYVFWYQQLPGTAPKLL IYSNNQRPSGVPDRFSGSKSGTSASLAISGLRSEDEADYYCAAWDDSL SASYVFGTGTKVTVLG | Variable light (VL) Anti-BCMA |
| 45 | QVQLVQSGAEVKKPGASVKVSCKASGYTFTDYYMHWVRQAPGQRLEWM GWINPNSGGTNYAQKFQDRITVTRDTSSNTGYMELTRLRSDDTAVYYC ARSPYSGVLDKWGQGTLVTVSS | Variable heavy (VH) Anti-BCMA |
| 46 | QSVLTQPPSVSGAPGQRVTISCTGSSSNIGAGFDVHWYQQLPGTAPKL LIYGNSNRPSGVPDRFSGSKSGTSASLAITGLQAEDEADYYCQSYDSS LSGYVFGTGTKVTVLG | Variable light (VL) Anti-BCMA |
| 47 | RASQDISKYLN | CDR L1 |
| 48 | SRLHSGV | CDR L2 |
| 49 | GNTLPYTEG | CDR L3 |
| 50 | DYGVS | CDR H1 |
| 51 | VIWGSETTYYNSALKS | CDR H2 |
| 52 | YAMDYWG | CDR H3 |
| 53 | EVKLQESGPGLVAPSQSLSVTCTVSGVSLPDYGVSWIRQPPRKGLEWL GVIWGSETTYYNSALKSRLTIIKDNSKSQVFLKMNSLQTDDTAIYYCA KHYYYGGSYAMDYWGQGTSVTVSS | VH |
| 54 | DIQMTQTTSSLSASLGDRVTISCRASQDISKYLNWYQQKPDGTVKLLI YHTSRLHSGVPSRFSGSGSGTDYSLTISNLEQEDIATYFCQQGNTLPY TFGGGTKLEIT | VL |
| 55 | DIQMTQTTSSLSASLGDRVTISCRASQDISKYLNWYQQKPDGTVKLLI YHTSRLHSGVPSRFSGSGSGTDYSLTISNLEQEDIATYFCQQGNTLPY TFGGGTKLEITGSTSGSGKPGSGEGSTKGEVKLQESGPGLVAPSQSLS VTCTVSGVSLPDYGVSWIRQPPRKGLEWLGVIWGSETTYYNSALKSRL TIIKDNSKSQVFLKMNSLQTDDTAIYYCAKHYYYGGSYAMDYWGQGTS VTVSS | scFv |
| 56 | KASQNVGTNVA | CDR L1 |
| 57 | SATYRNS | CDR L2 |
| 58 | QQYNRYPYT | CDR L3 |
| 59 | SYWMN | CDR H1 |
| 60 | QIYPGDGDTNYNGKEKG | CDR H2 |
| 61 | KTISSVVDFYFDY | CDR H3 |
| 62 | EVKLQQSGAELVRPGSSVKISCKASGYAFSSYWMNWVKQRPGQGLEWI GQIYPGDGDTNYNGKFKGQATLTADKSSSTAYMQLSGLTSEDSAVYFC ARKTISSVVDFYFDYWGQGTTVTVSS | VH |
| 63 | DIELTQSPKFMSTSVGDRVSVTCKASQNVGTNVAWYQQKPGQSPKPLI YSATYRNSGVPDRFTGSGSGTDFTLTITNVQSKDLADYFCQQYNRYPY TSGGGTKLEIKR | VL |
| 64 | GGGGSGGGGSGGGGS | Linker |
| 65 | EVKLQQSGAELVRPGSSVKISCKASGYAFSSYWMNWVKQRPGQGLEWI GQIYPGDGDTNYNGKFKGQATLTADKSSSTAYMQLSGLTSEDSAVYFC ARKTISSVVDFYFDYWGQGTTVTVSSGGGGSGGGGSGGGGSDIELTQS PKFMSTSVGDRVSVTCKASQNVGTNVAWYQQKPGQSPKPLIYSATYRN SGVPDRFTGSGSGTDFTLTITNVQSKDLADYFCQQYNRYPYTSGGGTK LEIKR | scFv |
| 66 | HYYYGGSYAMDY | HC-CDR3 |
| 67 | HTSRLHS | LC-CDR2 |
| 68 | QQGNTLPYT | LC-CDR3 |
| 69 | gacatccagatgacccagaccacctccagcctgagcgccagcctgggc gaccgggtgaccatcagctgccgggccagccaggacatcagcaagtac ctgaactggtatcagcagaagcccgacggcaccgtcaagctgctgatc taccacaccagccggctgcacagcggcgtgcccagccggtttagcggc agcggctccggcaccgactacagcctgaccatctccaacctggaacag gaagatatcgccacctacttttgccagcagggcaacacactgccctac acctttggcggcggaacaaagctggaaatcaccggcagcacctccggc agcggcaagcctggcagcggcgagggcagcaccaagggcgaggtgaag ctgcaggaaagcggccctggcctggtggcccccagccagagcctgagc gtgacctgcaccgtgagcggcgtgagcctgcccgactacggcgtgagc tggatccggcagcccccaggaagggcctggaatggctgggcgtgatc tggggcagcgagaccacctactacaacagcgccctgaagagccggctg accatcatcaaggacaacagcaagagccaggtgttcctgaagatgaac agcctgcagaccgacgacaccgccatctactactgcgccaagcactac tactacggcggcagctacgccatggactactggggccagggcaccagc gtgaccgtgagcagc | Sequence encoding scFv |
| 70 | ASTKGPSVFPLAPCSRSTSESTAALGCLVKDYFPEPVTVSWNSGALTS GVHTFPAVLQSSGLYSLSSVVTVPSSSLGTKTYTCNVDHKPSNTKVDK RVESKYGPPCPSCPAPEFLGGPSVFLFPPKPKDTLMISRTPEVTCVVV DVSQEDPEVQFNWYVDGVEVHNAKTKPREEQFNSTYRVVSVLTVLHQD WLNGKEYKCKVSNKGLPSSIEKTISKAKGQPREPQVYTLPPSQEEMTK NQVSLTCLVKGFYPSDIAVEWESNGQPENNYKTTPPVLDSDGSFFLYS RLTVDKSRWQEGNVFSCSVMHEALHNHYTQKSLSLSLGK | Human IgG4 Fc (Uniprot P01861) |
| 71 | ASTKGPSVFPLAPCSRSTSESTAALGCLVKDYFPEPVTVSWNSGALTS GVHTFPAVLQSSGLYSLSSVVTVPSSNFGTQTYTCNVDHKPSNTKVDK TVERKCCVECPPCPAPPVAGPSVFLFPPKPKDTLMISRTPEVTCVVVD VSHEDPEVQFNWYVDGVEVHNAKTKPREEQFNSTFRVVSVLTVVHQDW LNGKEYKCKVSNKGLPAPIEKTISKTKGQPREPQVYTLPPSREEMTKN QVSLTCLVKGFYPSDISVEWESNGQPENNYKTTPPMLDSDGSFFLYSK LTVDKSRWQQGNVFSCSVMHEALHNHYTQKSLSLSPGK | Human IgG2 Fc (Uniprot P01859) |

## Claims

1. A method for assessing genomic integration of a transgene sequence, the method comprising:
(a) separating a high molecular weight fraction of deoxyribonucleic acid (DNA) of greater than or greater than about 10 kilobases (kb) from DNA isolated from one or more cells, said one or more cells comprising, or suspected of comprising, at least one engineered cell comprising a transgene sequence encoding a recombinant protein;
(b) from the high molecular weight fraction, determining the presence, absence or amount of the transgene sequence integrated into the genome of the one or more cell, wherein determining the presence, absence or amount of the transgene sequence is carried out by polymerase chain reaction (PCR).

2. The method of claim 1, wherein the method further comprises, prior to the separating in (a), isolating deoxyribonucleic acid (DNA) from the one or more cells.

3. The method of claim 1 or claim 2, wherein:
(i) the determining the presence, absence or amount of the transgene sequence in (b) comprises determining the copy number of the transgene sequence per diploid genome or per cell in the one or more cells; and/or
(ii) the one or more cells comprises a population of cells in which a plurality of cells of the population comprise the transgene sequence encoding the recombinant protein,
optionally wherein the copy number is an average or mean copy number per diploid genome or per cell among the population of cells.

4. The method of any of claims 1-3, wherein the method comprises, prior to the separating step in (a), introduction of a polynucleotide comprising the transgene sequence encoding the recombinant protein into the at least one engineered cell of the one or more cells, wherein the one or more cells are isolated from a sample that is blood or a blood-derived sample, or wherein the sample is or is derived from an apheresis or leukapheresis product.

5. The method of claim 4, wherein:
(a) the method comprises:
(i) incubation of the at least one engineered cell at a temperature greater than 25°C, optionally at or about 37°C ± 2°C, for no more than 96 hours following the introduction of the polynucleotide comprising the transgene sequence;
(ii) incubation of the at least one engineered cell at a temperature greater than 25°C, optionally at or about 37°C ± 2°C, for no more than 72 hours following the introduction of the polynucleotide comprising the transgene sequence; or
(iii) incubation of the at least one engineered cell at a temperature greater than 25°C, optionally at or about 37°C ± 2°C, for no more than 48 hours following the introduction of the polynucleotide comprising the transgene sequence; and/or
(b) the introduction of the polynucleotide is carried out by transduction with a viral vector comprising the polynucleotide, optionally wherein:
(i) the viral vector is a retroviral vector or a gammaretroviral vector,
Further optionally wherein the viral vector is a lentiviral vector; or
(i) the viral vector is an AAV vector, further optionally selected from among AAV1, AAV2, AAV3, AAV4, AAV5, AAV6, AAV7 or AAV8 vector.

6. The method of any of claims 1-5, wherein the one or more cell has been cryopreserved prior to the separating of the high molecular weight fraction of DNA in (a).

7. The method of any of claims 1-6, wherein:
(a) the one or more cell is a cell line; or
(b) the one or more cell is a primary cell obtained from a sample from a subject.

8. The method of any of claims 1-7, wherein the one or more cell is an immune cell, optionally wherein the immune cell is a T cell or an NK cell, further optionally wherein the T cell is a CD3+, CD4+ and/or CD8+ T cell.

9. The method of any of claims 1-8, wherein the separating is carried out by pulse field gel electrophoresis or size exclusion chromatography.

10. The method of any of claims 1-9, wherein:
(a) the high molecular weight fraction is of greater than or greater than about 15 kilobases (kb);
(b) the high molecular weight fraction is of greater than or greater than about 17.5 kilobases (kb); or
(c) the high molecular weight fraction is of greater than or greater than about 20 kilobases (kb).

11. The method of any of claims 1-10, wherein:
(a) the transgene sequence comprises a regulatory element operably linked to a nucleic acid sequence encoding the recombinant protein; and/or
(b) the PCR is quantitative polymerase chain reaction (qPCR), digital PCR or droplet digital PCR; and/or
(c) the PCR is carried out using one or more primers that is complementary to or is capable of specifically amplifying at least a portion of the transgene sequence, optionally wherein the one or more primers is complementary to or is capable of specifically amplifying sequences of the regulatory element.

12. The method of any of claims 1-11, wherein:
(a) the determining the amount of the transgene sequence comprises assessing the copy number of the transgene sequence per mass or weight of DNA isolated from the one or more cells, optionally per microgram of DNA isolated from the one or more cells; or
(b) the determining the amount of the transgene sequence comprises assessing the copy number of the transgene sequence per the one or more cells, optionally per CD3+, CD4+ and/or CD8+ cell, and/or per cell expressing the recombinant protein.

13. The method of any of claims 1-12, wherein:
(a) determining the amount of the transgene sequence comprises assessing the copy number of the transgene sequence in the high molecular weight fraction and normalizing the copy number to the copy number of a reference gene in the high molecular weight fraction or to a standard curve, optionally wherein the reference gene is a housekeeping gene, and/or the reference gene is a gene encoding albumin *(ALB)* or the reference gene is a gene encoding ribonuclease P protein subunit p30 *(RPP30);* and/or
(b) the copy number of a reference gene in the isolated DNA is carried out by PCR using one or more primers that is complementary to or is capable of specifically amplifying at least a portion of the reference gene.

14. The method of any of claims 1-13, wherein:
(a) the transgene sequence does not encode a complete viral gag protein; and/or
(b) the transgene sequence does not comprise a complete HIV genome, a replication competent viral genome, and/or accessory genes, which accessory genes are optionally Nef, Vpu, Vif, Vpr, and/or Vpx.

15. The method of any of claims 1-14, wherein the recombinant protein is a recombinant receptor.

16. The method of claim 15, wherein the recombinant receptor specifically binds to an antigen associated with a disease or condition or an antigen that is expressed in cells of the environment of a lesion associated with a disease or condition; optionally wherein:
(a) the disease or condition is a cancer; and/or
(b) the antigen is selected from αvβ6 integrin (avb6 integrin), B cell maturation antigen (BCMA), B7-H3, B7-H6, carbonic anhydrase 9 (CA9, also known as CAIX or G250), a cancer-testis antigen, cancer/testis antigen 1B (CTAG, also known as NY-ESO-1 and LAGE-2), carcinoembryonic antigen (CEA), a cyclin, cyclin A2, C-C Motif Chemokine Ligand 1 (CCL-1), CD19, CD20, CD22, CD23, CD24, CD30, CD33, CD38, CD44, CD44v6, CD44v7/8, CD123, CD133, CD138, CD171, chondroitin sulfate proteoglycan 4 (CSPG4), epidermal growth factor protein (EGFR), type III epidermal growth factor receptor mutation (EGFR vIII), epithelial glycoprotein 2 (EPG-2), epithelial glycoprotein 40 (EPG-40), ephrinB2, ephrin receptor A2 (EPHa2), estrogen receptor, Fc receptor like 5 (FCRL5; also known as Fc receptor homolog 5 or FCRH5), fetal acetylcholine receptor (fetal AchR), a folate binding protein (FBP), folate receptor alpha, ganglioside GD2, O-acetylated GD2 (OGD2), ganglioside GD3, glycoprotein 100 (gp100), glypican-3 (GPC3), G protein-coupled receptor class C group 5 member D(GPRC5D), Her2/neu (receptor tyrosine kinase erb-B2), Her3 (erb-B3), Her4 (erb-B4), erbB dimers, Human high molecular weight-melanoma-associated antigen (HMW-MAA), hepatitis B surface antigen, Human leukocyte antigen A1 (HLA-A1), Human leukocyte antigen A2 (HLA-A2), IL-22 receptor alpha(IL-22Rα), IL-13 receptor alpha 2 (IL-13Rα2), kinase insert domain receptor (kdr), kappa light chain, L1 cell adhesion molecule (L1-CAM), CE7 epitope of L1-CAM, Leucine Rich Repeat Containing 8 Family Member A (LRRC8A), Lewis Y, Melanoma-associated antigen (MAGE)-A1, MAGE-A3, MAGE-A6, MAGE-A10, mesothelin (MSLN), c-Met, murine cytomegalovirus (CMV), mucin 1 (MUC1), MUC16, natural killer group 2 member D (NKG2D) ligands, melan A (MART-1), neural cell adhesion molecule (NCAM), oncofetal antigen, Preferentially expressed antigen of melanoma (PRAME), progesterone receptor, a prostate specific antigen, prostate stem cell antigen (PSCA), prostate specific membrane antigen (PSMA), Receptor Tyrosine Kinase Like Orphan Receptor 1 (ROR1), survivin, Trophoblast glycoprotein (TPBG also known as 5T4), tumor-associated glycoprotein 72 (TAG72), Tyrosinase related protein 1 (TRP1, also known as TYRP1 or gp75), Tyrosinase related protein 2 (TRP2, also known as dopachrome tautomerase, dopachrome delta-isomerase or DCT), vascular endothelial growth factor receptor (VEGFR), vascular endothelial growth factor receptor 2 (VEGFR2), Wilms Tumor 1 (WT-1), a pathogen-specific or pathogen-expressed antigen, or an antigen associated with a universal tag, and/or biotinylated molecules, and/or molecules expressed by HIV, HCV, HBV or other pathogens.

17. The method of claim 15 or claim 16, wherein:
(a) the recombinant receptor is a recombinant T cell receptor (TCR) or a functional non-T cell receptor, and/or
(b) the recombinant receptor is a chimeric antigen receptor (CAR), optionally wherein the CAR comprises an extracellular antigen-recognition domain that specifically binds to the antigen and an intracellular signaling domain comprising an ITAM; further optionally wherein
(i) the intracellular signaling domain comprising an ITAM comprises an intracellular domain of a CD3-zeta (CD3ζ) chain, optionally a human CD3-zeta chain; and/or
(ii) the intracellular signaling domain further comprises a costimulatory signaling region, optionally wherein the costimulatory signaling region comprises a signaling domain of CD28 or 4-1BB, further optionally human CD28 or human 4-1BB.

18. A method for assessing a residual non-integrated transgene sequence, the method comprising:
(1) performing the method of any of claims 1-17, to determine the presence, absence or amount of the transgene sequence in the high molecular weight fraction of DNA, thereby assessing genomic integration of a transgene sequence;
(2) determining the presence, absence or amount of the transgene sequence in the isolated DNA without separating the high molecular weight fraction; and
(3) comparing the amount determined in (1) to the amount determined in (2), thereby determining the amount of the residual non-integrated recombinant sequence;
wherein the determining the presence, absence or amount in (2) is carried out by polymerase chain reaction (PCR).

19. The method of claim 18, wherein the PCR is quantitative polymerase chain reaction (qPCR), digital PCR or droplet digital PCR.

## Patentansprüche

1. Verfahren zur Bewertung der genomischen Integration einer Transgensequenz, wobei das Verfahren Folgendes umfasst:
(a) Abtrennen einer hochmolekularen Fraktion von Desoxyribonukleinsäure (DNA) größer als oder mit mehr als etwa 10 Kilobasen (kb) aus DNA, die aus einer oder mehreren Zellen isoliert wurde, wobei die eine oder die mehreren Zellen mindestens eine konstruierte Zelle umfassen oder vermutlich umfassen, die eine Transgensequenz umfasst, die für ein rekombinantes Protein codiert;
(b) aus der hochmolekularen Fraktion das Bestimmen des Vorhandenseins, Nichtvorhandenseins oder der Menge der Transgensequenz, die in das Genom der einen oder mehreren Zellen integriert ist, wobei das Bestimmen des Vorhandenseins, Nichtvorhandenseins oder der Menge der Transgensequenz durch Polymerasekettenreaktion (PCR) durchgeführt wird.

2. Verfahren nach Anspruch 1, wobei das Verfahren ferner vor dem Abtrennen in (a) das Isolieren von Desoxyribonukleinsäure (DNA) aus der einen oder den mehreren Zellen umfasst.

3. Verfahren nach Anspruch 1 oder Anspruch 2, wobei:
(i) das Bestimmen des Vorhandenseins, Nichtvorhandenseins oder der Menge der Transgensequenz in (b) das Bestimmen der Kopienzahl der Transgensequenz pro diploidem Genom oder pro Zelle in der einen oder den mehreren Zellen umfasst; und/oder
(ii) die eine oder die mehreren Zellen eine Population von Zellen umfassen, in der eine Vielzahl von Zellen der Population die Transgensequenz umfassen, die für das rekombinante Protein codiert,
optional wobei die Kopienzahl eine durchschnittliche oder mittlere Kopienzahl pro diploidem Genom oder pro Zelle unter der Population von Zellen ist.

4. Verfahren nach einem der Ansprüche 1-3, wobei das Verfahren vor dem Trennschritt in (a) die Einführung eines Polynukleotids, das die Transgensequenz umfasst, die für das rekombinante Protein codiert, in die mindestens eine konstruierte Zelle der einen oder mehreren Zellen umfasst, wobei die eine oder die mehreren Zellen aus einer Probe isoliert sind, die Blut oder eine von Blut abgeleitete Probe ist, oder wobei die Probe ein Apherese- oder Leukaphereseprodukt ist oder daraus abgeleitet ist.

5. Verfahren nach Anspruch 4, wobei:
(a) das Verfahren umfasst:
(i) Inkubation der mindestens einen konstruierten Zelle bei einer Temperatur von mehr als 25 °C, optional bei oder bei etwa 37 °C ± 2 °C, für nicht mehr als 96 Stunden nach der Einbringung des Polynukleotids, das die Transgensequenz umfasst;
(ii) Inkubation der mindestens einen konstruierten Zelle bei einer Temperatur von mehr als 25 °C, optional bei oder bei etwa 37 °C ± 2 °C, für nicht mehr als 72 Stunden nach der Einbringung des Polynukleotids, das die Transgensequenz umfasst; oder
(iii) Inkubation der mindestens einen konstruierten Zelle bei einer Temperatur von mehr als 25 °C, optional bei oder bei etwa 37 °C ± 2 °C, für nicht mehr als 48 Stunden nach der Einbringung des Polynukleotids, das die Transgensequenz umfasst; und/oder
(b) die Einbringung des Polynukleotids durch Transduktion mit einem viralen Vektor, der das Polynukleotid umfasst, durchgeführt wird, wobei optional:
(i) der virale Vektor ein retroviraler Vektor oder ein gammaretroviraler Vektor ist,
ferner optional wobei der virale Vektor ein lentiviraler Vektor ist; oder
(i) der virale Vektor ein AAV-Vektor ist, ferner optional ausgewählt aus AAV1-, AAV2-, AAV3-, AAV4-, AAV5-, AAV6-, AAV7- oder AAV8-Vektor.

6. Verfahren nach einem der Ansprüche 1-5, wobei die eine oder die mehreren Zellen vor dem Abtrennen der hochmolekularen Fraktion von DNA in (a) kryokonserviert wurden.

7. Verfahren nach einem der Ansprüche 1-6, wobei:
(a) die eine oder die mehreren Zellen eine Zelllinie sind; oder
(b) die eine oder die mehreren Zellen eine Primärzelle sind, die aus einer Probe aus einem Individuum erhalten wurde.

8. Verfahren nach einem der Ansprüche 1-7, wobei die eine oder die mehreren Zellen eine Immunzelle sind, optional wobei die Immunzelle eine T-Zelle oder eine NK-Zelle ist, ferner optional wobei die T-Zelle eine CD3⁺-, CD4⁺- und/oder CD8⁺-T-Zelle ist.

9. Verfahren nach einem der Ansprüche 1-8, wobei das Abtrennen durch Pulsfeldgelelektrophorese oder Größenausschlusschromatographie durchgeführt wird.

10. Verfahren nach einem der Ansprüche 1-9, wobei:
(a) der hochmolekulare Anteil größer oder mehr als etwa 15 Kilobasen (kb) ist;
(b) der hochmolekulare Anteil größer oder mehr als als etwa 17,5 Kilobasen (kb) ist; oder
(c) der hochmolekulare Anteil größer oder mehr als etwa 20 Kilobasen (kb) ist.

11. Verfahren nach einem der Ansprüche 1-10, wobei:
(a) die Transgensequenz ein regulatorisches Element in funktionsfähiger Verknüpfung mit einer Nukleinsäuresequenz umfasst, die für das rekombinante Protein codiert; und/oder
(b) die PCR eine quantitative Polymerasekettenreaktion (qPCR), digitale PCR oder digitale Tröpfchen-PCR ist; und/oder
(c) die PCR unter Verwendung eines oder mehrerer Primer durchgeführt wird, der/die komplementär zu mindestens einem Teil der Transgensequenz oder in der Lage zum spezifischen Amplifizieren desselben ist/sind, optional wobei der eine oder die mehreren Primer komplementär zu Sequenzen des regulatorischen Elements oder in der Lage zum spezifischen Amplifizieren derselben ist/sind.

12. Verfahren nach einem der Ansprüche 1-11, wobei:
(a) das Bestimmen der Menge der Transgensequenz die Bewertung der Kopienzahl der Transgensequenz pro Masse oder Gewicht der aus der einen oder den mehreren Zellen isolierten DNA, optional pro Mikrogramm der aus der einen oder den mehreren Zellen isolierten DNA, umfasst; oder
(b) das Bestimmen der Menge der Transgensequenz die Bewertung der Kopienzahl der Transgensequenz pro der einen oder den mehreren Zellen, optional pro CD3⁺-, CD4⁺- und/oder CD8⁺-Zelle und/oder pro Zelle, die das rekombinante Protein exprimiert, umfasst.

13. Verfahren nach einem der Ansprüche 1-12, wobei:
(a) das Bestimmen der Menge der Transgensequenz die Bewertung der Kopienzahl der Transgensequenz in der hochmolekularen Fraktion und das Normalisieren der Kopienzahl auf die Kopienzahl eines Referenzgens in der hochmolekularen Fraktion oder auf eine Standardkurve umfasst, wobei optional das Referenzgen ein Haushaltsgen ist und/oder das Referenzgen ein Gen ist, das Albumin (ALB) codiert, oder das Referenzgen ein Gen ist, das Ribonuclease-P-Protein-Untereinheit p30 (RPP30) codiert; und/oder
(b) die Kopienzahl eines Referenzgens in der isolierten DNA durch PCR unter Verwendung eines oder mehrerer Primer ermittelt wird, der/die komplementär zu mindestens einem Teil des Referenzgens oder in der Lage zum spezifischen Amplifizieren desselben ist/sind.

14. Verfahren nach einem der Ansprüche 1-13, wobei:
(a) die Transgensequenz kein vollständiges virales gag-Protein codiert; und/oder
(b) die Transgensequenz kein vollständiges HIV-Genom, kein replikationskompetentes virales Genom und/oder keine akzessorischen Gene umfasst, wobei es sich bei den akzessorischen Genen optional um Nef, Vpu, Vif, Vpr und/oder Vpx handelt.

15. Verfahren nach einem der Ansprüche 1-14, wobei das rekombinante Protein ein rekombinanter Rezeptor ist.

16. Verfahren nach Anspruch 15, wobei der rekombinante Rezeptor spezifisch an ein Antigen bindet, das mit einer Krankheit oder einem Leiden assoziiert ist, oder an ein Antigen, das in Zellen der Umgebung einer Läsion exprimiert wird, die mit einer Krankheit oder einem Leiden assoziiert ist; optional wobei:
(a) die Krankheit oder das Leiden ein Krebs ist; und/oder
(b) das Antigen ausgewählt ist aus ανβ6-Integrin (avb6-Integrin), B-Zell-Reifungsantigen (BCMA), B7-H3, B7-H6, Carboanhydrase 9 (CA9, auch bekannt als CAIX oder G250), einem Krebs-Hoden-Antigen, Krebs/Hoden-Antigen 1B (CTAG, auch bekannt als NY-ESO-1 und LAGE-2), Karzinoembryonal-Antigen (CEA), einem Cyclin, Cyclin A2, C-C-Motiv-Chemokin-Ligand 1 (CCL-1), CD19, CD20, CD22, CD23, CD24, CD30, CD33, CD38, CD44, CD44v6, CD44v7/8, CD123, CD133, CD138, CD171, Chondroitinsulfat Proteoglycan 4 (CSPG4), Epidermalwachstumsfaktor-Protein (EGFR), Typ-III-Epidermalwachstumsfaktor-Rezeptor-Mutation (EGFR vIII), epitheliales Glykoprotein 2 (EPG-2), epitheliales Glykoprotein 40 (EPG-40), EphrinB2, Ephrinrezeptor A2 (EPHa2), Östrogenrezeptor, Fc-Rezeptor-like-5 (FCRL5;
auch bekannt als Fc-Rezeptor-Homolog 5 oder FCRH5), fötalem Acetylcholinrezeptor (fötaler AchR), einem Folatbindungsprotein (FBP), Folatrezeptor-alpha, Gangliosid GD2, O-acetyliertem GD2 (OGD2), Gangliosid GD3, Glycoprotein 100 (gp100), Glypican-3 (GPC3), G-Protein-gekoppelter-Rezeptor-Klasse C Gruppe 5-Mitglied D(GPRC5D), Her2/neu (Rezeptortyrosinkinase erb-B2), Her3 (erb-B3), Her4 (erb-B4), erbB-Dimeren, humanem Hochmolekulargewicht-Melanom-assoziiertes-Antigen (HMW-MAA), Hepatitis-B-Oberflächenantigen, Human-Leukozytenantigen A1 (HLA-A1), Human-Leukozytenantigen A2 (HLA-A2), IL-22-Rezeptor alpha(IL-22Rα), IL-13-Rezeptor alpha 2 (IL-13Rα2), Kinase-Insert-Domäne-Rezeptor (kdr), kappa-Leichtkette, L1-Zelladhäsionsmolekül (L1-CAM), CE7-Epitop von L1-CAM, Leucine-Rich-Repeat-Containing-8-Familie-Mitglied A (LRRC8A), Lewis Y, Melanom-assoziiertes-Antigen (MAGE)-A1, MAGE-A3, MAGE-A6, MAGE-A10, Mesothelin (MSLN), c-Met, murinem Cytomegalovirus (CMV), Mucin 1 (MUC1), MUC16, natürliche Killer-Gruppe-2-Mitglied-D(NKG2D)-Liganden, Melan A (MART-1), neuralem Zell-Adhäsionsmolekül (NCAM), onkofötalem Antigen, Präferentiell-exprimiertes Antigenvon-Melanom (PRAME), Progesteronrezeptor, einem Prostata-spezifischen Antigen (PSCA), Prostataspezifisches-Membranantigen (PSMA), Rezeptortyrosinkinase-Like-Orphan-Rezeptor 1 (ROR1), Survivin, Trophoblasten-Glycoprotein (TPBG, auch bekannt als 5T4), tumor-assoziiertem Glycoprotein 72 (TAG72), Tyrosinase-related-Protein 1 (TRP1, auch bekannt als TYRP1 oder gp75), Tyrosinase-related-Protein 2 (TRP2, auch bekannt als Dopachrom-Tautomerase, Dopachrom-delta-Isomerase oder DCT), Gefäßendothelwachstumsfaktor-Rezeptor (VEGFR), Gefäßendothelwachstumsfaktor-Rezeptor 2 (VEGFR2), Wilms-Tumor 1 (WT-1), einem pathogenspezifischen oder pathogenexprimierten Antigen oder einem Antigen, das mit einem universellen Tag assoziiert ist, und/oder biotinylierten Molekülen und/oder Molekülen, die von HIV, HCV, HBV oder anderen Pathogenen exprimiert werden.

17. Verfahren nach Anspruch 15 oder Anspruch 16, wobei:
(a) der rekombinante Rezeptor ein rekombinanter T-Zell-Rezeptor (TCR) oder ein funktioneller Nicht-T-Zell-Rezeptor ist, und/oder
(b) der rekombinante Rezeptor ein chimärer Antigenrezeptor (CAR) ist, wobei optional der CAR eine extrazelluläre Antigenerkennungsdomäne, die spezifisch an das Antigen bindet, und eine intrazelluläre Signalgebungsdomäne, die ein ITAM umfasst, umfasst; ferner optional wobei
(i) die intrazelluläre Signalgebungsdomäne, die ein ITAM umfasst, eine intrazelluläre Domäne einer CD3-Zeta(CD3ζ)-Kette, optional eine humane CD3-Zeta-Kette umfasst; und/oder
(ii) die intrazelluläre Signalgebungsdomäne ferner eine costimulatorische Signalgebungsregion umfasst, wobei die costimulatorische Signalgebungsregion optional eine Signalgebungsdomäne von CD28 oder 4-1BB, ferner optional humanem CD28 oder humanem 4-1BB, umfasst.

18. Verfahren zur Bewertung einer nicht-integrierten Transgensequenz, wobei das Verfahren Folgendes umfasst:
(1) Durchführen des Verfahrens nach einem der Ansprüche 1-17 zum Bestimmen des Vorhandenseins, Nichtvorhandenseins oder der Menge der Transgensequenz in der hochmolekularen Fraktion von DNA, wodurch die genomische Integration einer Transgensequenz bewertet wird;
(2) Bestimmen des Vorhandenseins, Nichtvorhandenseins oder der Menge der Transgensequenz in der isolierten DNA, ohne Abtrennen der hochmolekularen Fraktion; und
(3) Vergleichen der in (1) bestimmten Menge mit der in (2) bestimmten Menge, wodurch die Menge der restlichen nicht-integrierten rekombinanten Sequenz bestimmt wird;
wobei das Bestimmen des Vorhandenseins, Nichtvorhandensein oder der Menge in (2) durch Polymerasekettenreaktion (PCR) durchgeführt wird.

19. Verfahren nach Anspruch 18, wobei es sich bei der PCR um quantitative Polymerasekettenreaktion (qPCR), digitale PCR oder digitale Tröpfchen-PCR handelt.

## Revendications

1. Procédé d'évaluation de l'intégration génomique d'une séquence transgénique, le procédé comprenant :
(a) la séparation d'une fraction à poids moléculaire élevé d'acide désoxyribonucléique (ADN) supérieure ou supérieure à environ 10 kilobases (kb) d'ADN isolée à partir d'une ou plusieurs cellules, ladite ou lesdites cellules comprenant, ou étant suspectée(s) de comprendre, au moins une cellule modifiée comprenant une séquence transgénique codant pour une protéine recombinante ;
(b) à partir de la fraction à poids moléculaire élevé, la détermination de la présence, de l'absence ou de la quantité de la séquence transgénique intégrée dans le génome de la ou des cellules, la détermination de la présence, de l'absence ou de la quantité de la séquence transgénique étant effectuée par réaction en chaîne par polymérase (PCR).

2. Procédé selon la revendication 1, dans lequel le procédé comprend en outre, avant la séparation en (a), l'isolement d'acide désoxyribonucléique (ADN) à partir de la ou des cellules.

3. Procédé selon la revendication 1 ou la revendication 2, dans lequel :
(i) la détermination de la présence, de l'absence ou de la quantité de la séquence transgénique en (b) comprend la détermination du nombre de copies de la séquence transgénique par génome diploïde ou par cellule dans la ou les cellules ; et/ou
(ii) la ou les cellules comprennent une population de cellules dans laquelle une pluralité de cellules de la population comprennent la séquence transgénique codant pour la protéine recombinante,
facultativement, le nombre de copies étant un nombre moyen ou médian de copies par génome diploïde ou par cellule parmi la population de cellules.

4. Procédé selon l'une quelconque des revendications 1 à 3, dans lequel le procédé comprend, avant l'étape de séparation en (a), l'introduction d'un polynucléotide comprenant la séquence transgénique codant pour la protéine recombinante dans l'au moins une cellule modifiée parmi la ou des cellules, dans lequel la ou les cellules sont isolées à partir d'un échantillon qui est du sang ou un échantillon dérivé du sang, ou dans lequel l'échantillon est ou est dérivé d'un produit d'aphérèse ou de leucaphérèse.

5. Procédé selon la revendication 4, dans lequel :
(a) le procédé comprend :
(i) l'incubation de l'au moins une cellule modifiée à une température supérieure à 25 °C, facultativement à ou à environ 37 °C ± 2 °C, pendant pas plus de 96 heures après l'introduction du polynucléotide comprenant la séquence transgénique ;
(ii) l'incubation de l'au moins une cellule modifiée à une température supérieure à 25 °C, facultativement à ou à environ 37 °C ± 2 °C, pendant pas plus de 72 heures après l'introduction du polynucléotide comprenant la séquence transgénique ; ou
(iii) l'incubation de l'au moins une cellule modifiée à une température supérieure à 25 °C, facultativement à ou à environ 37 °C ± 2 °C, pendant pas plus de 48 heures après l'introduction du polynucléotide comprenant la séquence transgénique ; et/ou
(b) l'introduction du polynucléotide est effectuée par transduction avec un vecteur viral comprenant le polynucléotide, facultativement où :
(i) le vecteur viral est un vecteur rétroviral ou un vecteur gammarétroviral,
en outre facultativement dans lequel le vecteur viral est un vecteur lentiviral ; ou
(i) le vecteur viral est un vecteur AAV, en outre facultativement choisi parmi un vecteur AAV1, AAV2, AAV3, AAV4, AAV5, AAV6, AAV7 ou AAV8.

6. Procédé selon l'une quelconque des revendications 1 à 5, dans lequel la ou les cellules ont été cryoconservées avant la séparation de la fraction à poids moléculaire élevé de l'ADN en (a).

7. Procédé selon l'une quelconque des revendications 1 à 6, dans lequel :
(a) la ou les cellules sont une lignée cellulaire ; ou
(b) la ou les cellules sont une cellule primaire obtenue à partir d'un échantillon provenant d'un sujet.

8. Procédé selon l'une quelconque des revendications 1 à 7, dans lequel la cellule ou les cellules sont une cellule immunitaire, facultativement dans lequel la cellule immunitaire est une cellule T ou une cellule NK, facultativement en outre dans lequel la cellule T est une cellule T CD3+, CD4+ et/ou CD8+.

9. Procédé selon l'une quelconque des revendications 1 à 8, dans lequel la séparation est effectuée par électrophorèse sur gel en champ pulsé ou par chromatographie d'exclusion stérique.

10. Procédé selon l'une quelconque des revendications 1 à 9, dans lequel :
(a) la fraction à poids moléculaire élevé est supérieure ou supérieure à environ 15 kilobases (kb);
(b) la fraction à poids moléculaire élevé est supérieure ou supérieure à environ 17,5 kilobases (kb) ; ou
(c) la fraction à poids moléculaire élevé est supérieure ou supérieure à environ 20 kilobases (kb).

11. Procédé selon l'une quelconque des revendications 1 à 10, dans lequel :
(a) la séquence transgénique comprend un élément régulateur fonctionnellement lié à une séquence d'acide nucléique codant pour la protéine recombinante ; et/ou
(b) la PCR est une réaction en chaîne par polymérase quantitative (qPCR), une PCR numérique ou une PCR numérique en gouttelettes ; et/ou
(c) la PCR est réalisée en utilisant une ou plusieurs amorces qui sont complémentaires de ou sont capables d'amplifier spécifiquement au moins une partie de la séquence transgénique, facultativement où la ou les amorces sont complémentaires ou sont capables d'amplifier spécifiquement des séquences de l'élément régulateur.

12. Procédé selon l'une quelconque des revendications 1 à 11, dans lequel :
(a) la détermination de la quantité de la séquence transgénique comprend l'évaluation du nombre de copies de la séquence transgénique par masse ou poids d'ADN isolé à partir de la ou des cellules, éventuellement par microgramme d'ADN isolé à partir de la ou des cellules ; ou
(b) la détermination de la quantité de la séquence transgénique comprend l'évaluation du nombre de copies de la séquence transgénique par cellule(s), facultativement par cellule CD3+, CD4+ et/ou CD8+, et/ou par cellule exprimant la protéine recombinante.

13. Procédé selon l'une quelconque des revendications 1 à 12, dans lequel :
(a) la détermination de la quantité de la séquence transgénique comprend l'évaluation du nombre de copies de la séquence transgénique dans la fraction à poids moléculaire élevé et la normalisation du nombre de copies au nombre de copies d'un gène de référence dans la fraction à poids moléculaire élevé ou à une courbe étalon, éventuellement le gène de référence étant un gène domestique, et/ou le gène de référence étant un gène codant pour l'albumine (ALB) ou le gène de référence étant un gène codant pour la sous-unité p30 de la protéine ribonucléase P (RPP30) ; et/ou
(b) le nombre de copies d'un gène de référence dans l'ADN isolé est effectué par PCR en utilisant une ou plusieurs amorces qui sont complémentaires de ou sont capables d'amplifier spécifiquement au moins une partie du gène de référence.

14. Procédé selon l'une quelconque des revendications 1 à 13, dans lequel :
(a) la séquence transgénique ne code pas pour une protéine gag virale complète ; et/ou
(b) la séquence transgénique ne comprend pas un génome complet du VIH, un génome viral compétent pour la réplication, et/ou des gènes accessoires, lesquels gènes accessoires étant éventuellement Nef, Vpu, Vif, Vpr, et/ou Vpx.

15. Procédé selon l'une quelconque des revendications 1 à 14, dans lequel la protéine recombinante est un récepteur recombinant.

16. Procédé selon la revendication 15, dans lequel le récepteur recombinant se lie spécifiquement à un antigène associé à une maladie ou affection ou un antigène qui est exprimé dans des cellules de l'environnement d'une lésion associée à une maladie ou affection ; facultativement où :
(a) la maladie ou l'affection est un cancer ; et/ou
(b) l'antigène est choisi parmi intégrine ανβ6 (intégrine avb6), antigène de maturation des lymphocytes B (BCMA), B7-H3, B7-H6, anhydrase carbonique 9 (CA9, également connue sous le nom de CAIX ou G250), antigène cancer-testicule, antigène cancer/testicule 1B (CTAG, également connu sous le nom de NY-ESO-1 et LAGE-2), antigène carcinoembryonnaire (ACE), cycline, cycline A2, ligand 1 de la chimiokine à motif C-C (CCL-1), CD19, CD20, CD22, CD23, CD24, CD30, CD33, CD38, CD44, CD44v6, CD44v7/8, CD123, CD133, CD138, CD171, protéoglycane 4 de sulfate de chondroïtine (CSPG4), protéine de facteur de croissance épidermique (EGFR), mutation du récepteur du facteur de croissance épidermique de type III (EGFR vIII), glycoprotéine épithéliale 2 (EPG-2), glycoprotéine épithéliale 40 (EPG-40), éphrineB2, récepteur d'éphrine A2 (EPHa2), récepteur d'œstrogène, récepteur Fc de type 5 (FCRL5 ;
également connu sous le nom de récepteur Fc homologue 5 ou FCRH5), récepteur de l'acétylcholine fœtale (AchR fœtal), protéine de liaison au folate (FBP), récepteur alpha du folate, ganglioside GD2, GD2 O-acétylé (OGD2), ganglioside GD3, glycoprotéine 100 (gp100), glypicane-3 (GPC3), récepteur couplé aux protéines G de classe C, groupe 5, membre D (GPRC5D), Her2/neu (récepteur tyrosine kinase erb-B2), Her3 (erb-B3), Her4 (erb-B4), dimères erbB, antigène associé au mélanome de haut poids moléculaire humain (HMW-MAA), antigène de surface de l'hépatite B, antigène leucocytaire humain A1 (HLA-A1), antigène leucocytaire humain A2 (HLA-A2), récepteur alpha de l'IL-22 (IL-22Rα), récepteur alpha de l'IL-13 2 (IL-13Rα2), récepteur du domaine d'insertion de la kinase (kdr), chaîne légère kappa, molécule d'adhésion cellulaire L1 (L1-CAM), épitope CE7 de L1-CAM, membre A de la famille 8 contenant des répétitions riches en leucine (LRRC8A), Lewis Y, antigène associé à un mélanome (MAGE)-A1, MAGE-A3, MAGE-A6, MAGE-A10, mésothéline (MSLN), c-Met, cytomégalovirus murin (CMV), mucine 1 (MUC1), MUC16, ligands du membre D du groupe 2 des cellules NK (NKG2D), Melan A (MART-1), molécule d'adhésion des cellules neurales (NCAM), antigène onco-fœtal, antigène préférentiellement exprimé du mélanome (PRAME), récepteur de la progestérone, antigène spécifique de la prostate, antigène des cellules souches de la prostate (PSCA), antigène membranaire spécifique de la prostate (PSMA), récepteur orphelin de type tyrosine kinase 1 (ROR1), survivine, glycoprotéine trophoblastique (TPBG également connue sous le nom de 5T4), glycoprotéine associée aux tumeurs 72 (TAG72), protéine apparentée à la tyrosinase 1 (TRP1, également connue sous le nom de TYRP1 ou gp75), protéine apparentée à la tyrosinase 2 (TRP2, également connue sous le nom de dopachrome tautomérase, dopachrome delta-isomérase ou DCT), récepteur du facteur de croissance endothélial vasculaire (VEGFR), récepteur 2 du facteur de croissance endothélial vasculaire (VEGFR2), tumeur de Wilms 1 (WT-1), un antigène spécifique ou exprimé par un pathogène, ou un antigène associé à une étiquette universelle, et/ou des molécules biotinylées, et/ou des molécules exprimées par le VIH, le VHC, le VHB ou d'autres pathogènes.

17. Procédé selon la revendication 15 ou la revendication 16, dans lequel :
(a) le récepteur recombinant est un récepteur de lymphocytes T recombinant (TCR) ou un récepteur fonctionnel non de lymphocytes T, et/ou
(b) le récepteur recombinant est un récepteur antigénique chimérique (CAR), facultativement où le CAR comprend un domaine de reconnaissance d'antigène extracellulaire qui se lie spécifiquement à l'antigène et un domaine de signalisation intracellulaire comprenant un ITAM ; en outre, facultativement dans lequel
(i) le domaine de signalisation intracellulaire comprenant un ITAM comprend un domaine intracellulaire d'une chaîne CD3-zêta (CD3ζ), facultativement une chaîne CD3-zêta humain ; et/ou
(ii) le domaine de signalisation intracellulaire comprend en outre une région de signalisation costimulatrice, éventuellement la région de signalisation costimulatrice comprenant un domaine de signalisation de CD28 ou 4-1BB, éventuellement CD28 humaine ou 4-1BB humaine.

18. Procédé d'évaluation d'une séquence transgénique résiduelle non intégrée, le procédé comprenant :
(1) la mise en oeuvre du procédé selon l'une quelconque des revendications 1 à 17, pour déterminer la présence, l'absence ou la quantité de la séquence transgénique dans la fraction à poids moléculaire élevé d'ADN, évaluant ainsi l'intégration génomique d'une séquence transgénique ;
(2) la détermination de la présence, de l'absence ou de la quantité de la séquence transgénique dans l'ADN isolé sans séparer la fraction à poids moléculaire élevé ; et
(3) la comparaison de la quantité déterminée en (1) à la quantité déterminée en (2), déterminant ainsi la quantité de la séquence recombinante résiduelle non intégrée ;
dans lequel la détermination de la présence, de l'absence ou de la quantité en (2) est effectuée par réaction en chaîne par polymérase (PCR).

19. Procédé selon la revendication 18, dans lequel la PCR est une réaction en chaîne par polymérase quantitative (qPCR), une PCR numérique ou une PCR numérique en gouttelettes.
